# EUROPEAN PATENT APPLICATION

(11) **EP 4 759 933 A2**
(43) Date of publication of application: **17.06.2026**
(21) Application number: 26164373.8
(22) Date of filing: 18.11.2021
(51) Int. Cl.: C12P 17/12

(54) **METHODS OF IMPROVING PRODUCTION OF MORPHINAN ALKALOIDS AND DERIVATIVES**

(30) Priority: 19.11.2020 US 202063116097 P
(62) Divisional of application: 21895623.3
(71) Applicant: Antheia, Inc., Menlo Park, CA 94025 (US)
(72) Inventor: SMOLKE, Christina, D., Menlo Park 94025 (US); THODEY, Catherine, Menlo Park 94025 (US); HAWKINS, Kristy, M., Menlo Park 94025 (US); TAKEOKA, Kenneth, Menlo Park 94025 (US); HANSEN, Douglas, Menlo Park 94025 (US)
(74) Representative: Carpmaels & Ransford LLP

(57) **Abstract**

Methods and engineered cells are provided for increasing activity of a norcoclaurine synthase in a microbial cell. The method comprises, within the engineered microbial cell, contacting an engineered norcoclaurine synthase with a substrate, wherein contacting the substrate with the engineered norcoclaurine synthase increases conversion, within the engineered microbial cell, in comparison to a non-engineered norcoclaurine synthase.

## Description

### CROSS-REFERENCE TO EARLIER FILED APPLICATION

The present application claims benefit to U.S. provisional application no. 63/116,097, filed November 19, 2020. which is incorporated by reference herein in its entirety.

### SEQUENCE LISTING

The present application contains a Sequence Listing which has been submitted electronically in ASCII format and is hereby incorporated by reference in its entirety. Said ASCII copy, created on November 18, 2021, is named 2218605-126WOI_SL.txt and is 585,313 bytes in size.

### SUMMARY OF THE INVENTION

The present disclosure provides methods for the production of divers e benzylisoquinoline alkaloids (BIAs) in engineered host cells. The present disclosure further provides compositions of diverse alkaloids produced in engineered host cells. Additionally, the present disclosure provides methods for the production of one or more Bet v 1-fold proteins in engineered host cells. Additionally, the present disclosure provides methods for the production of one or more engineered Bet v 1-fold proteins in engineered host cells. In particular cases, the disclosure provides methods for increasing production of diverse alkaloid products by engineered Bet v 1-fold proteins with N-terminal truncations. In further particular cases, the disclosure provides methods for increasing production of diverse alkaloid products by engineered Bet v 1-fold proteins with particular amino acid mutations that increase activity.

In some embodiments, the disclosure provides methods for increasing production of diverse alkaloid products through the epimerization of a *(S)*-1-benzylisoquinoline alkaloid to a *(R)*-1-benzylisoquinoline alkaloid via engineered epimerases in an engineered host cell. In further embodiments, the present disclosure provides methods for increasing production of diverse alkaloid products through the production of one or more Bet v 1-fold proteins in engineered cells in combination with the epimerization of *(S)-*reticuline to *(R)*-reticuline via an engineered epimerase comprising two separate enzymes encoding an oxidase and a reductase compared to the production of diverse alkaloid products through the epimerization of *(S)*-reticulinc to *(R)*-reticuline via a wild-type epimerase.

While engineered split epimerases may be composed of a separate oxidase enzyme and reductase enzyme that originate from a parent or wild-type epimerase, engineered epimerases may also comprise a separate oxidase enzyme and reductase enzyme that originate from separate parent or wild-type epimerases. Examples of parent epimerases having an oxidase and reductase component comprise amino acid sequences selected from the group consisting of: SEQ ID NOs: 1, 2. 3, 4, 5, 6, 7. 8, 9, 10, 11, 12, 13. 14. 15, and 16, as listed in **Table 1. Table 1** also illustrates an example of a DRS and DRR component respectively seen in SEQ ID NOs. 17 and 18.

In some embodiments, the disclosure provides methods for increasing production of diverse alkaloid products through the conversion of a promorphinan alkaloid to a morphinan alkaloid via thebaine synthases in an engineered host cell. In further embodiments, the present disclosure provides methods for increasing production of diverse alkaloid products through the conversion of salutaridinol-7-*O*-acetate to thebaine via a thebaine synthase. Examples of parent thebaine synthases comprise amino acid sequences selected from the group consisting of: SEQ ID NOs: 19, 20. 21, 22. 23, 24, 25, and 26 as listed in **Table 2.** In some cases, the thebaine synthase enzyme is a Bet v 1 fold protein. In further embodiments, the present disclosure provides methods for increasing production of diverse alkaloid products through the production of one or more thebaine synthases in engineered cells in combination with the epimerization of *(S)*-reticuline to *(R)*-reticuline via an engineered epimerase comprising two separate enzymes encoding an oxidase and a reductase compared to the production of diverse alkaloid products through the epimerization of *(S)*-reticuline to *(R)*-reticuline via a wild-type epimerase. **Table 2** also illustrates examples of a SalAT component, as seen in SEQ ID NOs. 27-34.

In some embodiments, the disclosure provides methods for increasing production of diverse alkaloid products through the conversion of a promorphinan alkaloid to a morphinan alkaloid via engineered thebaine synthases in an engineered host cell. In further embodiments. the present disclosure provides methods for increasing production of diverse alkaloid products through the conversion of salutaridinol-7-*O*-acetate to thebaine via an engineered thebaine synthase. In other embodiments, the engineered thebaine synthase has a N-terminal truncation relative to the wild-type thebaine synthase.

In some embodiments, the engineered thebaine synthase is a fusion enzyme. In further embodiments, the thebaine synthase is fused to an acetyl transferase enzyme In further embodiments, the thebaine synthase is encoded within an acetyl transferase enzyme. In other embodiments, the thebaine synthasc is fused to a reductase enzyme.

In some embodiments, the disclosure provides methods for increasing production of diverse alkaloid products through the conversion of a precursor morphinan alkaloid with a carbon-carbon double bond between carbons C-14 and C-8 into a product morphinan alkaloid with a carbon-carbon double bond between carbons C-8 and C-7 via neopinone isomerases in an engineered host cell. In further embodiments, the precursor morphinan alkaloid with a carbon-carbon double bond between carbons C-14 and C-8 is produced in the engineered cell via a heterologous biosynthetic pathway comprising a plurality of enzymes and starting with simple starting materials such as sugar and/or L-tyrosine. In further embodiments, the present disclosure provides methods for increasing production of diverse alkaloid products through the conversion of neopinone to codeinone via a ncopinone isomerase. Examples of parent neopinone isomerases comprise amino acid sequences selected from the group consisting of: SEQ ID NOs 43, 44, 45, 46, 47, 48, 49, 50. 51, 52. 53, 54, 55, 56. 57. and 58 as listed in **Table 3.** In some cases, the neopinone isomerase enzyme is a Bet v 1 fold protein.

In some embodiments, the disclosure provides methods for increasing production of diverse alkaloid products through the conversion of a precursor morphinan alkaloid with a carbon-carbon double bond between carbons C-14 and C-8 into a product morphinan alkaloid with a carbon-carbon double bond between carbons C-8 and C-7 via engineered neopinone isomerases in an engineered host cell. In further embodiments, the present disclosure provides methods for increasing production of diverse alkaloid products through the conversion of neopinone to codeinone via an engineered neopinone isomerase. In other embodiments, the engineered neopinone isomerase has a N-terminal truncation relative to the wild-type neopinone isomerase.

In some embodiments, the engineered neopinone isomerase is a fusion enzyme. In further embodiments, the neopinone isomerase is fused to an *O*-demethylase enzyme that acts on the morphinan alkaloid scaffold. In further embodiments. the neopinone isomerase is encoded within an *O*-demethylase enzyme. In other embodiments, the neopinone isomerase is fused to a reductase enzyme. In further embodiments, the neopinone isomerase is encoded within a reductase enzyme.

In some embodiments, the disclosure provides methods for increasing production of diverse alkaloid products through the condensation of BIA precursors to a 1-benzylisoquinoline alkaloid via norcoclaurine synthases in an engineered host cell. In further embodiments, the present disclosure provides methods for increasing production of diverse alkaloid products through the condensation of 4-HPAA and dopamine to norcoclaurine via a norcoclaunne synthase. Examples of parent norcoclaurine synthases comprise amino acid sequences selected from the group consisting of: SEQ ID NOs: 69, 70, 71, 72. 73. 74. 75. 76, 77. 78. 79. 80. 81. and 82 as listed in **Table 5.** In some cases, the norcoclaurine synthase enzyme is a Bet v 1 fold protein.

In some embodiments, the disclosure provides methods for increasing production of diverse alkaloid products through the condensation of BIA precursors to a 1-benzylisoquinoline alkaloid via engineered norcoclaurine synthases in an engineered host cell. In further embodiments, the present disclosure provides methods for increasing production of diverse alkaloid products through the condensation of 4-HPAA and dopamine to norcoclausine via an engineered norcoclaurine synthase. In other embodiments, the engineered norcoclaurine synthase has a N-terminal truncation relative to the wild-type norcoclaurine synthase. In other embodiments, the engineered norcoclaurine synthase has amino acid mutations relative to the the wild-type norcoclaurine synthase that increase its activity.

In some embodiments, the engineered norcoclaurine synthase is a fusion enzyme. In further embodiments, the norcoclaurine synthase is fused to a L-DOPA decarboxylase enzyme. In further embodiments, the norcoclaurine synthase is encoded within a L-DOPA decarboxylase enzyme.

In some cases, the method further comprises engineering the non-plant cell with a plurality of heterologous enzymes to produce the diverse benzylisoquinoline alkaloid products from simple starting materials such as sugar and/or L-tyrosine. In some examples, an engineered non-plant cell comprises a plurality of coding sequences each encoding an enzyme that is selected from the group of enzymes listed in **Table 11.** In some examples, the heterologous coding sequences may be operably connected. Heterologous coding sequences that are operably connected may be within the same pathway of producing a particular benzylisoquinoline alkaloid product via an engineered Bet v 1 fold protein, including an engineered thebaine synthase activity, an engineered neopinone isomerase activity, or an engineered norcoclaurine synthase activity.

In some cases, the method further comprises engineering the non-plant cell with a plurality of heterologous enzymes to increase the production of BIA precursors, including L-tyrosine and 4-HPAA. In some examples, an engineered non-plant cell comprises a plurality of coding sequences each encoding an enzyme that is selected from the group of enzymes listed in **Table 11.** In some examples, an engineered non-plant cell further comprises inactivating mutations in selected enzymes that result in reduced production of byproducts. In some examples, an engineered non-plant cell further comprises heterologous expression or overexpression of selected enzymes that result in reduced production of byproducts. In some examples, the byproducts comprise tyrosol, phenylethanol, or methionol. In some examples, an engineered non-plant cell further comprises inactivating mutations in selected enzymes that result in increased production of diverse benzylisoquinoline alkaloid products

### EMBODIMENTS

E1. A method of increasing activity of a norcoclaurine synthase in a microbial cell, comprising within the engineered microbial cell, contacting an engineered norcoclaurine synthase with a substrate, wherein contacting the substrate with the engineered norcoclaurine synthase increases conversion, within the engineered microbial cell, in comparison to a non-engineered norcoclaurine synthase.

E2. A method of producing a benzylisoquinoline alkaloid product in an engineered microbial cell, wherein the method comprises contacting an engineered norcoclaurine synthase with a substrate within the engineered microbial cell.

E3. The method of any one of embodiments E1-E2, wherein the engineered norcoclaunne synthase is derived from the Coptis japonica norcoclaurine synthase (CjNCS).

E4. The method of any one of embodiment E1-E3, wherein the nucleic acid sequence encoding the engineered norcoclaurine synthase is codon optimized.

E5 The method of embodiment E1, wherein the engineered norcoclaurine synthase has been truncated at the N-terminus.

E6 The method of embodiment E1, wherein the engineered norcoclaurine synthase is a mutated norcoclaurine synthase.

E7. The method of embodiment E6. wherein the mutated norcoclaurine synthase has at least one mutation at a residue position with reference to SEQ ID NO: 70, where the position is selected from the group consisting of amino acid residue 70, 81, 91, 101, 104, 147, 149, 151, and 155.

E8. The method of any of embodiments E6-E7. wherein the mutated noncoclaurine synthase comprises one or more of the following mutations with reference to SEQ ID NO: 70: M70I, D149T, and I155N.

E9. The method of any of embodiments E6-E8. wherein the mutated norcoclaurine synthase comprises the following mutations with reference to SEQ ID NO: 70: M70I, D149T and 1155N.

E10. The method of any one of embodiments 6-9, wherein the mutated norcoclaurine synthase consists of the following mutations with reference to SEQ ID NO: 70: M70I, D149T, and I155N and wherein the engineered norcoclaurine synthase has been truncated at the N-terminus.

E11. The method of any of embodiments E1-E10, wherein the engineered microbial cell produces a benzylisoquinoline alkaloid selected from the group consisting of benzylisoquinolines, promorphinans, morphinans, protoberberines, protopines, benzophenanthridines, secoberberines, phthalideisoquinolines, aporphines, bisbenzylisoquinolines, nal-opioids, and nor-opioids.

E12. An engineered microbial cell that produces a benzylisoquinoline alkaloid product, said engineered microbial cell comprising a byproduct inhibition alleviating modification selected from the group consisting of gene inactivation and enzyme expression.

E13. The engineered microbial cell of embodiment E12, wherein inactivation of a gene occurs in an enzyme selected from the group consisting of aromatic aminotransferase (AROS), aromatic aminotransferase (ARO9), phenylpyruvate decarboxylase (ARO10), pyruvate decarboxylase (PDC1), pyruvate decarboxylase (PDC5), pyruvate decarboxylase (PDC6), aldehyde reductase (ARI1), alcohol acetyltransferase 1 (ATF1), alcohol acetyltransferase 2 (ATF2), octanoyl-coenzyme A:ethanol acyltransferase (EHTl), acyl-coenzyme A:ethanol O-acyltransferase (EEB1). (putative) aryl-alcohol dehydrogenase (AAD3), NADPH-dependent aldo-keto reductase (YPR1), 3-methylbutanal reductase and NADPH-dependent methylglyoxal reductase (GRE2), alcohol dehydrogenase 1 (ADH1), alcohol dehydrogenase 2 (ADH2), alcohol dehydrogenase 3 (ADH3), alcohol dehydrogenase 4 (ADH4), alcohol dehydrogenase 5 (ADH5), alcohol dehydrogenase 6 (ADH6), alcohol dehydrogenase 7 (ADH7), aldehyde reductase (YDR541c), branched-chain amino-acid aminotransferase (BAT2), hexadecenal dehydrogenase (HFD1), prephenate dehydrogenase (TYR1), and prephenate dehydratase (PHA2).

E 14. The engineered microbial cell of any of embodiments E12-E13 wherein expression of a gene occurs in an enzyme selected from the group consisting of (4-Hydroxyphenylacetaldehyde synthase (HPAAS), aspartate-prephenate aminotransferase (PAT), arogenate dehydratase (ADT), arogenate dehydrogenase (AAT), phosphoketolase (PK), and Uridine 5'-diphosphoglucosyltransferase (UGT).

E15. The engineered microbial cell of any of embodiments E12-E14, wherein the reduced byproduct is selected from the group consisting of ty rosol, phenylethanol, and methionol.

E16. The engineered microbial cell of any of embodiments E12-E 15. wherein the engineered microbial cell produces a benzylisoquinoline alkaloid selected from the group consisting of benzylisoquinolines, promorphinans, morphinans, protoberberines, protopines, benzophenanthridines, secoberberines, phthalideisoquinolines, aporphines, bisbenzylisoquinolines, nal-opioids, and nor-opioids.

E17. A method of producing benzylisoquinoline alkaloid products using an engineered microbial cell of any of embodiments E12-E16

E18. An engineered microbial cell that produces benzylisoquinoline alkaloids, wherein the engineered microbial cell comprises a modification to improve S-adenosyl-L-methionine (SAM) recycling.

E19. The engineered microbial cell of embodiment E18, wherein the engineered microbial cell comprises overexpression of S-adenosyl-L-homocysteine hydrolase (SAH1), thereby increasing recovery of S-adenosyl-L-homocysteine (SAH).

E20. The engineered microbial cell of embodiment E19, wherein the SAH1 is a native SAH 1.

E21. The engineered microbial cell of any of embodiments E18-E20, wherein the engineered microbial cell comprises overexpression of methionine synthase (MET6), thereby increasing recovery of SAH.

E22. The engineered microbial cell of embodiment E21, wherein the MET6 is a native MET6.

E23. The engineered microbial cell of any of embodiments E18-E22, wherein the engineered microbial cell comprises overexpression of S-adenosylmethionine synthetase (SAM2), thereby increasing recovery of SAH.

E24. The engineered microbial cell of embodiments E23, wherein the SAM2 is a native SAM2.

E25. A method of producing benzylisoquinoline alkaloid products using an engineered microbial cell of any of embodiments E12-E25.

E26. The method of embodiment E6. wherein the mutated norcoclaurine synthase comprises the mutation I155N with reference to SEQ ID NO: 70.

E27. The method of embodiment E6. wherein the mutated norcoclaurine synthase consists of the mutation I155N with reference to SEQ ID NO: 70. and wherein the engineered norcoclaurine synthase has been truncated at the N-terminus.

E28. The method of embodiment E6. wherein the mutated norcoclaurine synthase comprises one or more of the mutations D149T and 1155N with reference to SEQ ID NO: 70.

E29. The method of embodiment E6, wherein the mutated norcoclaurine synthase comprises the mutations D149T and I155N with reference to SEQ ID NO: 70.

E30. The method of embodiment E6, wherein the mutated norcoclaurine synthase consists of the mutations D149T and I155N with reference to SEQ ID NO: 70, and wherein the engineered norcoclaurine synthase has been truncated at the N-terminus.

E31. The method of embodiment E6, wherein the mutated norcoclaurine synthase has at least 75% homology to SEQ. ID NO. 70.

E32. The method of embodiment E6. wherein the mutated norcoclaurine synthase has at least 80% homology to SEQ. ID NO. 70.

E33. The method of embodiment E6. wherein the mutated norcoclaurine synthase has at least 85% homology to SEQ. ID NO. 70.

E34. The method of embodiment E6. wherein the mutated norcoclaurine synthase has at least 90% homology to SEQ. ID NO. 70.

E35. The method of embodiment E6. wherein the mutated norcoclaurine synthase has at least 95% homology to SEQ. ID NO 70.

E36. An improved norcoclaurine synthase (NCS) variant, wherein: (i) the amino acid sequence of the NCS variant is at least 95% identical to SEQ ID NO:70: (ii) the NCS variant comprises an N-terminal truncation relative to the Coptis japonica norcoclaurine synthase of SEQ ID NO:69 (CjNCS), wherein the N-terminal truncation is a truncation of between 20 and 40 consecutive amino acid residues at the N-terminus of the CjNCS of SEQ ID NO:69; and (iii) yeast cells engineered to express the NCS variant are capable of increased production of norcoclaurine, relative to corresponding yeast cells engineered to express the CjNCS of SEQ ID NO:69 when tested under the same assay conditions.

E37. The NCS variant of embodiment E36. wherein: (a) the N-terminal truncation is a truncation of amino acids 1-22 (Δ1-22) of the CjNCS of SEQ ID NO:69; (b) the N-terminal truncation is a truncation of amino acids 1-36 (Δ1-24) of the CjNCS of SEQ ID NO:69; (c) the N-terminal truncation is a truncation of amino acids 1-26 (Δ1-26) of the CjNCS of SEQ ID NO:69; (d) the N-terminal truncation is a truncation of amino acids 1-28 (Δ1-28) of the CjNCS of SEQ ID NO:69; (e) the N-terminal truncation is a truncation of amino acids 1-32 (Δ1-32) of the CjNCS of SEQ ID NO:69; (f) the N-terminal truncation is a truncation of amino acids 1-34 (Δ1-34) of the CjNCS of SEQ ID NO 69; or (g) the N-terminal truncation is a truncation of amino acids 1-36 (Δ1-36) of the CjNCS of SEQ ID NO:69.

E38. The NCS variant of embodiment E37, wherein the NCS variant consists of the amino acid sequence of SEQ ID NO:70.

E39. An improved norcoclaurine synthase (NCS) vanant, wherein: (i) the amino acid sequence of the NCS variant is at least 95% identical to SEQ ID NO:70; (ii) the NCS variant comprises at least one amino acid substitution relative to SEQ ID NO:70. and the at least one amino acid substitution is at a position corresponding to amino acid residue 70, 81, 91, 101, 104, 147, 149, 151 or 155 of SEQ ID NO:70; and (iii) yeast cells engineered to express the NCS variant are capable of increased production of norcoclaurine, relative to corresponding yeast cells engineered to express the NCS of SEQ ID NO:70 when tested under the same assay conditions.

E40. The NCS variant of any of embodiments E36-E39, wherein the increased production of norcoclaurine is at least 1.25-fold the production of the corresponding yeast cells engineered to express the NCS of SEQ ID NO: 70.

E41. The NCS variant of any of embodiments E36-E40, wherein the increased production of norcoclaurine is at least 1.5-fold the production of the corresponding yeast cells engineered to express the NCS of SEQ ID NO: 70.

E42. The NCS vanant of any of embodiments E36-E41, wherein the increased production of norcoclaurine is at least 2.0-fold the production of the corresponding yeast cells engineered to express the NCS of SEQ ID NO: 70.

E43. The NCS variant of any of embodiments E36-E42, wherein the increased production of norcoclaurine is at least 5.0-fold the production of the corresponding yeast cells engineered to express the NCS of SEQ ID NO: 70.

E44. The NCS variant of any of embodiments E36-E43, wherein the increased production of norcoclaurine is at least 10.0-fold the production of the corresponding yeast cells engineered to express the NCS of SEQ ID NO: 69.

E45. The NCS variant of any of embodiments E36-E44, wherein the increased production of norcoclaurine is determined by assaying culture medium generated from culturing yeast cells in the culture medium for the concentration of norcoclaurine or a downstream product thereof using liquid chromatography mass spectrometry (LCMS).

E46. The NCS variant of any of embodiments E36-E45, wherein the NCS variant comprises at least one amino acid substitution relative to SEQ ID NO: 70 selected from: M70L, M70I and M70V; Y81F; K91A, K91V, K91S and K91L; D101S and D101A; F104L; L147Y, L 147H and L 147F; D149N, D149T and D149S; V151E, V151A, V151K, V151T and V151S; and I155Q, I155A, I155T. A155S and A155N.

E47. The NCS variant of any of embodiments E36-E46, wherein the NCS variant comprises the following amino acid substitutions relative to SEQ ID NO: 70: D149T and I155Q; D149T and I155N; or D149S and I155Q.

E48. The NCS variant of any of embodiments E36-E47, wherein the amino acid substitutions in the NCS variant relative to SEQ ID NO: 70 consist of. D149T and I155Q. D149T and 1155N; or D149S and I155Q.

E49. The NCS variant of any of embodiments E36-E48, wherein the NCS variant comprises the following amino acid substitutions relative to SEQ ID NO: 70: (a) F104L. D149N and I155Q; (b) K91S, D149T and 1I55Q; (c) K91A, D149T and I155Q; (d) K91V, D149S and I155N; (e) Y81F., K91A and D149N: or (f) Y81F, D149N and I155N.

E50. The NCS variant of any of embodiments E36-E49. wherein the amino acid substitutions in the NCS variant relative to SEQ ID NO: 70 consist of: (a) F104L, D149N. 1155Q; (b) K91S, D149T, I155Q; (c) K91A, D149T, I155Q; (d) K91A, D149T, I155Q; (e) K91V, D149S, I155N; (f) Y8IF, K91A, D149N; or (g) Y81F, D149N, I155N.

E51. The NCS variant of any of embodiments E36-E50, wherein the NCS variant comprises the following amino acid substitutions relative to SEQ ID NO:70: (a) K91S, D101S, F104L, D149T, I155Q; (b) N2S, M70L. F140A, D149T, I155N; (c) M70L, K91A. D149T, 1155N.

E52. The NCS variant of any one of embodiments E36-E51, wherein the amino acid substitutions in the engineered NCS relative to SEQ ID NO: 70 consist of: (a) K91S. D101S, F104L, D149T, I155Q: (b) N2S. M70L. F140A, D149T, I155N; (c) M70L, K91A, D149T, 1155N.

E53. The NCS vanant of any of embodiments E36-E52, wherein the NCS variant comprises one or more of the following mutations relative to SEQ ID NO: 70: M701. D149T, and I155N.

E54. The NCS vanant of any of embodiments E36-E53, wherein the NCS variant comprises the following mutations relative to SEQ ID NO: 70: M701. D149T and 1155N.

E55. The NCS variant of any of embodiments E36-E54, wherein the amino acid substitutions in the NCS variant relative to SEQ ID NO: 70 consist of: M70I, D149T, and I155N.

E56. The NCS variant of any of embodiments E36-E55, wherein the NCS variant is 170-176 amino acids in length.

E57. The NCS variant of any of embodiments E36-E56, wherein the NCS variant is 174-175 amino acids in length.

E58. The NCS variant of any of embodiments E36-E57, wherein the NCS variant is 173 amino acids in length.

E59. The NCS variant of any of embodiments E36-E58, wherein the amino acid sequence of the NCS variant is at least 97.5% identical to SEQ ID NO: 70.

E60. The NCS variant of any of embodiments E36-E59, wherein the NCS variant consists of the amino acid sequence of SEQ ID NO: 71.

E61. The NCS variant of any of embodiments E36-E60, wherein the NCS variant consists of the amino acid sequence of SEQ ID NO: 72.

E62. The NCS variant of any of embodiments E36-E61, wherein the NCS variant consists of the amino acid sequence of SEQ ID NO: 73.

E63. An improved norcoclaurine synthase (NCS) variant, wherein: (i) the amino acid sequence of the NCS variant is at least 95% identical to SEQ ID NO:74; (ii) the NCS variant comprises at least one amino acid substitution relative to SEQ ID NO:74, and the at least one amino acid substitution is at a position corresponding to amino acid residue 70. 81, 91, 101, 104, 147, 149, 151 or 155 of SEQ ID NO:70; and (iii) yeast cells engineered to express the NCS vanant are capable of increased production of norcoclaurine, relative to corresponding yeast cells engineered to express the NCS of SEQ ID NO:74.

E64. The NCS variant of any of embodiments E36-E63, wherein the increased production of norcoclaurine is at least 1.25-fold the production of the corresponding yeast cells engineered to express the NCS of SEQ ID NO: 74.

E65. The NCS variant of any of embodiments E36-E64. wherein the increased production of norcoclaurine is at least 1.5-fold the production of the corresponding yeast cells engineered to express the NCS of SEQ ID NO: 74.

E66. The NCS variant of any of embodiments E36-E65, wherein the increased production of norcoclaurine is at least 2.0-fold the production of the corresponding yeast cells engineered to express the NCS of SEQ ID NO: 74.

E67. The NCS variant of any of embodiments E36-E66, wherein the increased production of norcoclaurine is at least 5.0-fold the production of the corresponding yeast cells engineered to express the NCS of SEQ ID NO: 74.

E68. An improved norcoclaurine synthase (NCS) variant, wherein: (i) the amino acid sequence of the NCS variant is at least 95% identical to SEQ ID NO:75; (ii) the NCS variant comprises at least one amino acid substitution relative to SEQ ID NO:75. and the at least one amino acid substitution is at a position corresponding to amino acid residue 70. 81, 91, 101, 104, 147, 149, 151 or 155 of SEQ ID NO:70; and (iii) yeast cells engineered to express the NCS variant are capable of increased production of norcoclaurine, relative to corresponding yeast cells engineered to express the NCS of SEQ ID NO:75 when tested under the same assay conditions.

E69. An improved norcoclaurine synthase (NCS) variant, wherein: (i) the amino acid sequence of the NCS variant is at least 95% identical to SEQ ID NO:76: (ii) the NCS variant comprises at least one amino acid substitution relative to SEQ ID NO:76. and the at least one amino acid substitution is at a position corresponding to amino acid residue 70. 81, 91, 101, 104, 147, 149, 151 or 155 of SEQ ID NO:70; and (iii) yeast cells engineered to express the NCS variant are capable of increased production of norcoclaurine, relative to corresponding yeast cells engineered to express the NCS of SEQ ID NO:76 when tested under the same assay conditions.

E70. An improved norcoclaurine synthase (NCS) variant, wherein: (i) the amino acid sequence of the NCS variant is at least 95% identical to SEQ ID NO:77; (ii) the NCS variant comprises at least one amino acid substitution relative to SEQ ID NO:77. and the at least one amino acid substitution is at a position corresponding to amino acid residue 70, 81, 91, 101, 104, 147, 149, 151 or 155 of SEQ ID NO:70; and (iii) yeast cells engineered to express the NCS variant are capable of increased production of norcoclaurine, relative to corresponding yeast cells engineered to express the NCS of SEQ ID NO:77 when tested under the same assay conditions.

E71. An improved norcoclaurine synthase (NCS) vanant, wherein: (i) the amino acid sequence of the NCS variant is at least 95% identical to SEQ ID NO:78: (ii) the NCS variant comprises at least one amino acid substitution relative to SEQ ID NO:78. and the at least one amino acid substitution is at a position corresponding to amino acid residue 70. 81, 91. 101, 104, 147, 149, 151 or 155 of SEQ ID NO:70: and (iii) yeast cells engineered to express the NCS vanant are capable of increased production of norcoclaurine, relative to corresponding yeast cells engineered to express the NCS of SEQ ID NO:78 when tested under the same assay conditions.

E72. An improved norcoclaurine synthase (NCS) variant, wherein: (i) the amino acid sequence of the NCS variant is at least 95% identical to SEQ ID NO:79: (ii) the NCS variant comprises at least one amino acid substitution relative to SEQ ID NO:79, and the at least one amino acid substitution is at a position corresponding to amino acid residue 70, 81, 91, 101, 104, 147, 149, 151 or 155 of SEQ ID NO:70. and (iii) yeast cells engineered to express the NCS variant are capable of increased production of norcoclaurine, relative to corresponding yeast cells engineered to express the NCS of SEQ ID NO:79 when tested under the same assay conditions.

E73. An improved norcoclaurine synthase (NCS) variant, wherein. (i) the amino acid sequence of the NCS variant is at least 95% identical to SEQ ID NO:80; (ii) the NCS variant comprises at least one amino acid substitution relative to SEQ ID NO:80, and the at least one amino acid substitution is at a position corresponding to amino acid residue 70. 81, 91, 101. 104, 147, 149, 151 or 155 of SEQ ID NO:70; and (iii) yeast cells engineered to express the NCS variant are capable of increased production of norcoclaurine, relative to corresponding yeast cells engineered to express the NCS of SEQ ID NO:80 when tested under the same assay conditions.

E74. An improved norcoclaurine synthase (NCS) variant, wherein: (i) the amino acid sequence of the NCS variant is at least 95% identical to SEQ ID NO:81; (ii) the NCS variant comprises at least one amino acid substitution relative to SEQ ID NO:81, and the at least one amino acid substitution is at a position corresponding to amino acid residue 70, 81, 91, 101, 104, 147, 149, 151 or 155 of SEQ ID NO:70; and (iii) yeast cells engineered to express the NCS variant are capable of increased production of norcoclaurine, relative to corresponding yeast cells engineered to express the NCS of SEQ ID NO:81 when tested under the same assay conditions.

E75. An improved norcoclaurine synthase (NCS) variant, wherein: (i) the amino acid sequence of the NCS variant is at least 95% identical to SEQ ID NO:82; (ii) the NCS variant comprises at least one amino acid substitution relative to SEQ ID NO:82. and the at least one amino acid substitution is at a position corresponding to amino acid residue 70. 81, 91, 101. 104, 147, 149, 151 or 155 of SEQ ID NO:70; and (iii) yeast cells engineered to express the NCS variant are capable of increased production of norcoclaurine, relative to corresponding yeast cells engineered to express the NCS of SEQ ID NO:82 when tested under the same assay conditions.

E76. The NCS variant of any of embodiments E36-E75. wherein the increased production of norcoclaurine is determined by assaying culture medium generated from culturing yeast cells in the culture medium for the concentration of norcoclaurine or a downstream product thereof using liquid chromatography mass spectrometry (LCMS).

E77. The NCS variant of any of embodiments E36-E76, wherein the NCS variant comprises at least one amino acid substitution relative to SEQ ID NO: 70 selected from: M70L. M701 and M70V; Y81F; K91A, K9IV. K91S and K91L: D101S and D101A; F104L; L147Y, L147H and L147F: D149N, D149T and D149S; V151E, V151A, V151K, V151T and V151S; and 1155Q. I155A, I155T, A155S and A155N.

E78. The NCS variant of any of embodiments E36-E77. wherein the NCS variant comprises the following amino acid substitutions relative to SEQ ID NO: 70: D149T and 1155Q; D149T and I155N; or D149S and I155Q.

E79. The NCS variant of any of embodiments E36-E78, wherein the amino acid substitutions in the NCS variant relative to SEQ ID NO: 70 consist of: D149T and I155Q; D149T and I155N; or D149S and I155Q

E80. The NCS variant of any of embodiments E36-E79, wherein the NCS variant comprises the following amino acid substitutions relative to SEQ ID NO: 70: (a) F104L, D149N and I155Q; (b) K91S, D149T and I155Q; (c) K91A, D149T and I155Q; (d) K91V. D149S and 1155N; (e) Y81F, K91A and D149N; or (f) Y81F. D149N and 1155N.

E81. The NCS vanant of any of embodiments E36-E80, wherein the amino acid substitutions in the NCS variant relative to SEQ ID NO: 70 consist of: (a) F 104L. D149N, 1155Q; (b) K91S, D149T, 1155Q; (c) K91A, D149T, I155Q; (d) K91A, D149T. I155Q; (e) K91V, D149S, I155N; (f) Y81F, K91A, D149N; or (g) Y81F, D149N, I155N.

E82. The NCS variant of any of embodiments E36-E81, wherein the NCS variant comprises the following amino acid substitutions relative to SEQ ID NO: 70: (a) K91S, D101S, F104L, D149T. I155Q; (b) N2S, M70L, F140A, D149T, I155N; (c) M70L, K91A, D149T, I155N.

E83. The NCS variant of any of embodiments E36-E82, wherein the amino acid substitutions in the engineered NCS relative to SEQ ID NO: 70 consist of: (a) K91S, D101S, F104L, D149T, I155Q; (b) N2S, M70L, F140A, D149T, I155N; (c) M70L. K91A, D149T. 1155N.

E84. The NCS variant of any of embodiments E36-E83, wherein the NCS variant comprises one or more of the following mutations relative to SEQ ID NO: 70: M70I, D149T, and 1155N.

E85. The NCS variant of any of embodiments E36-E84, wherein the NCS variant comprises the following mutations relative to SEQ ID NO: 70: M701, D149T and 1155N.

E86. The NCS variant of any of embodiments E36-E85, wherein the amino acid substitutions in the NCS variant relative to SEQ ID NO: 70 consist of: M701. D149T, and I155N.

E87. The NCS variant of any of embodiments E36-E86, wherein the amino acid sequence of the NCS variant is at least 97.5% identical to SEQ ID NO:74.

E88. The NCS variant of any of embodiments E36-E87. wherein the amino acid sequence of the NCS variant is at least 97.5% identical to SEQ ID NO:75.

E89. The NCS variant of any of embodiments E36-E88. wherein the amino acid sequence of the NCS variant is at least 97.5% identical to SEQ ID NO:76.

E90. The NCS variant of any of embodiments E36-E89. wherein the amino acid sequence of the NCS variant is at least 97.5% identical to SEQ ID NO:77.

E91. The NCS variant of any of embodiments E36-E90, wherein the amino acid sequence of the NCS variant is at least 97.5% identical to SEQ ID NO:78.

E92. The NCS variant of any of embodiments E36-E91, wherein the amino acid sequence of the NCS variant is at least 97.5% identical to SEQ ID NO:79.

E93. The NCS variant of any of embodiments E36-E92, wherein the amino acid sequence of the NCS variant is at least 97.5% identical to SEQ ID NO:80.

E94. The NCS variant of any of embodiments E36-E93, wherein the amino acid sequence of the NCS variant is at least 97.5% identical to SEQ ID NO:81.

E95. The NCS variant of any of embodiments E36-E94, wherein the amino acid sequence of the NCS variant is at least 97.5% identical to SEQ ID NO:82.

E96. An engineered norcoclaurine synthase comprising an N-terminal truncation and/or one or more amino acid mutations compared to a non-engineered wild-type norcoclaurine synthase, wherein the engineered norcoclaurine synthase has increased condensation activity compared to a non-engineered wild-type norcoclaurine synthase.

E97. The engineered norcoclaurine synthase of embodiment E96. wherein the engineered norcoclaurine synthase comprises an N-tenninal truncation compared to a non-engineered wild-type norcoclaurine synthase.

E98. The engineered norcoclaurine synthase of embodiment E96, wherein the engineered norcoclaurine synthase comprises one or more amino acid mutations compared to a non-engineered wild-type norcoclaurine synthase.

E99. The engineered norcoclaurine synthase of any one of embodiments E96-E98. wherein the engineered norcoclaurine synthase comprises at least 90% sequence identity to the amino acid sequence of SEQ ID NO: 69. 70, 71, 72, 73, 74. 75. 76. 77, 78, 79, 80. 81, or 82.

E100. The engineered norcoclaurine synthase of any one of embodiments E96-E99. wherein the engineered norcoclaurine synthase comprises at least one mutation at one or more amino acid residue positions selected from the group consisting of amino acid residue 70, 81, 91, 101, 104, 147, 149, 151, and 155, with reference to the amino acid sequence of SEQ. ID. NO: 70.

E101. The engineered norcoclaurine synthase of any one of embodiments E96-E100, wherein the engineered norcoclaurine synthase condenses a BIA precursor.

E102. The engineered norcoclaurine synthase of embodiment E101, wherein the BIA precursor is one or more of norcoclaurine (NC) and norlaudanosoline (NL).

E103. A method of increasing production of thebaine in an engineered microbial cell, the method comprising: (a) introducing into the engineered microbial cell a heterologous poly nucleotide sequence that encodes a uridine 5'-diphospho-glucosyltransferase; (b) expressing the uridine 5'-diphospho-glucosyltransferase within the engineered microbial cell; and (c) producing increased levels of thebaine within the engineered microbial cell compared to a non-engineered microbial cell. wherein the uridine 5 diphospho-glucosyltransferase converts a phenol to an aryl beta-D-glucoside within the engineered microbial cell.

E104. The method of embodiment E103, wherein the heterologous polynucleotide sequence that encodes the uridine 5'-diphospho-glucosyltransferase is positioned at a HXT5 locus in the engineered microbial cell.

E105. The method of any of embodiments E103-E104, wherein the heterologous polynucleotide sequence that encodes the uridine 5'-diphospho-glucosyltransferase construct is positioned at an EGH1 locus in the engineered microbial cell.

E106. The method of any of embodiments E103-E015, wherein the heterologous polynucleotide sequence that encodes the uridine 5'-diphospho-glucosyltransferase construct is positioned at a HST2 locus in the engineered microbial cell.

E107. The method of embodiment E106, wherein BAT2 is deleted from the background of the nucrobial cell.

E108. The method of any of embodiments E103-E107, wherein the engineered microbial cell further comprises an inactivation mutation comprising a deletion of EGH1.

E109. The method of any of embodiments E103-E108, wherein the expression of the uridine 5'-diphospho-glucosyltransferase within the engineered microbial cell reduces the accumulation of fusel alcohols.

E110. The method of embodiment E109. wherein the expression of the uridine 5'-diphospho-glucosyltransferase reduces the accumulation of tyrosol within the engineered microbial cell.

E111. The method of embodiment E109, wherein the expression of the uridine 5'-diphospho-glucosyltransferase reduces the accumulation of phenylethanol within the engineered microbial cell.

E112. The method of embodiment E109, wherein the expression of the uridine 5'-diphospho-glucosyltransferase reduces the accumulation of methionol within the engineered microbial cell.

E113. A method of producing a benzylisoquinoline alkaloid (BIA) product in an engineered host cellthe method comprising: (a) expressing an engineered norcoclaurine synthase in the engineered host cell. (b) contacting the engineered norcoclaurine synthase with a BIA-precursor substrate; and (c) producing the BIA product within the host cell; wherein the engineered norcoclaurine synthase comprises an N-terminal truncation and/or one or more amino acid mutations compared to a non-engineered wild-type norcoclaurine synthase, and has increased condensation activity compared to a non-engineered norcoclaurine synthase; and wherein the engineered host cell produces more BIA product than a non-engineered host cell.

E114. The method of embodiment E113, wherein the engineered norcoclaurine synthase comprises an N-terminal truncation compared to a non-engineered wild-type norcoclaurine synthase.

E115. The method of embodiment E113 or E114, wherein the engineered norcoclaurine synthase comprises one or more amino acid mutations compared to a non-engineered wild-type norcoclaurine synthase.

E116. The method of any one of embodiments E113-E115, wherein the engineered norcoclaurine synthase comprises at least 90% sequence identity to the amino acid sequence of SEQ ID NO: 69. 70. 71, 72. 73. 74. 75. 76. 77. 78. 79. 80. 81, or 82.

E117. The method of any one of embodiments E113-E116, wherein the engineered norcoclaurine synthase comprises at least one amino acid mutation at one or more residue positions selected from the group consisting of amino acid residue 70, 81, 91, 101, 104, 147, 149, 151, and 155 with reference to the amino acid sequence of SEQ. ID. NO: 70.

E118. The method of any one of embodiments E113-E117, wherein the engineered host cell produces a BIA selected from the group consisting of benzylisoquinolines, promorphinans, morphinans, protoberberines, protopines, benzophenanthridines, secoberberines, phthalideisoquinolines, aporphines, bisbenzylisoquinolines, nal-opioids. and nor-opioids.

E119. An engineered host cell that produces a benzylisoquinoline alkaloid (BIA) product, the engineered host cell comprising: (i) one or more engineered biosynthetic enzymes that reduces accumulation of one or more byproducts that inhibits the production of the BIA product, and/or (ii) an inactivation of one or more genes that results in the reduction of accumulation of one or more byproduct that inhibits the production of the BIA product.

E120. The engineered host cell of embodiment E119, wherein the one or more engineered biosynthetic enzymes comprises at least one amino acid modification compared to a non-engineered wild-type biosynthetic synthase.

E121. The engineered host cell of embodiment E119 or E120, wherein the inactivation of one or more genes occurs in one or more genes that encodes an enzyme selected from the group consisting of aromatic aminotransferase (ARO8). aromatic aminotransferase (ARO9), phenylpyruvate decarboxylase (ARO10), pyruvate decarboxylase (PDC1), pyruvate decarboxylase (PDCS), pyruvate decarboxylase (PDC6), aldehyde reductase (ARI1), alcohol acetyltransferase 1 (ATF1), alcohol acetyltransferase 2 (ATF2), octanoyl-coenzyme A:ethanol acyltransferase (EHT1), acyl-coenzyme A:ethanol O-acyltransferase (EEB1), (putative) aryl-alcohol dehydrogenase (AAD3). NADPH-dependent aldo-keto reductase (YPR1), 3-methylbutanal reductase and NADPH-dependent methylglyoxal reductase (GRE2), alcohol dehydrogenase 1 (ADH1), alcohol dehydrogenase 2 (ADH2), alcohol dehydrogenase 3 (ADH3), alcohol dehydrogenase 4 (ADH4), alcohol dehydrogenase 5 (ADH5), alcohol dehydrogenase 6 (ADH6), alcohol dehydrogenase 7 (ADH7), aldehyde reductase (YDR541c), branched-chain amino-acid aminotransferase (BAT2), hexadecenal dehydrogenase (HFD1), prephenate dehydrogenase (TYR1), and prephenate dehydratase (PHA2).

E122. The engineered host cell of any one of embodiments E119-E121, wherein the one or more engineered biosynthetic enzymes is selected from the group consisting of (4-Hydroxyphenylacetaldehyde synthase (HPAAS). aspartate-prephenate aminotransferase (PAT), arogenate dehydratase (ADT), arogenate dehydrogenase (AAT). phosphoketolase (PK), and Uridine 5'-diphosphoglucosyltransferase (UGT)

E123. The engineered host cell of any one of embodiments E119-E122, wherein the one or more engineered biosynthetic enzymes and/or the inactivation of one or more genes reduces the accumulation of a by product selected from the group consisting of tyrosol, phenylethanol, and methionol.

E124. The engineered host cell of any one of embodiments E119-E123, wherein the engineered host cell produces a BIA product selected from the group consisting of benzyhsoquinolines, promorphinans, morphinans. protoberberines. protopines, benzophenanthridines. secoberberines, phthalideisoquinolines. aporphines, bisbenzylisoquinolines. nal-opioids, and nor-opioids.

E125. A method of producing one or more BIA products using the engineered host cell of anyone of embodiments E119-E124.

E126. An engineered host cell that produces a benzylisoquinoline alkaloid (BIA) product. the engineered host cell comprising one or more modifications that increases S-adenosyl-L-methionine (SAM) recycling compared to a non-engineered host cell.

E127. The engineered host cell of embodiment E126. wherein the engineered host cell comprises one or more modifications that cause overexpression of S-adenosyl-L-homocysteine hydrolase (SAH1) compared to a non-engineered host cell. thereby increasing recovery of S-adenosyl-L-homocysteine (SAH) in the engineered host cell.

E128. The engineered host cell of embodiment E127, wherein the SAH1 is a native SAH1.

E129. The engineered host cell of any one of embodiments E126-E128, wherein the engineered host cell comprises one or more modifications that cause overexpression of methionine synthase (MET6) compared to a non-engineered host cell, thereby increasing recovery of SAH in the engineered host cell.

E130. The engineered host cell of embodiment E129, wherein the MET6 is a native MET6.

E131. The engineered host cell of any one of embodiments E126-E130, wherein the engineered host cell comprises one or more modifications that cause overexpression of S-adenosylmethionine synthetase (SAM2) compared to a non-engineered host cell, thereby increasing recovery of SAH in the engineered host cell.

E132. The engineered host cell of embodiment E131, wherein the SAM2 is a native SAM2.

E133. A method of producing a benzylisoquinoline alkaloid products (BIA) product using an engineered host cell of any one of embodiments E126-E132.

E134. The method of any one of embodiments E113-E115, wherein the engineered norcoclaurine synthase is a fusion enzyme.

E135. The method of embodiment E134, wherein the engineered norcoclaurine synthase is fused to a L-3,4-dibydroxyphenylalanine (L-DOPA) decarboxylase enzyme.

E136. The method of any one embodiments E113-E118, E125, and E133-E135, wherein the engineered host cell is a microbial cell.

E137. The engineered host cell of any one of embodiments E119-E124 and E126-E132, wherein the engineered host cell is a microbial cell.

### INCORPORATION BY REFERENCE

All publications, patents. and patent applications mentioned in this specification are herein incorporated by reference to the same extent as if each individual publication, patent. or patent application was specifically and individually indicated to be incorporated by reference.

### BRIEF DESCRIPTION OF THE DRAWINGS

The novel features of the invention are set forth with particularity in the appended claims. A better understanding of the features and advantages of the invention will be obtained by reference to the following detailed description that sets forth illustrative embodiments, in which the principles of the invention are utilized. and the accompanying drawings of which.
**FIG. 1** illustrates a biosynthetic scheme for conversion of glucose to 4-HPAA, dopamine, 3.4-DHPAA, and 1-benzylisoquinoline alkaloids to reticuline, in accordance with some embodiments of the invention.
**FIG. 2** illustrates examples of tyrosine hydroxylase activities, and synthesis, recycling. and salvage pathways of tetrahydrobiopterin associated with tyrosine 3-monooxygenase activities. in accordance with some embodiments of the invention.
**FIG. 3** illustrates a biosynthetic scheme for conversion of L-tyrosine to reticuline via norcoclaurine and norlaudanosoline, in accordance with some embodiments of the invention.
**FIG. 4** illustrates a biosynthetic scheme for conversion of L-tyrosine to morphinan alkaloids, including natural and semi-synthetic opioids, in accordance with some embodiments of the invention.

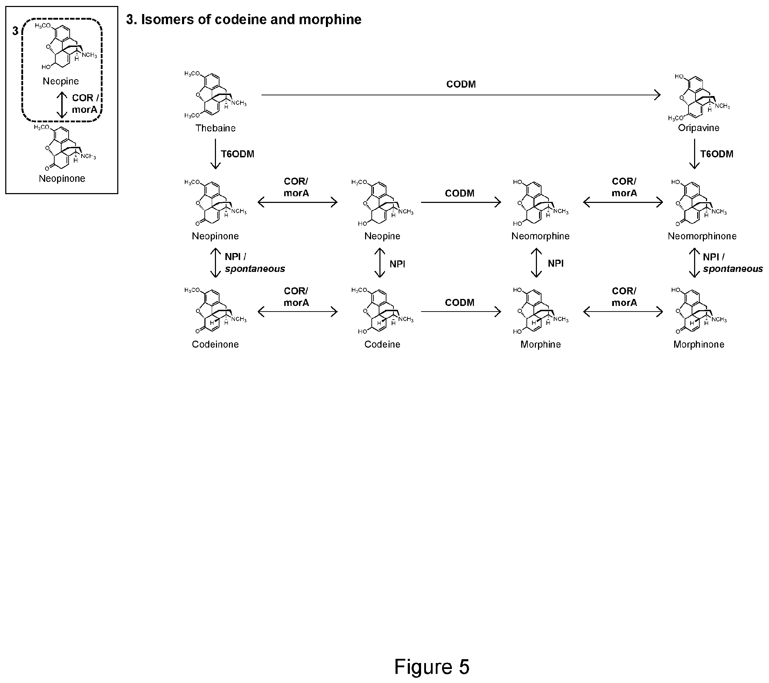

**FIG. 5** illustrates a biosynthetic scheme for production of natural opioids, including isomers of codeine and morphine. in accordance with some embodiments of the invention.
**FIG. 6** illustrates a biosynthetic scheme for production of nor-opioids and nal-opioids, in accordance with some embodiments of the invention.
**FIG. 7** illustrates a biosynthetic scheme for production of noscapine and related pathway metabolites, in accordance with some embodiments of the invention.
**FIG. 8** illustrates a biosynthetic scheme for production of sanguinarine and related pathway metabolites. in accordance with some embodiments of the invention.
**FIG. 9** illustrates a biosynthetic scheme for production of berberine and related pathway metabolites. in accordance with some embodiments of the invention.
**FIG. 10** illustrates a biosynthetic scheme for production of bisBIAs and related pathway metabolites. in accordance with some embodiments of the invention.
**FIG. 11** illustrates an enzyme having opioid 6-*O*-demethylase activity. in accordance with some embodiments of the invention.
**FIG. 12** illustrates an enzyme having opioid 3-*O*-demethylase activity. in accordance with some embodiments of the invention.
**FIG. 13** illustrates an enzyme having opioid N-demethylase activity, in accordance with some embodiments of the invention.

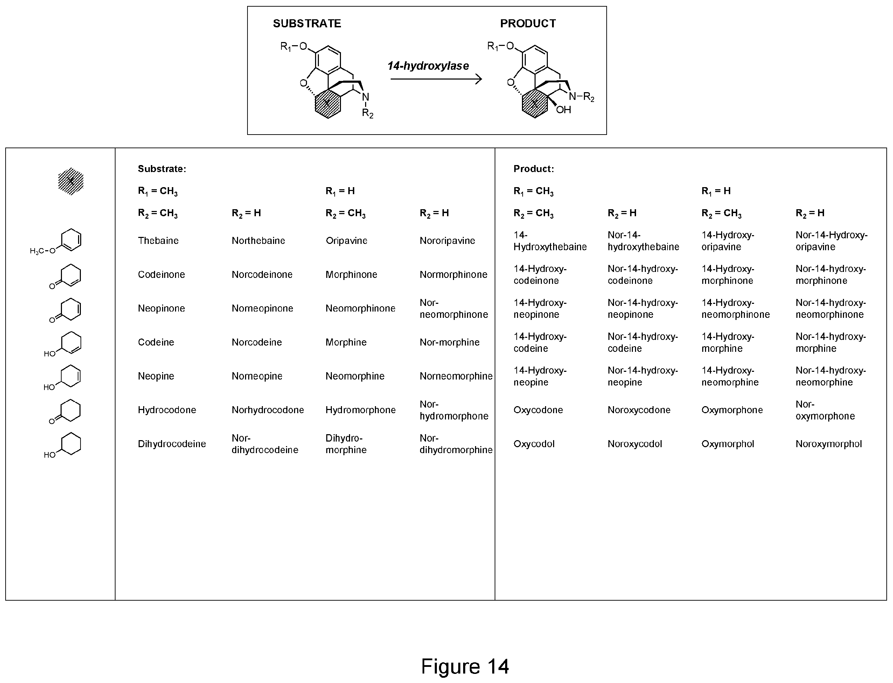

**FIG. 14** illustrates an enzyme having opioid 14-hydroxylase activity, in accordance with some embodiments of the invention.
**FIG. 15** illustrates an enzyme having opioid alcohol oxidoreductase activity, in accordance with some embodiments of the invention.
**FIG. 16** illustrates an enzyme having opioid reductase activity. in accordance with some embodiments of the invention.
**FIG. 17** illustrates an enzyme having opioid isomerase activity. in accordance with some embodiments of the invention.
**FIG. 18** illustrates an enzyme having *N*-methyltransferase activity. in accordance with some embodiments of the invention.
**FIG. 19** illustrates yeast platform strains for the production of reticuline from L-tyrosine, in accordance with some embodiments of the invention.
**FIG. 20** illustrates yeast strains for the production of thebaine and hydrocodone from L-tyrosine, in accordance with some embodiments of the invention.
**FIGS. 21A-C** illustrates the production of morphinan alkaloids from sugar and L-tyrsoine from engineered yeast strains, in accordance with embodiments of the invention.
**FIG. 22** illustrates an enzyme having norcoclaurine synthase activity. in accordance with embodiments of the invention.
**FIG. 23** depicts a phylogenetic tree of selected plant Be 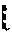 v I proteins with predicted NCS activity. Represented species are *Coptis japonica*, *Thalictrum flavum*, *Argemone mexicana*, *Sinopodophyllum hexandrum*, *Papaver bracteatum*, *Papaver somniferum*, and *Cordalvis saxicola*, in accordance with embodiments of the invention.
**FIG. 24** depicts N-terminal truncations of CjNCS (SEQ ID NO: 69) and the effect on enzymatic activity, in accordance with embodiments of the invention.
**FIG. 25** depicts the key residues identified in a directed evolution screen of NCS (SEQ ID NO: 70) **(Table 6)** mapped to the crystal structure of TfNCS (PDB: 5N8Q), in accordance with embodiments of the invention.
**FIG. 26** depicts the key residues for improving norcoclaurine synthase activity in the template NCS parent (SEQ ID NO: 70) and in NCS variants from *Coptis japonica*, *Thalictrum flavum*, *Argrmone mexicana, Sinopodophyllum hexandrum*, *Papaver bracteatum*, *Papaver somniferum,* and *Cordalyis Saxicola* (SEQ ID NOS 69 and 75-82, respectively, m order of appearance), in accordance with embodiments of the invention.
**FIG. 27** depicts engineered NCS variants with enhanced norcoclaurine synthase activity, in accordance with embodiments of the invention.
**FIG. 28** depicts noreoclaurine synthase activity in the presence of increasing dopamine concentration, in accordance with embodiments of the invention.
**FIG. 29** depicts a bioprocess for thebaine, in accordance with embodiments of the invention.
**FIG. 30** depicts another bioprocess for thebaine, in accordance with embodiments of the invention.
**FIG. 31** depicts a bioprocess for noscapine, in accordance with embodiments of the invention.
**FIG. 32** illustrates a biosynthetic scheme for conversion of glucose to 4-HPAA, dopamine, 3,4-DHPAA, and 1-benzylisoquinoline alkaloids to reticulinc. in accordance with some embodiments of the invention.
**FIG. 33** illustrates a biosynthetic scheme for conversion of chorismate to tyrosine and phenylalanine through the arogenate intermediate. in accordance with some embodiments of the invention.
**FIG. 34** illustrates a biosynthetic scheme for glycolysis with the phosphoketalase providing a route to acetyl-CoA, in accordance with some embodiments of the invention.
**FIG. 35** depicts a bioprocess for thebaine, in accordance with embodiments of the invention.
**FIG. 36** depicts a bioprocess for thebaine. in accordance with embodiments of the invention.
**FIG. 37** depicts a bioprocess for thebaine. in accordance with embodiments of the invention.
**FIG. 38** depicts a bioprocess for thebaine, in accordance with embodiments of the invention.
**FIG. 39** illustrates a biosynthetic scheme for the recycling of methionine in accordance with embodiments of the invention.
**FIG. 40** depicts a bioprocess for thebaine, in accordance with embodiments of the invention.

### DETAILED DESCRIPTION OF THE INVENTION

The present disclosure provides methods for the production of diverse benzylisoquinoline alkaloids (BIAs) in engineered host cells. The present disclosure further provides compositions of diverse alkaloids produced in engineered host cells. Additionally, the present disclosure provides methods for the production of one or more Bet v 1-fold proteins in host cells engineered with a plurality of heterologous enzymes to produce a diverse benzylisoquinoline alkaloid product from simple starting materials such as sugar and/or L-tyrosine. Additionally, the present disclosure provides methods for the production of one or more engineered Bet v 1-fold proteins in engineered host cells. In particular cases. the disclosure provides methods for increasing production of diverse alkaloid products by engineered Bet v 1-fold proteins with N-terminal truncations. In further particular cases, the disclosure provides methods for increasing production of diverse alkaloid products by engineered Bet v 1-fold proteins with particular amino acid mutations that increase activity. In particular cases. the disclosure provides methods for producing benzylisoquinolines, promorphinans. morphinans, protoberberines, protopines, benzophenanthridines, secoberberines, phthalideisoquinolines, aporphines, bisbenzylisoquinolines, nal-opioids, nor-opioids, and others through the increased conversion of precursor BIAs to a benzylisoquinoline alkaloid product in an engineered host cell. In further particular cases, the method comprises engineering the host cell with a plurality of heterologous enzymes to increase the production of BIA precursors. including L-tyrosine and 4-HPAA. In further particular examples. an engineered host cell further comprises inactivating mutations in selected enzymes that result in increased production of diverse benzylisoquinoline alkaloid products or decreased production of byproducts. In further particular examples, an engineered host cell further comprises heterologous expression or overexpression of selected enzymes that result in increased production of diverse benzylisoquinoline alkaloid products or decreased production of byproducts. In further particular cases, the byproducts comprise tyrosol, phenylethanol, or methionol.

### Benzylisoquinoline Alkaloids (BIAs) of Interest

Host cells which produce BIAs of interest are provided. In some examples, engineered strains of host cells such as the engineered strains of the invention provide a platform for producing benzylisoquinoline alkaloids of interest and modifications thereof across several structural classes including. but not limited to. precursor BIAs. benzylisoquinolines, promorphinans, morphinans, protoberberines, protopines. benzophenanthridines, secoberberines, phthalideisoquinohnes, aporphines, bisbenzylisoquinolines, nal-opioids, nor-opioids, and others. Each of these classes is meant to include biosynthetic precursors, intermediates. and metabolites thereof. of any convenient member of an engineered host cell biosynthetic pathway that may lead to a member of the class. Non-limiting examples of compounds are given below for each of these structural classes. In some cases, the structure of a given example may or may not be characterized itself as a benzylisoquinoline alkaloid. The present chemical entities are meant to include all possible isomers, including single enantiomers, racemic mixtures, optically pure forms, mixtures of diastereomers. and intermediate mixtures.

Benzylisoquinoline alkaloid precursors may include. but are not limited to. norcoclaurine (NC) and norlaudanosoline (NL), as well as NC and NL precursors, such as tyrosine, ty ramine, 4-hydroxyphenylacetaldehyde (4-HPAA). 4-hydroxyphenylpyruvic acid (4-HPPA), L-3,4-dihydroxyphenylalanine (L-DOPA), 3,4-dihydroxyphenylacetaldehyde (3,4-DHPAA), and dopamine. In some embodiments, the one or more BIA precursors are 3,4-dihydroxyphenylacetaldehyde (3,4-DHPAA) and dopamine. In certain instances, the one or more BIA precursors are 4-hydroxyphenylacetaldehyde (4-HPAA) and dopamine. In particular. NL and NC may be synthesized, respectively, from precursor molecules via a Pictet-Spengler condensation reaction, where the reaction may occur spontaneously or may by catalyzed by any convenient enzymes.

Benzylisoquinolines may include, but are not limited to, norcoclaurine, norlaudanosoline. coclaurine, 3'-hydroxycoclaurine, 4'-*O*-methylnorlaudanosoline, 4'-*O*-methyl-laudanosoline, *N-*methylnorcoclaurine, laudanosoline, *N*-methylcoclaurine, 3'-hydroxy-*N*-methylcoclaurine, reticuline, norreticuline, papaverine, laudanine, laudanosine, tetrahydropapaverine. 1,2-dihydropapaverine, and orientaline.

Promorphinans may include. but are not limited to. salutaridine. salutaridinol. and salutaridinol-7-O-acetate.

Morphinans may include. but are not limited to, thebaine, codeinone, codeine, morphine, morphinone, oripavine, neopinone, neopine, neomorphine, hydrocodone, dihydrocodeine, 14-hydroxycodeinone, oxycodone, 14-hydroxycodeine, morphinone, hydromorphone, dihydromorphine, dihydroctorphine, ethylmorphinc. etorphine, metopon, buprenorphine, pholcodine, heterocodeine, and oxymorphone.

Protoberberines may include. but are not limited to. scoulerine, cheilanthifoline. stylopine, nandinine, jatrorrhizine. stepholidine. discretamine, *cis*-*N*-methylstylopine, tetrahydrocolumbamine, palmatine, tetrahydropalmatine, columbamine, canadine. *N*-methylcanadine, 1-hydroxycanadine, berberine. *N*-methyl-ophiocarpine, 1,13-dihydroxy-*N*-methylcanadine, and 1-hydroxy-10-*O*-acetyl-*N-*methylcanadine.

Protopines may include. but are not limited to. protopine, 6-hydroxyprotopine. allocryptopine, cryptopine, muramine. and thalictricine.

Benzophenanthridines may include, but are not limited to. dihydrosanguinarine, sanguinarine, dihydrocheilirubine, cheilirubine, dihydromarcapine, marcapine, and chelerythrine.

Secoberberines may include, but are not limited to, 4'-*O*-desmethylmacrantaldehyde, 4'-O-desmethylpapaveroxine, 4'-*O*-desmethyl-3-*O*-acetylpapaveroxine, papaveroxine, and 3-*O-*aceteylpapaveroxine.

Phthalideisoquinolines may include. but are not limited to. narcotolinehemiacetal, narcotinehemiacetal, narcotoline, noscapine. adlurnidine, adlumine, (+) or (-)-bicuculline, capnoidine, carlumine. corledine, corlumidine, decumbenine, 5'-*O*-demethylnarcotine, (+) or (-)-α or β-hydrastine, and hypecournine.

Aporphines may include. but are not limited to. magnoflorine. corytuberine, apomorphine. boldine, isoboldine, isothebaine, isocorytuberine, and glaufine.

Bisbenzylisoquinolines may include. but are not limited to, berbamunine, guattegaumerine. dauricine. and liensinine.

Nal-opioids may include. but are not limited to, naltrexone, naloxone, nalmefene, nalorphine, nalorphine, nalodeine, naldemedine, naloxegol, 6β-naltresol, naltrindole, methylnaltrexone, methylsamidorphan, alvimopan. axelopran, bevenpran, dinicotinate, levallorphan, samidorphan, buprenorphine, dezocine, eptazocine, butorphanol, levorphanol, nalbuphine, pentazocine, phenazocine, norbinaltorphimine, and diprenorphine.

Nor-opioids may include, but are not limited to, norcodeine, noroxycodone, northebaine, norhydrocodone, nordihydro-codeine, nor-14-hydroxy-codeine, norcodeinone, nor-14-hydroxy-codeinone, normorphine, noroxymorphone, nororipavine, norhydro-morphone, nordihydro-morphine, nor-14-hydroxy -morphine, normorphinone, and nor-14-hydroxy-moφhinone.

Other compounds that may be produced by the engineered strains of the invention may include, but are not limited to, rhoeadine, pavine, isopavine, and cularine.

In certain embodiments, the engineered strains of the invention may provide a platform for producing compounds related to tetrahydrobiopterin synthesis including, but not limited to, dihydroneopterin triphosphate. 6-pyruvoy tetrahydropterin, 5,6,7,8-tctrnhydrobiopterin, 7,8-dihydrobiopterin, tetrahydrobiopterin 4a-carbinolamine, quinonoid dihydrobiopterin, and biopterin.

### Host Cells

Any convenient cells may be utilized in the subject host cells and methods. In some cases, the host cells are non-plant cells. In some instances, the host cells may be characterized as microbial cells. In certain cases, the host cells are insect cells, mammalian cells, bacterial cells, fungal cells, or yeast cells. Any convenient type of host cell may be utilized in producing the subject BIA-producing cells. see, e.g., US2008/0176754. US2014/0273109. PCT/US2014/063738. PCT/US2016/030808. PCT/US2015/060891, PCT/US2016/031506. and PCT/US2017/057237, the disclosures of which are incorporated by reference in their entirety. Host cells of interest include, but are not limited to, bacterial cells, such as *Bacillus subtilis, Escherichia coli. Streptomyces, Anabaena, Arthrobacter, Acetobacter, Acetobacterium, Bacillus, Bifidobacterium, Brachybacterium, Brevibacterium, Carnobacterium, Clostridium, Corynebacterium, Enterobacter, Escherichia, Gluconacetobacter, Gluconobacter, Hafiria, Halomonas, Klebsiella, Kocuria, Lactobacillus, Leucononstoc, Macrococcus, Methylomonas, Methylobacter, Methylocella, Methylococcus, Microbacterium, Micrococcus, Microcystis, Afoorella, Oenococcus. Pediococcus, Prochlorococcus, Propionibacterium, Proteus, Pseudoalteromonas, Pseudomonas, Psychrobacter, Rhodobacter, Rhodococcus, Rhodopseudomonas, Serratia, Staphylococcus, Streptococcus, Streptomyces, Synechococcus*, *Synechocystis*, *Tetragenococcus, Weissella, Zymomonas, and Salmonella typhimuium* cells, insect cells such as *Drosophila melanogaster* S2 and *Spodoptera frugiperda* Sf9 cells, and yeast cells such as *Saccharomyces cerevisiae, Schizosaccharonyces pombe,* and *Pichia* pastoris cells. In some examples, the host cells are yeast cells or *E. coli* cells. In some cases, the host cell is a yeast cell. In some instances, the host cell is from a strain of yeast engineered to produce a BIA of interest, such as a *(R)*-1-benzylisoquinoline alkaloid. In some instances, the host cell is from a strain of yeast engineered to produce enzymes of interest. In some instances, the host cell is from a strain of yeast engineered to produce an engineered epimerase. In some embodiments. an engineered epimerase may be an engineered split epimerase. In some embodiments, an engineered epimerase may be an engineered fused epimerase. In some embodiments, epimerase activity may be encoded by separate oxidase and reductase enzymes. Additionally, in some embodiments an engineered epimerase may be able to more efficiently convert a (S)-1-benzylisoquinoline alkaloid to a (R)-1-benzylisoquinoline alkaloid relative to a parent epimerase. In some embodiments, a parent epimerase may be a wild-type epimerase. In some embodiments, a parent epimerase may be substantially similar to a wild-type epimerase. In some cases, a parent epimerase that is substantially similar to a wild-type epimerase may have an amino acid sequence that is at least 50% or more, 55% or more, 60% or more, 65% or more. 70% or more, 75% or more, 80% or more, 81% or more, 82% or more, 83% or more, 84% or more, 85% or more, 86%, or more, 87% or more, 88% or more, 89% or more, 90% or more, 91% or more. 92% or more, 93% or more, 94% or more, 95% or more, 96% or more, 97% or more, 98% or more. or 99% or more similar to an amino acid sequence of a wild-type epimerase. In some embodiments. an engineered epimerase may be separated into smaller enzymes that exhibit oxidase and reductase activities that more efficiently convert a *(S)*-1-benglisoquinoline alkaloid to a *(R)*-1-benzylisoquinoline alkaloid relative to its corresponding parent epimerase.

In some instances. the host cell is from a strain of yeast engineered to produce a thebaine synthase. The thebaine synthase may be able to more efficiently convert a salutaridinol-7-*O*-acetate to a thebaine relative to a spontaneous reaction. In some instances, the host cell is from a strain of yeast engineered to produce an engineered thebaine synthase. In some embodiments. an engineered thebaine synthase may be an engineered fusion enzyme. Additionally. the engineered thebaine synthase may be able to more efficiently convert a salutaridinol-7-*O*-acetate to a thebaine relative to a parent thebaine synthase. In some embodiments. the parent thebaine synthase may be a wild-type thebaine synthase. In some embodiments. a parent thebaine synthase may be substantially similar to a wild-type thebaine synthase. In some cases, a parent thebaine synthase that is substantially similar to a wild-type thebaine synthase may have an amino acid sequence that is at least 50% or more. 55% or more. 60% or more. 65% or more, 70% or more. 75% or more. 80% or more. 81% or more. 82% or more. 83% or more. 84% or more, 85% or more, 86% or more, 87% or more, 88% or more, 89% or more. 90% or more. 91% or more. 92% or more. 93% or more. 94% or more. 95% or more, 96% or more, 97% or more, 98% or more, or 99% or more similar to an amino acid sequence of a wild-type thebaine synthase. The engineered thebaine synthase may be engineered as a fusion enzyme to another enzyme to more efficiently convert a salutaridinol-7-*O*-acetate to a thebaine relative to the parent thebaine synthase.

In some instances, the host cell is from a strain of yeast engineered to produce a ncopinone isomerase. The neopinone isomerase may be able to more efficiently convert a neopinone to a codeinone relative to a spontaneous reaction. In some instances. the host cell is from a strain of yeast engineered to produce an engineered neopinone isomerase. In some embodiments, an engineered neopinone isomerase may be an engineered fusion enzyme. Additionally. the engineered neopinone isomerase may be able to more efficiently convert a neopinone to a codeinone relative to a parent neopinone isomerase. In some embodiments. the parent neopinone isomerase may be a wild-type neopinone isomerase. In some embodiments, a parent neopinone isomerase may be substantially similar to a wild-type neopinone isomerase. In some cases. a parent ncoprinone isomerase that is substantially similar to a wild-type neopinone isomerase may have an amino acid sequence that is at least 50% or more, 55% or more, 60% or more, 65% or more, 70% or more, 75% or more. 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more similar to an amino acid sequence of a wild-type neopinone isomerase. The engineered neopinone isomerase may be engineered as a fusion enzyme to another enzyme to more efficiently convert a neopinoite to a codeinone relative to the parent neopinone isomerase.

In some instances, the host cell is from a strain of yeast engineered to produce an engineered norcoclaurine synthase. Additionally, the engineered norcoclaurine synthase may be able to more efficiency convert a 4-HPAA and dopamine to a norcoclaurine relative to a parent norcoclaurine synthase. Additionally, the engineered norcoclaurine synthase may be able to more efficienctly convert a 3.4-DHPA and dopamine to a norlaudanosoline relative to a parent norcoclaurine synthase. In some embodiments, the parent norcoclaurine synthase may be a wild-type norcoclaurine synthase. In some embodiments, a parent norcoclaurine synthase may be substantially similar to a wild-type norcoclaurine synthase. In some cases, a parent norcoclaurine synthase that is substantially similar to a wild-type norcoclaurine synthase may have an amino acid sequence that is at least 75% or more, 80% or more, 81% or more, 82% or more, 83% or more, 84% or more. 85% or more. 86% or more, 87% or more, 88% or more, 89% or more, 90% or more, 91% or more, 92% or more, 93% or more, 94% or more, 95% or more, 96% or more, 97% or more, 98% or more. or 99% or more similar to an amino acid sequence of a wild-type norcoclaurine synthase.

Any of the host cells described in US2008/0176754, US2014/0273109, PCTUS2014/063738, PCT/US2016/030808. PCT/US2015/060891. PCT/US2016/031506. PCT/US2017/057237. and US Provisional Application No. 62/627.264 by Smolke et al. may be adapted for use in the subject cells and methods. In certain embodiments, the yeast cells may be of the species Saccharomyces *cerevisiae* (*S. cerevisiae).* In certain embodiments, the yeast cells may be of the species *Schizosaccharomyces pombe.* In certain embodiments, the yeast cells may be of the species *Pichia pastoris.* Yeast is of interest as a host cell because cytochrome P450 proteins are able to fold properly into the endoplasmic reticulum membrane so that their activity is maintained. In some examples, cytochrome P450 proteins are involved in some biosynthetic pathways of interest. In additional examples, cytochrome P450 proteins are involved in the production of BIAs of interest. In further examples. cytochrome P450 proteins are involved in the production of an enzyme of interest.

Yeast strains of interest that find use in the invention include. but are not limited to, CEN.PK (Genotype: *MAT*a/α *ura3-52*/*ura3-52 trp1-289*/*trp1-289 leu2-3_112*/*leu2*-*3_112 his3* Δ*1*/*his3* Δ*1 MAL2-8C*/*MAL2-8CSUC2*/*SUC2*), S288C. W303, D273-10B, X2180. A364A, ∑1278B, AB972. SK1, and FL100. In certain cases. the yeast strain is any of S288C (MATα: SUC2 mal mel gal2 CUP1 flol flo8-1 hapl). BY4741 (MATα: his3Δ1; leu2Δ0; metl5Δ0; ura3Δ0). BY4742 (MATα; his3Al; leu2Δ0; lys2Δ0; ura3Δ0), BY4743 (MATa/MATα; his3Δ1/his3Δ1; le2Δ0/leu2Δ0; met15Δ0/MET15; LYS2/lys2Δ0; ura3Δ0/ura3Δ0), and WAT11 or W*(R).* derivatives of the W303-B strain (MATa; ade2-1; his3-11, -15; leu2-3,-112; ura3-1; canR; cyr+) which express the*Arabidopsis thaliana* NADPH-P450 reductase ATR1 and the yeast NADPH-N50 reductase CPR1, respectively. In another embodiment the yeast cell is W303alpha (MATα: his3-11,15 trp1-1 leu2-3 ura3-1 ade2-1). The identity and genotype of additional yeast strains of interest may be found at EUROSCARF (web.uni-fratikfurt.de/fb15/mikro/euroscarf/col_index.html).

In some instances, the host cell is a fungal cell. In certain embodiments, the fungal cells may be of the Aspergillus species and strains *include Aspergillus niger* (ATCC 1015. ATCC 9029, CBS 513.88), *Aspergillus oryzae* (ATCC 56747, RIB40), *Aspergillus terreu.s* (NIH 2624, ATCC 20542) and *Aspergillus nidulans* (FGSC A4).

In certain embodiments. heterologous coding sequences may be codon optimized for expression in Aspergillus sp. and expressed from an appropriate promoter. In certain embodiments. the promoter may be selected from phosphoglycerate kinase promoter (PGK), MbfA promoter, cytochrome c oxidase subunit promoter (CoxA), SrpB promoter. TvdA promoter, malate dehydrogenase promoter (MdhA), beta-mannosidase promoter (ManB). In certain embodiments, a terminator may be selected from glucoamylase terminator (GlaA) or TrpC terminator. In certain embodiments, the expression cassette consisting of a promoter. heterologous coding sequence. and terminator may be expressed from a plasmid or integrated into the genome of the host. In certain embodiments, selection of cells maintaining the plasmid or integration cassette may be performed with antibiotic selection such as hygromycin or nitrogen source utilization, such as using acetamide as a sole nitrogen source. In certain embodiments, DNA constructs may be introduced into the host cells using established transformation methods such as protoplast transformation, lithium acetate, or electroporation. In certain embodiments, cells may be cultured in liquid ME or solid MEA (3 % malt extract. 0.5 % peptone, and ±1.5 % agar) or in Vogel's minimal medium with or without selection.

In some instances, the host cell is a bacterial cell. The bacterial cell may be selected from any bacterial genus. Examples of genuses from which the bacterial cell may come include*Anabaena*, Arthrobacter, Acetobacter, Acetobacterium. *Bacillus, Bifidobacterium, Brachyhacterium, Brevibacterium, Carnobacterium, Clostridium, Corynebacterium, Enterobacter, Escherichia, Gluconacetobaciter, Gluconobacter, Hafnia, Halomonas, Klebsiella, Kocuria, Lactobacillus, Leucononstoc, Macrococcus. Methylomonas, Methylobacter, Methylocella, Methylococcus, Microbacterium, Micrococcus, Microcystis, Moorella, Oenococcus, Pediococcus, Prochloracoccus, Propionibacterium, Proteus, Pseudoalteromonas, Pseudomonas, Psychrobacter, Rhodobacter, Rhodococcus, Rhodopseudomonas, Serratia, Staphylococcus, Streptococcus, Streptomyces, Synechococcus, Synechocustis, Tetragenococcus, Weissella,* and *Zymomonas.* Examples of bacterial species which may be used with the methods of this disclosure include *Arthrobacter nicotiance, Acetobacter aceti, Arthrobacter arilaitensis, Bacillus cereus, Bacillus coagulans, Bacillus licheniformus, Bacillus pumilus, Bacillus sphaericus, Bacillus stearothermophilus, Bacillus subtilis, Bifidobacterium adolescentis, Brachybacterium tyrofermentans, Brevibacterium linens, Carnobacterium divergens, Corynebacterium flavescens, Enterococcus faecium. Gluconacetobacter europaeus, Gluconacetobacter johannae*, *Gluconobacter oxydans, Hafnia alvei, Halomonas elongata, Kocuria rhizophila, Lactobacillus acidifarinae, Lactobacillus jensenii, Lactococcus lactis, Lactobacillus yamanashiensis, Leuconostoc citreum, Macrococcus caseolyticus, Microbacterium foliorum, Micrococcus lylae, Oenococcus oeni, Pediococcus acidilactici, Propionibacterium acidipropionici, Proteus vulgaris, Pseudomonas fluorescens. Psychrobacter celer, Staphylococcus condiments, Streptococcus thermophilus, Streptomyces griseus, Tetragenococcus halophilus, Weissella cibaria, Weissel*/*a koreensis, Zymomonas mobilis, Corynebacterium glutamicum, Bifidobacterium bifidum*/*breve*/*longum, Streptomyces lividans, Streptomyces coelicolor, Lactobacillus plantarum, Lactobacillus sakei, Lactobacillus* cases, *Pseudoalteromonas citrea, Pseudomonas putida, Clostridium ljungdahlii*/*aceticum*/*acetobutylicum*/*beijerinckii*/*butyricum,* and *Morella themocellum*/*thermoacetica.*

In certain embodiments, the bacterial cells may be of a strain of *Escherichia coli.* In certain embodiments, the strain of *E. coli* may be selected from BL21, DH5α, XL1-Blue, HB101, BL21. and K12. In certain embodiments, heterologous coding sequences may be codon optimized for expression in *E. coli* and expressed from an appropriate promoter. In certain embodiments, the promoter may be selected from T7 promoter, tac promoter, trc promoter, tetracycline-inducible promoter (tet).lac operon promoter (lac).lacOI promoter. In certain embodiments, the expression cassette consisting of a promoter, heterologous coding sequence, and terminator may be expressed from a plasmid or integrated into the genome. In certain embodiments, the plasmid is selected from pUC19 or pBAD. In certain embodiments, selection of cells maintaining the plasmid or integration cassette may be performed with antibiotic selection such as kanamycin, chloramphenicol, streptomycin, spectinomycin, gentamycin, erythromycin or ampicillin. In certain embodiments. DNA constructs may be introduced into the host cells using established transformation methods such as conjugation, heat shock chemical transformation. or electroporation. In certain embodiments, cells may be cultured in liquid Luria-Bertani (LB) media at about 37°C with or without antibiotics.

In certain embodiments, the bacterial cells may be a strain of *Bacillus subtilis.* In certain embodiments, the strain of *B. subtilis* may be selected from I779, GP25, RO-NN-1, 168, BSn5. BEST195, 1A382. and 62178. In certain embodiments, heterologous coding sequences may be codon optimized for expression in Bacillus sp. and expressed from an appropriate promoter. In certain embodiments, the promoter may be selected from grac promoter. p43 promoter, or trnQ promoter. In certain embodiments, the expression cassette consisting of the promoter, heterologous coding sequence, and terminator may be expressed from a plasmid or integrated into the genome. In certain embodiments, the plasmid is selected from pHP13 pE194, pC194, pHT01, or pHT43. In certain embodiments, integrating vectors such as pDG364 or pDG1730 may be used to integrate the expression cassette into the genome. In certain embodiments, selection of cells maintaining the plasmid or integration cassette may be performed with antibiotic selection such as erythromycin, kanamycin, tetracycline, and spectinomycin. In certain embodiments. DNA constructs may be introduced into the host cells using established transformation methods such as natural competence, heat shock, or chemical transformation. In certain embodiments, cells may be cultured in liquid Luria-Bertani (LB) media at 37°C or M9 medium plus glucose and tryptophan.

### Genetic Modifications to Host Cells

The host cells may be engineered to include one or more modifications (such as two or more, three or more, four or more, five or more, or even more modifications) that provide for the production of BIAs of interest. Additionally or alternatively, the host cells may be engineered to include one or more modifications (such as two or more, three or more, four or more, five or more, or even more modifications) that provide for the production of enzymes of interest. In some cases, a modification is a genetic modification, such as a mutation, addition, or deletion of a gene or fragment thereof, or transcription regulation of a gene or fragment thereof. As used herein, the term "mutation" refers to a deletion, insertion, or substitution of an amino acid(s) residue or nucleotide(s) residue relative to a reference sequence or motif. The mutation may be incorporated as a directed mutation to the native gene at the original locus. In some cases, the mutation may be incorporated as an additional copy of the gene introduced as a genetic integration at a separate locus, or as an additional copy on an episomal vector such as a 2µ or centromeric plasmid. In certain instances, the substrate inhibited copy of the enzyme is under the native cell transcriptional regulation. In some instances, the substrate inhibited copy of the enzyme is introduced with engineered constitutive or dynamic regulation of protein expression by placing it under the control of a synthetic promoter. In some examples, the object of one or more modifications may be a native gene. In some examples, the object of one or more modifications may be a non-native gene. In some examples, a non-native gene may be inserted into a host cell. In further examples, a non-native gene may be altered by one or more modifications prior to being inserted into a host cell.

An engineered host cell may overproduce one or more BIAs of interest. By overproduce is meant that the cell has an improved or increased production of a BIA molecule of interest relative to a control cell (e.g.. an unmodified cell). By improved or increased production is meant both the production of some amount of the BIA of interest where the control has no BIA of interest production, as well as an increase of about 10% or more, such as about 20% or more, about 30% or more, about 40% or more, about 50% or more, about 60% or more, about 80% or more, about 100% or more, such as 2-fold or more, such as 5-fold or more, including 10-fold or more in situations where the control has some BIA of interest production.

An engineered host cell may overproduce one or more (*S*)-1-benzylisoquinoline alkaloids. In some cases, the engineered host cell may produce some amount of the *(S)*-1-benzylisoquinoline alkaloid of interest where the control has no *(S)*-1-benzylisoquinoline alkaloid production, as well as an increase of about 10% or more, such as about 20% or more, about 30% or more, about 40% or more, about 50% or more, about 60% or more, about 80% or more, about 100% or more, such as 2-fold or more, such as 5-fold or more, including 10-fold or more in situations where the control has some *(S)*-1-benzylisoquinoline alkaloid of interest production.

An engineered host cell may further overproduce one or more *(R)*-1-benzylisoquinoline alkaloids. In some cases, the engineered host cell may produce some amount of the *(R)-1-*benzylisoquinoline alkaloid of interest where the control has no (*R*)-1-benzylisoquinoline alkaloid production, as well as an increase of about 10% or more, such as about 20% or more, about 30% or more, about 40% or more, about 50% or more, about 60% or more, about 80% or more, about 100% or more, such as 2-fold or more, such as 5-fold or more, including 10-fold or more in situations where the control has some *(R)*-1-benzy lisoquinoline alkaloid of interest production. An engineered host cell may further overproduce one or more of 1-benzylisoquinoline alkaloids.

An engineered host cell may further overproduce one or more morphinan alkaloids. In some cases, the engineered host cell may produce some amount of the morphinan alkaloid of interest where the control has no morphinan alkaloid production, as well as an increase of about 10% or more, such as about 20% or more, about 30% or more, about 40% or more, about 50% or more, about 60% or more, about 80% or more, about 100% or more, such as 2-fold or more, such as 5-fold or more, including 10-fold or more in situations where the control has some morphinan alkaloid of interest production. In some cases, the morphinan alkaloid is formed from a 1-benzylisoquinoline alkaloid product, or derivative thereof, of an epimerization reaction cataly zed by an engineered epimerase within an engineered host cell. The engineered epimerase may comprise two separate enzymes that work to produce an epimerase reaction. An engineered host cell may further overproduce one or more of promorphinan, nor-opioid, or nal-opioid alkaloids.

In some cases, the engineered host cell having an engineered split epimerase is capable of producing an increased amount of *(R)*-reticuline relative to a host cell having an engineered fused epimerase. In some cases, the engineered host cell having modifications to an oxidase portion of an engineered epimerase is capable of producing an increased amount of *(R)*-reticuline relative to a control host cell that lacks the one or more modifications to the oxidase portion of the engineered epimerase (e.g., as described herein). In certain instances, the increased amount of *(R)*-reticuline is about 10% or more relative to the control host cell, such as about 20% or more, about 30% or more, about 40% or more, about 50% or more, about 60% or more, about 80% or more, about 100% or more, about 2-fold or more, about 5-fold or more, or even about 10-fold or more relative to the control host cell. In some cases, *(R)-*reticuline is the product of an epimerization reaction cataly zed by at least one engineered epimerase within an engineered host cell. In these cases. *(S)*-reticuline may be the substrate of the epimerization reaction.

In some cases, the engineered host cell is capable of producing an increased amount of thebaine relative to a control host cell that lacks the one or more modifications (e.g.. as described herein). In some cases, the engineered host cell having a thebaine synthase is capable of producing an increased amount of thebaine relative to a host cell that lacks a thebaine synthase. In some cases, the engineered host cell having an engineered thebaine synthase is capable of producing an increased amount of thebaine relative to a host cell having a parent thebaine synthase (e.g.. as described herein). In certain instances, the increased amount of thebaine is about 10% or more relative to the control host cell, such as about 20% or more, about 30% or more, about 40% or more, about 50% or more, about 60% or more, about 80% or more, about 100% or more, about 2-fold or more, about 5-fold or more, or even about 10-fold or more relative to the control host cell. In some cases, thebaine is the product of a thebaine synthase reaction within an engineered host cell. In some cases, thebaine is the product of a thebaine synthase reaction catalyzed by at least one engineered thebaine synthase within an engineered host cell. In these cases, salutaridinol-7-*O*-acetate may be the substrate of the thebaine synthase reaction.

In some cases, the engineered host cell is capable of producing an increased amount of codeinone, or morphinan alkaloid product downstream from codeinone in a biosynthetic pathway, relative to a control host cell that lacks the one or more modifications (e.g.. as described herein). In some cases, the engineered host cell having a neopinone isomerase is capable of producing an increased amount of codeinone, or morphinan alkaloid product downstream from codeinone in a biosynthetic pathway, relative to a host cell that lacks a neopinone isomerase. In some cases, the engineered host cell having an engineered neopinone isomerase is capable of producing an increased amount of codeinone, or morphinan alkaloid product downstream from codeinone in a biosynthetic pathway, relative to a host cell having a parent neopinone isomerase (e.g., as described herein). In certain instances, the increased amount of codeinone, or morphinan alkaloid product downstream from codeinone in a biosynthetic pathway, is about 10% or more relative to the control host cell, such as about 20% or more, about 30% or more, about 40% or more, about 50% or more, about 60% or more, about 80% or more, about 100% or more, about 2-fold or more, about 5-fold or more, or even about 10-fold or more relative to the control host cell. In some cases, codeinone is the product of a neopinone isomerase reaction within an engineered host cell. In some cases, codeinone is the product of a neopinone isomerase reaction catalyzed by at least one engineered neopinone isomerase within an engineered host cell. In these cases, neopinone may be the substrate of the neopinone isomerase reaction.

In some cases, the engineered host cell having an engineered norcoclaurine synthase is capable of producing an increased amount of one or more BIAs of interest relative to a control host cell having a parent norcoclaurine synthase. In certain instances, the increased amount of the benzylisoquinoline alkaloids of interest is about 10% or more relative to the control host cell, such as about 20% or more, about 30% or more, about 40% or more, about 50% or more, about 60% or more, about 80% or more, about 100% or more, about 2-fold or more, about 5-fold or more, or even about 10-fold or more relative to the control host cell. In some cases, norcoclaurine is the product of a condenstation reaction catalyzed by at least one engineered norcoclaurine synthase within an engineered host cell. In these cases, 4-HPAA and dopamine may be the substrates of the condensation reaction. In some cases, norlaudanosoline is the product of a condenstation reaction catalyzed by at least one engineered norcoclaurine synthase within an engineered host cell. In these cases, 3,4-DHPA and dopamine may be the substrates of the condensation reaction. An engineered host cell may further produce an increased amount of one or more of BIAs in the structural classes of benzylisoquinolines, promorphinans, morphinans, protoberberines, protopines, benzophenanthridines, secoberberines, phthalideisoquinolines, aporphines, bisbenzylisoquinolines, nal-opioids, nor-opioids, and others.

Additionally, an engineered host cell may overproduce one or more enzymes of interest. By overproduce is meant that the cell has an improved or increased production of an enzyme of interest relative to a control cell (e.g., an unmodified cell). By improved or increased production is meant both the production of some amount of the enzyme of interest where the control has no production, as well as an increase of about 10% or more, such as about 20% or more, about 30% or more, about 40% or more, about 50% or more, about 60% or more, about 80% or more, about 100% or more, such as 2-fold or more, such as 5-fold or more, including 10-fold or more in situations where the control has some enzyme of interest production.

An engineered host cell may overproduce one or more engineered DRS-DRR enzymes. In some cases, the engineered host cell may produce some amount of the engineered DRS-DRR epimerase where the control has no DRS-DRR enzyme production, or where the control has a same level of production of wild-type epimerases in comparison to the engineered host cell, as well as an increase of about 10% or more, such as about 20% or more, about 30% or more, about 40% or more, about 50% or more, about 60% or more, about 80% or more, about 100% or more, such as 2-fold or more, such as 5-fold or more, including 10-fold or more in situations where the control has some DRS-DRR enzyme production. In some cases, an engineered DRS-DRR epimerase may be an engineered split epimerase. In some cases, an engineered DRS-DRR epimerase may be an engineered fused epimerase.

An engineered host cell may overproduce one or more thebaine synthase enzymes. In some cases, the engineered host cell may produce some amount of the thebaine synthase enzyme where the control has no thebaine synthase enzyme production, as well as an increase of about 10% or more, such as about 20% or more, about 30% or more, about 40% or more, about 50% or more, about 60% or more, about 80% or more, about 100% or more, such as 2-fold or more, such as 5-fold or more, including 10-fold or more in situations where the control has some thebaine synthase enzyme production.

An engineered host cell may overproduce one or more engineered thebaine synthase enzymes. In some cases, the engineered host cell may produce some amount of the engineered thebaine synthase where the control has no thebaine synthase enzyme production, or where the control has a same level of production of wild-type thebaine synthase in comparison to the engineered host cell, as well as an increase of about 10% or more, such as about 20% or more, about 30% or more, about 40% or more, about 50% or more, about 60% or more, about 80% or more, about 100% or more, such as 2-fold or more, such as 5-fold or more, including 10-fold or more in situations where the control has some thebaine synthase enzyme production. In some cases, an engineered thebaine synthase may be an engineered fusion enzyme.

An engineered host cell may further overproduce one or more enzymes that are derived from the thebaine synthase enzyme. In some cases, the engineered host cell may produce some amount of the enzymes that are denved from the thebaine synthase enzyme. where the control has no production of enzymes that are derived from the thebaine synthase enzyme, as well as an increase of about 10% or more, such as about 20% or more, about 30% or more, about 40% or more, about 50% or more, about 60% or more, about 80% or more, about 100% or more, such as 2-fold or more, such as 5-fold or more, including 10-fold or more in situations where the control has some production of enzymes that are derived from the thebaine synthase enzyme.

An engineered host cell may overproduce one or more neopinone isomerase enzymes. In some cases, the engineered host cell may produce some amount of the neopinone isomerase enzyme where the control has no neopinone isomerase enzyme production, as well as an increase of about 10% or more, such as about 20% or more, about 30% or more, about 40% or more, about 50% or more, about 60% or more, about 80% or more, about 100% or more, such as 2-fold or more, such as 5-fold or more, including 10-fold or more in situations where the control has some neopinone isomerase enzyme production.

An engineered host cell may overproduce one or more engineered neopinone isomerase enzymes. In some cases, the engineered host cell may produce some amount of the engineered neopinone isomerase where the control has no neopinone isomerase enzyme production, or where the control has a same level of production of wild-type neopinone isomerase in comparison to the engineered host cell, as well as an increase of about 10% or more, such as about 20% or more, about 30% or more, about 40% or more, about 50% or more, about 60% or more, about 80% or more, about 100% or more, such as 2-fold or more, such as 5-fold or more, including 10-fold or more in situations where the control has some neopinone isomerase enzyme production. In some cases, an engineered neopinone isomerase may be an engineered fusion enzyme.

An engineered host cell may further overproduce one or more enzymes that are derived from the neopinone isomerase enzyme. In some cases, the engineered host cell may produce some amount of the enzymes that are derived from the neopinone isomerase enzyme, where the control has no production of enzymes that are derived from the neopinone isomerase enzyme, as well as an increase of about 10% or more, such as about 20% or more, about 30% or more, about 40% or more, about 50% or more, about 60% or more, about 80% or more, about 100% or more, such as 2-fold or more, such as 5-fold or more, including 10-fold or more in situations where the control has some production of enzymes that are derived from the neopinone isomerase enzyme.

An engineered host cell may overproduce one or more engineered norcoclaurine synthase enzymes In some cases, the engineered host cell may produce some amount of the engineered norcoclaurine synthase where the control has no norcoclaurine synthase enzyme production, or where the control has a same level of production of wild-type norcoclaurine synthase in comparison to the engineered host cell, as well as an increase of about 10% or more, such as about 20% or more, about 30% or more, about 40% or more, about 50% or more, about 60% or more, about 80% or more, about 100% or more, such as 2-fold or more, such as 5-fold or more, including 10-fold or more in situations where the control has some norcoclaurine synthase enzyme production.

Additionally, an engineered host cell may overproduce one or more bisbenzylisoquinoline alkaloids (bisBIAs). In particular, an engineered host cell is capable of producing an increased amount of bisbenzylisoquinoline alkaloids (bisBlAs) relative to a control host cell that lacks the one or more modifications (e.g., as described herein), including modifications related to harboring an engineered epimerase. In certain instances, the increased amount of bisBiAs is about 10% or more relative to the control host cell, such as about 20% or more, about 30% or more, about 40% or more, about 50% or more, about 60% or more, about 80% or more, about 100% or more, about 2-fold or more, about 5-fold or more, or even about 10-fold or more relative to the control host cell. In some cases, the bisBIA is formed from at least one BIA monomer that is the product, or derivative thereof, of an epimerization reaction cataly zed by an engineered epimerase within an engineered host cell. The engineered epimerase may comprise two separate enzymes that work to produce an epimerase reaction. An engineered host cell may further overproduce one or more of cepharanthine, fangehinoline, liensinine, neferine, tubocurarine, dauricine. tetrandrine, curine, berbamunine, guattegaumenne. 2'-nonberbamunine, and berbamine.

In some cases, the one or more (such as two or more, three or more. or four or more) modifications may be selected from: an engineered thebaine synthase modification; an engineered neopinone isomerase modification; an engineered split epimerase modification; an engineered norcoclaurine synthase modification: an enzyme expression modification; an inactivation modification; and a byproduct inhibition alleviating modification, or a combination thereof. A cell that includes one or more modifications may be referred to as an engineered cell.

### Substrate Inhibition Alleviating Mutations

In some instances, the engineered host cells are cells that include one or more substrate inhibition alleviating mutations (such as two or more, three or more, four or more, five or more, or even more) in one or more biosynthetic enzyme genes of the cell. In some examples, the one or more biosynthetic enzyme genes are native to the cell (e.g., is present in an unmodified cell). In some examples, the one or more biosynthetic enzyme genes are non-native to the cell As used herein, the term "substrate inhibition alleviating mutation" refers to a mutation that alleviates a substrate inhibition control mechanism of the cell.

A mutation that alleviates substrate inhibition reduces the inhibition of a regulated enzyme in the cell of interest relative to a control cell and provides for an increased level of the regulated compound or a downstream biosynthetic product thereof. In some cases, by alleviating inhibition of the regulated enzyme is meant that the IC₅₀ of inhibition is increased by 2-fold or more, such as by 3-fold or more. 5-fold or more. 10-fold or more, 30-fold or more. 100-fold or more, 300-fold or more. 1000-fold or more, or even more. By increased level is meant a level that is 110% or more of that of the regulated compound in a control cell or a downstream product thereof, such as 120% or more, 130% or more, 140% or more, 150% or more, 160%, or more, 170% or more, 180% or more. 190% or more, or 200%, or more. such as at least 3-fold or more, at least 5-fold or more, at least 10-fold or more or even more of the regulated compound in the engineered host cell or a downstream product thereof

A variety of substrate inhibition control mechanisms and biosynthetic enzymes in the engineered host cell that are directed to regulation of levels of BIAs of interest, or precursors thereof, may be targeted for substrate inhibition alleviation. The engineered host cell may include one or more substrate inhibition alleviating mutations in one or more biosynthetic enzyme genes. The one or more mutations may be located in any convenient biosynthetic enzyme genes where the biosynthetic enzyme is subject to regulatory control. In some embodiments, the one or more biosynthetic enzyme genes encode one or more tyrosine hydroxylase enzymes. In certain instances, the one or more substrate inhibition alleviating mutations are present in a biosynthetic enzyme gene that is TyrH. In some embodiments, the engineered host cell may include one or more substrate inhibition alleviating mutations in one or more biosynthetic enzyme genes such as one of those genes described in **Table 11.**

In certain embodiments, the one or more substrate inhibition alleviating mutations are present in the TyrH gene. The TyrH gene encodes ty rosinc hydroxylase, which is an enzyme that converts tyrosine to L-DOPA. However, TyrH is inhibited by its substrate, tytosine. Mammalian tyrosine hydroxylase activity, such as that seen in humans or rats, can be improved through mutations to the TyrH gene that relieve substrate inhibition. In particular, substrate inhibition from tyrosine can be relieved by a point mutation W166Y in the TyrH gene. The point mutation W166Y in the TyrH gene may also improve the binding of the cosubstrate of tyrosine hydroxylase. BH₄, to catalyze the reaction of tyrosine to L-DOPA. The mutants of TyrH, when expressed in yeast strains to produce BIAs from sugar (such as those described in United States Provisional Patent Application Serial No. 61/899.496) can significantly improve the production of BIAs.

Any convenient numbers and types of mutations may be utilized to alleviate a substrate inhibition control mechanism. In certain embodiments, the engineered host cells of the present invention may include 1 or more. 2 or more. 3 or more. 4 or more, 5 or more, 6 or more, 7 or more, 8 or more, 9 or more, 10 or more, 11 or more. 12 or more. 13 or more, 14 or more, or even 15 or more substrate inhibition alleviating mutations, such as 1, 2. 3. 4, 5. 6. 7. 8. 9. 10. 11, 12. 13, 14 or 15 substrate inhibition alleviating mutations in one or more biosynthetic enzyme genes within the engineered host cell.

### Cofactor Recovery Promoting Mechanisms

In some instances, the engineered host cells are cells that include one or more cofactor recovery promoting mechanisms (such as two or more, three or more, four or more, five or more, or even more) in one or more biosynthetic enzyme genes of the cell. In some examples, the one or more biosynthetic enzyme genes are native to the cell (e.g.. is present in an unmodified cell). In some examples, the one or more biosynthetic enzyme genes are non-native to the cell. As used herein, the term "cofactor recovery promoting mechanism" refers to a mechanism that promotes a cofactor recovery control mechanism of the cell.

A variety of cofactor recovery control mechanisms and biosynthetic enzymes in the engineered host cell that are directed to regulation of levels of BIAs of interest, or precursors thereof, may be targeted for cofactor recovery promotion. The engineered host cell may include one or more cofactor recovery promoting mechanism in one or more biosynthetic enzyme genes. In some examples, the engineered host cell may include a heterologous coding sequence that encodes dihydrofolate reductase (DHFR). When DHFR is expressed, it may convert 7,8-dihydrobiopterin (BH₂) to the tetrahydrobioplerin (BH₄), thereby recovering BH₄ as a TyrH cosubstrate. In some examples, the engineered host cell may include one or more cofactor recovery promoting mechanisms in one or more biosynthetic enzyme genes such as one of those genes described in **Table 11.**

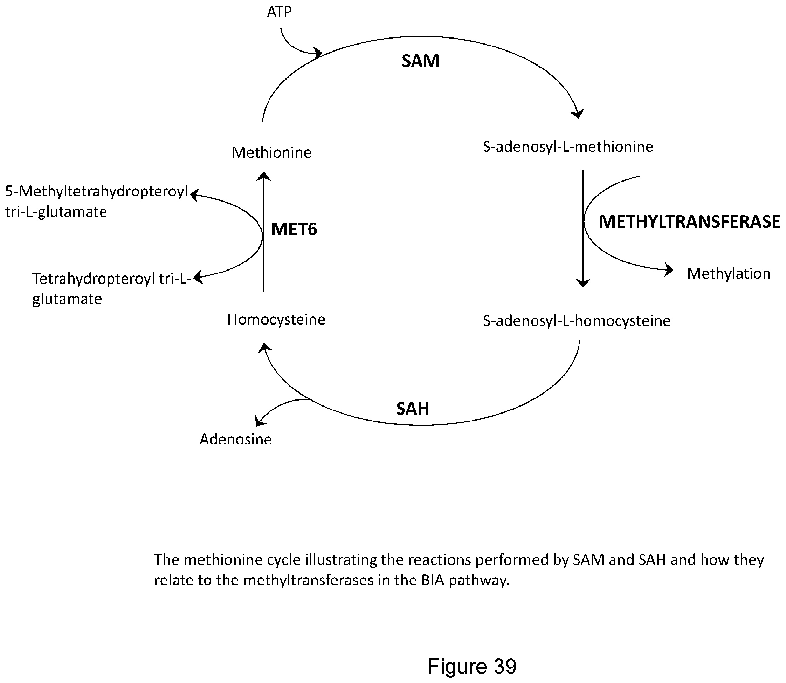

One important cofactor for production of BIAs of interest is S-adenosyl-L-methionine (SAM) used by multiple methyltransferase enzymes. As SAM is utilized in this reaction, it is converted to S-adenosyl-L-homocysteine (SAH), homocysteine, methionine, and then back to SAM. This pathway is illustrated in **FIG. 39** and may be targeted for modification to increase cofactor recovery. In some examples, the engineered host cell may include overexpression of the native S-adenosyl-L-homocysteine hydrolase (SAH1). In some examples, the engineered host cell may include overexpression of the native methionine synthase (MET6). In some examples, the engineered host cell may include overexpression of the native S-adenosylmethionine synthetase (SAM2). When one or more of these genes is overexpressed, it may increase recovery of SAH to SAM. In some examples. the engineered host cell may include one or more cofactor recycling genes described in **Table 11.**

Any convenient numbers and types of mechanisms may be utilized to promote a cofactor recovery control mechanism. In certain embodiments, the engineered host cells of the present invention may include 1 or more, 2 or more. 3 or more. 4 or more, 5 or more, 6 or more, 7 or more, 8 or more, 9 or more, 10 or more, 11 or more, 12 or more, 13 or more, 14 or more, or even 15 or more cofactor recovery promoting mechanisms such as l. 2. 3. 4. 5, 6, 7. 8, 9, 10, 11, 12, 13, 14 or 15 cofactor recovery promoting mechanisms in one or more biosynthetic enzyme genes within the engineered host cell.

### Product Inhibition Alleviating Mutations

In some instances. the engineered host cells are cells that include one or more product inhibition alleviating mutations (such as two or more. three or more. four or more, five or more. or even more) in one or more biosynthetic enzyme genes of the cell. In some examples, the one or more biosynthetic enzyme genes are native to the cell (e.g.. is present in an unmodified cell). In some examples. the one or more biosynthetic enzyme genes are non-native to the cell. As used herein. the term "product inhibition alleviating mutation" refers to a mutation that alleviates a short term and/or long term product inhibition control mechanism of an engineered host cell. Short term product inhibition is a control mechanism of the cell in which there is competitive binding at a cosubstrate binding site. Long term product inhibition is a control mechanism of the cell in which there is irreversible binding of a compound away from a desired pathway.

A mutation that alleviates product inhibition reduces the inhibition of a regulated enzyme in the cell of interest relative to a control cell and provides for an increased level of the regulated compound or a downstream biosynthetic product thereof. In some cases. by alleviating inhibition of the regulated enzyme is meant that the IC₅₀ of inhibition is increased by 2-fold or more, such as by 3-fold or more, 5-fold or more, 10-fold or more. 30-fold or more. 100-fold or more, 300-fold or more, 1000-fold or more. or even more. By increased level is meant a level that is 110% or more of that of the regulated compound in a control cell or a downstream product thereof. such as 120% or more. 130% or more, 140% or more, 150% or more. 160% or more, 170% or more, 180% or more, 190% or more, or 200% or more, such as at least 3-fold or more, at least 5-fold or more, at least 10-fold or more or even more of the regulated compound in the engineered host cell or a downstream product thereof.

A variety of product inhibition control mechanisms and biosynthetic enzymes in the engineered host cell that are directed to regulation of levels of BIAs of interest may be targeted for product inhibition alleviation. The engineered host cell may include one or more product inhibition alleviating mutations in one or more biosynthetic enzyme genes. The mutation may be located in any convenient biosynthetic enzyme genes where the biosynthetic enzyme is subject to regulatory control. In some embodiments. the one or more biosynthetic enzyme genes encode one or more tyrosine hydroxylise enzymes. In certain instances, the one or more product inhibition alleviating mutations are present in a biosynthetic enzyme gene that is TyrH. In some embodiments, the engineered host cell includes one or more product inhibition alleviating mutations in one or more biosynthetic enzyme genes such as one of those genes described in

### Table 11.

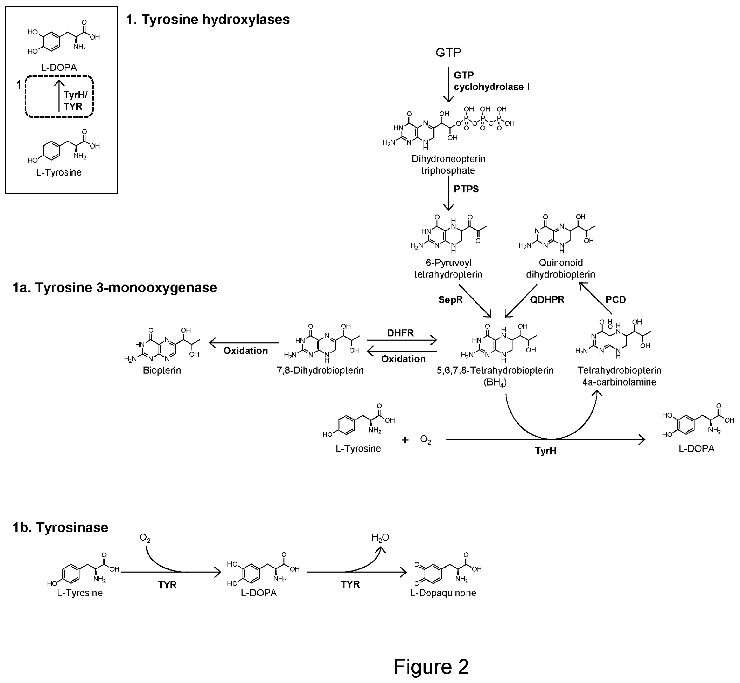

In certain embodiments, the one or more product inhibition alleviating mutations are present in the TyrH gene. The TyrH gene encodes tyrosine hydroxylase, which is an enzyme that converts tyrosine to L-DOPA. TyrH requires tetrahydrobiopterin (BH₄) as a cosubstrate to catalyze the hydroxylation reaction. Some microbial strains, such as *Saccharomyces cerevisiae,* do not naturally produce BH₄, but can be engineered to produce this substrate through a four-enzyme synthesis and recycling pathway, as illustrated in **FIG. 2. FIG.** 2 illustrates examples of synthesis, recycling, and salvage pathways of tetrahydrobiopterin, in accordance with some embodiments of the invention. **FIG. 2** provides the use of the enzymes PTPS. pyruvoyl tetrahydropterin synthase; SepR, sepiapterin reductase; PCD, pterin 4a-carbinolamine dehydratase; QDHPR. dihydropteridine reductase: and DHFR, dihydrofolate reductase. Of the enzymes that are illustrated in **FIG. 2****.** yeast synthesizes an endogenous GTP cyclohydrolase 1. GTP and dihydroneoplerin triphosphate are naturally synthesized in yeast. Additionally, other metabolites in FIG. 2 are not naturally produced in yeast.

TyrH is inhibited by its product L-DOPA, as well as other catecholamines, particularly dopamine. Mammalian tyrosine hydroxylase activity, such as from humans or rats, can be improved through mutations that relieve product inhibition. For example, short term product inhibition. such as competitive binding at the cosubstrate binding site, can be relieved by a point mutation W166Y on the TyrH gene. In particular. the point mutation W166Y on the TyrH gene may improve binding of the cosubstrate. Additionally, short term product inhibition to relieve competitive binding at the cosubstrate binding site may be improved by a point mutation S40D on the TyrH gene. Short tenn product inhibition may also be improved by the joint mutations of R37E, R38E on the TyrH gene. In particular, R37E. R38E mutations may together specifically improve tyrosine hydroxylase activity in the presence of dopamine.

Additionally, long term product inhibition may be relieved by point mutations on the TyrH gene. Long term product inhibition relief may include the irreversible binding of catecholamine to iron in the active site such that there is less catecholamine present to act as a product inhibitor of tyrosine hydroxylase activity. Long term product inhibition can be relieved by the mutations E332D and Y371F, respectively, in the TyrH gene.

Combinations of the mutations can be made (such as two or three or more mutations at once) to relieve multiple types of substrate and product inhibition to further improve the activity of TyrH. The mutants of TyrH, when expressed in yeast strains to produce BIAs from sugar (such as those described in United States Provisional Patent Application Serial No. 61/899.496) can significantly improve the production of BIAs.

Any convenient numbers and types of mutations may be utilized to alleviate a product inhibition control mechanism. In certain embodiments, the engineered host cells of the present invention may include 1 or more, 2 or more, 3 or more, 4 or more, 5 or more, 6 or more, 7 or more, 8 or more, 9 or more, 10 or more, 11 or more. 12 or more, 13 or more. 14 or more, or even 15 or more product inhibition alleviating mutations, such as 1, 2, 3, 4, 5, 6, 7. 8. 9, 10, 11, 12, 13, 14 or 15 product inhibition alleviating mutations in one or more biosynthetic enzyme genes within the engineered host cell.

### Feedback Inhibition Alleviating Mutations

In some instances, the engineered host cells are cells that include one or more feedback inhibition alleviating mutations (such as two or more. three or more, four or more, five or more, or even more) in one or more biosynthetic enzyme genes of the cell. In some cases, the one or more biosynthetic enzyme genes are native to the cell (e.g., is present in an unmodified cell). Additionally or alternatively, in some examples the one or more biosynthetic enzyme genes are non-native to the cell. As used herein, the term "feedback inhibition alleviating mutation" refers to a mutation that alleviates a feedback inhibition control mechanism of an engineered host cell. Feedback inhibition is a control mechanism of the cell in which an enzyme in the synthetic pathway of a regulated compound is inhibited when that compound has accumulated to a certain level, thereby balancing the amount of the compound in the cell. A mutation that alleviates feedback inhibition reduces the inhibition of a regulated enzyme in the engineered host cell relative to a control cell. In this way, engineered host cell provides for an increased level of the regulated compound or a downstream biosynthetic product thereof. In some cases, by alleviating inhibition of the regulated enzyme is meant that the IC₅₀ of inhibition is increased by 2-fold or more, such as by 3-fold or more. 5-fold or more, 10-fold or more, 30-fold or more. 100-fold or more, 300-fold or more. 1000-fold or more, or even more. By increased level is meant a level that is 110% or more of that of the regulated compound in a control cell or a downstream product thereof, such as 120% or more. 130% or more. 140%, or more, 150% or more, 160% or more, 170% or more. 180% or more. 190% or more, or 200% or more, such as at least 3-fold or more, at least 5-fold or more, at least 10-fold or more or even more of the regulated compound in the host cell or a downstream product thereof.

A variety of feedback inhibition control mechanisms and biosynthetic enzymes that are directed to regulation of levels of BIAs of interest may be targeted for alleviation in the host cell. The host cell may include one or more feedback inhibition alleviating mutations in one or more biosynthetic enzyme genes native to the cell. The one or more mutations may be located in any conveinent biosynthetic enzyme genes where the biosynthetic enzyme is subject to regulatory control. In some embodiments, the one or more biosynthetic enzyme genes may encode one or more enzymes selected from a 3-deoxy-d-arabinose-heptulosonate-7-phosphate (DAHP) synthase and a chorismate mutase. In some embodiments, the one or more biosynthetic enzyme genes encode a 3-deoxy-d-arabinose-heptulosonate-7-phosphate (DAHP) synthase. In some instances, the one or more biosynthetic enzyme genes may encode a chorismate mutase. In certain instances, the one or more feedback inhibition alleviating mutations may be present in a biosynthetic enzyme gene selected from ARO4 and ARO7. In certain instances, the one or more feedback inhibition alleviating mutations may be present in a biosynthetic enzyme gene that is ARO4. In certain instances, the one or more feedback inhibition alleviating mutations are present in a biosynthetic enzyme gene that is ARO7. In some embodiments, the engineered host cell may include one or more feedback inhibition alleviating mutations in one or more biosynthetic enzyme genes such as one of those genes described in **Table 11.**

Any convenient numbers and types of mutations may be utilized to alleviate a feedback inhibition control mechanism. As used herein, the term "mutation" refers to a deletion, insertion, or substitution of an amino acid(s) residue or nucleotide(s) residue relative to a reference sequence or motif. The mutation may be incorporated as a directed mutation to the native gene at the original locus. In some cases, the mutation may be incorporated as an additional copy of the gene introduced as a genetic integration at a separate locus, or as an additional copy on an episomal vector such as a 2µ or centromeric plasmid. In certain instances, the feedback inhibited copy of the enzyme is under the native cell transcriptional regulation. In some instances, the feedback inhibited copy of the enzyme is introduced with engineered constitutive or dy namic regulation of protein expression by placing it under the control of a synthetic promoter.

In certain embodiments, the one or more feedback inhibition alleviating mutations may be present in the ARO4 gene. ARO4 mutations of interest may include, but are not limited to, substitution of the lysine residue at position 229 with a leucine, a substitution of the glutamine residue at position 166 with a lysine residue. or a mutation as described by Hartmann M. et al. ((2003) Proc Natl Acad Sci U S A 100(3):862-867) or Fukuda et al. ((1992) J Ferment Bioeng 74(2):117-119). In some instances, mutations for conferring feedback inhibition may be selected from a mutagenized library of enzyme mutants. Examples of such selections may include rescue of growth of o-fluoro-D,L,-phenylalanine or growth of aro3 mutant yeast strains in media with excess tyrosine as described by Fukuda et al. ((1990) Breeding of Brewing Yeast Producing a Large Amount of Beta-Phenylethyl Alcohol and Beta-Phentlethyl Acetate Agr Biol Chem Tokyo 54(1):269-271).

In certain embodiments, the engineered host cells of the present invention may include 1 or more, 2 or more, 3 or more, 4 or more, 5 or more, 6 or more, 7 or more. 8 or more. 9 or more, 10 or more, 11 or more. 12 or more, 13 or more. 14 or more, or even 15 or more feedback inhibition alleviating mutations, such as 1, 2, 3, 4, 5, 6, 7, 8. 9. 10, 11, 12. 13, 14 or 15 feedback inhibition alleviating mutations in one or more biosynthetic enzyme genes within the engineered host cell.

### Byproduct Inhibition Alleviating Modifications

The host cells may include one or more modifications (such as two or more, three or more, four or more, five or more, or even more modifications) of one or more biosynthetic enzyme genes of the cell that are directed to alleviating byproduct inhibition. in some examples, the one or more biosynthetic enzyme genes are native to the cell. In some examples, the one or more biosynthetic enzyme genes are non-native to the cell. Any convenient biosynthetic enzyme genes of the cell may be targeted for modification to alleviate accumulation of key byproducts. As used herein, the term "byproduct inhibition alleviating modification" refers to a modification that reduces the accumulation of a key inhibitory byproduct of an engineered host cell. Byproduct inhibition is a mechanism of the cell in which accumulation of a particular byproduct compound of fermentation inhibits production of BIAs of interest when that compound has accumulated to a certain level. A modification that alleviates byproduct inhibition reduces the accumulation of one or more byproduct compounds in the engineered host cell relative to a control cell. In this way, the engineered host cell provides for a decreased level of the byproduct compound and/or an increased level of the BIAs of interest. By increased level is meant a level that is 110% or more of that of the BIAs of interest in a control cell, such as 120% or more, 130% or more, 140% or more, 150% or more. 160% or more, 170% or more. 180% or more, 190% or more, or 200% or more, such as at least 3-fold or more, at least 5-fold or more, at least 10-fold or more or even more of the BIAs of interest in the control cell. By decreased level is meant a level that is reduced by 10% or more, such as by 20% or more. 30% or more. 40% or more, 50% or more, 60% or more, 70% or more, 80% or more, 90% or more. 95% or more. 97% or more, or 99% or more, of that of the byproduct compound in a control cell. Modifications of host cell processes of interest that may be adapted for use in the subject host cells are described in U.S. Publication No. 20140273109 (14/211.611) by Smolke et al., the disclosure of which is herein incorporated by reference in its entirety.

A variety of byproduct inhibition alleviating modifications and biosynthetic enzymes in the engineered host cell that are directed to modifying the accumulation of levels of byproducts of interest may be targeted for modification. The engineered host cell may include one or more byproduct inhibition alleviating mechanism in one or more biosynthetic enzyme genes. In some examples, the byproducts of interest are fusel alcohols. In some examples, the byproducts are tyrosol, phenylethanol, or methionol. In some examples, the engineered host cell may include one or more byproduct inhibition alleviating mechanisms in one or more biosynthetic enzyme genes such as one of those genes described in **Table 11.**

In some examples, the engineered host cell may include one or more heterologous coding sequences that encode one or more biosynthetic enzymes. In some examples. the biosynthetic enzymes are 4-hydroxyphenylacetaldehyde synthase (HPAAS). When HPAAS is expressed, it may convert L-tyrosine to 4-HPAA. In some examples, the biosynthetic enzymes are prephenate-aspartate anunotransferase (PAT), arogenate dehydratase (ADT), and arogenate dehydrogenase (AAT). When PAT, AUT, and AAT are expressed, it may convert chorismate to L-tyrosine and L-phenylalamine through arogenate. In some examples, the biosynthetic enzymes are phosphoketolase (PK). When PK is expressed. it may convert fructose-6-phosphate and xylulose-5-phosphate to acetyl-phosphate. In some examples, the biosynthetic enzymes are uridine 5'-diphospho-glucosyltransferase (UGT). When UGT enzyme is expressed, it may convert a phenol to an aryl beta-D-glucose. In cases where UGT is expressed, it may be in combination with an inactivating mutation in EGH1 to increase the availability of the substrate UDP-glucose.

In some examples, the engineered host cell may include one or more inactivating mutations in one or more genes that encode biosynthetic enzymes. In some examples, the one or more inactivating mutations are in ARO8, ARO9. ARO10, PDC1, PDC5, PDC6. ARII, ATF1, ATF2, EHT1, EEB1, AAD3, YPR1, GRE2, ADH1, ADH2. ADH3, ADH4, ADH5, ADH6, ADH7, YPR1, YDR541c, BAT2, HFD1, TYR1, PHA2.

Any convenient numbers and types of modifications may be utilized to alleviate a byproduct inhibition mechanism. In certain embodiments, the engineered host cells of the present invention may include 1 or more. 2 or more. 3 or more. 4 or more. 5 or more. 6 or more. 7 or more. 8 or more. 9 or more. 10 or more. 11 or more, 12 or more, 13 or more. 14 or more, or even 15 or more byproduct inhibition alleviating modifications, such as 1, 2, 3. 4, 5, 6, 7, 8, 9, 10. 11, 12, 13, 14 or 15 byproduct inhibition alleviating modifications in one or more biosynthetic enzyme genes within the engineered host cell.

### Transcriptional Modulation Modifications

The host cells may include one or more transcriptional modulation modifications (such as two or more, three or more, four or more, five or more, or even more modifications) of one or more biosynthetic enzyme genes of the cell. In some examples, the one or more biosynthetic enzyme genes are native to the cell. In some examples, the one or more biosynthetic enzyme genes are non-native to the cell. Any convenient biosynthetic enzyme genes of the cell may be targeted for transcription modulation. By transcription modulation is meant that the expression of a gene of interest in a modified cell is modulated. e.g.. increased or decreased, enhanced or repressed, relative to a control cell (e.g., an unmodified cell). In some cases, transcriptional modulation of the gene of interest includes increasing or enhancing expression. By increasing or enhancing expression is meant that the expression level of the gene of interest is increased by 2-fold or more, such as by 5-fold or more and sometimes by 25-, 50-, or 100-fold or more and in certain embodiments 300-fold or more or higlier, as compared to a control. i.e.. expression in the same cell not modified (e.g., by using any convenient gene expression assay). Alternatively, in cases where expression of the gene of interest in a cell is so low that it is undetectable, the expression level of the gene of interest is considered to be increased if expression is increased to a level that is easily detectable, In certain instances, transcriptional modulation of the gene of interest includes decreasing or repressing expression. By decreasing or repressing expression is meant that the expression level of the gene of interest is decreased by 2-fold or more, such as by 5-fold or more and sometimes by 25-. 50-, or 100-fold or more and in certain embodiments 300-fold or more or higher, as compared to a control. In some cases, expression is decreased to a level that is undetectable. Modifications of host cell processes of interest that may be adapted for use in the subject host cells are described in U.S. Publication No. 20140273109 (14/211,611) by Smolke et al., the disclosure of which is herein incorporated by reference in its entirety.

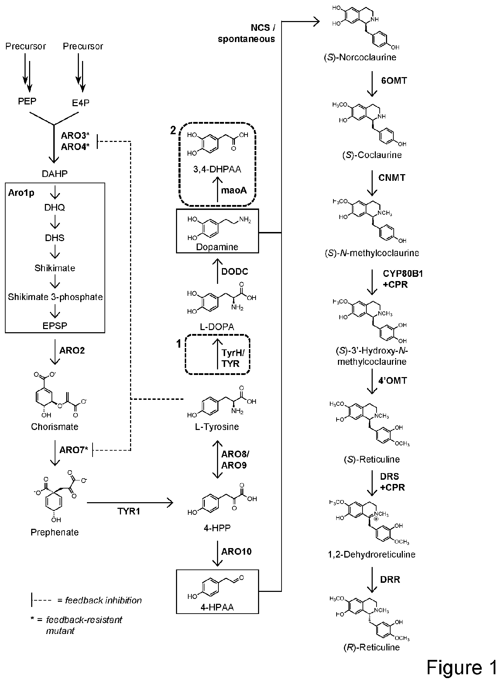

Any convenient biosynthetic enzyme genes may be tmnscriptionally modulated, and include but are not limited to, those biosynthetic enzymes described in FIG. 1, In particular. FIG. 1 illustrates a biosynthetic scheme for conversion of glucose to 4-HPAA, dopamine, and 3,4-DHPAA, in accordance with some embodiments of the invention. Examples of enzymes described in FIG. 1 include ARO3. ARO4, ARO1, ARO7, TYR1, TYR. TyrH, DODC, MAO, ARO10, ARO9, and ARO8. In some instances, the one or more biosynthetic enzyme genes may be selected from ARO10, ARO9. ARO8, and TYR1. In some cases, the one or more biosynthetic enzyme genes may be ARO10. In certain instances, the one or more biosynthetic enzyme genes may be ARO9. In some embodiments, the one or more biosynthetic enzyme genes may be TYR1. In some embodiments, the host cell includes one or more transcriptional modulation modifications to one or more genes such as one of those genes described in **Table 11.**

In some embodiments, the transcriptional modulation modification may include a substitution of a strong promoter for a native promoter of the one or more biosynthetic enzyme genes or the expression of an additional copy(ies) of the gene or genes under the control of a strong promoter. The promoters driving expression of the genes of interest may be constitutive promoters or inducible promoters, provided that the promoters may be active in the host cells. The genes of interest may be expressed from their native promoters. Additionally or alternatively, the genes of interest may be expressed from non-native promoters. Although not a requirement, such promoters may be medium to high strength in the host in which they are used. Promoters may be regulated or constitutive In some embodiments, promoters that are not glucose repressed, or repressed only mildly by the presence of glucose in the culture medium, may be used. There are numerous suitable promoters, examples of which include promoters of glycolytic genes such as the promoter of the *B. subtilis* tsr gene (encoding fructose biphosphate aldolase) or GAPDH promoter from yeast *S*. *cerevisine* (coding for glyceraldehyde-phosphate dehydrogenase) (Bitter G. A., Meth. Enzymol. 152:673 684 (1987)). Other strong promoters of interest include, but are not limited to, the ADH1 promoter of baker's yeast (Ruohonen L., et al, J. Biotechnol. 39:193 203 (1995)), the phosphate-starvation induced promoters such as the PHOS promoter of yeast (Hinnen, A., et al. in Yeast Genetic Engineering. Barr. P. J.. et al. eds. Butterworths (1989), the alkaline phosphatase promoter from *B. licheniformis* (Lee. J. W. K.. et al.. J. Gen. Microbiol. 137:1127 11.33 (1991)), GPD1, and TEF1. Yeast promoters of interest include, but are not limited to, inducible promoters such as Gal1-10, Gal1, Gall, GalS. repressible promoter Met25, tetO, and constitutive promoters such as glyceraldehyde 3-phosphate dehydrogenase promoter (GPD), alcohol dehydrogenase promoter (ADH), translation-elongation factor-1-alpha promoter (TEF), cytochrome c-oxidase promoter (CYC1), MRP7 promoter, etc. In some instances, the strong promoter is GPD1. In certain instances, the strong promoter is TEF1. Autonomously replicating yeast expression vectors containing promoters inducible by hormones such as glucocorticoids, steroids, and thyroid honnones are also known and include, but are not limited to, the glucorticoid responsive element (GRE) and thyroid hormone responsive element (TRE), see e.g., those promoters descnbed in U.S. Pat. No. 7,045,290. Vectors containing constitutive or inducible promoters such as alpha factor, alcohol oxidase, and PGH may be used. Additionally any convenient promoter/enhancer combination (as per the Eukaryotic Promoter Data Base EPDB) could also be used to drive expression of genes of interest. It is understood that any convenient promoters specific to the host cell may be selected. e.g., *E. coli.* In some cases, promoter selection may be used to optimize transcription, and hence, enzyme levels to maximize production while minimizing energy resources.

### Inactivating Mutations

The engineered host cells may include one or more inactivating mutations to an enzyme or protein of the cell (such as two or more, three or more, four or more, five or more, or even more). The inclusion of one or more inactivating mutations may modify the flux of a synthetic pathway of an engineered host cell to increase the levels of a BIA of interest or a desirable enzy me or precursor leading to the same. In some examples, the one or more inactivating mutations are to an enzy me native to the cell. Additionally or alternatively, the one or more inactivating mutations are to an enzyme non-native to the cell. As used herein, by "inactivating mutation" is meant one or more mutations to a gene or regulatory DNA sequence of the cell, where the mutation(s) inactivates a biological activity of the protein expressed by that gene of interest. In some cases, the gene is native to the cell. In some instances, the gene encodes an enzyme that is inactivated and is part of or connected to the synthetic pathway of a BIA of interest produced by the host cell. In some instances, an inactivating mutation is located in a regulatory DNA sequence that controls a gene of interest. In certain cases, the inactivating mutation is to a promoter of a gene. Any convenient mutations (e.g., as described herein) may be utilized to inactivate a gene or regulatory DNA sequence of interest. By "inactivated" or "inactivates" is meant that a biological activity of the protein expressed by the mutated gene is reduced by 10% or more, such as by 20% or more. 30% or more, 40% or more. 50% or more, 60% or more. 70% or more. 80% or more, 90% or more, 95% or more, 97% or more, or 99% or more, relative to a control protein expressed by a non-mutated control gene. In some cases, the protein is an enzyme and the inactivating mutation reduces the activity of the enzyme.

In some examples, the engineered host cell includes an inactivating mutation in an enzyme or protein native to the cell. Any convenient enzymes may be targeted for inactivation. Enzymes of interest may include, but are not limited to those enzymes, described in **Table 11** whose action in the synthetic pathway of the engineered host cell tends to reduce the levels of a BIA of interest. In some cases, the enzyme has glucose-6-phosphate dehydrogenase activity. In certain embodiments, the enzyme that includes an inactivating mutation is ZWF1. In some cases, the enzyme has alcohol dehydrogenase activity. In some embodiments, the enzyme that includes an inactivating mutation is selected from ADH2, ADH3, ADH4, ADH5, ADH6, ADH7. and SFA1. In certain embodiments, the enzyme that includes an inactivating mutation(s) is ADH2. In certain embodiments, the enzyme that includes an inactivating mutation(s) is ADH3. In certain embodiments, the enzyme that includes an inactivating mutation(s) is ADH4. In certain embodiments, the enzyme that includes an inactivating mutation(s) is ADH5. In certain embodiments, the enzyme that includes an inactivating mutation(s) is ADH6. In certain embodiments, the enzyme that includes an inactivating mutation(s) is ADH7. In some cases, the enzyme has aldehyde oxidoreductase activity. In certain embodiments, the enzyme that includes an inactivating mutation is selected from ALD2, ALD3, ALD4, ALD5, and ALD6, In certain embodiments, the enzyme that includes an inactivating mutation(s) is ALD2, In certain embodiments. the enzyme that includes an inactivating mutation(s) is ALD3. In certain embodiments, the enzyme that includes an inactivating mutation(s) is ALD4. In certain embodiments, the enzyme that includes an inactivating mutation(s) is ALD5. In certain embodiments, the enzyme that includes an inactivating mutation(s) is ALD6. In some cases, the enzyme has aldehyde reductase activity. In some embodiments, the enzyme that includes an inactivating mutation is ARI1. In some cases, the enzyme has aryl-alcohol dehy drogenasc activity. In some embodiments, the enzyme that includes an inactivating mutation is selected from AAD4, AAD6. AAD10, AAD14, AAD15, AAD16. In certain embodiments, the enzyme that includes an inactivating mutation(s) is AAD4. In certain embodiments, the enzyme that includes an inactivating mutation(s) is AAD6. In certain embodiments, the enzyme that includes an inactivating mutation(s) is AAD10. In certain embodiments, the enzyme that includes an inactivating mutation(s) is AAD14. In certain embodiments, the enzyme that includes an inactivating mutation(s) is AAD15. In certain embodiments, the enzyme that includes an inactivating mutation(s) is AAD16. In some examples, the engineered host cell includes an inactivating mutation in a transcription regulator native to the cell. Transcriptional regulators of interest may include, but are not limited to those proteins, described in **Table 11.** In some cases, the protein has activity as a transcriptional regulator of phospholipid biosynthetic genes. In some embodiments, the transcriptional regulator that includes an inactivating mutation is OPII. In some embodiments, the host cell includes one or more inactivating mutations to one or more genes described in **Table 11.**

In some examples, the engineered host cell includes an inactivating mutation in an enzyme or protein native to the cell. Enzymes of interest may include, but are not limited to those enzymes, described in **Table 11** whose action in the synthetic pathway of the engineered host cell is part of the Erlich pathway to produce fusel alcohols. In some cases, the enzyme has phenylpyruvate decarboxylase activity. In certain embodiments, the enzyme that includes an inactivating mutation is ARO10. In some cases, the enzyme has pyruvate decarboxylase activity. In some embodiments, the enzyme that includes an inactivating mutation is selected from PDC1. PDC5. and PDC6. In certain embodiments, the enzyme that includes an inactivating mutation(s) is PDC1. In certain embodiments, the enzyme that includes an inactivating mutation(s) is PDC5. In certain embodiments, the enzyme that includes an inactivating mutation(s) is PDC6. In some cases, the enzyme has aromatic aminotransferase activity. In some embodiments, the enzyme that includes an inactivating mutation is selected from ARO8 and ARO9. In certain embodiments, the enzyme that includes an inactivating mutation(s) is ARO8. In certain embodiments, the enzyme that includes an inactivating mutation(s) is ARO9. In some cases, the enzyme has prephenate dehydrogenase activity. In certain embodiments, the enzy me that includes an inactivating mutation(s) is TYR1. In some cases, the enzyme has prephenate dehydratase activity. In certain embodiments, the enzyme that includes an inactivating mutation(s) is PHA2. In some embodiments, the host cell includes one or more inactivating mutations to one or more genes described in **Table 11.**

### Epimerization Modifications

Some methods, processes, and systems provided herein describe the conversion of *(S)*-1-benzylisoquinoline alkaloids to (*R*)-1-benzylisoquinoline alkaloids. Some of these methods, processes, and systems may comprise an engineered host cell. In some examples. the conversion of *(S)*-1-benzylisoquinoline alkaloids to (*R*)-1-benzylisoquinoline alkaloids is a key step in the conversion of a substrate to a diverse range of alkaloids. In some examples, the conversion of (*S*)-1-benzylisoquinoline alkaloids to (*R*)-1-benzylisoquinoline alkaloids comprises an epimerization reaction via an engineered epimerase. In some cases, epimerization of a substrate alkaloid may be performed by oxidizing an (*S*)-substrate to the corresponding Schiff base or imine intermediate, then stereospecifically reducing this intermediate to an (*R*)-product as provided in **FIG. 1** and as represented generally in **Scheme 1.** As provided in **Scheme 1,** R₁, R₂. R₃, and R₄ may be H or CH₃. R₃ may be H. OH, or OCH₃.

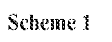

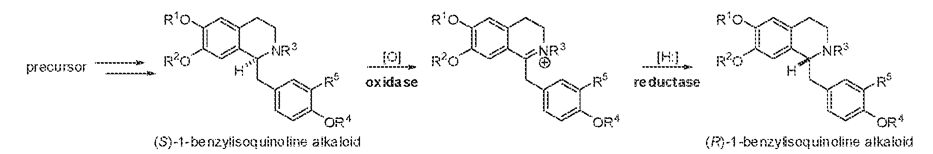

In some examples, the conversion of the *(S)*-substrate to the *(R)*-product may involve at least one oxidation reaction and at least one reduction reaction. In some cases, an oxidation reaction is optionally followed by a reduction reaction. In some cases, at least one of the oxidation and reduction reactions is carried out in the presence of an enzyme. In some cases, at least one of the oxidation and reduction reactions is catalyzed by an engineered epimerase. In some cases, the oxidation and reduction reactions are both carried out in the presence of an engineered fused epimerase. In some cases, the oxidation and reduction reactions are both carried out in the presence of an engineered split epimerase having a separately expressed oxidase component and reductase component, respectively. In some cases, an engineered epimerase is useful to catalyze the oxidation and reduction reactions The oxidation and reduction reactions may be catalyzed by the same engineered epimerase.

In some methods, processes and systems described herein, an oxidation reaction may be performed in the presence of an enzyme that is part of an engineered epimerase. In some examples, the engineered epimerase may has e an oxidase component. In some cases, the oxidase component may be a component of an engineered fused epimerase. In some case, the oxidase component may be independently expressed as part of an engineered split epimerase. The oxidase may use a *(S)*-1-benzylisoquinoline as a substrate. The oxidase may convert the *(S)*-substrate to a corresponding imine or Schiff base derivative. The oxidase may be referred to as 1,2-dehydroreticuline synthase (DRS). Non-limiting examples of enzymes suitable for oxidation of (*S*)-1-benzylisoquinoline alkaloids in this disclosure include a cytochrome P450 oxidase, a 2-oxoglutarate-dependent oxidase, and a flavoprotein oxidase. For example. *(S)*-tetrahydroprotoberberine oxidase (STOX. E.C 1.3.3.8) may oxidize *(S)-*norreticuline and other *(S)*-1-benzylisoquinoline alkaloids to 1,2-dchydronorreticuline and other corresponding 1,2-dehydro products. In some examples, a protein that comprises an oxidase domain of any one of the preceding examples may perform the oxidation. In some examples, the oxidase may catalyze the oxidation reaction within a host cell, such as an engineered host cell, as described herein. In some cases, the oxidase may have one or more activity-increasing components.

In some examples, a reduction reaction may follow the oxidation reaction. The reduction reaction may be performed by an enzyme that is part of an engineered epimerase. In some examples, the reductase may use an imine or Schiff base derived from a 1-benzylisoquinoline as a substrate. The reductase may convert the imine or Scluff base derivative to a (*R*)-1-benzylisoquinoline. The reductase may be referred to as 1,2-dehydroreticuline reductase (DRR). Non-limiting examples of enzymes suitable for reduction of an imine or Schiff base derived from an *(S)*-1-benzylisoquinoline alkaloid include an aldo-keto reductase (e.g., a codeinone reductase-like enzyme (EC 1.1.1.247)) and a short chain dehydrogenase (e.g.. a salutaridine reductase-like enzyme (EC 1.1.1.248)). In some examples, a protein that comprises a reductase domain of any one of the preceding examples may perform the reduction. In a further embodiment, the reduction is stereospecific. In some examples, the reductase may catalyze the reduction reaction within a host cell, such as an engineered host cell, as described herein.

An example of an enzyme that can perform an epimerization reaction that converts *(S)*-1-benzylisoquinoline alkaloids to *(R)*-1-benzylisoquinoline alkaloids includes an epimerase having an oxidase domain and a reductase domain. In particular, the epimerase may have a cytochrome P450 oxidase 82Y2-like domain. Additionally, the epimerase may have a codeinone reductase-like domain. An epimerase having a cytochrome P450 oxidase 82Y2-like domain and also having a codeinone reductase-like domain may be referred to as a DRS-DRR enzyme. In particular, a DRS-DRR enzyme may be a fusion enzyme that is a fusion epimerase. Further, when a DRS-DRR enzyme is modified by at least one activity-increasing modification, the fusion enzyme may be an engineered fusion epimerase.

Examples of amino acid sequences of a DRS-DRR enzyme that may be used to perform the conversion of *(S)*-1-benzylisoquinoline alkaloids to *(R)-*1-benzylisoquinoline alkaloids are set forth in **Table 1.** An amino acid sequence for an epimerase that is utilized in converting an *(S)*-1-benzylisoquinoline alkaloid to an *(R)*-1-benzylisoquinoline alkaloid may be 50% or more identical to a given amino acid sequence as listed in **Table 1.** For example, an amino acid sequence for such an epimerase may comprise an amino acid sequence that is at least 50% or more, 55% or more, 60% or more, 65% or more. 70% or more. 75% or more. 80% or more, 81% or more, 82% or more, 83% or more, 84% or more, 85% or more, 86% or more. 87% or more. 88% or more. 89% or more. 90% or more. 91% or more, 92% or more, 93% or more, 94% or more, 95% or more, 96% or more. 97% or more. 98% or more, or 99% or more identical to an amino acid sequence as provided herein. Additionally, in certain embodiments, an "identical" amino acid sequence contains at least 80%-99% identity at the amino acid level to the specific amino acid sequence. In some cases an "identical" amino acid sequence contains at least about 85%, 86%, 87%, 88%, 89%, 90%, 91%, 92%, 93%. 94% and more in certain cases, at least 95%, 96%, 97%. 98% and 99% identity, at the amino acid level. In some cases, the amino acid sequence may be identical but the DNA sequence is altered such as to optimize codon usage for the host organism, for example.

Amino acid residues of homologous epimerases may be referenced according to the numbering scheme of SEQ ID NO. 16, and this numbering system is used throughout the disclosure to refer to specific amino acid residues of epimerases which are homologous to SEQ ID NO. 16. Epimcrases homologous to SEQ ID NO. 16 may have at least about 50%, 55%, 60%, 65%, 70%, 75%, 80%, 85%. 90%, 95%, 97%, 98%, or 99% sequence identity to SEQ ID NO. 16. In some cases, an amino acid referred to as position 50 in a homologous epimerase may not be the 50^{th} amino acid in the homologous epimerase, but would be the amino acid which corresponds to the amino acid at position 50 in SEQ ID NO. 16 in a protein alignment of the homologous epimerase with SEQ ID NO. 16. In some cases, homologous enzymes may be aligned with SEQ ID NO. 16 either according to primary sequence, secondary structure, or tertiary structure.

An engineered host cell may be provided that produces an engineered epimerase that converts (*S*)-1-benzylisoquinoline alkaloid to (*R*)-1-benzylisoquinoline alkaloid, wherein the epimerase comprises an amino acid sequence selected from the group consisting of: SEQ ID NOs: 1, 2. 3, 4, 5. 6. 7, 8, 9. 10, 11, 12, 13, 14, 15, 16, 17, and 18, and having one or more activity-enhancing modifications. The epimerase that is produced within the engineered host cell may be recovered and purified so as to form a biocatalyst. In some cases, the epimerase may be split into one or more enzymes. Additionally, one or more enzymes that are produced by splitting the epimerase may be recovered from the engineered host cell. These one or more enzymes that result from splitting the epimerase may also be used to catalyze the conversion of (*S*)-1-benzylisoquinoline alkaloids to (*R*)-1-benzylisoquinoline alkaloids. Additionally, the use of an engineered split epimerase may be used to increase the production of benzy lisoquinoline alkaloid products within a cell when compared to the production of benzylisoquinoline alkaloid products within a cell utilizing a fused epimerase.

In additional cases, the one or more enzymes that are recovered from the engineered host cell that produces the epimerase may be used in a process for converting a (*S*)-1-benzylisoquinoline alkaloid to a (*R*)-1-benzylisoquinoline alkaloid. The process may include contacting the (*S*)-1-benzylisoquinoline alkaloid with an epimerase in an amount sufficient to convert said (*S*)-1-benzylisoquinoline alkaloid to (*R*)-1-benzylisoquinoline alkaloid. In some examples. the (*S*)-1-benzylisoqiuinoline alkaloid may be contacted with a sufficient amount of the one or more enzymes such that at least 5% of said (*S*)-1-benzylisoquinoline alkaloid is com erted to (*R*)-1-benzylisoquinoline alkaloid. In further examples, the *(S)*-l-benzvlisoquinoline alkaloid may be contacted with a sufficient amount of the one or more enzymes such that at least 10% at least 15%, at least 20% at least 25%, at least 30% at least 35%, at least 40%, at least 45%. at least 50%, at least 55%, at least 60%, at least 65%, at least 70%, at least 75%, at least 80%, at least 82%. at least 84%, at least 86%, at least 88%, at least 90% at least 91%, at least 92% at least 93% at least 94%, at least 95%, at least 96% at least 97%, at least 98%, at least 99%, at least 99.5%. at least 99.7%. or 100% of said *(S)*-1-benzylisoquinoline alkaloid is converted to (*R*)-1-benzylisoquinoline alkaloid.

The one or more enzymes that may be used to convert a (*S*)-1-benzylisoquinoline alkaloid to a (*R*)-1-benzylisoquinoline alkaloid may contact the (*S*)-1-benzylisoquinoline alkaloid in vitro. Additionally, or alternatively, the one or more enzymes that may be used to convert a *(S)*-1-benzylisoquinoline alkaloid to a (*R*)-1-benzylisoquinoline alkaloid may contact the (*S*)-1-benzylisoquinoline alkaloid in vivo. Additionally, the one or more enzymes that may be used to convert a (*S*)-1-benzylisoquinoline alkaloid to a (*R)-*1-benzylisoquinoline alkaloid may be provided to a cell having the (*S*)-1-benzylisoquinoline alkaloid within, or may be produced within an engineered host cell.

In some examples, the methods provide for engineered host cells that produce an alkaloid product, wherein the epimerization of a *(S)*-substrate to a *(R)*-product may comprise a key step in the production of an alkaloid product. In some examples, the alkaloid produced is a *(R)*-1-benzylisoquinoline alkaloid. In still other embodiments, the alkaloid produced is derived from a *(R)*-1-benz^{y}lisoquinoline alkaloid, including, for example, 4-ring promorphinan and S-ring morphinan alkaloids. In another embodiment, a (*S*)-1-benzylisoquinoline alkaloid is an intermediate toward the product of the engineered host cell. In still other embodiments, the alkaloid product is selected from the group consisting of 1-benzylisoquinoline, morphinan, promorphinan, nor-opioid, nal-opioid, or bisbenzylisoquinoline alkaloids.

In some examples, the *(S)*-substrate is a *(S)*-1-benzylisoquinoline alkaloid selected from the group consisting of *(S)*-norreticuline. *(S)*-reticuline, *(S)*-tetrahydropapaverine, *(S)*-norcoclaurine, *(S)-*coclaurine, (*S*)-*N*-methylcoclaurine, *(S)*-3⁻-hydroy-*N*-methylcoclaurine, *(S)*-norisoorientaline, *(S)-*orientaline, *(S)*-isoorientaline. *(S)*-norprotosinomenine. (*S*)-protosinomenine, *(S)*-norlaudanosoline, *(S)-*laudanosoline, (*S*)-4'-O-methyllaudanosoline, *(S)*-6-O-methylnorlaudanosoline, *(S)*-4'-*O-*methylnorlaudanosoline.

In some examples. the *(S)*-substrate is a compound of Formula 1: 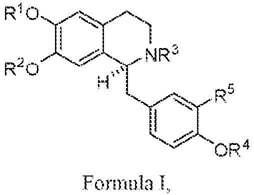 or a salt thereof, wherein:
R¹, R², R³, and R⁴ are independently selected from hydrogen and methyl; and
R⁵ is selected from hydrogen, hydroxy, and methoxy.

In some other examples, at least one of R¹, R², R³, R⁴, and R⁵ is hydrogen.

In still other examples. the *(S)*-substrate is a compound of Formula II: 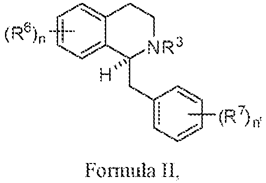 or a salt thereof, wherein:
R³ is selected from hydrogen and C₁-C₄ alkyl:
R⁶ and R⁷ are independently selected at each occurrence from hydroxy, fluoro, chloro, bromo, carboxaldehyde. C₁-C₂ acyl, C₁-C₄ alkyl. and C₁-C₄ alkoxy:
n is 0, 1, 2, 3. or 4 ; and
n' is 0, 1, 2, 3, 4 or 5.

When a bond is drawn across a ring, it means substitution may occur at a non-specific ring atom or position. For example, in Formula II shown above, the hydrogen of any -CH- in the 6-membered ring may be replaced with R⁷ to form -CR⁷-.

In some examples. R⁶ and R⁷ are independently methyl or methoxy. In some other examples, n and n' are independently 1 or 2. In still other embodiments, R³ is hydrogen or methyl.

In some examples, the methods provide for engineered host cells that produce alkaloid products from *(S)*-reticuline. The epimerization of *(S)*-reticuline to *(R)*-reticuline may comprise a key step in the production of diverse alkaloid products from a precursor In some examples, the precursor is L-tyrosine or a sugar (e.g., glucose). The diverse alkaloid products can include, without limitation, 1-benzylisoquinoline, morphinan, promorphinan, nor-opioid, or nal-opioid alkaloids.

Any suitable carbon source may be used as a precursor toward an epimerized 1-benzylisoquinoline alkaloid. Suitable precursors can include, without limitation, monosaccharides (e.g., glucose, fructose, galactose, xylose), oligosaccharides (e.g., lactose, sucrose, raffinose), polysaccharides (e.g., starch, cellulose), or a combination thereof. In some examples, unpurified mixtures from renewable feedstocks can be used (e.g.. consteep liquor, sugar beet molasses, barley malt, biomass hydrolysate). In still other embodiments, the carbon precursor can be a one-carbon compound (e.g., methanol, carbon dioxide) or a two-carbon compound (e.g.. ethanol). In yet other embodiments, other carbon-containing compounds can be utilized, for example, methylamine, glucosamine, and amino acids (e.g., L-tyrosine). In some examples, a 1-benzylisoquinoline alkaloid may be added directly to an engineered host cell of the invention, including, for example, norlaudanosoline, laudanosoline, norreticuline, and reticuline. In still further embodiments, a 1-benzylisoquinoline alkaloid may be added to the engineered host cell as a single enantiomer (e.g.. a *(S)-*1-benzylisoquinoline alkaloid). or a mixture of enantiomers, including, for example, a racemic mixture.

In some examples, the methods provide for the epimerization of a stereocenter of a 1-benzylisoquinoline alkaloid, or a derivative thereof, using an engineered epimerase. In a further embodiment, the method comprises contacting the 1-benzylisoquinoline alkaloid with an engineered epimerase. The engineered epimerase may invert the stereochemistry of a stereocenter of a 1-benzylisoquinoline alkaloid, or derivative thereof, to the opposite stereochemistry. In some examples, the engineered epimerase converts a (*S)*-1-benzylisoquinoline alkaloid to a *(R)*-1-benzylisoquinoline alkaloid. In some examples of this conversion of a *(S)*-1-benzylisoquinoline alkaloid to a *(R)*-1-benzylisoquinoline alkaloid utilizing the engineered epimerase. the *(S)*-1-benzylisoquinoline alkaloid is selected from the group consisting of *(S)*-norreticuline. *(S)-*(reticuline) *(S)*-tetrahydropapaverine, *(S)*-norcoclaurine, *(S)-*coclaurine, (*S*)-*N*-methylcoclaurine, *(S)*-3-hydroxy-*N*-methylcoclaurine, *(S)*-norisorientaline, *(S)-*orientaline, *(S)*-isoorientaline, *(S)*-norprotosinomenine, *(S)*-protosinomenine, *(S)*-norlaudanosoline, *(S)-*laudanosoline. (*S*)-4'-O-methyllaudanosoline, *(S)*-6-*O*-methylnorlaudanosoline, and *(S)*-4'*-O-*methylnorlaudanosoline.

In still other embodiments, the 1-benzylisoquinoline alkaloid that is epimerized using an engineered epimerase may comprise two or more stereocenters, wherein only one of the two or more stereocenters is inverted to produce a diastereomer of the substrate (e.g., (*S*,*R*)-1-benzylisoquinoline alkaloid converted to (*R*,*R*)-1-benzylisoquinoline alkaloid). In some examples where only one stereocenter of a 1-benzylisoquinoline alkaloid is inverted when contacted with the at least one enzyme, the product is referred to as an epimer of the 1-benzylisoquinoline alkaloid.

In some examples, the 1-benzylisoquinoline alkaloid is presented to the enzyme as a single stereoisomer. In some other examples, the 1-benzylisoquinoline alkaloid is presented to the enzyme as a mixture of stereoisomers. In still further embodiments, the mixture of stereoisomers may be a racemic mixture. In some other examples, the mixture of stereoisomers may be enriched in one stereoisomer as compared to another stereoisomer.

In some examples, a 1-benzylisoquinoline alkaloid, or a derivative thereof, is recovered. In some examples, the 1-benzylisoquinoline alkaloid is recovered from a cell culture. In still further embodiments, the recovered 1-benzylisoquinoline alkaloid is enantiomerically enriched in one stereoisomer as compared to the original mixture of 1-benzylisoquinoline alkaloids presented to the enzyme. In still further embodiments, the recovered 1-benzylisoquinoline alkaloid has an enantiomeric excess of at least 50%, at least 55%, at least 60%, at least 65%, at least 70%, at least 75%, at least 80%, at least 82%, at least 84%. at least 86%. at least 88%, at least 90% at least 91%, at least 92%, at least 93%, at least 94%. at least 95% at least 96%, at least 97%, at least 98%, at least 99%, at least 99.5%, at least 99.7%. or 100%.

In some examples, a promorphinan, or a derivative thereof, is recovered. In some examples, the promorphinan is recovered from a cell culture. In still further embodiments, the recovered promorphinan has an enantiomeric excess of at least 50%, at least 55% at least 60%, at least 65%, at least 70%, at least 75%, at least 80%, at least 82%, at least 84%, at least 86%, at least 88%, at least 20%, at least 91%, at least 92% at least 93%, at least 94%, at least 95%, at least 96%, at least 97%, at least 98%, at least 99% at least 99.5% at least 99.7%, or 100%.

In some examples, a morphinan, or a derivative thereof, is recovered. In some examples, the morphinan is recovered from a cell culture. In still further embodiments, the recovered morphinan has an enantiomeric excess of at least 50%, at least 55%, at least 60%, at least 65%, at least 70% at least 75%, at least 80%, at least 82%, at least 84%, at least 86%, at least 88%, at least 90%, at least 91%, at least 92%, at least 93%, at least 94%, at least 95%, at least 96%, at least 97% at least 98%, at least 99%, at least 99.5%, at least 99.7%, or 100%.

In some examples, a bisbenzylisoquinoline, or a derivative thereof, is recovered. In some examples, the bisbenzylisoquinoline is recovered from a cell culture. In still further embodiments, the recovered bisbenzylisoquinoline is enantiomerically enriched in one stereoisomer as compared to the original mixture of bisbenzylisoquinoline presented to the enzyme. In still further embodiments, the recovered bisbenzylisoquinoline has an enantiomeric excess of at least 50%, at least 55%, at least 60%, at least 65%, at least 70%, at least 75%, at least 80%, at least 82%, at least 84%, at least 86%, at least 88%, at least 90% at least 91%, at least 92%, at least 93%, at least 94%, at least 95% at least 96%, at least 97%, at least 98%, at least 99%, at least 99.5%, at least 99.7%, or 100%.

In some examples, a nal-opioid, or a derivative thereof, is recovered. In some examples, the nal-opioid is recovered from a cell culture. In still further embodiments, the recovered nal-opioid has an enantiomeric excess of at least 50%, at least 55%, at least 60%, at least 65%, at least 70%, at least 75%, at least 80%, at least 82%, at least 84%, at least 86%, at least 88%, at least 90%, at least 91%, at least 92%, at least 93%, at least 94%, at least 95%, at least 96%, at least 97%, at least 98%, at least 99%, at least 99.5%, at least 99.7%, or 100%.

In some examples, a nor-opioid, or a derivative thereof, is recovered. In some examples, the nor-opioid is recovered from a cell culture. In still further embodiments, the recovered nor-opioid has an enantiomeric excess of at least 50%, at least 55%, at least 60%, at least 65%, at least 70%, at least 75%, at least 80%, at least 82%, at least 84%, at least 86%, at least 88%, at least 90%, at least 91%, at least 92%, at least 93%, at least 94%, at least 95%, at least 96%, at least 97%, at least 98%, at least 99%, at least 99.5%, at least 99.7%, or 100%.

"Isomers" are different compounds that have the same molecular formula. "Stereoisomers" are isomers that differ only in the way the atoms are arranged in space. "Enantiomers" are a pair of stereoisomers that are non superimposable mirror images of each other. A 1:1 mixture of a pair of enantiomers is a "racemic" mixture. "Diastereoisomers" or "diastereomers" are stereoisomers that have at least two asymmetric atoms but are not mirror images of each other. The term "epimer" as used herein refers to a compound having the identical chemical formula but a different optical configuration at a particular position. For example, the (*R,S*) and (*S*,*S*) stereoisomers of a compound are epimers of one another. In some examples, a 1-benzylisoquinoline alkaloid is converted to its epimer (e.g.. epi-1-benzylisoquinoline alkaloid). The absolute stereochemistry is specified according to the Cahn-Ingold-Prelog R-S system. When a compound is a pure enantiomer, the stereochemistry at each chiral carbon can be specified by either R or S. Resolved compounds whose absolute configuration is unk now n can be designated (+) or (-) depending on the direction (dextro- or levorotatory) in which they rotate plane polarized light at the wavelength of the sodium D line. Certain compounds described herein contain one or more asymmetric centers and can thus give rise to enantiomers, diastereomers, and other stereoisomeric forms that can be defined, in terms of absolute stereochemistry, as *(R)-* or *(S)*-.

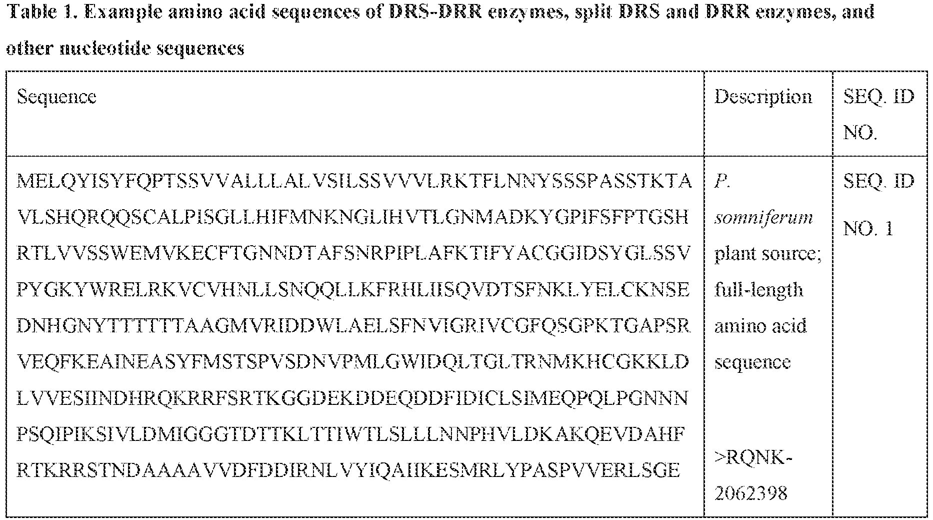

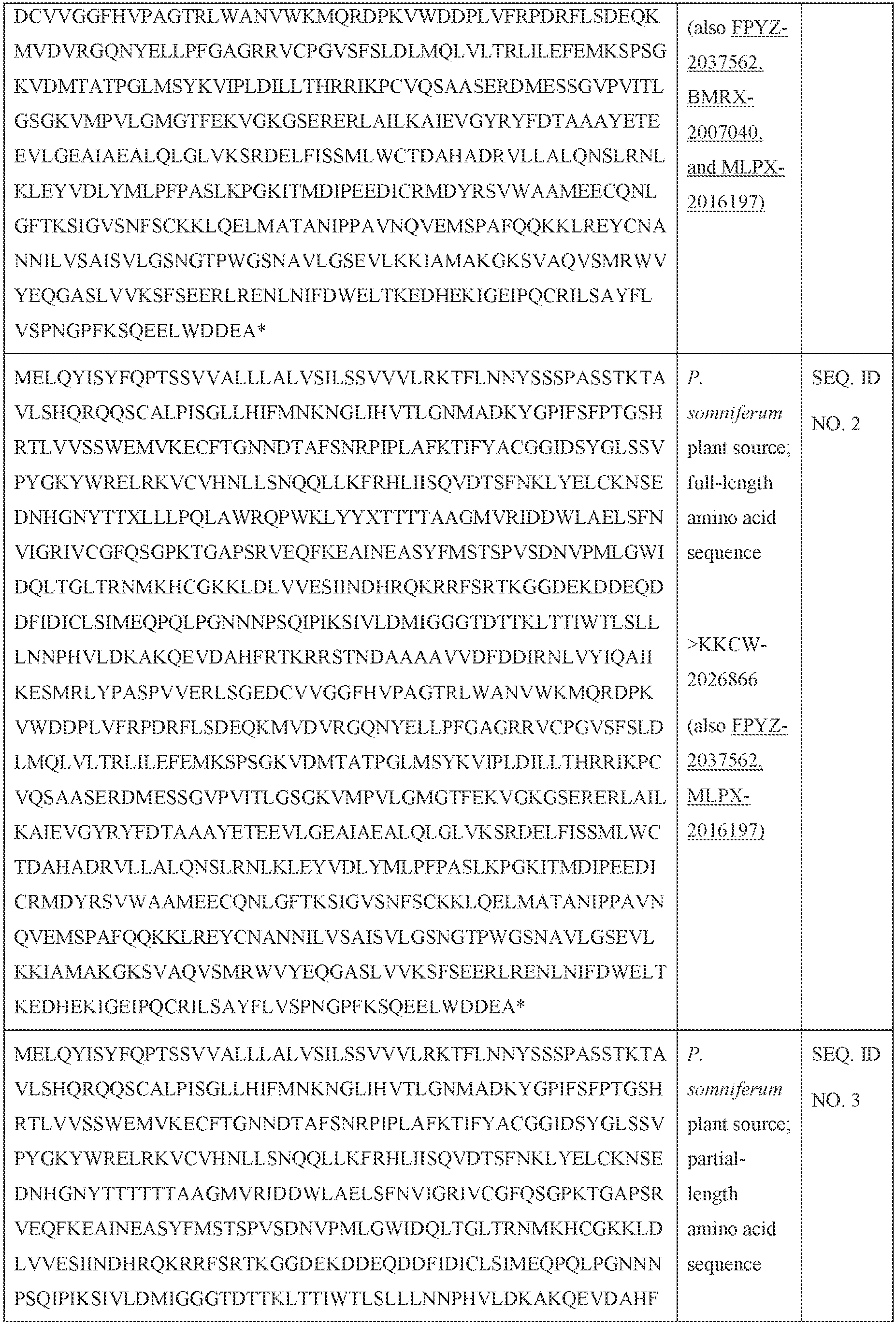

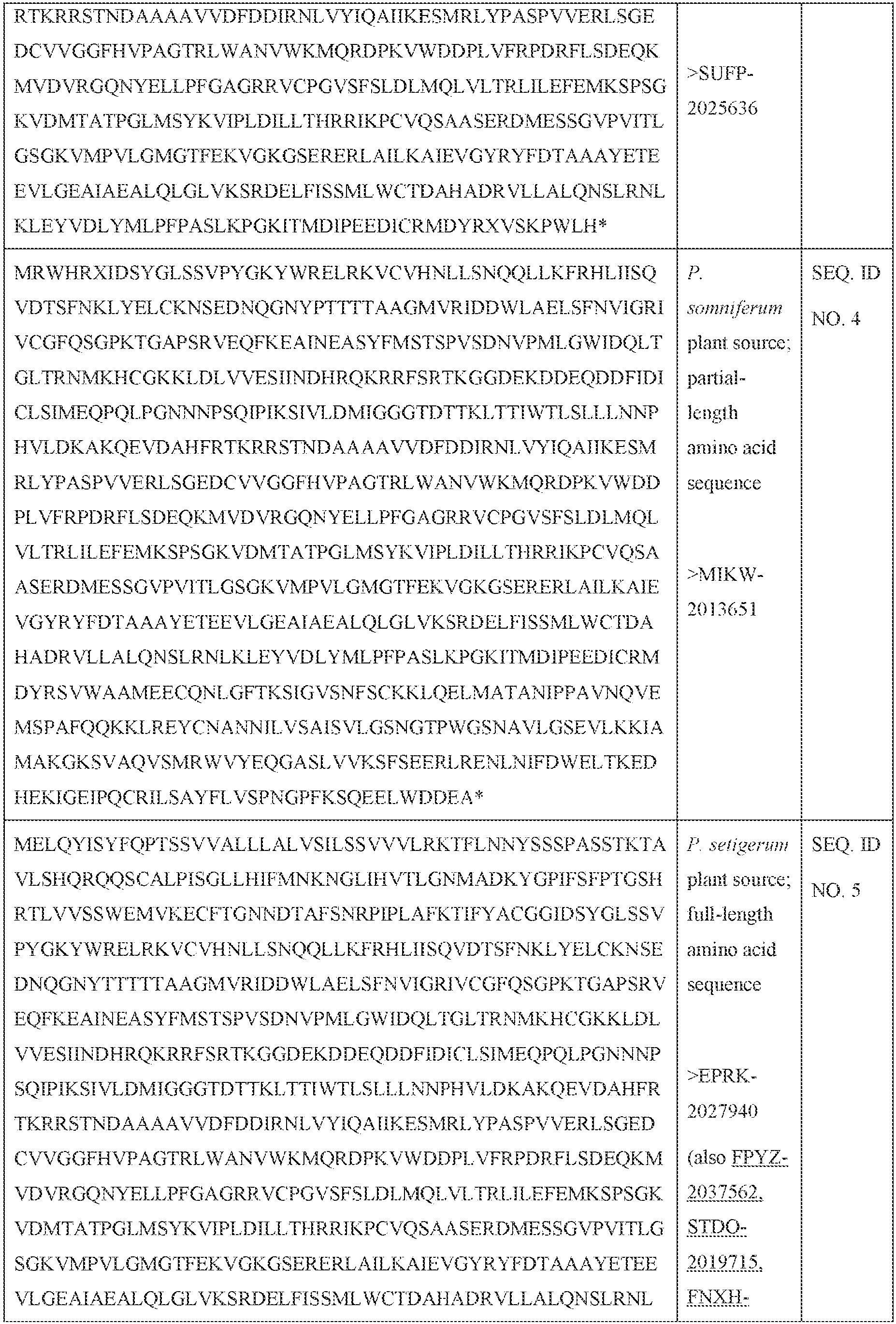

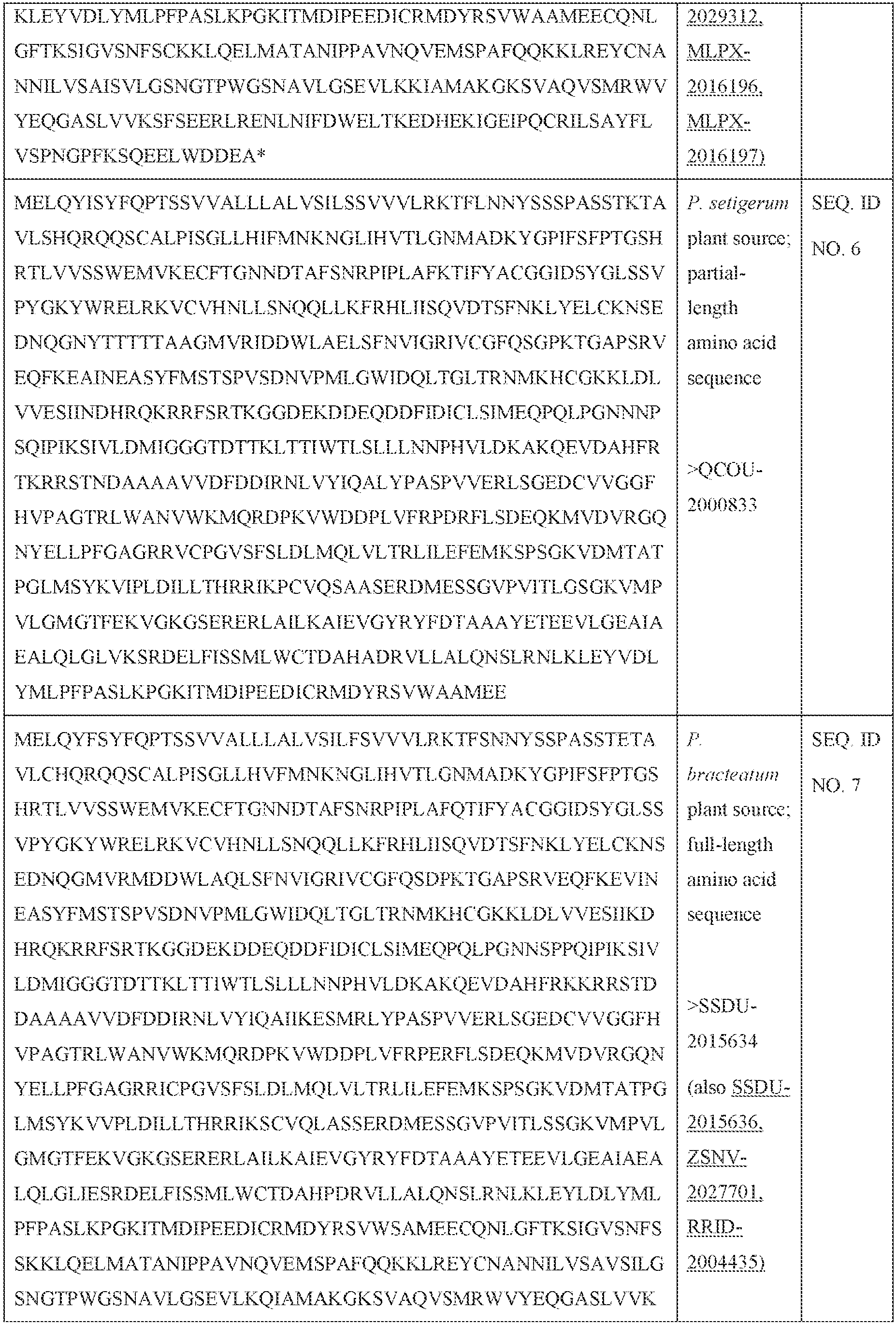

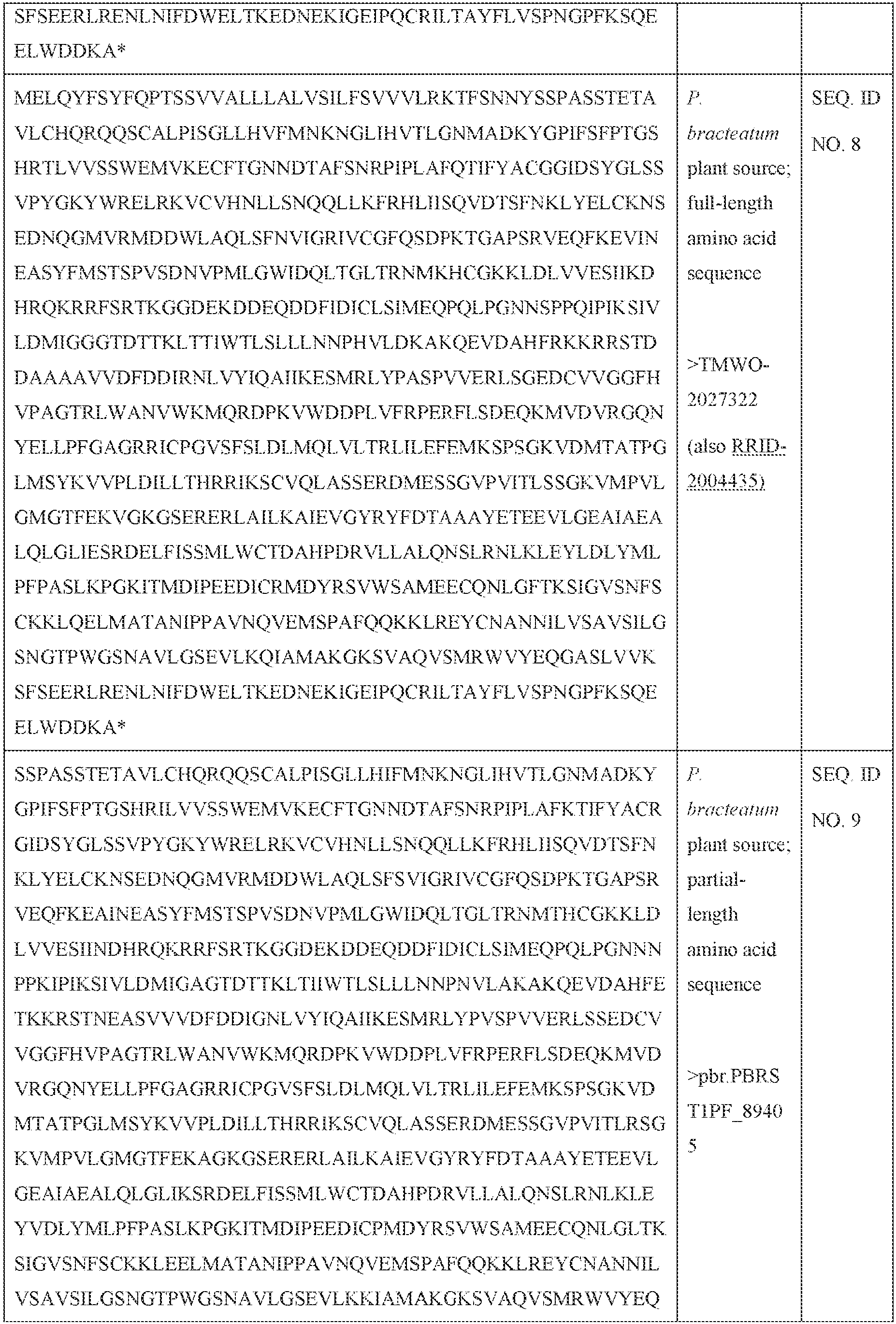

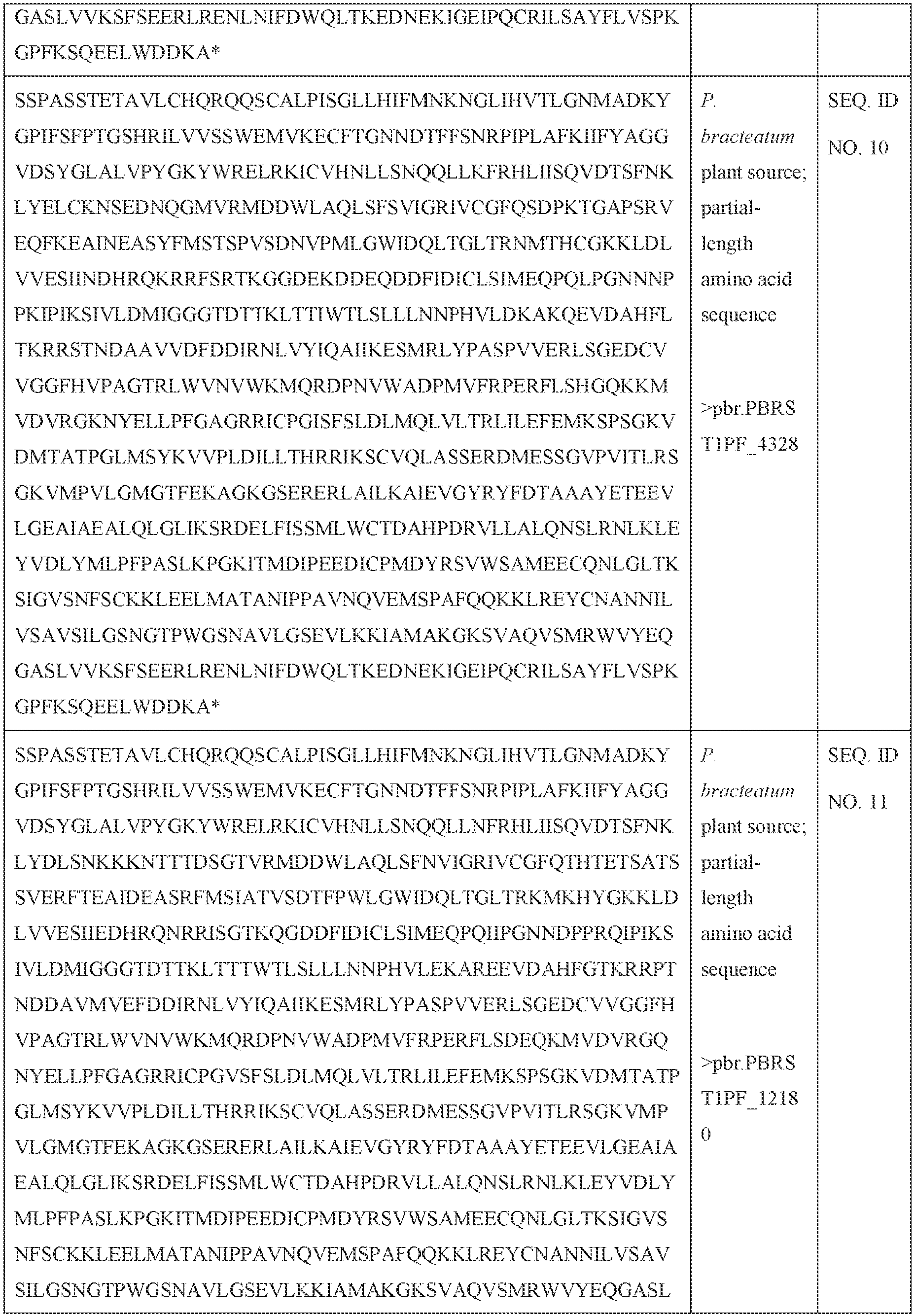

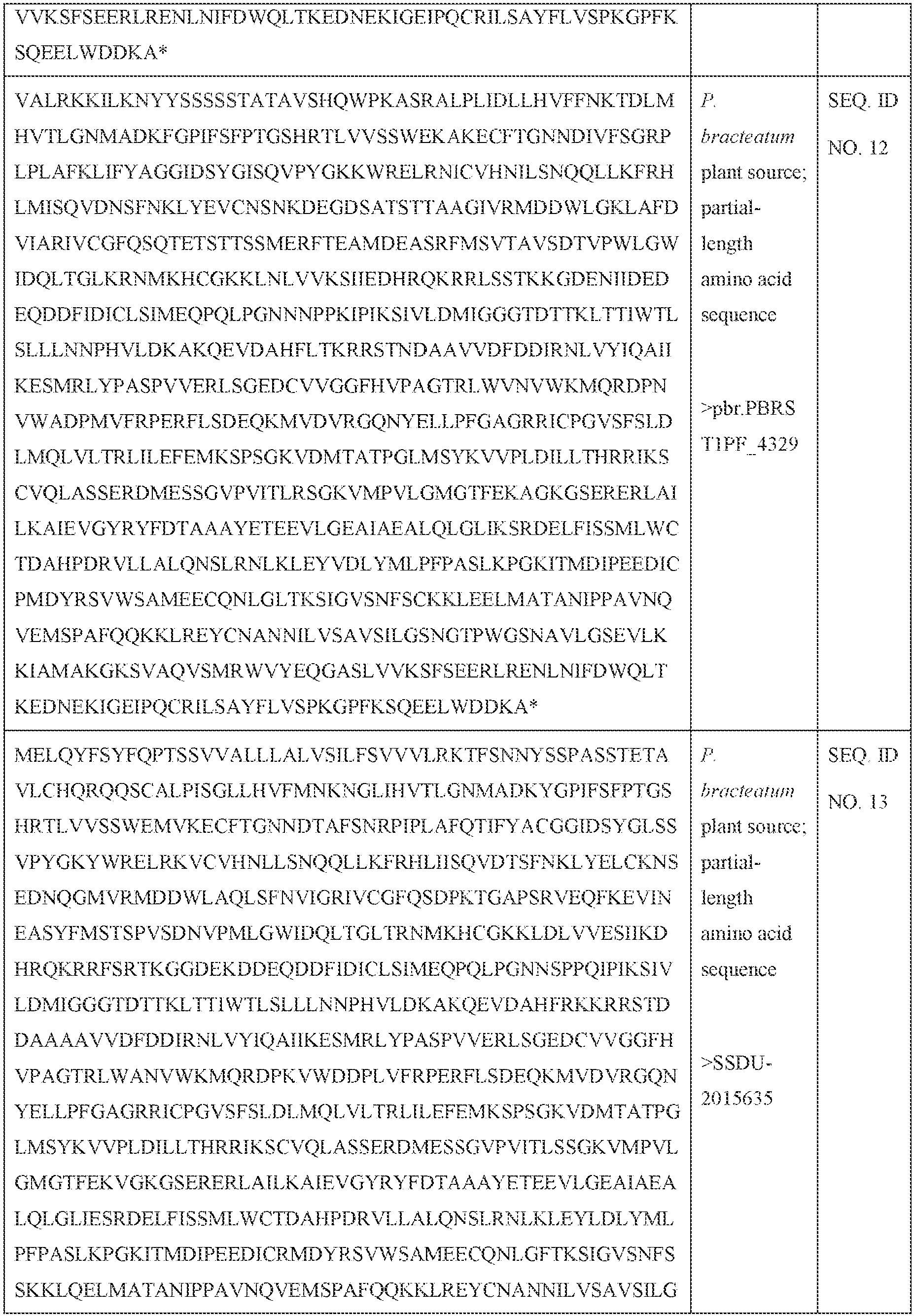

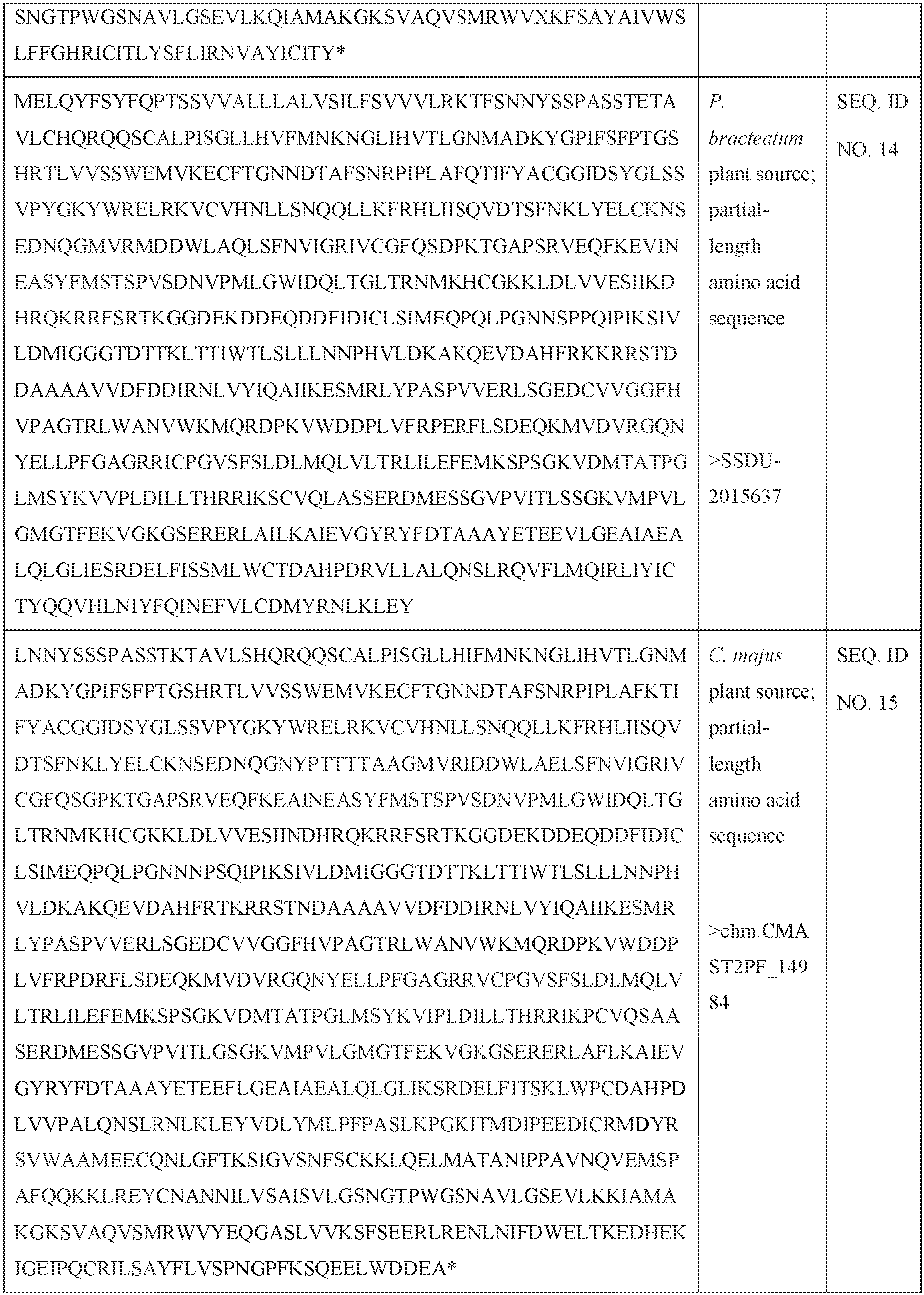

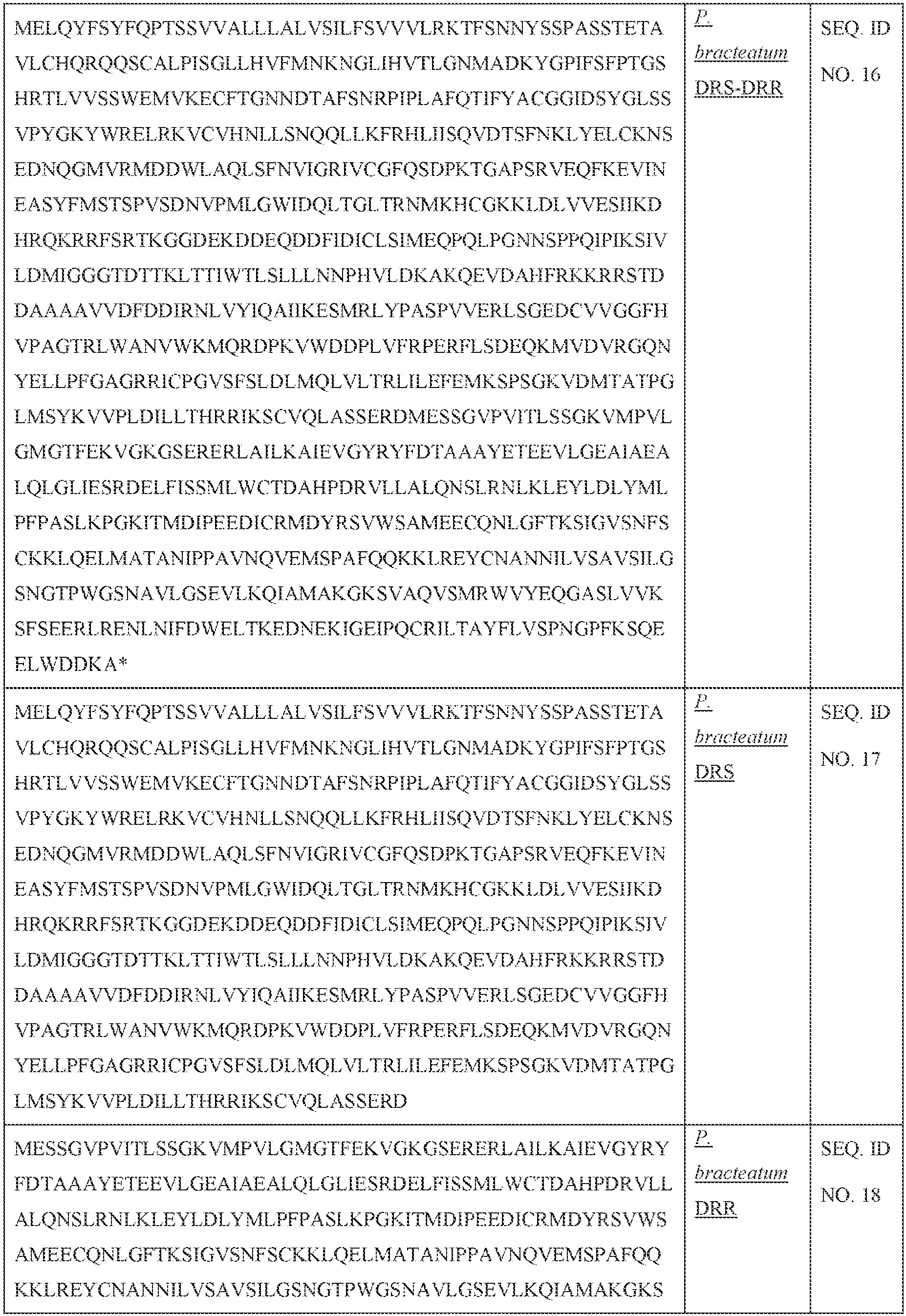

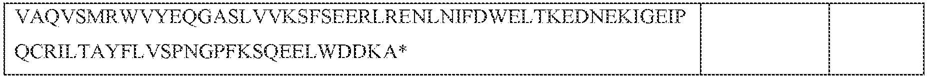

### Morphinan Alkaloid Generating Modifications

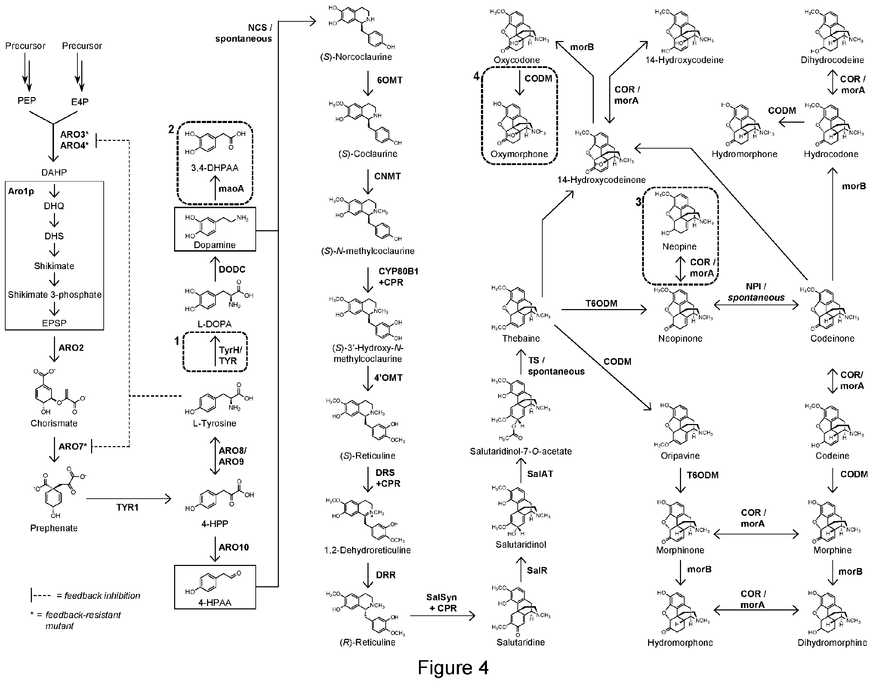

Some methods, processes, and systems provided herein describe the conversion of promorphinan alkaloids to morphinan alkaloids. Some of the methods, processes, and systems describe the conversion of a tetracyclic scaffold to a pentacyclic scaffold **(****FIG. 4****).** Some of the methods, processes, and systems may comprise an engineered host cell. In some examples, the production of pentacyclic thebaine, or a morphinan alkaloid, from a tetracyclic precursor, or a promorphinan alkaloid is described. In some examples, the conversion of promorphinan alkaloids to thebaine are key steps in the conversion of a substrate to a diverse range of benzylisoquinoline alkaloids.

In some examples, the tetracyclic precursor may be salutaridine, salutaridinol, or salutaridinol-7-O-acetate. The tetracyclic precursor may be converted to pentacyclic thebaine by closure of an oxide bridge between C-4 and C-5. In some examples, the tetracyclic precursor salutaridine may be prepared for ring closure by stepwise hydroxy lation and O-acetylation at C-7. Ring closure may be activated by elimination of an acetate leaving group. In some examples, the allylic elimination and oxide ring closure that generates thebaine occurs spontaneously. In other examples, the ring closure reaction that generates pentacyclic thebaine is promoted by factors such as pH or solvent. In other examples, the thebaine-generating nng closure reaction is promoted by contact with a protein or enzyme. These conversion steps are provided in **FIG. 4** and represented generally in Scheme 2. R₁, R₂, and R₃ may be H or CH₃. R₄ may be CH₃, CH₃CH₂, CH₃CH₃CH₃, or other appropriate alkyl group. In some cases, R₁, R₂, R₃, and R₄, may be CH: as provided in **FIG. 4****.**

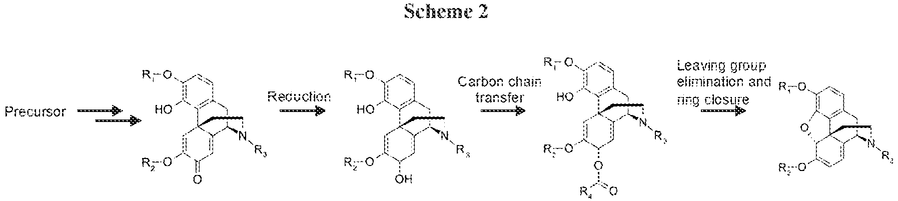

In some examples, the first enzyme that prepares the tetracyclic precursor is salutaridine reductase (SalR). In some cases. SalR hydroxylates the substrate salutaridine at the C-7 position (see Formula III). The product of this reaction may be one or more salutaridinol epimers. In some examples, the product is (7S)-salutaridinol. In some examples, the salutaridine reductase may catalyze the reduction reaction within a host cell, such as an engineered host cell, as described herein.

In some examples, the second enzyme that prepares the tetracyclic precursor is salutaridinol 7-O-acetyltransferase (SalAT). In some cases. SalAT transfers the acetyl from acetyl-CoA to the 7-OH of salutaridinol (see Formula IV). In other cases. SalAT may utilize a novel cofactor such as n-propionyl-CoA and transfer the propionyl to the 7-OH of salutaridinol. In some examples, the product of SalAT is (7*S*)-salutaridinol-7-O-acetate. In some examples, the salutaridinol 7-*O*-acetyltransferase may catalyze the acetyl transfer reaction within a host cell, such as an engineered host cell, as described herein.

In some examples, the tetracyclic precursor of thebaine is (7*S*)-salutaridinol-7-O-acetate. In some examples (7S)-salutaridinol-7-O-acetate is unstable and spontaneously eliminates the acetate at C-7 and closes the oxide bridge between C-4 and C-5 to form thebaine (see Formula V). In some examples, the rate of elimination of the acetate leat ing group is promoted by pH. In some examples, the allylic elimination and oxide bridge closure is catalyzed by an enzyme with thebaine synthase activity, or a thebaine synthase. In some examples, this enzyme is a Bet v 1-fold protein. In some examples, this enzyme is an engineered thebaine synthase, an engineered SalAT. a dirigent (DIR) protein, or a chalcone isomerase (CHI). In some examples, the enzyme encoding thebaine synthase activity may catalyze the ring closure reaction within a host cell, such as an engineered host cell, as described herein.

In some examples, the salutaridine reductase enzyme may be SalR or a SalR-like enzyme from plants in the *Ranunculales* order that biosynthesize thebain, for example *Papaver somniferum.* In other examples, the enzyme with salutaridine reductase activity may be from mammals or any other vertebrate or invertebrate that biosynthesizes endogenous morphine.

In some examples, the salutaridinol 7-*O*-acetyltransferase enzyme may be SalAT or a SalAT-like enzyme from plants in the *Ranunculates* order that biosynthesize thebaine, for example *P. somniferum.* In other examples, the enzyme with salutaridinol 7-*O*-acetyltransferase activity may be from mammals or any other vertebrate or invertebrate that biosynthesizes endogenous morphine.

In some examples, the thebaine synthase (TS) enzyme may be a Bet v 1 fold protein from plants in the *Ranunculales* order that biosynthesize thebaine, for example *P. somniferum.* In some examples, the Bet v 1 protein includes the following domains in order from the N-terminus to C-terminus: a β-strand, one or two α-helices, six β-strands, and one or two α-helices. The protein is organized such that it has a Bet v 1 fold and an active site that accepts large, bulky, hydrophobic molecules, such as morphinan alkaloids. This protein may be any plant Bet v 1 protein, pathogenesis-related 10 protein (PR-10), a major latex protein (MLP), fruit or pollen allergen, plant hormone binding protein (e.g., binding to cytokinin or brassinosteroids), plant polyketide cyclase-like protein, or norcoclaurine synthase (NCS)-related protein that has a Bet v 1 fold. Other non-plant examples of the Bet v 1 fold protein are polyketide cyclases, activator of Hsp90 ATPase homolog 1 (AHA1) proteins, SMU440-like proteins (e.g., from *Streptococcus mutans*). PA1206-related proteins (e.g., from *Pseudomonas aeruginosa*). CalC calicheamicin resistance protein (e.g., from *Micromonospora echinospora*), and the CoxG protein from carbon monoxide metabolizing *Oligotropha carboxidovorans.* Further examples from Bet v 1-related families include START lipid transfer proteins, phosphatidylinositol transfer proteins, and ring hydroxylases.

In some examples, the thebaine synthase enzyme may be a dirigent protein from plants in the *Ranunculates* order that biosynthesize thebaine, for example *P. somniferum.* In other examples, the enzyme may be any dirigent protein from plants.

In some examples, the thebaine synthase enzyme may be a chalcone isomerase protein from plants in the *Ranunculates* order that biosynthesize thebaine, for example *P. somniferum.* In other examples, the enzyme may be any chalcone isomerase protein from plants.

In some examples, the thebaine synthase enzyme may be a SalAT-like enzyme from plants in the *Ranunculates* order that biosynthesize thebaine, for example *P. somniferum.* In other examples, the enzyme may be any SalAT-like protein from plants.

In some examples, the enzyme with thebaine synthase activity may be from mammals or any other vertebrate or invertebrate that biosynthesizes endogenous morphine.

In some examples, combinations of the above enzymes together with additional accessory proteins may function to convert various tetracyclic precursors into thebaine. In some examples, these enzymes catalyze the reactions within a host cell, such as an engineered host cell, as described herein.

Examples of amino acid sequences for thebaine synthase activity are set forth in **Table 2.** An amino acid sequence for a thebaine synthase that is utilized in a tetracyclic precursor to thebaine may be 50% or more identical to a given amino acid sequence as listed in **Table 2.** For example, an amino acid sequence for such a thebaine synthase may comprise an amino acid sequence that is at least 50% or more, 55% or more. 60% or more. 65% or more. 70% or more, 75% or more, 80% or more, 81% or more, 82% or more. 83% or more. 84% or more. 85% or more, 86% or more, 87% or more, 88% or more, 89% or more, 90% or more, 91% or more, 92% or more, 93% or more, 94%, or more. 95% or more. 96% or more. 97% or more. 98% or more, or 99% or more identical to an amino acid sequence as provided herein. Additionally, in certain embodiments, an "identical" amino acid sequence contains at least 80%-99% identity at the amino acid level to the specific amino acid sequence. In some cases an "identical" amino acid sequence contains at least about 85%, 86%, 87%, 88%, 89%, 90%, 91%, 92%, 93%, 94% and more in certain cases, at least 95%. 96%. 97%, 98% and 99% identity, at the amino acid level. In some cases, the amino acid sequence may be identical but the DNA sequence is altered such as to optimize codon usage for the host organism, for example.

An engineered host cell may be provided that produces a salutaridine reductase, salutaridinol 7-*O*-acetyltransferase, and thebaine synthase that converts a tetracyclic precursor into thebaine, wherein the the baine synthase comprises an amino acid sequence selected from the group consisting of SEQ ID NOs: 19, 20. 21, 22, 23, 24, 25, and 26 as listed in **Table 2**. In some cases, the thebaine synthase may form a fusion protein with other enzymes. The enzymes that are produced within the engineered host cell may be recovered and purified so as to form a biocatalyst. These one or more enzymes may also be used to catalyze the conversion of a tetracyclic promorphinan precursor to the baine.

In other examples, the thebaine synthase comprises an amino acid sequence selected from the group consisting of SEQ ID NOs: 27, 28, 29. 30. 31, 32. 33, and 34 as listed in **Table 2.**

In additional cases, the one or more enzymes that are recovered from the engineered host cell may be used in a process for converting a tetracyclic promorphinan precursor to a thebaine. The process may include contacting the tetracyclic promorphinan precursor with the recovered enzymes in an amount sufficient to convert said tetracyclic promorphinan precursor to thebaine. In some examples, the tetracyclic promorphinan precursor may be contacted with a sufficient amount of the one or more enzymes such that at least 5% of said tetracyclic promorphinan precursor is converted to thebaine. In further examples, the tetracyclic promorphinan precursor may be contacted with a sufficient amount of the one or more enzymes such that at least 10%, at least 15%, at least 20%, at least 25%, at least 30%, at least 35%, at least 40%, at least 45%, at least 50%, at least 55%, at least 60%, at least 65%, at least 70%, at least 80%, at least 82%, at least 84%, at least 86%, at least 88%, at least 90%, at least 91%, at least 92%, at least 93%, at least 94%, at least 95%, at least 96%, at least 97%, at least 98%, at least 99%, at least 99.5%, at least 99.7%, or 100% of said tetracyclic promorphinan precursor is converted to thebaine.

In some examples, process conditions are implemented to support the formation of thebaine in engineered host cells. In some cases, engineered host cells are grown at pH 3.3, and once high cell density is reached the pH is adjusted to pH 8.0 to support continued production of thebaine at higher pH. In some cases, the engineered host cells produce additional enzymes to convert sugar and other simple precursors, such as tyrosine, to thebaine. In some cases, the SalAT enzyme has been engineered to exhibit higher activity at pH 8.0 and is expressed from a late stage promoter.

In some examples, one or more of the enzymes converting a tetracyclic promorphinan precursor to a thebaine are localized to cellular compartments. In some examples. SalR, SalAT. and thebaine synthase (TS) may be modified such that they encode targeting sequences that localize them to the endoplasmic reticulum membrane of the engineered host cell. In particular, in certain instances, the host cell may be engineered to increase production of salutaridinol or thebaine or products for which the aine is a precursor from reticuline or its precursors by localizing TS and/or SalR and/or SalAT to organelles in the yeast cell TS and/or SalR and/or SalAT may be localized to the yeast endoplasmic reticulum in order to decrease the spatial distance between TS and/or SalR and/or SalAT and CYP2D2 or CYP2D6 or SalSyn or an engineered cytochrome P450 enzyme that catalyzes the conversion of reticuline to salutaridine. By increased production is meant both the production of some amount of the compound of interest where the control has no production of the compound of interest, as well as an increase of 10% or more, such as 50% or more, including 2-fold or more, e.g.. 5-fold or more, such as 10-fold or more in situations where the control has some production of the compound of interest.

In other examples, SalAT and TS may be co-localized in to a single protein fusion. In some examples, the fusion is created between SalAT and TS by one of several methods, including, direct fusion, co-localization to a yeast organelle, or by enzy me co-localization tools such as leucine zippers, protein scaffolds that utilize adaptor domains, or RNA scaffolds that utilize aptamers. Co-localizing the thebaine synthesis enzyme may facilitate substrate channeling between the active sites of the enzymes and limit the diffusion of unstable intermediates such as salutaridinol-7-*O*-acetate.

In some examples, an engineered salutaridinol 7-O-acetyltransferase (SalAT) enzyme is used in converting a tetracyclic promorphinan precursor to a thebaine. In some examples, a SalAT enzy me is engineered to combine two functions: (1) the transfer of an acyl group from acetyl-CoA to the 7-OH of salutaridinol, and (2) the subsequent elimination of the acety 1 group and closure of an oxide bridge between carbons C4 and C5 to form thebaine.

In some examples, an enzyme with salutaridinol 7-O-acetyltmnsfemse activity is fused to a peptide with a Bet v 1 fold. In some examples, salutaridinol 7-O-acety transferase enzyme and the Bet v 1 fold protein may be fused in any order from N-terminus to C-terminus, C-terminus to N-terminus, N-terminus to N-terminus, or C-terminus to C-terminus. In some examples, the two protein sequences may be fused directly or fused through a peptide linker region.

In some examples, an enzyme with salutaridinol 7-O-acetyltransferase activity is fused to a peptide with a Bet v 1 fold by circular permutation. In some cases, the N- and C-termini of SalAT are fused and the Bet v 1 sequence is then inserted randomly within this sequence. In some cases, the resulting fusion protein library is screened for thebaine production. In other cases, a circular permutation SalAT library is first screened for activity in the absence of Bet v 1. In other cases, the N- and C-termini of SalAT are fused and the enzyme is digested and blunt end cloned. In other cases, this library of circularly permuted SalAT is screened for salutaridinol 7-*O*-acetyltransferase activity. In other cases, active variants from the circularly permuted SalAT library are then used to design protein fusions with a peptide with a Bet v 1 fold.

The one or more enzymes that may be used to convert a tetracyclic promorphinan precursor to a thebaine may contact the tetracyclic promorphinan precursor in vitro. Additionally, or alternatively, the one or more enzymes that may be used to convert a tetracyclic promorphinan precursor to thebaine may contact the tetracyclic promorphinan precursor in vivo. Additionally, the one or more enzymes that may be used to convert a tetracyclic promorphinan precursor to thebaine may be provided to a cell having the tetracyclic promorphinan precursor within, or may be produced within an engineered host cell.

In some examples, the methods provide for engineered host cells that produce an alkaloid product, wherein the conversion of a tetracyclic promorphinan precursor to a thebaine may comprise a key step in the production of an alkaloid product. In some examples, the alkaloid product is a thebaine. In still other embodiments, the alkaloid product is derived from a the baine, including for example, downstream morphinan alkaloids. In another embodiment, a tetracyclic promorphinan precursor is an intermediate toward the product in of the engineered host cell. In still other embodiments, the alkaloid product is selected from the group consisting of morphinan, nor-opioid, or nal-opioid alkaloids.

In some examples, the substrate of the reduction reaction is a compound of Formula III: 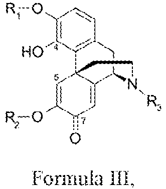
or a salt thereof, wherein
R₁, R₂, and R₃ are independently selected from hydrogen and methyl.

In some other examples. R₁, R₂, and R₃ are methyl, and the reduction reaction is catalyzed by a salutaridine reductase.

In some examples, the substrate of the carbon chain transfer reaction is a compound of Formula IV: 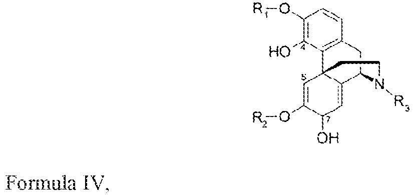 or a salt thereof, wherein:
R₁, R₂, and R₃ are independently selected from hydrogen and methyl.

In some other examples. R₁, R₂, and R₃ are methyl, and the carbon chain transfer reaction is catalyzed by a salutaridinol 7-*O*-acetyltransferase.

In some examples, the substrate of thebaine synthase is a compound of Formula V: 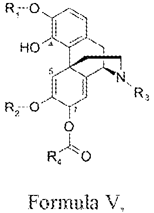 or a salt thereof, wherein:
R₁, R₂, and R₂ are independently selected from hydrogen and methyl; and
R₄ is selected from methyl, ethyl, propyl, and other appropnate alkyl group.

In some other examples. R₁, R₂, R₃, and R₄ are methyl, and the ring closure reaction is catalyzed by a thebaine synthase. In some examples, the thebaine synthase is a Bet v 1 protein.

In some examples, the methods provide for engineered host cells that produce alkaloid products from salutaridine. The conversion of salutardine to thebaine may comprise a key step in the production of diverse alkaloid products from a precursor. In some examples, the precursor is L.-tyrosine or a sugar (e.g., glucose). The diverse alkaloid products can include, without limitation, morphinan, nor-opioid, or nal-opioid alkaloids.

Any suitable carbon source may be used as a precursor toward a pentacyclic morphinan alkaloid. Suitable precursors can include, without limitation, monosaccharides (e.g., glucose, fructose, galactose, xylose), oligosaccharides (e.g.. lactose, sucrose, raffinose), polysaccharides (e.g.. starch, cellulose), or a combination thereof. In some examples, unpurified mixtures from renewable feedstocks can be used (e.g., cornsteep liquor, sugar beet molasses, barley malt, biomass hydrolysate). In still other embodiments, the carbon precursor can be a one-carbon compound (e.g., methanol, carbon dioxide) or a two-carbon compound (e.g., ethanol). In yet other embodiments, other carbon-containing compounds can be utilized, for example, methylamine, glucosamine, and amino acids (e.g.. L-tyrosine). In some examples, a 1-benzylisoquinoline alkaloid may be added directly to an engineered host cell of the invention, including, for example, norlaudanosoline, laudanosoline, norreticuline, and reticuline.

In some examples, the benzylisoquinoline alkaloid product, or a derivative thereof, is recovered. In some examples, the benzylisoquinoline alkaloid product is recovered from a cell culture. In some examples, the benzylisoquinoline alkaloid product is a morphinan, nor-opioid, or nal-opioid alkaloid.

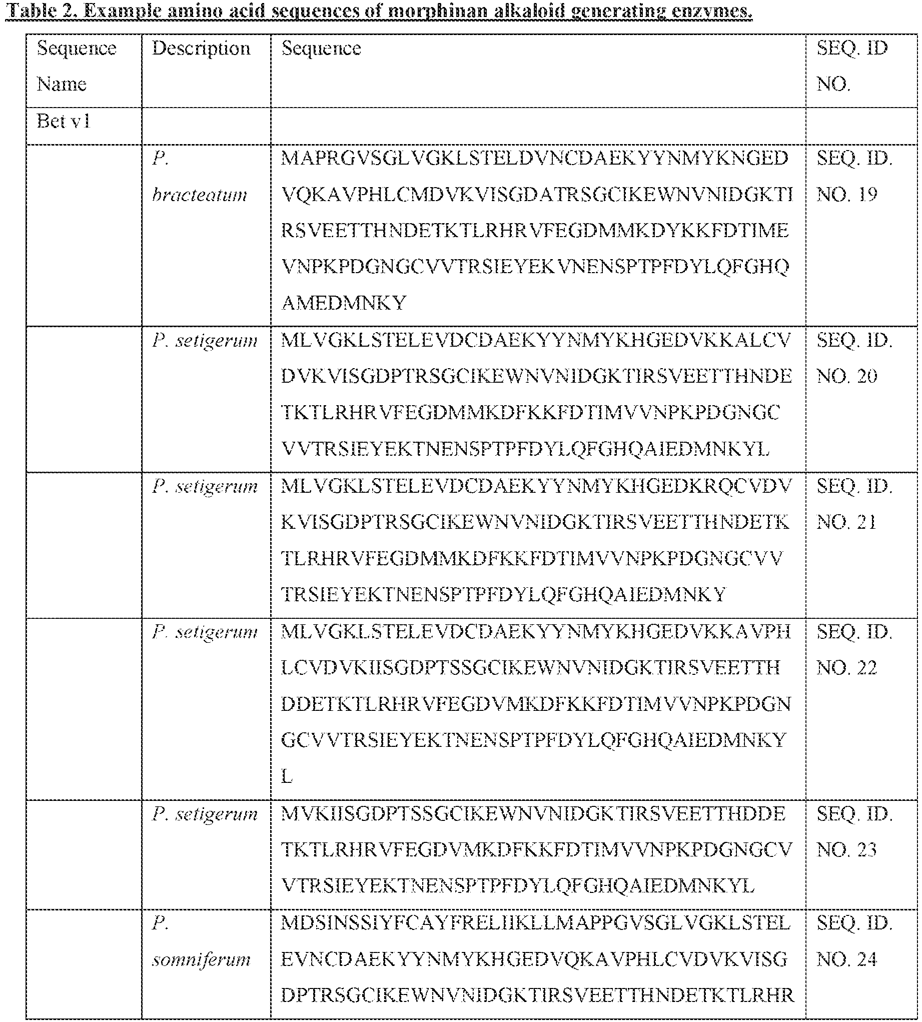

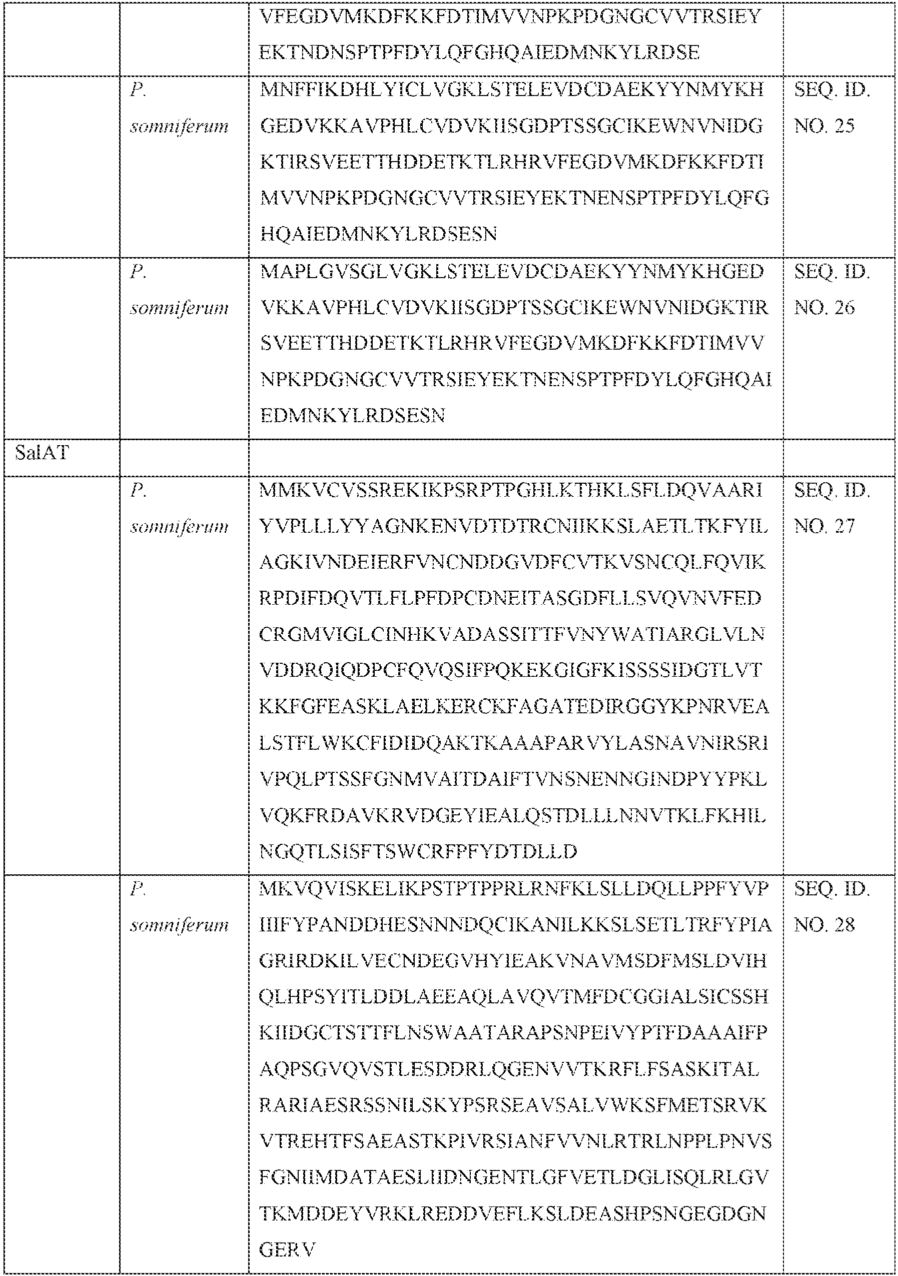

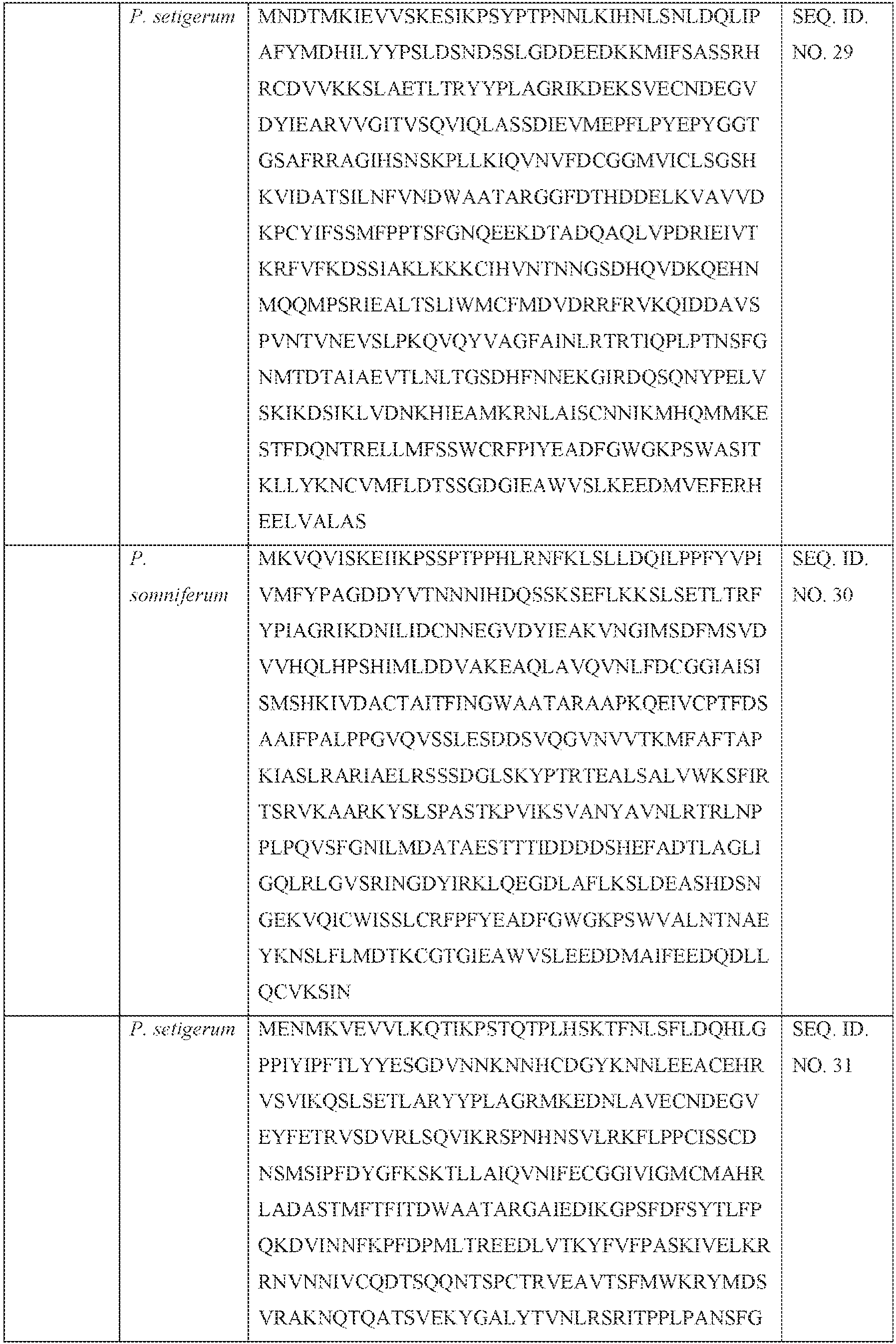

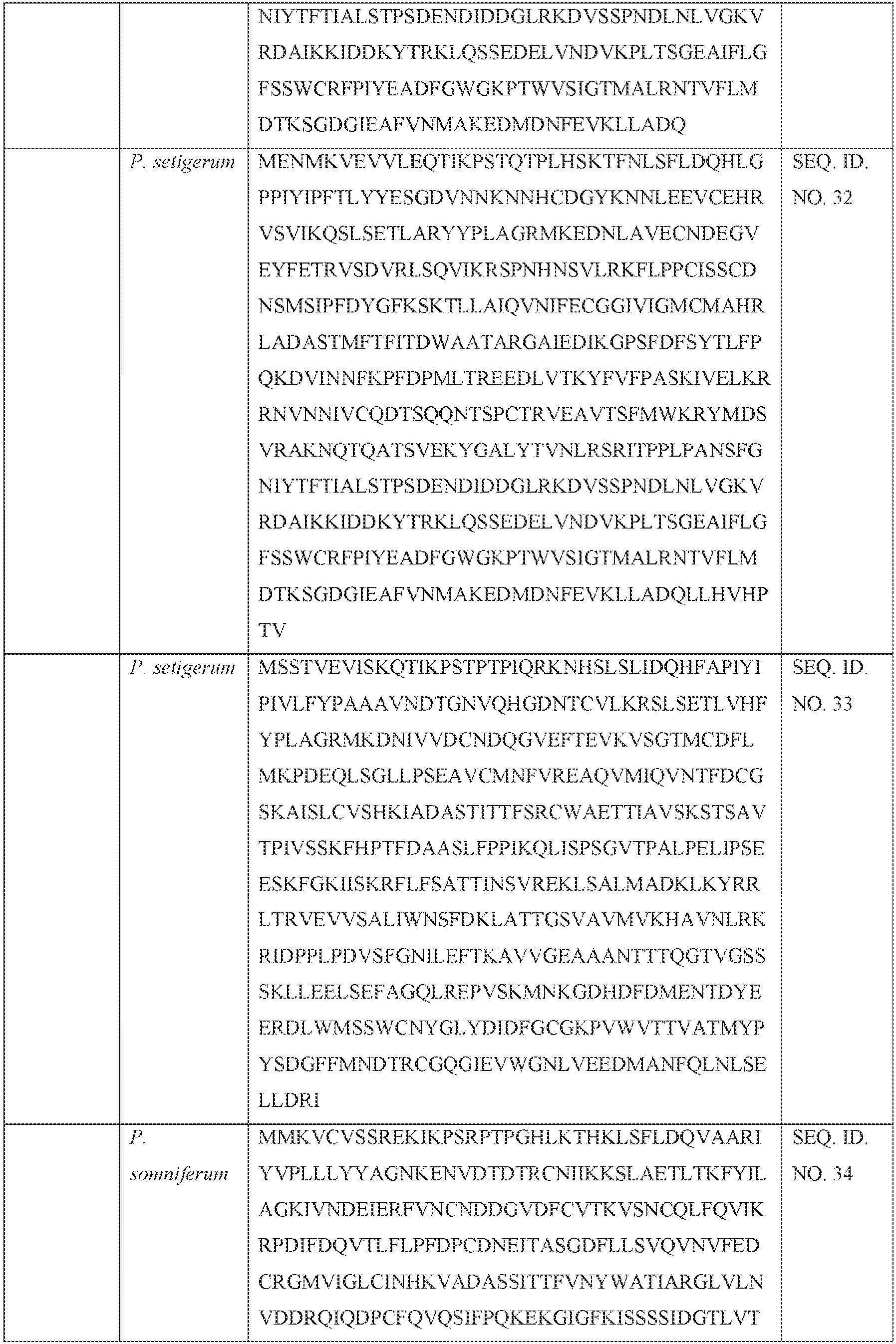

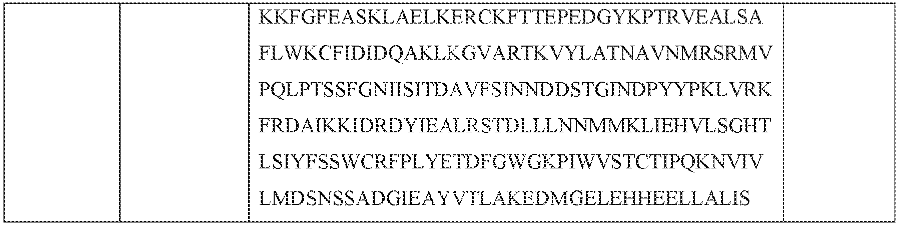

### Morphinan Alkaloid Isomerization Modifications

Some methods, processes, and systems provided herein describe the production of morphinan alkaloid isomers. Some of the methods, processes, and systems describe the conversion of a precursor morphinan alkaloid with a carbon-carbon double bond between carbons C-14 and C-8 into a product morphinan alkaloid with a carbon-carbon double bond between carbons C-8 and C-7 (**FIG. 4**). Some of the methods, processes, and systems may comprise an engineered host cell. In some examples, the conversion of a precursor morphinan alkaloid with a carbon-carbon double bond between carbons C-14 and C-8 into a product morphinan alkaloid with a carbon-carbon double bond between carbons C-8 and C-7 are significant steps in the conversion of a precursor to a diverse range of benzylisoquinoline alkaloids.

In some examples, the production of precursor morphinan alkaloids with a carbon-carbon double bond between carbons C-14 and C-8 occurs within the engineered host cell comprising a plurality of heterologous enzymes for converting simple starting materials to the precursor morphinan alkaloids. In some examples, the simple starting materials are sugar and/or L-tyrosine.

In some examples, the isomer precursor morphinan alkaloid may be neopinone, neopine, neomorphine, or neomorphinone The precursor morphinan alkaloid may be convened to the desired isomer by rearrangement of a carbon-carbon double bond between carbons C-14 and C-8 and carbons C-8 and C-7. In some cases, examples of the products formed by isomerization may be codeinone, codeine, morphine, or morphinone. In some examples, the rearrangement that generates the desired isomer occurs spontaneously. In other examples, the rearrangement that generates the desired isomer is promoted by factors such as pH and solvent. In other examples, the carbon-carbon double bond is transposed by contact with a protein or enzyme. The isomerization conversion step is provided in **FIG. 4** and represented generally in **Scheme 3.** R₁, R₂, R₃, and R₄ may be O, OH, H, CH₃, or other appropriate alkyl groups.

### Scheme 3

In some examples, the first enzyme that generates an isomer precursor morphinan alkaloid is 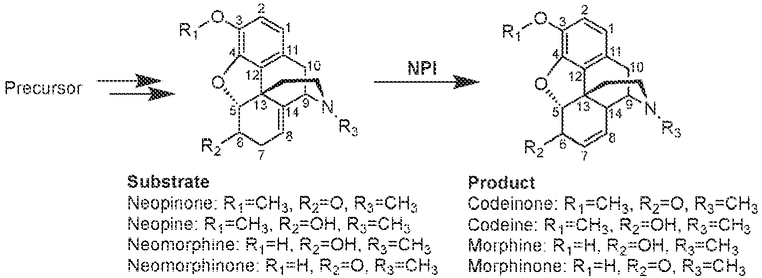 the baine 6-*O*-demethylase (T6ODM). In some cases. T6ODM *O*-demethylates the substrate thebaine at the C-6 position. In some examples, the product of this reaction is neopinone. In some examples, the T6ODM may catalyze the *O-*demethylation reaction within a host cell, such as an engineered host cell, as described herein.

In some examples, the isomer precursor morphinan alkaloid is neopinone. In some examples, neopinone undergoes isomerization to codeinone. In some examples, partitioning from neopinone to codeinone may reach equilibnum in aqueous solution such that neopinone and codeinone exist at steady state concentrations. In some examples, the rate of conversion of neopinone to codeinone is promoted by pH. In some examples, the rearrangement of neopinone to codeinone is catalyzed by an enzyme with neopinone isomerase activity. In some examples, this enzyme is a Bet v 1-fold protein. In some examples, this enzyme is a neopinone isomerase (NPI). In some examples, this enzyme is an engineered protein with a tnmcation of its N-terminal sequence. In some examples, the NPI may catalyze the isomerization reaction within a host cell, such as an engineered host cell, as described herein.

In some examples, the enzyme that acts on codeinone is codeinone reductase (COR). In some cases. COR reduces the ketone at position C-6 of codeinone to form a hydroxyl. In some examples, the product of this reaction is codeine. In some examples. COR is selected from numerous gene duplication and alternative splicing isoforms to exhibit the highest activity when paired with the protein encoding the neopinone isomerase activity. In some examples, the COR may catalyze the reduction reaction within a host cell, such as an engineered host cell, as described herem.

In some examples, the enzyme that acts on codeinone is morphinone reductase (morB). In some cases, morB saturates the carbon-carbon double bond between C-7 and C-8 of codeinone. In some examples, the product of this reaction is hydrocodone. In some examples, the morB may catalyze the reduction reaction within a host cell, such as an engineered host cell, as described herein.

In some examples, the thebaine 6-*O*-demethylase enzyme may be T6ODM or a T60DM-like enzyme from plants in the *Ranunculales* order that biosynthesize morphine, for example *Papaver somniferum.* In some examples, T60DM may be a T6ODM-like enzyme from plants that biosynthesize benzy lisoquinoline alkaloids, for example *P. bracteatum*, *P. rhoeas*, *P. nudicaule*, and *P. orientale.* In some examples, the plant enzyme is a 2-oxoglutarate/Fe(II)-dependent dioxygenase that uses 2-oxoglutarate and oxygen and generates succinate and carbon dioxide when demethylating thebaine to produce neopinone. In some examples. T6ODM can also demethylate oripavine to generate neomorphinone.

In other examples, the enzyme with thebaine 6-*O*-demethylase activity may be from mammals oranother vertebrate or invertebrate that biosynthesizes endogenous morphinan alkaloids.

In some examples, the neopinone isomerase (NPI) enzyme may be a Bet v 1-fold protein from plants in the *Ranunculales* order that biosynthesize morphine, for example *Papaver* somniferum. In some examples, NPI may be a NPI-like enzyme from plants that biosynthesize benzylisoquinoline alkaloids, for example *P. bracteatum*, *P. rhoeas*, *P. nudicaule*, and *P. orientale.* In some examples, the Bet v 1 protein includes the following domains in order from the N-terminus to the C-terminus: a β-strand, one or two α-helices, six β-strands, and one or two α-helices. In some examples, a truncation is performed at the N-terminus of the enzyme to remove all or part of the first domain. In some examples, the enzyme may have one or more activity-increasing components as discussed herein and as described in **Examples 6 and 7.** In some examples, the protein is organized such that it has a Bet v 1 fold and an active site that accepts large, bulky, hydrophobic molecules, such as the morphinan alkaloids. In some examples, the protein may be any plant Bet v 1 protein, pathogenesis-related 10 protein (PR-10), a major latex protein (MLP), fruit or pollen allergen, plant hormone binding protein (e.g.. binding to cytokinin or brassinosteroids), plant polyketide cyclase-like protein, or norcoclaurine synthase (NCS)-related protein that has a Bet v 1 fold. In some examples, the function of the Bet v 1-fold protein is to catalyze a reaction that can also occur spontaneously.

In other examples, the enzyme with neopinone isomerase activity may be from mammals or another vertebrate or invertebrate that biosynthesizes endogenous morphinan alkaloids.

In some examples, the codeinone reductase enzyme may be COR or a COR-like enzyme from plants in the *Ranunculales* order that biosynthesize morphine, for example *P. somniferum.* In some examples, COR may be a COR-like enzyme from plants that biosynthesize benzylisoquinoline alkaloids, for example *P. bracteatum*, *P. rhoeas*, *P. nudicaule*, and *P. orientale.* In some examples, the plant enzyme is an oxidoreductase that uses NADPH as a cofactor in the reversible reduction of codeinone to codeine. In some examples, the COR enzyme is a particular gene duplication or splicing variant selected to have select kinetic parameters, for example a higher rate of activity for one or more reactions (K_{cat}), improved binding affinity to one or more substrates (K_{M}), enhanced specificity for substrate codeinone over neopinone, or enhanced thermostability. In some examples, the COR enzyme may act to reduce other morphinan alkaloid substrates, for example neopinone, morphinone, neomorphinone, hydrocodone, hydromorphone, oxycodone, oxymorphone, 14-hydroxycodeinone, or 14-hydroxymorphinone. In some examples, the products of COR activity are neopine, morphine, neomorphine, dihydrocodeine, dihydromorphine, oxycodol, oxymorphol, 14-hydroxcodeine, or 14-hydroxy morphine.

In some examples, the morphinone reductase enzyme may be morB or a morB-like enzyme from bacteria in the *Pseudomonas* genus. In some examples, morphinone reductase may be an alkene reductase enzyme from a gram-negative bacterium. In some examples, the bacterial enzyme is a a/ß-bamel flavoprotein that uses NADH and FMN as cofactors to saturate the carbon-carbon double bond between C-7 and C-8 of codeinone. In some examples, the morB enzyme has select kinetic parameters, for example a higher rate of activity for one or more reactions (K_{cat}), improved substrate binding affinity for one or more substrates (K_{M}), enhanced specificity for one substrate, or enhanced thermostability. The morB enzyme may also reduce other morphinan substrates, for example morphinone, neomorphinone, codeine, morphine, neopine, neomorphine, 14-hydroxycodeinone, or 14-hydroxymorphinone. Examples of products of morB activity are hydromorphone, dihydrocodeine, dihydromorphine, oxycodone, or oxymorphone.

In other examples, combinations of the above enzymes together with additional accessory proteins may function in the production of select morphinan alkaloid isomers. In some examples, these enzymes catalyze the reactions within a host cell, such as an engineered host cell, described herein.

Examples of amino acid sequences for neopinone isomerase activity are set forth in **Table 3.** An amino acid sequence for a neopinone isomerase that is utilized in converting a precursor morphinan alkaloid with a carbon-carbon double bond between carbons C-14 and C-8 into a product morphinan alkaloid with a carbon-carbon double bond between carbons C-8 and C-7 may be 50% or more identical to a given amino acid sequence is listed in **Table 3.** For example, an amino acid sequence for such a neopinone isomerase may comprise an amino acid sequence that is at least 50% or more, 55% or more, 60% or more. 65% or more. 70% or more, 75% or more, 80% or more, 81% or more, 82% or more, 83% or more. 84% or more. 85% or more. 86% or more, 87% or more, 88% or more, 89% or more, 90% or more, 91% or more, 92% or more, 93% or more, 94% or more, 95% or more, 96% or more, 97% or more, 98% or more, or 99% or more identical to an amino acid sequence as provided herein. Additionally, in certain embodiments, an "identical" amino acid sequence contains at least 80%-99% identity at the amino acid level to the specific amino acid sequence. In some cases, an "identical" amino acid sequence contains at least about 85%, 86%, 87%, 88%, 89%, 90%, 91%, 92%, 93%, 94% and more in certain cases, at least 95%, 96%, 97%, 98% and 99% identity, at the amino acid level. In some cases, the amino acid sequence may be identical but the DNA sequence is altered such as to optimize codon usage for the host organism, for example.

An engineered host cell may be provided that produces a thebaine 6-O-demethylase, neopinone isomerase, and codeinone reductase that converts a precursor morphinan alkaloid isomer into a desired product morphinan alkaloid isomer by rearrangement of a carbon-carbon double bond between carbons C-14 and C-8 and carbons C-8 and C-7. wherein the neopinone isomerase comprises an amino acid sequence selected from the group consisting of SEQ ID NOs: 54, 55. 56. 57, and 58. In some cases, the neopinone isomerase may physicially interact with one or more pathway enzymes. In some cases, the physicial interaction may change the activity of the one or more pathway enzymes. In some cases, the neopinone isomerase may form a fusion protein with one or more other enzymes. Enzymes that are produced within the engineered host cell may be recovered and purified so as to form a biocatalyst. These one or more enzymes may also be used to catalyze the conversion of a precursor morphinan alkaloid with a carbon-carbon double bond between carbons C-14 and C-8 into a product morphinan alkaloid with a carbon-carbon double bond between carbons C-8 and C-7.

In other examples, the neopinone isomerase comprises an amino acid sequence selected from the group consisting of SEQ ID NOs: 43, 44. 45. 46, 47, 48, 49, 50. 51. 52. 53, 54, 55, 56, 57. and 58 as listed in **Table 3**.

Examples of amino acid sequences for codeinone reductase activity are set forth in **Table 4.** An amino acid sequence for a codeinone reductase that is utilized in reducing a ketone at the C-6 position of a morphinan alkaloid to a hydroxyl at that position may be 50% or more identical to a given amino acid sequence as listed in **Table 4.** For example, an amino acid sequence for such a codeinone reductase may comprise an amino acid sequence that is at least 50% or more, 55% or more, 60% or more, 65% or more, 70% or more. 75% or more. 80% or more. 81% or more, 82% or more, 83% or more, 84% or more, 85% or more. 86% or more, 87% or more, 88% or more, 89% or more, 90% or more, 91% or more, 92% or more. 93% or more. 94% or more. 95% or more, 96% or more. 97% or more. 98% or more, or 99% or more identical to an amino acid sequence as provided herein. Additionally, in certain embodiments, an "identical" amino acid sequence contains at least 80%-99% identity at the amino acid level to the specific amino acid sequence. In some cases, an "identical" amino acid sequence contains at least about 85%. 86%. 87%. 88%. 89%, 90%, 91%, 92%, 93%, 94% and more in certain cases, at least 95%, 96%, 97%. 98% and 99% identity, at the amino acid level. In some cases, the amino acid sequence may be identical but the DNA sequence is altered such as to optimize codon usage for the host organism, for example.

An engineered host cell may be provided that produces a the baine 6-O-demethylase, neopinone isomerase, and codeinone reductase that converts a precursor morphinan alkaloid isomer into a desired product morphinan alkaloid isomer by rearrangement of a carbon-carbon double bond between carbons C-14 and C-8 and carbons C-8 and C-7 and reduction of a ketone at the C-6 position to a hydroxyl, wherein the codeinone reductase comprises an amino acid sequence selected from the group consisting of SEQ ID NOs: 59, 60, 61, 62. 63. 64, 65, 66, 67, and 68 as listed in **Table 4.** In some cases, the codeinone reductase may interact with or form a fusion protein with other enzymes. The enzymes that are produced within the engineered host cell may be recovered and purified so as to form a biocatalyst. These one or more enzymes may also be used to catalyze the conversion of a precursor morphinan alkaloid with a carbon-carbon double bond between carbons C-14 and C-8 into a product morphinan alkaloid with a carbon-carbon double bond between carbons C-8 and C-7.

In additional cases, the one or more enzymes that are recovered from the engineered host cell may be used in a process for converting a precursor morphinan alkaloid with a carbon-carbon double bond between carbons C-14 and C-8 into a product morphinan alkaloid with a carbon-carbon double bond between carbons C-8 and C-7. The process may include contacting the precursor morphinan alkaloid isomer with the recovered enzymes in an amount sufficient to convert said precursor morphinan alkaloid isomer to the desired morphinan alkaloid isomer product. In some examples, the precursor morphinan alkaloid isomer may be contacted with a sufficient amount of the one or more enzymes such that at least 5% of said precursor morphinan alkaloid isomer is converted to the desired product morphinan alkaloid isomer. In further examples, the precursor morphinan alkaloid isomer may be contacted with a sufficient amount of the one or more enzymes such that at least 10%, at least 15%, at least 20%, at least 25%, at least 30%, at least 35%, at least 40%, at least 43%, at least 50%, at least 55%, at least 60%, at least 65%, at least 70%, at least 80%, at least 82%, at least 84%, at least 86%, at least 88%, at least 90%, at least 91%, at least 92%, at least 93%, at least 94%, at least 95%, at least 96%, at least 97%, at least 98%, at least 99%, at least 99.5%, at least 99.7%, or 100% of said precursor morphinan alkaloid isomer is converted to the desired product morphinan alkaloid isomer.

In some examples, process conditions are implemented to support the formation of the desired product morphinan alkaloid isomer in engineered host cells. In some cases, engineered host cells are grown at pH 3.3. and once high cell density is reached the pH is adjusted to pH 6-6.5 to support continued production of the desired product morphinan alkaloid isomers at higher pH. In some cases, the engineered host cells produce additional enzymes to convert sugar and other simple starting materials, such as tyrosine, to the desired product morphinan alkaloid isomers.

In some examples, one or more of the enzymes converting a precursor morphinan alkaloid with a carbon-carbon double bond between carbons C-14 and C-8 to a product morphinan alkaloid with a carbon-carbon double bond between carbons C-8 and C-7 are localized to cellular compartments. In some examples, T6ODM. COR or morB, and NPI may be modified such that they encode targeting sequences that localize them to the endoplasmic reticulum membrane of the engineered host cell. In particular, in certain instances, the host cell may be engineered to increase production of product morphinan alkaloid isomers or its precursors by localizing NPI and/or T6ODM and/or COR and/or morB to organelles in the yeast cell. NPI and/or T6ODM and/or COR and/or morB may be localized to the yeast endoplasmic reticulum in order to decrease the spatial distance between these enzymes. By increased production is meant both the production of some amount of the compound of interest where the control has no production of the compound of interest, as well as an increase of 10% or more, such as 50% or more, including 2-fold or more, e.g., 5-fold or more, such as 10-fold or more in situations where the control has some production of the compound of interest.

In other examples. T6ODM and NPI may be co-localized in to a single protein fusion. In other examples, COR or morB and NPI may be co-localized in to a single protein fusion. In some examples, the fusion is between the proteins is created by one of several methods, including, direct fusion, co-localization to a yeast organelle, or by enzyme co-localization tools such as leucine zippers, protein scaffolds that utilize adaptor domains, or RNA scaffolds that utilize aptamers. Co-localizing the neopinone isomerase enzyme may facilitate substrate channeling between the active sites of the enzymes and limit the diffusion of unstable intermediates such as neopinone and codeinone.

In some examples, an engineered T6ODM enzyme is used in converting between morphinan alkaloid isomers. In some examples, a T6ODM enzyme is engineered to combine two functions: (1) the *O*-demethylation of thebaine at the C-6 position, and (2) the rearrangement of a carbon-carbon double bond between carbons C-14 and C-8 and carbons C-8 and C-7.

In some examples, an enzyme with thebaine 6-*O*-demethylase activity is fused to a peptide with a Bet v 1 fold. In some examples, the thebaine 6-*O*-demethylase enzyme and the Bet v 1 fold protein may be fused in any order from N-termimts to C-terminus. C-terminus to N-terminus, N-terminus to N-terminus, or C-terminus to C-terminus. In some examples, the two protein sequences may be fused directly or fused through a peptide linker region.

In some examples, an enzyme with thebaine 6-*O*-demethylase activity is fused to a peptide with a Bet v 1 fold by circular permutation. In some cases, the N- and C-termini of T6ODM are fused and the Bet v 1 sequence is then inserted randomly within this sequence. In some cases, the resulting fusion protein library is screened for production of the desired morphinan alkaloid isomer product. In other cases, a circular permutation T6ODM library is first screened for activity in the absence of Bet v 1. In other cases, the N- and C-tennini of T6ODM are fused and the enzyme is digested and blunt end cloned. In other cases, this library of circularly permuted T6ODM is screened for thebaine 6-*O*-demethylase activity. In other cases, active variants from the circularly permuted T6ODM library are then used to design protein fusions with a peptide with a Bet v 1 fold.

In some examples, an engineered COR or morB enzyme is used in converting between morphinan alkaloid isomers. In some examples, a COR or morB enzyme is engineered to combine two functions: (1) the rearrangement of a carbon-carbon double bond between carbons C-14 and C-8 and carbons C-8 and C-7. and (2) the reduction of a morphinan alkaloid isomer product.

In some examples, an enzyme with opioid reductase activity is fused to a peptide with a Bet v 1 fold. In some examples, the COR or morB enzyme and the Bet v 1 fold protein may be fused in any order from N-terminus to C-terminus, C-ternunus to N-terminus, N-terminus to N-terminus, or C-tenninus to C-terminus. In some examples, the two protein sequences may be fused directly or fused through a peptide linker region.

In some examples, an enzyme with opioid reductase activity is fused to a peptide with a Bet v 1 fold by circular pennutation. In some cases, the N- and C-tennini of COR or morB are fused and the Bet v 1 sequence is then inserted randomly within this sequence. In some cases, the resulting fusion protein library is screened for production of the desired morphinan alkaloid isomer product. In other cases, a circular permutation COR or morB library is first screened for activity in the absence of Bet v 1. In other cases, the N- and C-termini of COR or morB are fused and the enzyme is digested and blunt end cloned. In other cases, this library of circularly permuted COR or morB is screened for opioid reductase activity. In other cases, active variants from the circularly permuted COR or morB library are then used to design protein fusions with a peptide with a Bet v 1 fold

The one or more enzymes that may be used to convert a precursor morphinan alkaloid with a carbon-carbon double bond between carbons C-14 and C-8 to a product morphinan alkaloid with a carbon-carbon double bond between carbons C-8 and C-7 may contact the precursor morphinan alkaloid isomer in vitro. Additionally, or alternatively, the one or more enzymes that may be used to convert a precursor morphinan alkaloid with a carbon-carbon double bond between carbons C-14 and C-8 to a product morphinan alkaloid with a carbon-carbon double bond between carbons C-8 and C-7 may contact the precursor morphinan alkaloid isomer in vivo. Additionally, the one or more enzymes that may be used to convert a precursor morphinan alkaloid with a carbon-carbon double bond between carbons C-14 and C-8 to a product morphinan alkaloid with a carbon-carbon double bond between carbons C-8 and C-7 may be provided to a cell having the precursor morphinan alkaloid isomer within, or may be produced within an engineered host cell.

In some examples, the methods provide for engineered host cells that produce an alkaloid product, wherein the conversion of a precursor morphinan alkaloid with a carbon-carbon double bond between carbons C-14 and C-8 to a product morphinan alkaloid with a carbon-carbon double bond between carbons C-8 and C-7 may comprise a significant step in the production of an alkaloid product. In some examples, the alkaloid product is a codeinone. In still other embodiments, the alkaloid product is derived from a codeinone, including for example, downstream morphinan alkaloids. In another embodiment, a precursor morphinan alkaloid with a carbon-carbon double bond between carbons C-14 and C-8 is an intermediate toward the product in of the engineered host cell. In still other embodiments, the alkaloid product is selected from the group consisting of morphinan, nor-opioid, or nal-opioid alkaloids.

In some examples, the substrate of the *O*-demethylation reaction is a compound of Formula VI: 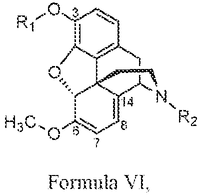 or a salt thereof, wherein:
R₁, and R₂ are independently selected from hydrogen and methyl.

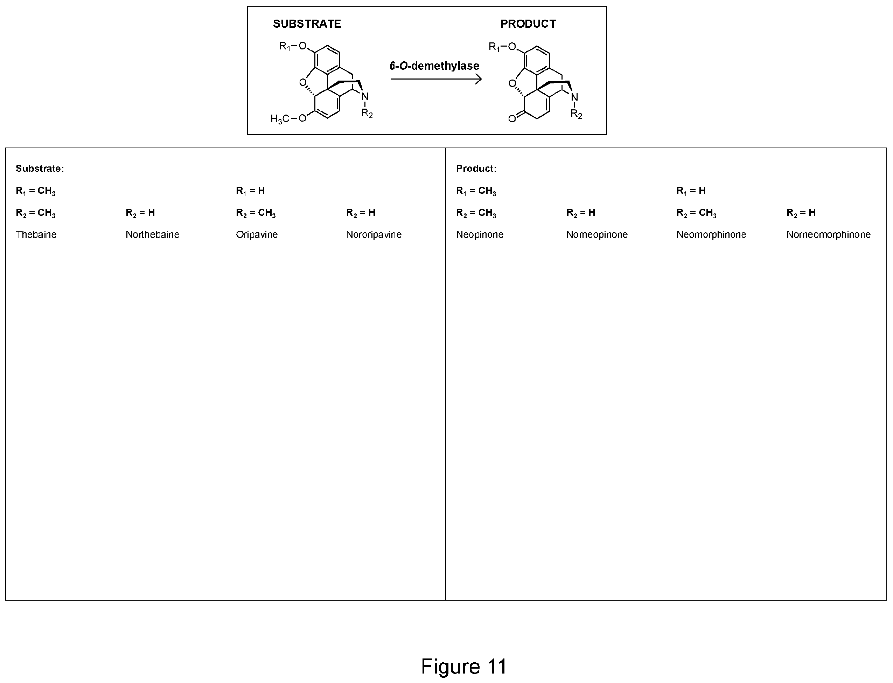

In some other examples. R₁ and R₂ are methyl, and the *O*-demethylation reaction is catalyzed by a thebaine 6-*O*-demethylase. Other examples of 6-*O*-demethylation reactions are provided in **FIG. 11****.**

In some examples, the substrate of the isomenzation reaction is a compound of Formula VII: 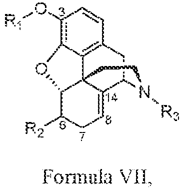
or a salt thereof, wherein.
R₁, and R₃ are independently selected from hydrogen and methyl, and R₂ is independently selected from hydroxyl and oxygen.

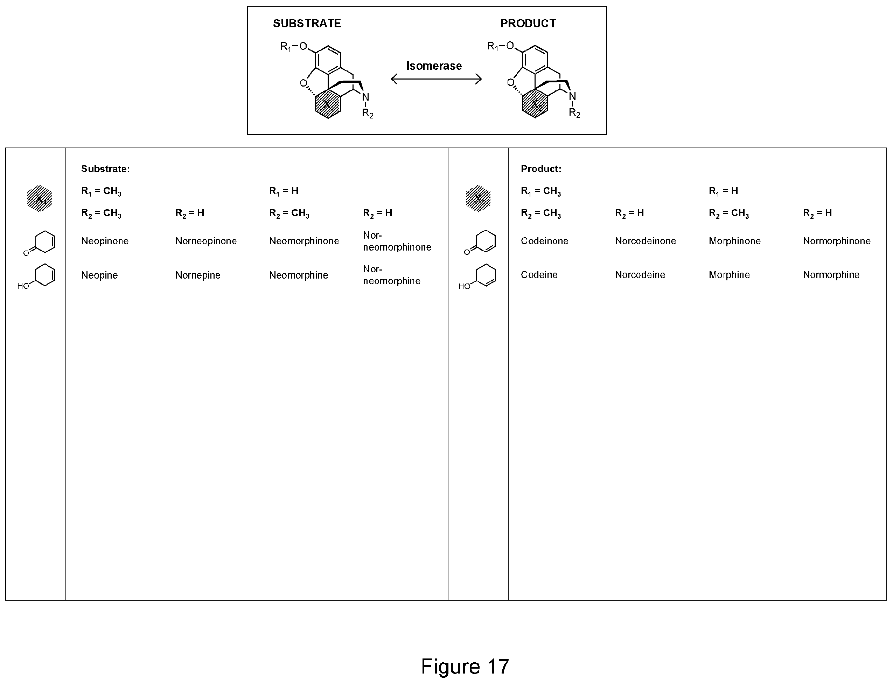

In some other examples, R₁, and R₃ are methyl and R₁ is oxygen, and the isomerization reaction is catalyzed by a neopinone isomerase. Other examples of isomerization reactions are provided in **FIG. 17****.**

In some examples, the substrate of the reduction reaction is a compound of Formula VIII: 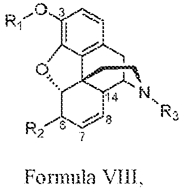 or a salt thereof, wherein:
R₁, and R₃ are independently selected from hydrogen and methyl; and R₂ is independently selected from hydroxyl and oxygen.

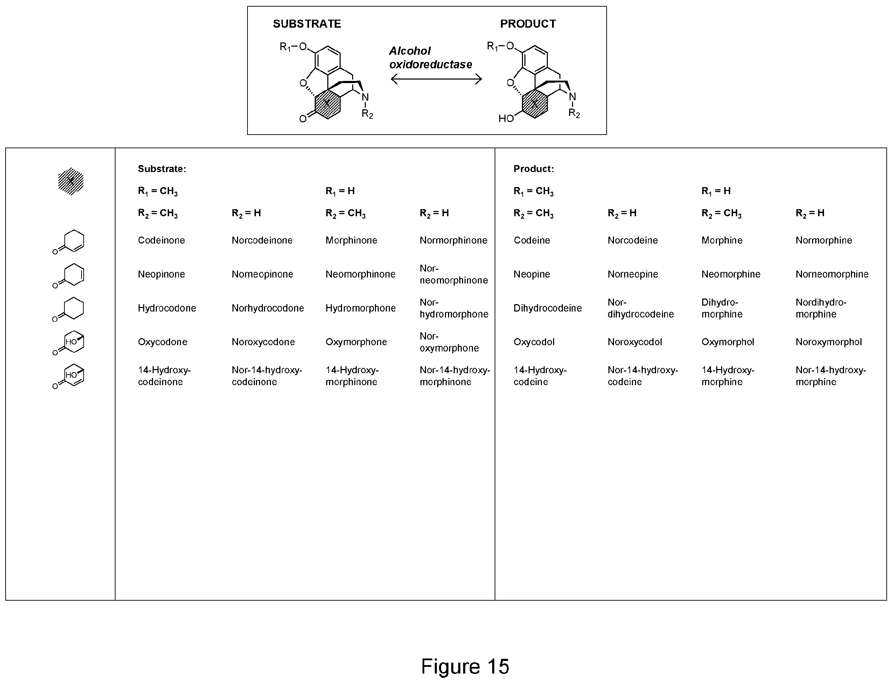

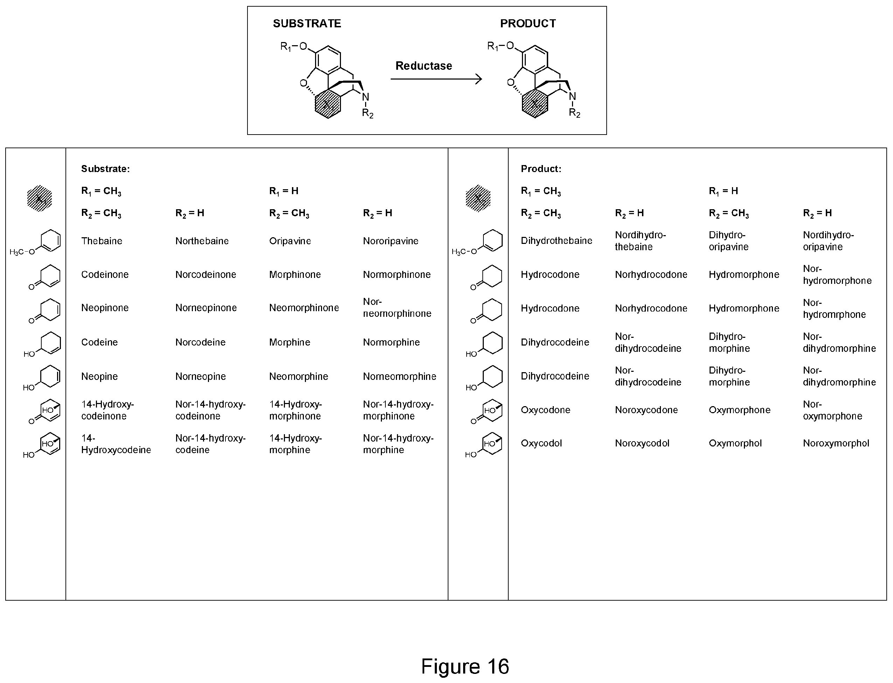

In some other examples. R₁ and R₃ are methyl and R₂ is oxygen, and the reduction reaction is catalyzed by a codeinone reductase. In some other examples, the reduction reaction is catalyzed by a morphinone reductase. Other examples of reduction reactions are provided in **FIGs. 15** **and** **16****.**

In some examples, the methods provide for engineered host cells that produce morphinan alkaloid products from neopinone. The conversion of neopinone to codeinone may comprise a significant step in the production of diverse morphinan alkaloid products from a simple starting material. In some examples, the simple starting material is L-tyrosine or a sugar (e.g.. glucose). The diverse alkaloid products can include, without limitation, morphinan, nor-opioid, or nal-opioid alkaloids.

In some examples, the engineered host cells are grown through a fed-batch fermentation process in which the simple starting material is fed over time and converted to the precursor morphinan alkaloid continuously over time in the engineered host cell, thereby providing a constant source of the precursor morphinan alkaloid. In some examples, the continuous source of precursor morphinan alkaloid is isomerized to the product morphinan alkaloid isomer continuously over time and then converted to the downstream alkaloid product through one or more enzymes that act on the morphinan alkaloid isomer in the engineered host cell, thereby providing a constant pull of the product isomer to the downstream alkaloid product. In some examples, the dynamic system process (e.g.. continuous supply of the precursor morphinan alkaloid and continuous conversion of the product morphinan alkaloid isomer to a downstream alkaloid product) is a beneficial component to achieving increased production of desired alkaloid products through an enhanced reversible isomerization reaction.

In some cases, the pairing of a neopinone isomerase with a COR variant exhibiting particular kinetic properties is a beneficial component to achieving increased production of desired alkaloid products in an engineered host cell. In some cases, the pairing of a neopinone isomerase with a morB variant exhibiting particular kinetic properties is a beneficial component to achieving increased production of desired alkaloid products in an engineered host cell.

Any suitable carbon source may be used as a starting material toward a morphinan alkaloid. Suitable precursors can include, without limitation, simple starting materials such as monosaccharides (e.g., glucose, fructose, galactose, xylose), oligosaccharides (e.g., lactose, sucrose, raffinose), polysaccharides (e.g.. starch, cellulose), or a combination thereof. In some examples, unpurified mixtures from renewable feedstocks can be used (e.g., cornsteep liquor, sugar beet molasses, barley malt, biomass hydrolysate). In still other embodiments, the carbon precursor can be a one-carbon compound (e.g., methanol, carbon dioxide) or a two-carbon compound (e.g., ethanol). In yet other embodiments, other carbon-containing compounds can be utilized, for example, methylamine, glucosamine, and amino acids (e.g., L-tyrosine).

In some examples, the benzylisoquinoline alkaloid product, or a derivative thereof, is recovered. In some examples, the benzylisoquinoline alkaloid product is recovered from a cell culture. In some examples, the benzylisoquinoline alkaloid product is a morphinan, nor-opioid, or nal-opioid alkaloid.

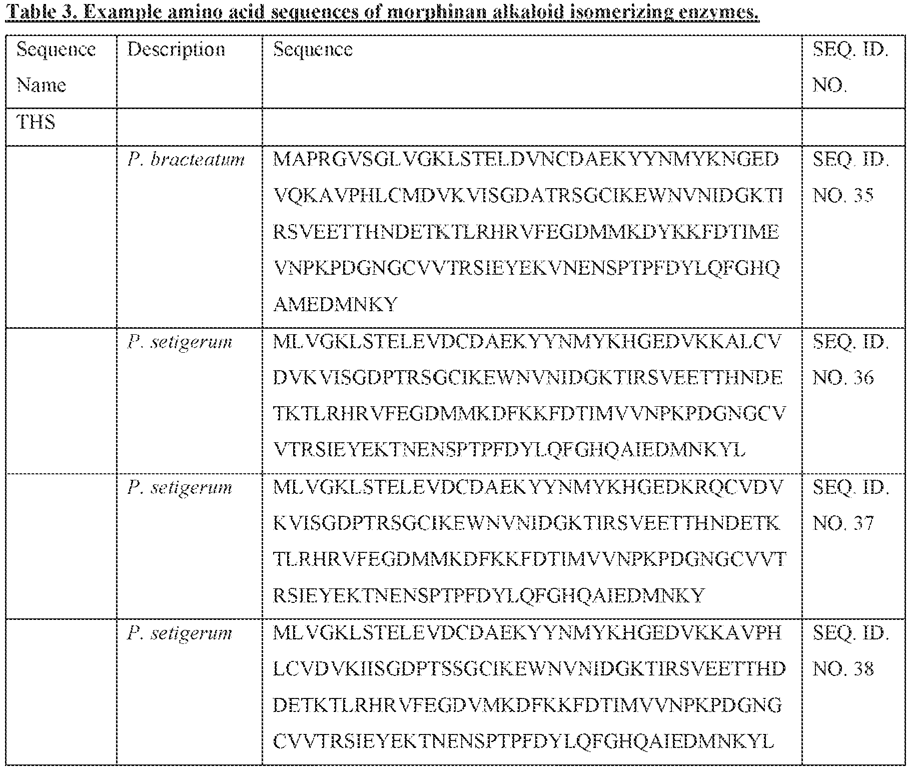

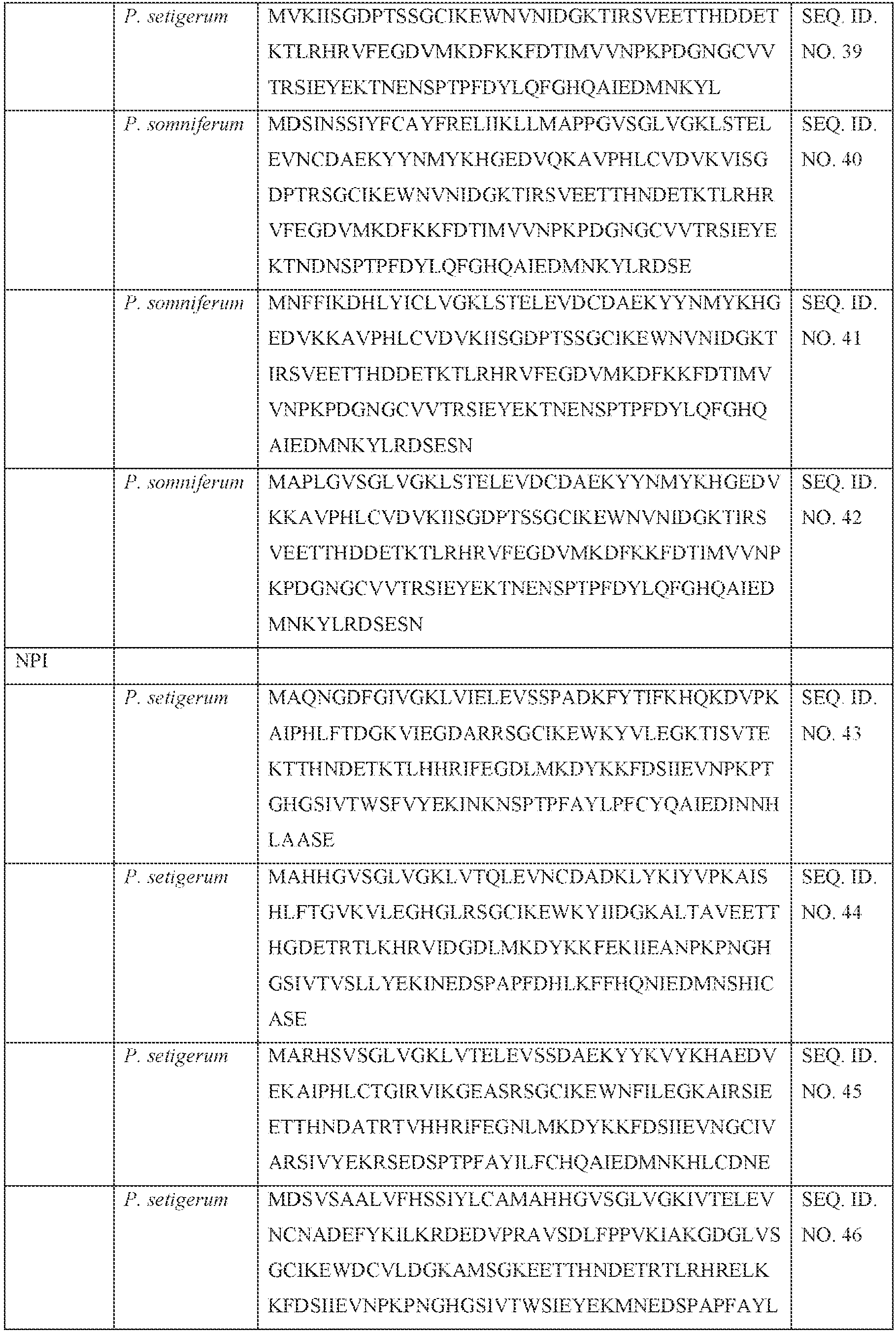

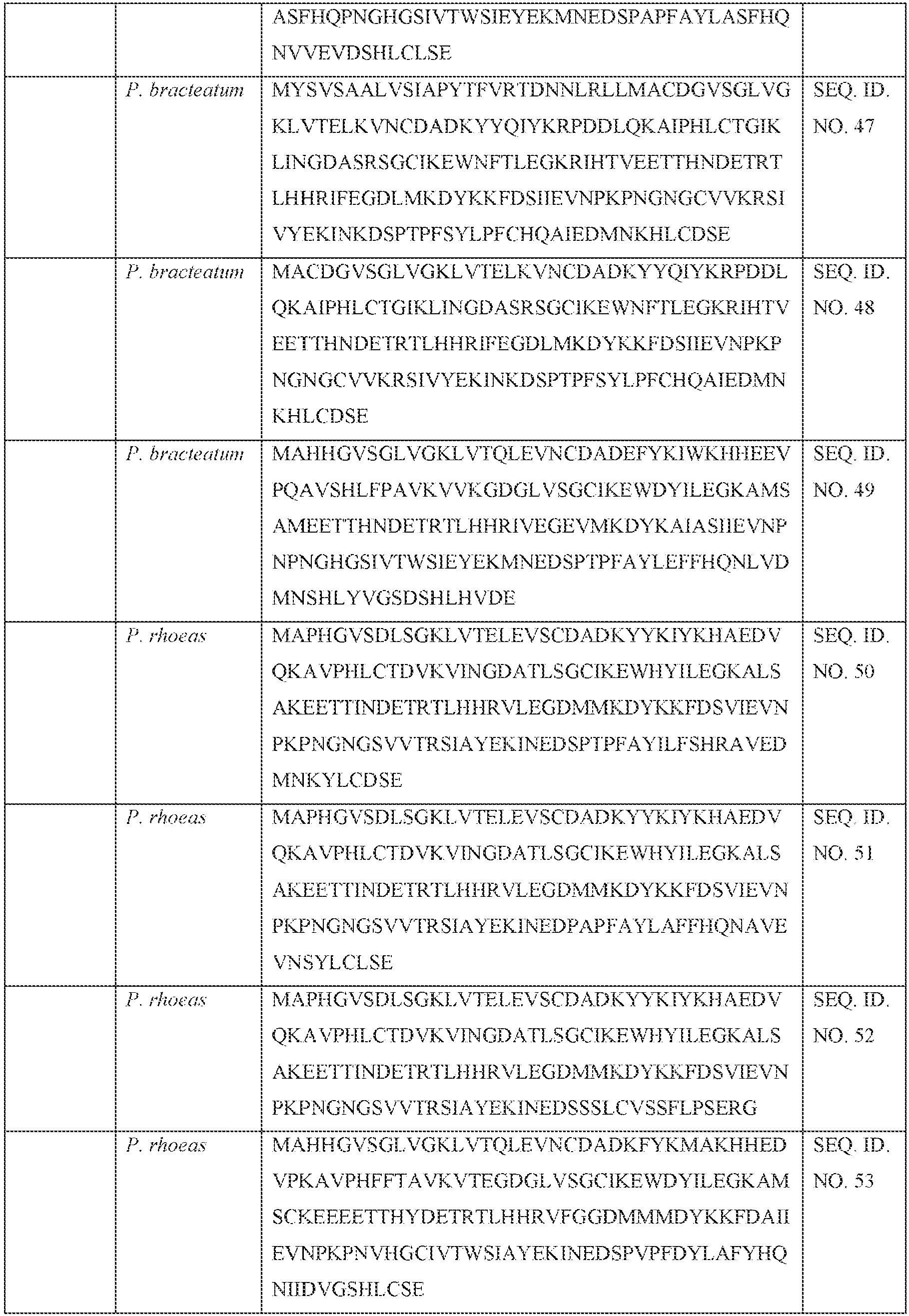

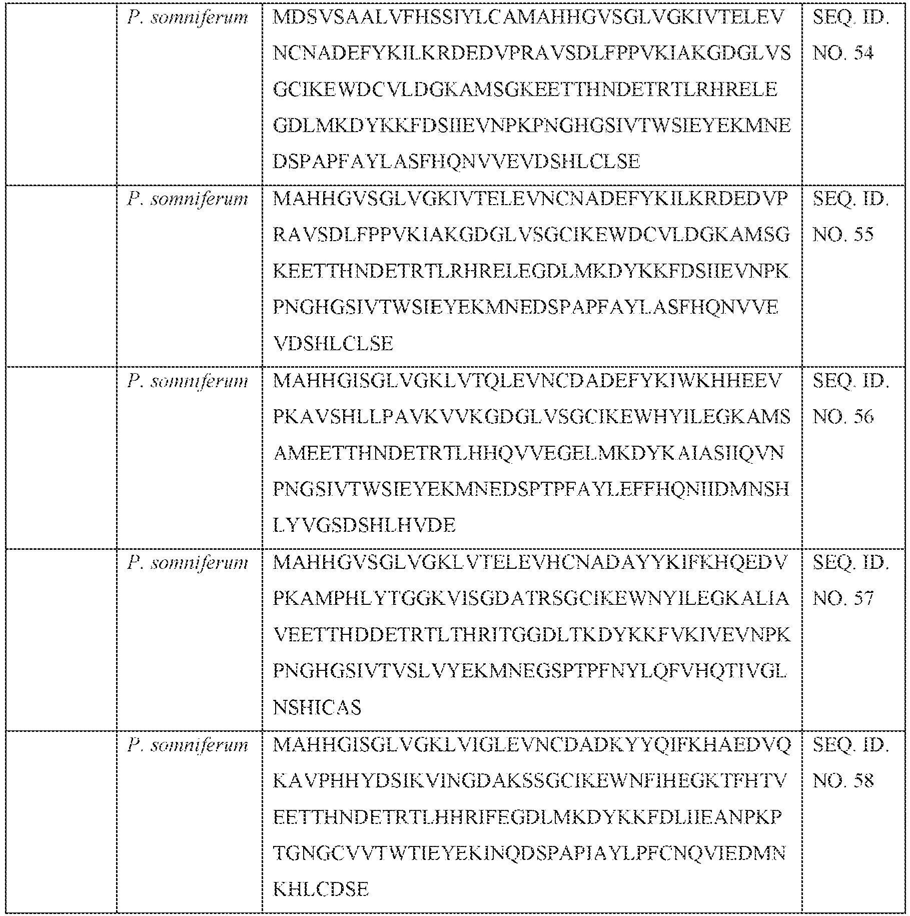

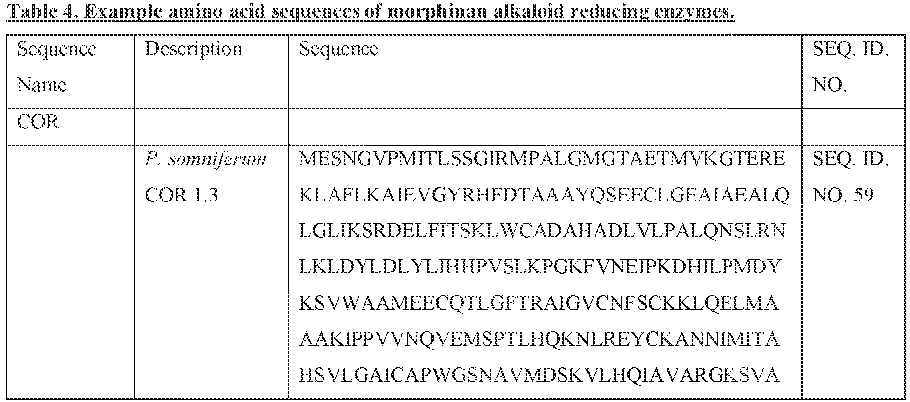

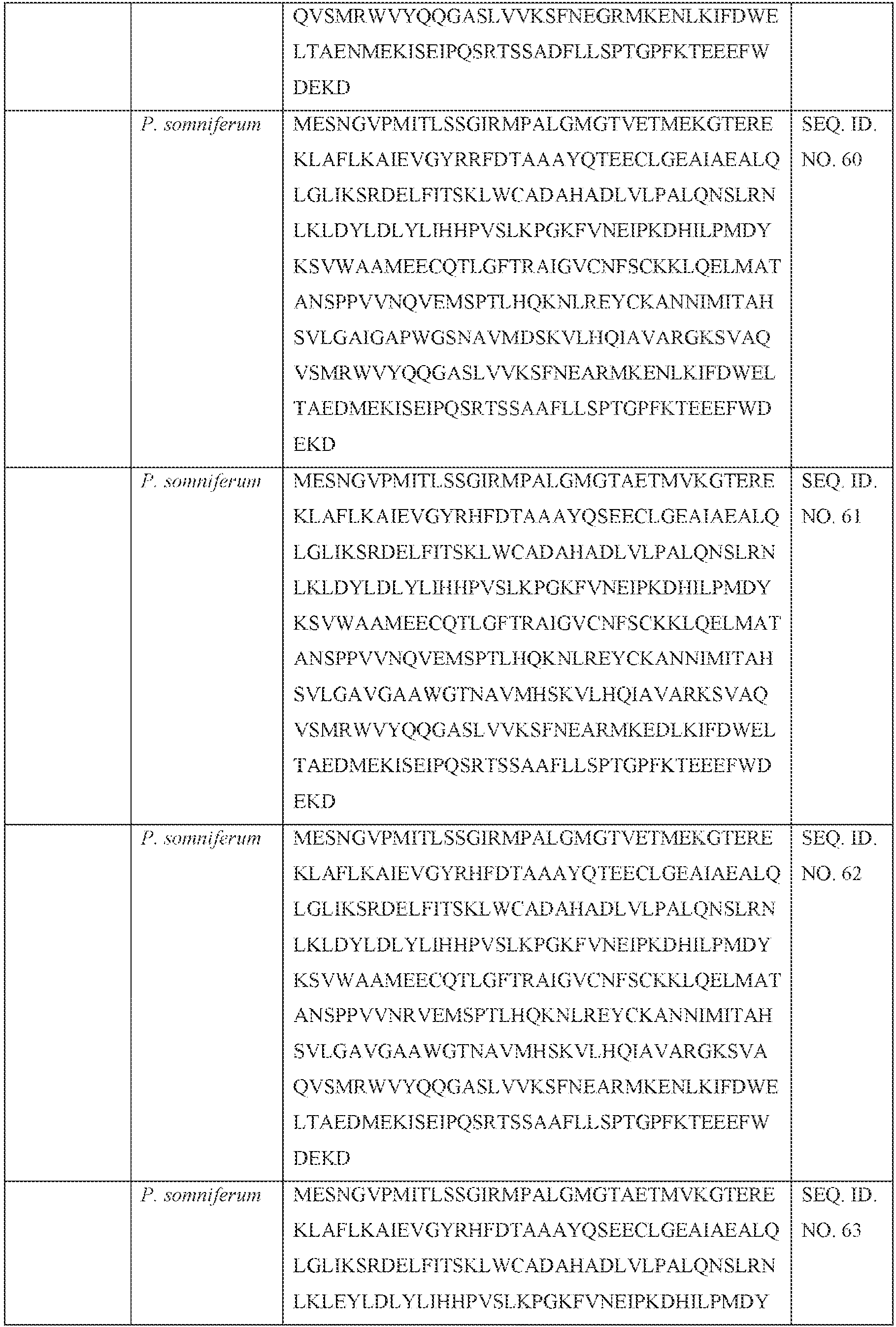

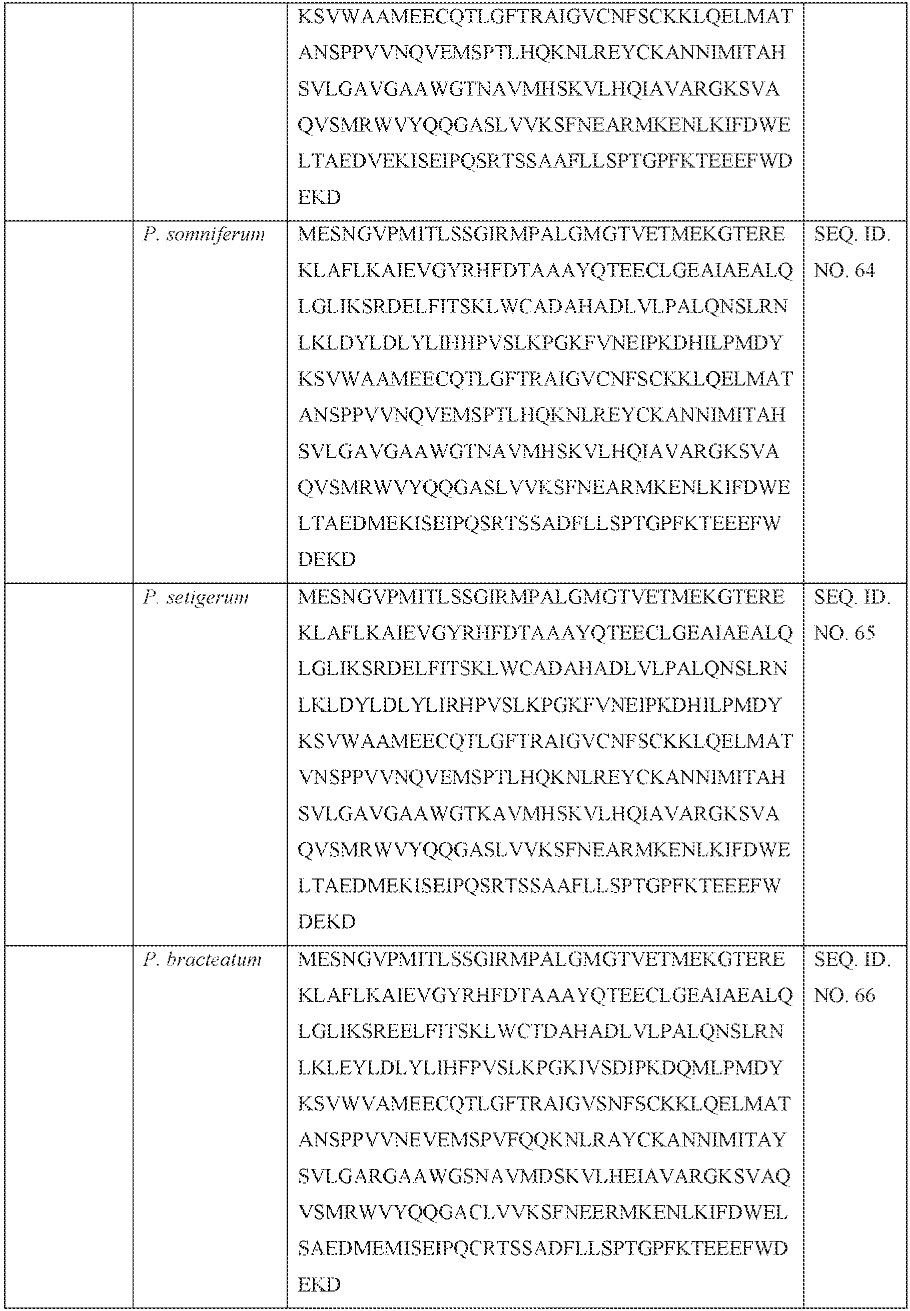

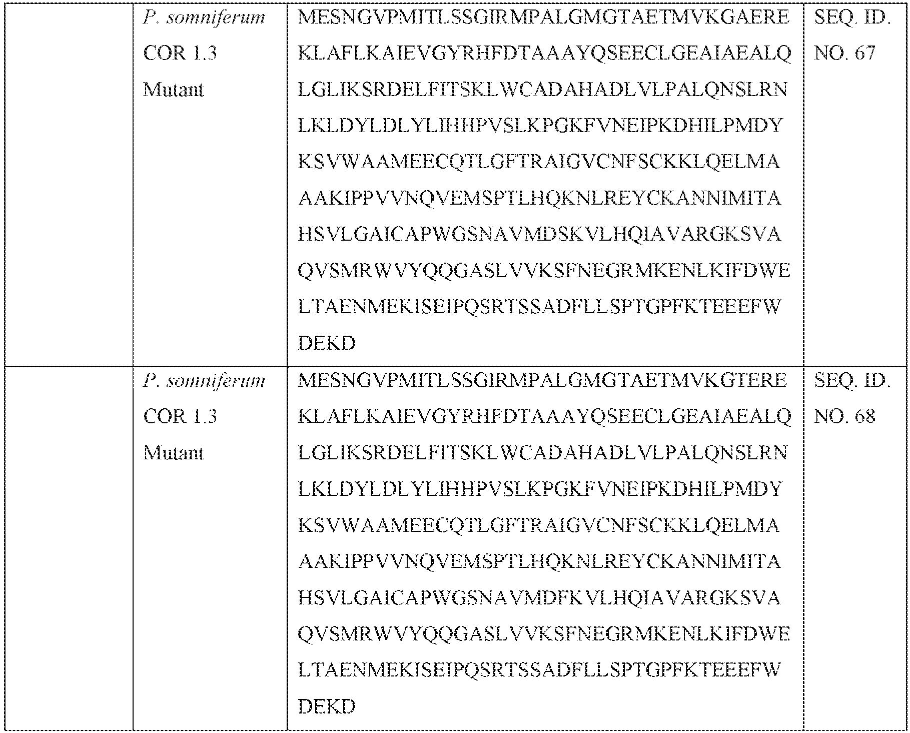

### Benzylisoquinoline Alkaloid Generating Modifications

Some methods, processes, and systems provided herein describe the conversion of BIA precursors to 1-benzylisoquinoline alkaloids. Some of these methods, processes, and systems may comprise an engineered host cell. In some examples, the production of nococlaurine, or a 1-benzylisoquinoline alkaloid, from 4-HPAA and dopamine, or BIA precursors, is described. In some examples, the production of norlaudonosoline, or a 1-benzylisoquinoline alkaloid, from 3,4-DHPA and dopamine, or BIA precursors, is described. In some examples, the conversion of BIA precursors to 1-benzylisoquinoline alkaloids is a key step in the conversion of a substrate to a diverse range of benzylisoquinoline alkaloids.

In some examples, the BIA precursors may be 4-HPAA and dopamine. In some examples, the BIA precursors may be 3,4-DHPA and dopamine. In some cases, a condensation reaction between two BIA precursors occurs between a amine of a first substrate and an aldehyde of a second substrate to generate an iminium ion followed by carbon-carbon bond formation between the C-6 of the first substrate and C-1 of the second substrate as provided in **FIG. 1** and as represented generally in **Scheme 4.** As provided in **Scheme 4.** R₁, R₂, R₃, and R₄ may be H or OH.

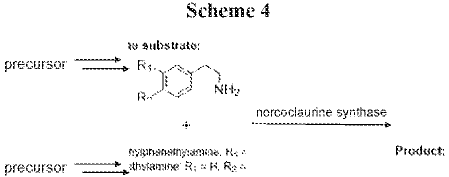

In some examples, the condensation of the BIA precursors to the 1-benzylisoquinoline alkaloid product may occur spontaneously. In some examples, the condensation reaction is promoted by conditions such as pH or solvent. In other examples, the 1-benzylisoquinoline alkaloid-generating Pictet-Spengler cyclization reaction is promoted by contact with a protein or enzyme with norcoclaurine synthase activity, or a norcoclaurine synthase. In some examples, this enzyme is a Bet v 1-fold protein. In some examples, this enzyme is an engineered norcoclaurine synthase. In some examples, this enzyme is an engineered norcoclaurine synthase with a truncation of its N-terminal sequence. In some examples, the enzyme encoding norcoclaurine synthase activity may catalyze the condensation reaction within a host cell, such as an engineered host, as described herein.

In some examples, the norcoclaurine synthase enzyme may be a Bet v 1 fold protein from plants in the *Ranunculales* order that biosynthesize thebaine, for example *P. somniferum*. In some examples, the norcoclaurine synthase enzyme may be a Bet v 1 fold protein from plants in the *Ranunculales* order that biosynthesize benzylisoquinoline alkaloids, for example *C. japonica* or *E. californica*. In some examples, the Bet v 1 protein includes the following domains in order from the N-terminus to C-terminus: a β-strand, one or two α-helices, six β-strands, and one or two α-helices. In some examples, a truncation is performed at the N-terminus of the enzyme to remove all or part of the first domain. In some examples, the enzyme may have one or more activity-increasing components as discussed herein and as described in **Examples 14, 15, and 16.** The protein is organized such that it has a Bet v 1 fold and an active site that accepts large, bulky, hydrophobic molecules, such as 1-benzylisoquinoline alkaloids. This protein may be any plant Bet v 1 protein.

In some examples, the enzyme with norcoclaurine synthase activity may be from mammals or any other vertebrate or invertebrate that biosynthesizes endogenous morphine.

In some examples, the norcoclaurine synthase may be combined with additional accessory proteins that may function to convert any BIA precursors into 1-benzylisoquinoline alkaloids. In some examples, these enzymes catalyze the reactions within a host cell, such as an engineered host, as described herein.

Examples of amino acid sequences for norcoclaurine synthases are set forth in **Table 5.** An amino acid sequence for a norcoclaurine synthase that is utilized in converting BIA precursors to 1-benzylisoquinoline alkaloid may be 75% or more identical to a given amino acid sequence as listed in **Tables 6, 7, and 8.** For example, an amino acid sequence for such a norcoclaurine synthase may comprise an amino acid sequence that is at least 75% or more, 80% or more, 81% or more, 82% or more, 83% or more, 84% or more, 85% or more. 86% or more, 87% or more, 88% or more, 89% or more, 90% or more, 91% or more, 92% or more, 93% or more. 94% or more, 95% or more, 96% or more, 97% or more, 98% or more, or 99% or more identical to an amino acid sequence as provided herein. Additionally, in certain embodiments, an "identical" amino acid sequence contains at least 80%-99% identity at the amino acid level to the specific amino acid sequence. In some cases an "identical" amino acid sequence contains at least about 85%, 86%, 87%, 88%, 89%, 90%. 91%, 92%, 93%, 94% and more in certain cases, at least 95%, 96%, 97%. 98% and 99% identity, at the amino acid level. In some cases, the amino acid sequence may be identical but the DNA sequence is altered such as to optimize codon usage for the host organism, for example.

Amino acid residues of homologous norcoclaurine synthases may be referenced according to the numbering scheme of SEQ ID NO. 70. and this numbering system is used throughout the disclosure to refer to specific amino acid residues of norcoclaurine synthases which are homologous to SEQ ID NO. 70. Norcoclaurine synthases homologous to SEQ ID NO. 70 may have at least about 70%, 75%, 80%, 85%, 90%, 95%, 97%. 98%, or 99% sequence identity to SEQ ID NO. 70. In some cases, an amino acid referred to as position 50 in a homologous norcoclaurine synthase may not be the 50^{th} amino acid in the homologous norcoclaurine synthase, but would be the amino acid which corresponds to the amino acid at position 50 in SEQ ID NO. 70 in a protein alignment of the homologous norcoclaurine synthase with SEQ ID NO. 70. In some cases, homologous enzymes may be aligned with SEQ ID NO. 70 either according to primary sequence, secondary structure, or tertiary structure.

An engineered host cell may be provided that produces an engineered norcoclaurine synthase that converts BIA precursors to 1-benzylisoquinoline alkaloid, wherein the engineered norcoclaurine synthase comprises an amino acid sequence selected from the group consisting of: SEQ ID NOs: 69, 70, 71. 72. 73, 74, 75, 76. 77. 78. 79. 80, 81, and 82, and having one or more activity-enhancing modifications as described in **Tables 6, 7, and 8.** The engineered norcoclaurine synthase that is produced within the engineered host cell may be recovered and purified so as to form a biocatalyst. In some cases, the engineered norcoclaurine synthase may have a N-terminal truncation. These engineered norcoclaurine synthase enzymes may also be used to catalyze the conversion of BIA precursors to 1-benzylisoquinoline alkaloids. Additionally, the use of an engineered norcoclaurine synthase may be used to increase the production of benzylisoquinoline alkaloid products within a cell when compared to the production of benzylisoquinoline alkaloid products within a cell utilizing a parent norcoclaurine synthase.

In additional cases, the one or more enzymes that are recovered from the engineered host cell that produces the norcoclaurine synthase may be used in a process for converting BIA precursors to a 1-benzylisoquinoline alkaloid. The process may include contacting the BIA precursors with the recovered enzymes in an amount sufficient to convert said BIA precursors to 1-benzylisoquinoline alkaloid. In examples, the BIA precursors may be contacted with a sufficient amount of the one or more enzymes such that at least 5% of said BIA precursors is converted to 1-benzylisoquinoline alkaloid. In further examples, the BIA precursors may be contacted with a sufficient amount of the one or more enzymes such that at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 82%, at least 84%, at least 86%, at least 88%, at least 90%, at least 91%, at least 92%, at least 93%, at least 94%, at least 95%, at least 96%, at least 97%, at least 98%, at least 99%, at least 99.5%, at least 99.7%, or 100% of said BIA precursors are converted to 1-benzylisoquinoline alkaloid.

In some examples, one or more enzymes converting BIA precursors to a 1-benzylisoquinoline alkaloid are localized to cellular compartments. In some examples, Bet v 1 may be modified such that it encodes targeting sequences that localize it to the endoplasmic reticulum membrane of the engineered host cell. In particular, in certain instances, the host cell may be engineered to increase production of norcoclaurine or norlaudanosoline or products for which norcoclaurine or norlaudanosline is a precursor from BIA precursors by localizing Bet v 1 to organelles in the yeast cell. Bet v 1 and/or DODC may be localized to the yeast endoplasmic reticulin in order to decrease the spatial distance between Bet v 1 and/or DODC. By increased production is meant both the production of some amount of the compound of interest where the control has no production of the compound of interest, as well as an increase of 10% or more, such as 50% or more, including 2-fold or more. e.g.. 5-fold or more, such as 10-fold or more in situations where the control has some production of the compound of interest.

In other examples. DODC and Bet v 1 may be co-localized to a single protein fusion. In some examples, the fusion is created between DODC and Bet v 1 by one of several methods, including, direct fusion, co-localization to a yeast organelle, or by enzyme co-localization tools such as leucine zippers, protein scaffolds that utilize adapter domains, or RNA scaffolds that utilize aptamers. Co-localizing the norcoclaurine synthase enzyme may facilitate substrate channeling between the active sites of the enzymes and limit the diffusion of unstable intermediates such as 4-HPAA.

In some examples, an enzyme with DODC activity is fused to a peptide with a Bet v 1 fold. In some examples, the DODC enzyme and the Bet v 1 fold protein may be fused in any order from N-terminus to C-terminus, C-terminus to N-tenninus. N-terminus to N-terminus, or C-terminus to C-terminus. In some examples, the two protein sequences may be fused directly or fused through a peptide linker region.

In some examples, an enzyme with DODC activity is fused to a peptide with a Bet v 1 fold by circular permutation. In some cases, the N- and C-tenmni of DODC are fused and the Bet v 1 sequence is then inserted randomly within this sequence. In some cases, the resulung fusion protein library is screened for 1-benzylisoquinoline alkaloid production.

The one or more enzymes that may be used to convert BIA precursors to a 1-benzylisoquinoline alkaloid may contact the BIA precursors in vitro. Additionally, or alternatively, the one or more enzymes that may be used to convert BIA precursors to a 1-benzylisoquinoline alkaloid may contact the BIA precursors in vivo. Additionally, the one or more enzymes that may be used to convert BIA precursors to a 1-benzylisoquinoline alkaloid may be provided to a cell having the BIA precursors within, or may be produced within an engineered host cell.

In some examples, the methods provide for engineered host cells that produce an alkaloid product, wherein the condensation of BIA precursors to a 1-benzylisoquinoline alkaloid product may comprise a key step in the production of an alkaloid product. In some examples, the alkaloid produced is a 1-benzylisoquinoline alkaloid. In still other embodiments, the alkaloid produced is derived from a 1-benzylisoquinoline alkaloid, including, for example. 4-nng promorphinan and 5-ring morphinan alkaloids. In another embodiment, a BIA precursor is an intermediate toward the product of the engineered host cell. In still other embodiments, the alkaloid product is selected from the group consisting of 1-benzylisoquinoline, promorphinan, morphinan, protoberberine, protopine, benzophenanthridine, secoberberine, phthalideisoquinoline, aporphine, bisbenzylisoquinoline, nal-opioid, or nor-opioid akaloids.

In some examples, the BIA precursor substrates are selected from the group consisting of 4-HPAA, 3,4-DHPA, and dopamine.

In some examples, the first BIA precursor substrate, or amine substrate, is a compound of Formula IX: 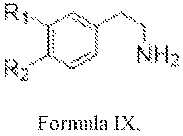 or a salt thereof, wherein:
R₁ and R₂ are independently selected from hydrogen and hydroxy.

In some other examples, R₁ and R₂ are hydroxy, and the first BIA precursor substrate is dopamine.

In some examples, the second BIA precursor substrate, or aldehyde substrate, is a compound of Formula X: 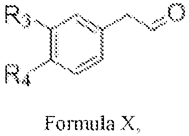 or a salt thereof, wherein:
R₃ and R₄ are independently selected from hydrogen and hydroxy.

In some examples. R₃ is a hydrogen and R₄ is a hydroxy, and the second BIA precursor is 4-HPAA.

In other examples, R₃ and R₄ are hydroxy, and the second BIA precursor is 3,4-DHPAA.

In some examples, the methods provide for engineered host cells that produce alkaloid products from BIA precursors. In some cases, the condensation of 4-HPAA and dopamine to norcoclaurine may comprise a key step in the production of diverse alkaloid products from a precursor. In some cases, the condensation of 3,4-DHPA and dopamine to norlaudanosoline may comprise a key step in the production of diverse alkaloid products from a precursor. In some examples, the precursor is L-tyrosine or a sugar (e.g., glucose). The diverse alkaloid products can include, without limitation, 1-benzylisoquinoline, promorphinan, morphinan, protoberberine, protopine, benzophenanthridine, secoberberine, phthalideisoquinoline, aporphine, bisbenzylisoquinoline, nal-opioid, and nor-opioid akaloids.

Any suitable carbon source may be used as a precursor toward a 1-benzylisoquinoline alkaloid. Suitable precursors can include, without limitation, monosaccharides (e.g.. glucose, fructose, galactose, xylose), oligosaccharides (e.g., lactose, sucrose, raffinose), polysaccharides (e.g.. starch, cellulose), or a combination thereof. In some examples, unpurified mixtures from renewable feedstocks can be used (e.g.. cornsteep liquor, sugar beet molasses, barley malt, biomass hydrolysate) In still other embodiments, the carbon precursor can be a one-carbon compound (e.g.. methanol, carbon dioxide) or a two-carbon compound (e.g., ethanol). In yet other embodiments, other carbon-containing compounds can be utilized, for example, methylamine, glucosamine, and amino acids (e.g.. L-tyrosine). In some examples, a BIA precursor substrate may be added directly to an engineered host cell of the invention, including, for example. 4-HPAA. 3.4-DHPA. and/or dopamine.

In some examples, a benzylisoquinoline alkaloid product, or a derivative thereof, is recovered. In some examples, the benzylisoquinoline alkaloid product is recovered from a cell culture. In some examples, the benzylisoquinoline alkaloid product is a 1-benzylisoquinoline, promorphinan, morphinan, protoberberine, protopine, benzophenanthridine, secoberberine, phthalideisoquinoline, aporphine, bisbenzylisoquinoline, nal-opioid, or nor-opioid akaloids.

### BisBIA Generating Modifications

Some methods, processes, and systems provided herein describe the increased production of bisbenzylisoquinoline alkaloids (bisBIAs) by utilizing two separate epimerase enzymes derived from a parent epimerase enzyme when compared to production of the bisBIAs by utilizing a corresponding fused enzyme. In some examples, a corresponding fused enzyme comprises a fused epimerase having corresponding oxidase and reductase regions to the two separate epimerase enzymes. In some examples, the two separate epimerase enzymes may comprise an oxidase and a reductase BisBIAs are dimeric molecules that may be formed by coupling reactions between two BIA monomers. In some examples, bisBIAs may be formed by carbon-oxygen coupling reactions. In other examples, bisBIAs may be formed by carbon-carbon coupling reactions. In some examples, the bisBIA dimeric molecule is a homodimer, comprising two identical BIA monomers. In some examples, an engineered host cell may produce one BIA monomer. In these examples, the BIA monomers may form homodimers when contacted with one or more coupling enzymes. In other examples, the bisBIA dimeric molecule is a heterodimer, comprising two different BIA monomers. For example, a bisBIA may be a heterodimer that comprises BIA monomers that are enantiomers of each other. In some examples, an engineered host cell may produce two or more BIA monomers. In these examples, the BIA monomers may form homodimers and heterodimers when contacted with one or more coupling enzymes.

Some of these methods, processes, and systems that describe the production of bisBIAs may comprise an engineered host cell. In some examples, the engineered host cell may be engineered to produce BIA monomers which, in turn, may be used as building block molecules for forming bisBIAs. Examples of BIA monomers that may be used to form bisBlAs include coclaurine, *N*-methylcoclaurine, laudanine, norcoclaurine, norlaudanosoline. 6-*O*-methyl-norlaudanosoline, 3'-hydroxy-*N-*methylcoclaurine, 3'-hydroxycoclaurine, reticuline, norreticuline, norlaudanine, laudanosine, and papaverine. In particular, engineered host cells may synthesize BIA monomers from norcoclaurine or norlaudanosoline by expression of heterologous enzymes including *O*-methyltransferases, *N-*methyltransferases, and 3'-hydroxylases. Examples of *O*-methyltransferases may include norcoclaurine 6-*O*-methyltransferase (6OMT). Further examples of *O*-methyltransferases may include catechol *O-*methyltransferase (COMT). Further examples of *N*-methyltransferases may include coclaurine *N-*methyltransferase (CNMT). Examples of 3'hydroxylases may include *N*-methylcoclaurine 3'-hydroxylase (CYP80B1).

The engineered host cells may produce either *(S)* or *(R)* enantiomers of various BIA monomers. Additionally or alternatively, the engineered host cells may produce a mixture of both enantiomers. The ratio of *(S)* and *(R)* enantiomers may be detennined by the substrate and product specificities of the one or more enzymes that synthesize the BIA monomers. Alternatively, the amount of each enantiomer present may be modified by the expression and engagement of the two separate oxidase and reductase enzymes of the engineered epimerase that performs the epimerization of one stereoisomer into another. In some cases, the amount of each enantiomer present may be modified by the expression and engagement of the engineered fused epimerase that performs the epimerization of one stereoisomer into another.

These BIA monomers may be fused into a dimeric bisBIA scaffold. In particular, the BIA monomers may be fused into a dimeric bisBIA scaffold utilizing one or more enzymes that are produced by the engineered host cell. Additionally or alternatively, the BIA monomers may be fused into a dimeric bisBIA scaffold utilizing one or more enzymes that are provided to the BIA monomers from a source that is external to the engineered host cell. The one or more enzymes may be used to form carbon-oxygen and/or carbon-carbon coupling reactions to fuse two BIA monomers at one, two, or three positions. In some examples, two BIA monomers may be linked by an ether bridge. In some examples, a direct carbon-carbon bond may be used to connect the two BIA monomers. In some examples, a bisBIA that is formed by fusing two BIA monomers may comprise one diphenyl ether linkage. In some examples, two BIA monomers may be fused to form a bisBIA that comprises two diphenyl ether linkages. In some examples, a bisBIA that is formed from two BIA monomers may comprise three diphenyl ether linkages. In some examples, the bisBIA may comprise one diphenyl ether linkage and one benzyl phenyl ether linkage. In some cases, the bisBIA may comprise one benzyl phenyl ether linkage and two diphenyl ether linkages.

In some examples, the BIA monomers may be contacted with a sufficient amount of the one or more enzymes that may be used to form coupling reactions to fuse two BIA monomers such that at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 82%, at least 84%, at least 86%, at least 88%, at least 90%, at least 91%, at least 92%, at least 93%, at least 94%, at least 95%, at least 96%, at least 97%, at least 98%, at least 99%, at least 99.5%, at least 99.7%, or 100% of said BIA monomers are converted to bisBIAs. The one or more enzymes that may be used to dimerize the BIA monomers into bisBIAs may contact the BIA monomers *in vitro*. Additionally, or alternatively, the one or more enzymes that may be used to dimerize the BIA monomers into bisBIAs may contact the BIA monomers *in vivo.* Additionally, the one or more bisBIA dimerizing enzyme may be expressed in a host cell that produces BIA monomers. Alternatively, the BIA monomers may be provided to the engineered host cell that expresses the bisBIA dimerizing enzyme. Alternatively, the one or more bisBIA dimerizing enzymes may be provided to a cell having BIA monomers within.

In some examples, the bisbenzylisoquinoline alkaloid is a compound of any one of Formulas Va-Vu: 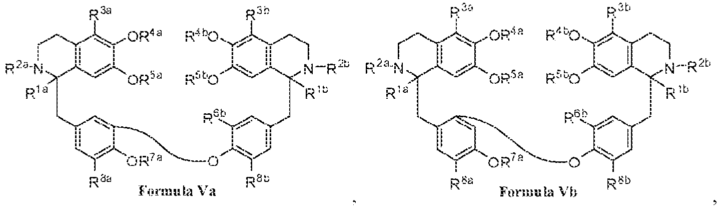 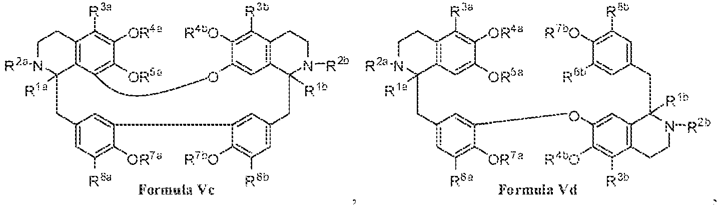 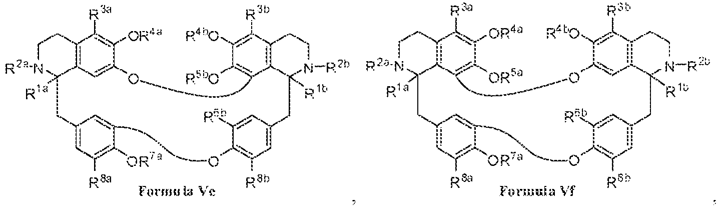 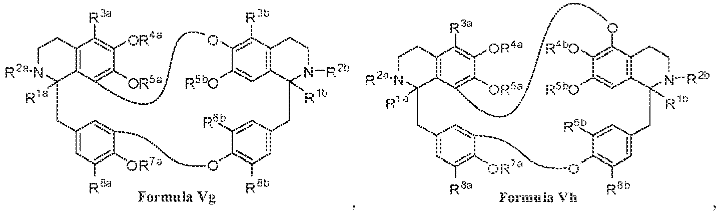 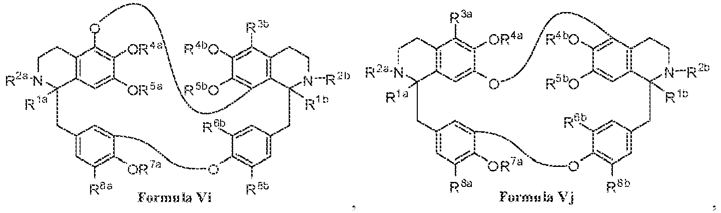 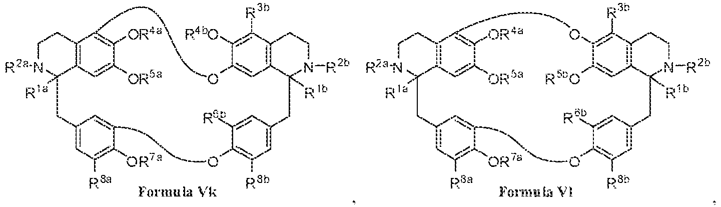 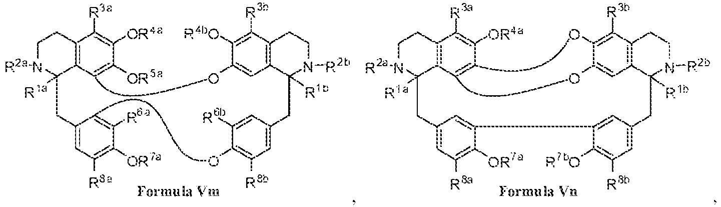 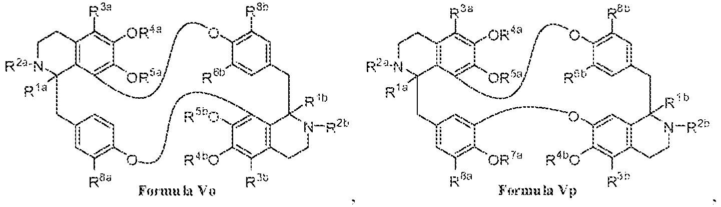 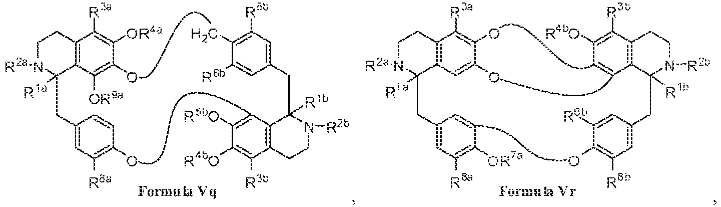 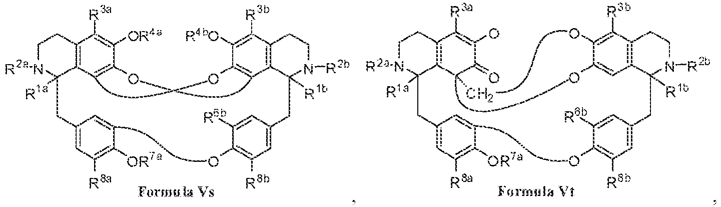 and 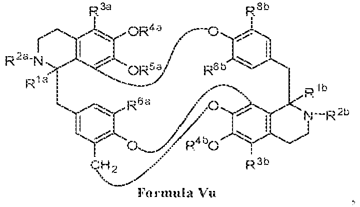 or a salt thereof, wherein:
R^{1a}, R^{1b}, R^{2a}, and R^{2b} are independently selected from hydrogen and C₁-C₄ alkyl;
R^{3a}, R^{3b}, R^{6a}, R^{6b}, R^{8a}, and R^{8b} are independently selected from hydrogen, hydroxy, fluoro, chloro, bromo, carboxaldehyde, C₁-C₄ acyl, C₁-C₄ alkyl, and C₁-C₄ alkoxy;
R^{4a} and R^{5a} are independently selected from hydrogen and C₁-C₄ alkyl, or R^{4a} and R^{5a} together form a methylene bridge:
   R^{4b} and R^{5b} are independently selected from hydrogen and C₁-C₄ alkyl, or R^{4b} and R^{5b} together form a methylene bridge; and
   R^{7a}, R^{7b}, and R^{9a} are independently selected from hydrogen and C₁-C₄ alkyl.

In some examples, R^{1a} and R^{1b} are each hydrogen: R^{2a} and R^{2b} are each methyl; R^{3a} and R^{3b} are each hydrogen; R^{4a} and R^{5a} are independently hydrogen or methyl: R^{4b} and R^{5b} are independently hydrogen or methyl, or R^{4b} and R^{5b} together form a methylene bridge: R^{6a}, R^{6b}, R^{8a}, and R^{8b} are each hydrogen, and R^{7a}, R^{7b}, and R^{9a} are independently hydrogen or methyl.

As illustrated above, the bisBIA compounds of Formulas Va. Vb, and Vd are formed by fusing two BIA monomers using a carbon-oxygen coupling reaction. Additionally, the bisBIA compounds of Formulas Vc, Vf, and Vh are formed by fusing two BIA monomers using both a carbon-oxygen coupling reaction and a carbon-carbon coupling reaction. Further, the bisBIA compounds of Formulas Ve, Vg, Vi, Vj, Vk, Vl, Vm, Vo. Vp, and Vq are formed by fusing two BIA monomers using two carbon-oxygen coupling reactions. The bisBIA compound of Formula Vn is formed by fusing two BIA monomers using two carbon-oxygen coupling reactions and a carbon-carbon coupling reaction. Additionally, the bisBIA compound of Formula Vr is formed by fusing two BIA monomers using three carbon-oxygen coupling reactions.

The one or more enzymes that may be used to form the coupling reactions may include known cytochrome P450s such as *Berberis stolonifera* CYP80A1 or similar cytochrome P450 enzymes from other plants that naturally synthesize these compounds. Alternatively, the coupling reaction may be performed by an enzyme that is not a cytochrome P450. The one or more enzymes that may be used to form the coupling reactions may be engineered to accept non-native substrates. Accordingly, the one or more enzymes that may be used to form the coupling reactions may be used to generate non-natural bisBIA molecules. In some examples, the one or more enzymes may fuse a natural BIA monomer with a non-natural BIA monomer to produce a non-natural bisBIA molecule. In other examples, the one or more enzymes may fuse two non-natural BIA monomers to produce a non-natural bisBIA molecule. Enzyme engineered strategies may be used to identify one or more enzymes that may be used to form the coupling reactions that fuse BIA monomers to produce bisBIAs. In some examples, enzyme engineering strategies may include site directed mutagenesis, random mutagenesis and screening. DNA shuffling, and screening.

Once bisBIAs are formed, the bisBIAs may be further derivatized or modified. The bisBIAs may be derivatized or modified utilizing one or more enzymes that are produced by the engineered host cell. In particular, the bisBlAs may be derivatized or modified by contacting the bisBIAs with one or more enzymes that are produced by the engineered host cell. Additionally or alternatively, the bisBlAs may be derivatized or modified by contacting the bisBlAs with one or more enzymes that are provided to the bisBlAs from a source that is external to the engineered host cell. The one or more enzymes that may be used to derivatize or modify the bisBIAs may be used to perform tailoring reactions. Examples of tailoring reactions include oxidation, reduction, *O*-methylation, *N*-methylation, *O*-demethylation, acetylation, methylenedioxybridge formation, and *O*,*O*-demethylenation. A bisBIA may be derivatized or modified using one or more tailoring reactions.

Examples of tailoring reactions are provided in **Tables 11 and 16**. In some examples, tailoring enzymes may be used to catalyze carbon-carbon coupling reactions performed on a bisBIA, or a derivative thereof. Examples of tailoring enzymes that may be used to catalyze carbon-carbon coupling reactions include a Berberine bridge enzyme (BBE) from *Papaver somniferum*, *Eschscholzia californica*, *Coptis japonica*, *Berberis stolonifer*, *Thalictrum flavum*, or another species. Salutaridine synthase (SalSyn) from *Papaver somniferum* or another species, and Corytuberine synthase (CorSyn) from *Coptis japonica* or another species. Non-linuting examples of reactions that can be catalyzed by tailoring enzymes are shown in **Scheme 5,** wherein R^{a}, R^{b}, R^{c}, and R^{d} are independently selected from hydrogen, hydroxy, fluoro, chloro, bromo, carboxaldehyde, C₁-C₄ acyl, C₁-C₄ alkyl, and C₁-C₄ alkoxy. In some examples, R^{a}, R^{b}, and the carbon atoms to which they are attached optionally form a carbocycle or heterocycle. In some examples, R^{c}, R^{d}, and the carbon atoms to which they are attached optionally form a carbocycle or heterocycle.

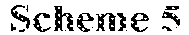

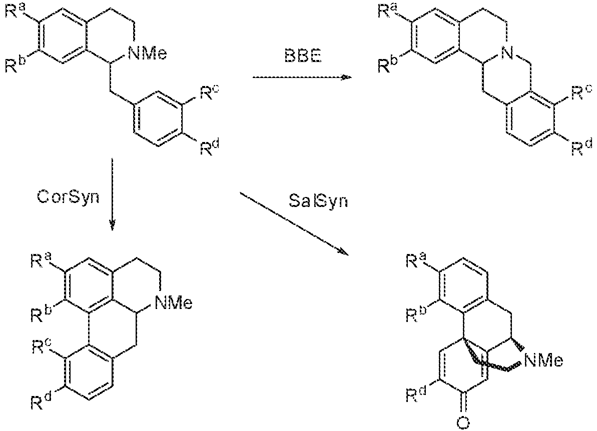

In some examples, tailoring enzymes may be used to catalyze oxidation reactions performed on a bisBIA, or a derivative thereof. Examples of tailoring enzymes that may be used to catalyze oxidation reactions include a Tetrahydroprotoberberine oxidase (STOX) from *Coptis japonica*, *Argemone mexicana*, *Berberis wilsonae*, or another species: Dihydrobenzophenanthridine oxidase (DBOX) from *Papaver somniferum* or another species; Methylstylopine hydroxylase (MSH) from *Papaver somniferum* or another species; and Protopine 6-hydroxylase (P6H) from *Papaver somniferum*, *Eschscholzia californica*, or another species.

Tailoring enzymes may also be used to catalyze methylenedioxy bridge formation reactions performed on a bisBIA. or a derivative thereof. Examples of tailoring enzymes that may be used to catalyze methylenedioxy bridge formation reactions include a Stylopine synthase (StySyn) from *Papaver somniferum*, *Eschscholzia californica*, *Argemone mexicana*, or another species; Cheilanthifoline synthase (CheSyn) from *Papaver somniferum*, *Eschscholzia californica*, *Argemone mexicana*, or another species: and Canadine synthase (CAS) from *Thalictrum flavum*, *Coptis chinensis*, or another species.

In other examples, tailoring enzymes may be used to catalyze *O*-methylation reactions performed on a bisBIA, or a derivative thereof. Examples of tailoring enzymes that may be used to catalyze *O*-methylation reactions include a Norcoclaurine 6-*O*-methyltransferase (6OMT) from *Papaver somniferum*, *Thalictrum flavum*, *Coptis japonica*, *Papaver bracteatum*, or another species; 3'hydroxy-*N-*methylcoclaurine 4'-*O*-methyltransferase (4'OMT) from *Papaver somniferum*, *Thalictrum flavum*, *Coptis japonica, Coptis chinensis*, or another species: Reticuline 7-*O*-methyltransferase (7OMT) from *Papaver somniferum*, *Eschscholzia californica*, or another species: and Scoulerine 9-*O*-methyltransferase (9OMT) from *Papaver somniferum*, *Thalictrum flavum*, *Coptis japonica*, *Coptis chinensis*, or another species.

Additionally, tailoring enzymes may be used to catalyze *N*-methylation reactions performed on a bisBIA, or a derivative thereof. Examples of tailoring enzymes that may be used to catalyze *N-*methylation reactions include Coclaurine *N*-methyltransferase (CNMT) from *Papaver somniferum*, *Thalictrum flavum*, *Coptis japonica*, or another species: Tetrahydroprotoberberine N-methyltransferase (TNMT) from *Papaver somniferum*, *Eschscholzia californica*, *Papaver bracteatum*, or another species.

Further, tailoring enzymes may be used to catalyze *O*-demethylation reactions performed on a bisBIA. or a derivative thereof. Examples of tailoring enzymes that may be used to catalyze *O-*demethylation reactions include Thebaine demethylase (T6ODM) from *Papaver somniferum* or another species; and Codeine demethylase (CODM) from *Papaver somniferum*, or another species.

Tailoring enzymes may also be used to catalyze reduction reactions performed on a bisBIA, or a derivative thereof. Examples of tailoring enzymes that may be used to catalyze reduction reactions include Salutaridine reductase (SalR) from *Papaver somniferum*, *Papaver bracteatum*, or another species; Codeinone reductase (COR) from *Papaver somniferum* or another species; and Sanguinarine reductase (SanR) from *Eschscholzia californica* or another species. In other examples, tailoring enzymes may be used to catalyze acetylation reactions performed on a bisBIA, or a derivative thereof. An example of a tailoring enzyme that may be used to catalyze acetylation reactions includes Salutaridine acetyltransferase (Sa1AT) from *Papaver somniferum* or another species.

### O-Demethylation Modifications

Some methods, processes, and systems provided herein describe the conversion of a first benzylisoquinoline alkaloid to a second benzylisoquinoline alkaloid by the removal of an *O*-linked methyl group. Some of these methods, processes, and systems may comprise an engineered host cell. In some examples, the conversion of a first benzylisoquinoline alkaloid to a second benzylisoquinoline alkaloid is a key step in the conversion of a substrate to a nor-opioids or nal-opioids. In some examples, the conversion of a first alkaloid to a second alkaloid comprises a demethylase reaction.

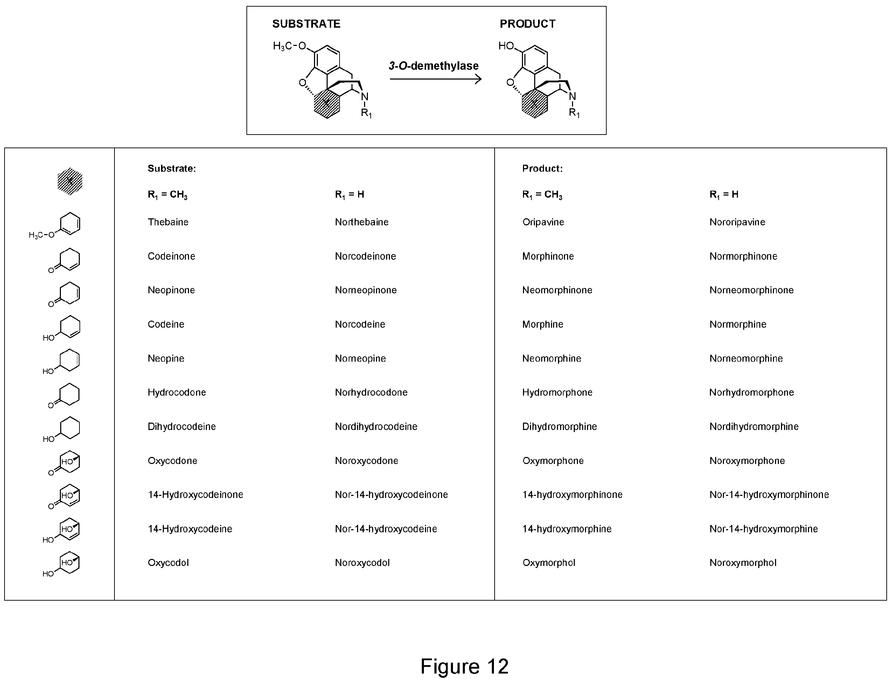

**FIG. 12** illustrates an enzyme having opioid 3-*O*-demethylase (ODM) activity, in accordance with some embodiments of the invention. Specifically, the enzy me may act on morphinan alkaloid structures to remove the methyl group from the oxygen bound to carbon 3.

Examples of amino acid sequences of ODM enzymes are set forth in **Table 12.** An amino acid sequence for an ODM that is utilized in converting a first alkaloid to a second alkaloid may be 50% or more identical to a given amino acid sequence as listed in **Table 12**. For example, an amino acid sequence for such an epimerase may comprise an amino acid sequence that is at least 50% or more, 55% or more, 60% or more. 65% or more. 70% or more, 75% or more. 80% or more. 81% or more, 82% or more, 83% or more, 84% or more, 85% or more, 86% or more, 87% or more, 88% or more, 89% or more, 90% or more, 91% or more, 92% or more. 93% or more. 94% or more. 95% or more, 96% or more, 97% or more, 98% or more, or 99% or more identical to an amino acid sequence as provided herein. Additionally, in certain embodiments, an "identical" amino acid sequence contains at least 80%-99% identity at the amino acid level to the specific amino acid sequence. In some cases an "identical" amino acid sequence contains at least about 85%. 86%, 87%, 88%, 89%, 90%, 91%, 92%, 93%, 94% and more in certain cases, at least 95%, 96%, 97%, 98% and 99% identity, at the amino acid level. In some cases, the amino acid sequence may be identical but the DNA sequence is altered such as to optimize codon usage for the host organism, for example.

An engineered host cell may be provided that produces an ODM that converts a first alkaloid to a second alkaloid, wherein the ODM comprises a given amino acid sequence as listed in **Table 12.** An engineered host cell may be provided that produces one or more ODM enzymes. The ODM that is produced within the engineered host cell may be recovered and purified so as to form a biocatalyst. The process may include contacting the first alkaloid with an ODM in an amount sufficient to convert said first alkaloid to a second alkaloid. In some examples, the first alkaloid may be contacted with a sufficient amount of the one or more enzymes such that at least 5% of said first alkaloid is converted to a second alkaloid. In further examples, the first alkaloid may be contacted with a sufficient amount of the one or more enzymes such that at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 82%, at least 84%, at least 86%, at least 88%, at least 90%, at least 91%, at least 92%, at least 93%, at least 94%, at least 95%, at least 96%, at least 97%, at least 98%, at least 99%, at least 99.5%, at least 99.7%, or 100% of said first alkaloid is converted to a second alkaloid.

The one or more enzymes that may be used to convert a first alkaloid to a second alkaloid may contact the first alkaloid in vitro. Additionally, or alternatively, the one or more enzymes that may be used to convert a first alkaloid to a second alkaloid may contact the first alkaloid in vivo. In some examples, the one or more enzymes that may be used to convert a first alkaloid to a second alkaloid may be provided to a cell having the first alkaloid within. In some examples, the one or more enzymes that may be used to convert a first alkaloid to a second alkaloid may be produced within an engineered host cell.

In some examples, the methods provide for engineered host cells that produce an alkaloid product, wherein the *O*-demethylation of a substrate to a product may comprise a key step in the production of an alkaloid product. In some examples, the alkaloid produced is a nor-opioid or a nal-opioid. In still other embodiments, the alkaloid produced is derived from a nor-opioid or a nal-opioid. In another embodiment, a first alkaloid is an intermediate toward the product of the engineered host cell. In still other embodiments, the alkaloid product is selected from the group consisting of morphine, oxymorphine, oripavine, hydromorphone, dihydromorphine, 14-hydrogmorphine, morphinone, and 14-hydroxymorphinone.

In some examples, the substrate alkaloid is an opioid selected from the group consisting of codeine, oxycodone, thebaine, hydrocodone, dihydrocodeine, 14-hydroxycodeine, codeinone, and 14-hydroxycodeinone.

### N-Demethylation Modifications

Some methods, processes, and systems provided herein describe the conversion of a first alkaloid to a second alkaloid by the removal of an *N*-linked methyl group. Some of these methods. processes, and systems may comprise an engineered host cell. In some examples, the conversion of a first alkaloid to a second alkaloid is a key step in the conversion of a substrate to a nor-opioids or nal-opioids. In some examples, the conversion of a first alkaloid to a second alkaloid comprises a demethylase reaction.

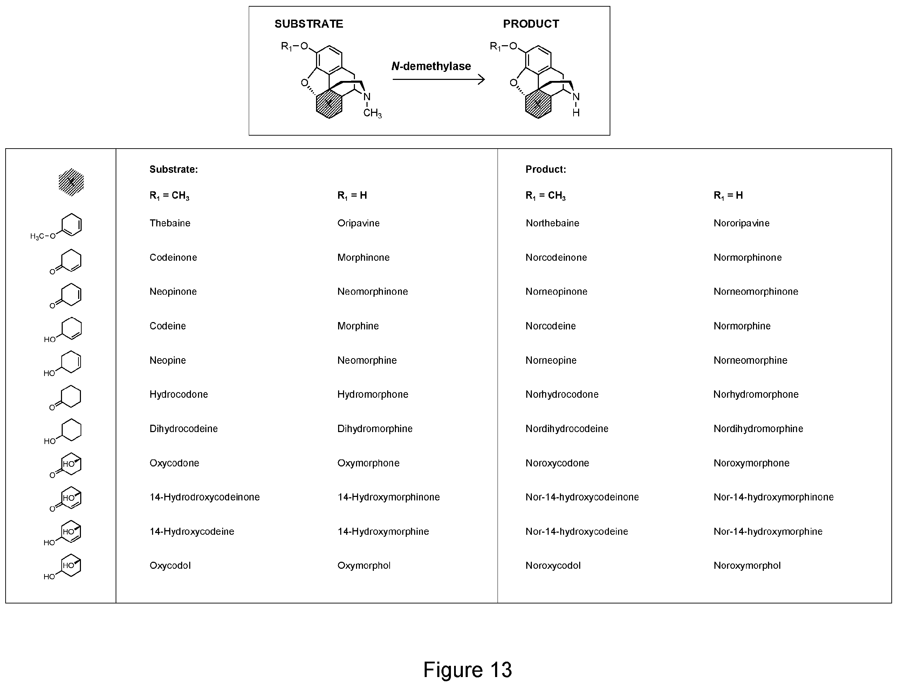

**FIG. 13** illustrates an enzyme having opioid *N*-demethylase activity, in accordance with some embodiments of the invention. Specifically, the enzyme may act on morphinan alkaloid structures to remove the methyl group from the nitrogen.

Examples of an amino acid sequence of an *N*-demethylase (NDM) enzyme that may be used to perform the conversion a first alkaloid to a second alkaloid are provided in **Table 13.** An amino acid sequence for an NDM that is utilized in converting a first alkaloid to a second alkaloid may be 50% or more identical to a given amino acid sequence as listed in **Table 13.** For example, an amino acid sequence for such an epimerase may comprise an amino acid sequence that is at least 50% or more, 55% or more, 60% or more, 65% or more, 70% or more, 75% or more, 80% or more, 81% or more, 82% or more, 83% or more. 84% or more. 85% or more. 86% or more. 87% or more. 88% or more. 89% or more. 90% or more. 91% or more. 92% or more. 93% or more, 94% or more, 95% or more, 96% or more, 97% or more. 98% or more, or 99% or more identical to an amino acid sequence as provided herein. Additionally, in certain embodiments, an "identical" amino acid sequence contains at least 80%-99% identity at the amino acid level to the specific amino acid sequence. In some cases an "identical" amino acid sequence contains at least about 85%, 86%, 87%, 88%. 89%, 90%, 91%, 92%. 93%, 94% and more in certain cases, at least 95%, 96%, 97%, 98% and 99% identity, at the amino acid level. In some cases, the amino acid sequence may be identical but the DNA sequence is altered such as to optimize codon usage for the host organism, for example.

An engineered host cell may be provided that produces an NDM that converts a first alkaloid to a second alkaloid, wherein the NDM comprises an amino acid sequence as listed in Table 13. An engineered host cell may be provided that produces one or more NDM enzymes. The NDM that is produced within the engineered host cell may be recovered and purified so as to form a biocatalyst. The process may include contacting the first alkaloid with an NDM in an amount sufficient to convert said first alkaloid to a second alkaloid. In some examples, the first alkaloid may be contacted with a sufficient amount of the one or more enzymes such that at least 5% of said first alkaloid is converted to a second alkaloid. In further examples, the first alkaloid may be contacted with a sufficient amount of the one or more enzymes such that at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 82%, at least 84%, at least 86%, at least 88%, at least 90%, at least 9 1%, at least 92%, at least 93%, at least 94%, at least 95%, at least 96%, at least 97%, at least 98%, at least 99%, at least 99.5%, at least 99.7%, or 100% of said first alkaloid is converted to a second alkaloid.

The one or more enzymes that may be used to convert a first alkaloid to a second alkaloid may contact the first alkaloid in vitro. Additionally, or alternatively, the one or more enzymes that may be used to convert a first alkaloid to a second alkaloid may contact the first alkaloid in vivo. In some examples, the one or more enzymes that may be used to convert a first alkaloid to a second alkaloid may be provided to a cell having the first alkaloid within. In some examples, the one or more enzymes that may be used to convert a first alkaloid to a second alkaloid may be produced within an engineered host cell.

In some examples, the methods provide for engineered host cells that produce an alkaloid product, wherein the *N*-demethylation of a substrate to a product may comprise a key step in the production of an alkaloid product. In some examples, the alkaloid produced is a nor-opioid or a nal-opioid. In still other embodiments, the alkaloid produced is derived from a nor-opioid or a nal-opioid. In another embodiment, a first alkaloid is an intermediate toward the product of the engineered host cell. In still other embodiments, the alkaloid product is selected from the group consisting of norcodeine, noroxycodone, northebaine, norhydrocodone, nordihydro-codeine, nor-14-hydroxy-codeine, norcodeinone, nor-14-hydroxy-codeinone, normorphine, noroxymorphone, nororipavine, norhydro-morphone, nordihydro-morphine, nor-14-hydroxy-morphine, normorphinone, and nor-14-hydroxy-morphinone.

In some examples, the substrate alkaloid is an opioid selected from the group consisting of codeine, oxycodone, thebaine, hydrocodone, dihydrocodeine. 14-hydroxycodeine, codeinone, 14-hydroxycodeinone, morphine, oxymorphone, oripavine, hydromorphone, dihydromorphine, 14-hydroxy-morphine, morphinone, and 14-hydroxy-morphinone.

### N-Methyltransferase Modifications

Some methods, processes, and systems provided herein describe the conversion of a first alkaloid to a second alkaloid by the addition of an *N*-linked sidechain group. Some methods, processes, and systems provided herein describe the conversion of a first alkaloid to a second alkaloid by the transfer of a sidechain group from a cosubstrate to the first alkaloid. Some of these methods, processes, and systems may comprise an engineered host cell. In some examples, the conversion of a first alkaloid to a second alkaloid is a key step in the conversion of a substrate to a nal-opioid. In some examples, the conversion of a first alkaloid to a second alkaloid comprises a methyltransferase reaction.

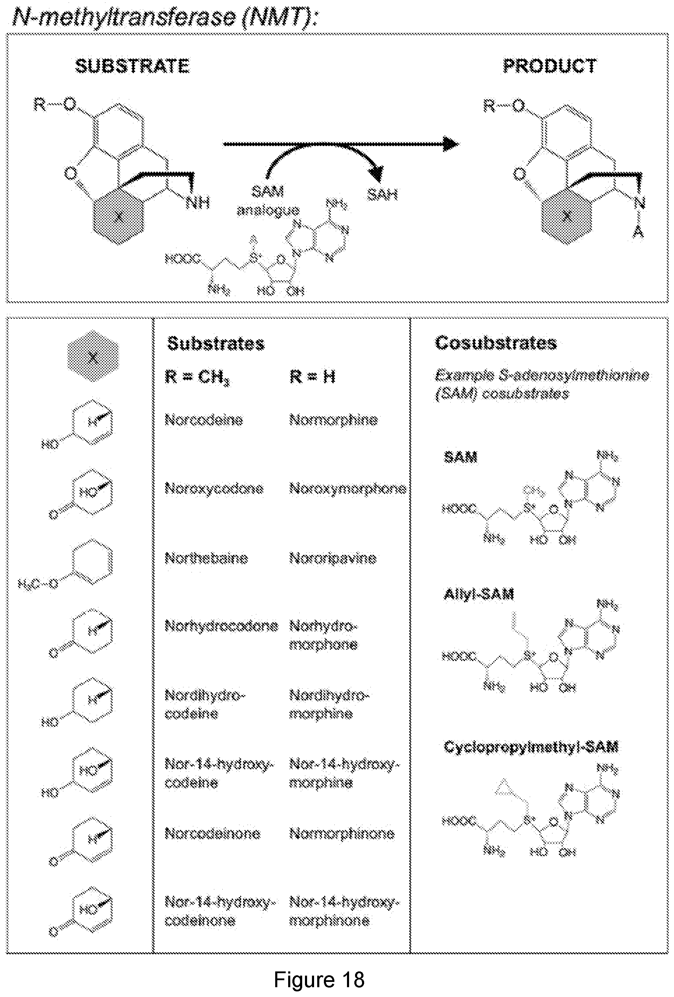

**FIG. 18** illustrates an enzyme having N-methy ltransferase (NMT) activity, in accordance with some embodiments of the invention. Specifically, the enzyme may act on morphinan alkaloid structures to add a methyl group or other carbon moiety to the nitrogen. S-Adenosyl methionine (SAM) may act as the donor of the functional group (methyl, allyl, cyclopropylmethyl, or other).

Examples of amino acid sequences of NMT enzymes are set forth in **Table 14.** An amino acid sequence for an NMT that is utilized in converting a first alkaloid to a second alkaloid may be 50% or more identical to a given amino acid sequence as listed in **Table 14.** For example, an amino acid sequence for such an epimerase may comprise an amino acid sequence that is at least 50% or more. 55% or more, 60% or more, 65% or more, 70% or more, 75% or more. 80% or more. 81% or more, 82% or more, 83% or more, 84% or more, 85% or more. 86% or more, 87% or more, 88% or more, 89% or more, 90% or more, 91% or more, 92% or more, 93% or more, 94% or more, 95% or more, 96% or more, 97% or more, 98% or more, or 99% or more identical to an amino acid sequence as provided herein. Additionally, in certain embodiments, an "identical" amino acid sequence contains at least 80%-99% identity at the amino acid level to the specific amino acid sequence. In some cases an "identical" amino acid sequence contains at least about 85%. 86%. 87%, 88%, 89%, 90%. 91%, 92%, 93%, 94% and more in certain cases, at least 95%, 96%, 97%. 98% and 99% identity, at the amino acid level. In some cases, the amino acid sequence may be identical but the DNA sequence is altered such as to optimize codon usage for the host organism, for example.

An engineered host cell may be provided that produces an NMT that converts a first alkaloid to a second alkaloid, wherein the NMT comprises an amino acid sequence as provided in **Table 14.** An engineered host cell may be provided that produces one or more NMT enzymes. The NMT that is produced within the engineered host cell may be recovered and purified so as to form a biocatalyst. The process may include contacting the first alkaloid with an NMT in an amount sufficient to convert said first alkaloid to a second alkaloid. In some examples, the first alkaloid may be contacted with a sufficient amount of the one or more enzymes such that at least 5% of said first alkaloid is converted to a second alkaloid. In further examples, the first alkaloid may be contacted with a sufficient amount of the one or more enzymes such that at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 82%, at least 84%, at least 86%, at least 88%, at least 90%, at least 91%, at least 92%, at least 93%, at least 94%, at least 95%, at least 96%, at least 97%, at least 98%, at least 99%, at least 99.5%, at least 99.7%, or 100% of said first alkaloid is converted to a second alkaloid.

The one or more enzymes that may be used to convert a first alkaloid to a second alkaloid may contact the first alkaloid in vitro. Additionally, or alternatively, the one or more enzymes that may be used to convent a first alkaloid to a second alkaloid may contact the first alkaloid in vivo. In some examples, the one or more enzymes that may be used to convert a first alkaloid to a second alkaloid may be provided to a cell having the first alkaloid within. In some examples, the one or more enzymes that may be used to convert a first alkaloid to a second alkaloid may be produced within an engineered host cell.

In some examples, the methods provide for engineered host cells that produce an alkaloid product, wherein the *N*-methyltransferase of a substrate to a product may comprise a key step in the production of an alkaloid product. In some examples, the alkaloid produced is a nal-opioid. In still other embodiments, the alkaloid produced is derived from a nor-opioid or a nal-opioid. In another embodiment, a first alkaloid is an intermediate toward the product of the engincered host cell. In still other embodiments, the alkaloid product is selected from the group including naloxone, naltrexone, and nalmefene.

In some examples, the substrate alkaloid is an opioid selected from the group consisting of norcodeine, noroxycodone, northebaine, norhydrocodone, nordihydro-codeine, nor-14-hydroxy-codeine, norcodeinone, nor-14-hydroxy-codeinone, normorphine, noroxymorphone, nororipavine, norhydro-morphone, nordihydro-morphine, nor-14-hydroxy-morphine, normorphinone, and nor-14-hydroxy-morphinone. In some examples, the cosubstrate is S-adenosylmethionine, allyl-S-adenosylmethionine, or cyclopropylmethyl-S-adenosylmethionine.

### Heterologous Coding Sequences

In some instances, the engineered host cells harbor one or more heterologous coding sequences (such as two or more, three or more, four or more, five or more) which encode activity(ies) that enable the engineered host cells to produce desired enzymes of interest and/or BIAs of interest, e.g., as described herein. As used herein, the term "heterologous coding sequence" is used to indicate any polynucleotide that codes for, or ultimately codes for, a peptide or protein or its equivalent amino acid sequence. e.g., an enzyme, that is not normally present in the host organism and may be expressed in the host cell under proper conditions. As such. "heterologous coding sequences" includes multiple copies of coding sequences that are normally present in the host cell, such that the cell is expressing additional copies of a coding sequence that are not normally present in the cells. The heterologous coding sequences may be RNA or any type thereof, e.g.. mRNA. DNA or any type thereof. e.g., cDNA, or a hybrid of RNA/DNA. Coding sequences of interest include, but are not limited to, full-length transcription units that include such features as the coding sequence, introns, promoter regions. 3'-UTRs, and enhancer regions.

In some examples, the engineered host cells may compnse a plurality of heterologous coding sequences each encoding an enzyme, such as an enzyine listed in Table 11. In some examples, the plurality of enzymes encoded by the plurality of heterologous coding sequences may be distinct from each other. In some examples, some of the plurality of enzymes encoded by the plurality of heterologous coding sequences may be distinct from each other and some of the plurality of enzymes encoded by the plurality of heterologous coding sequences may be duplicate copies.

In some examples, the heterologous coding sequences may be operably connected. Heterologous coding sequences that are operably connected may be within the same pathway of producing a particular benzylisoquinoline alkaloid product and/or epimerase product. In some examples, the operably connected heterologous coding sequences may be directly sequential along the pathway of producing a particular benzylisoquinoline alkaloid product and/or epimerase product. In some examples, the operably connected heterologous coding sequences may have one or more native enzymes between one or more of the enzymes encoded by the plurality of heterologous coding sequences. In some examples, the heterologous coding sequences may have one or more heterologous enzymes between one or more of the enzymes encoded by the plurality of heterologous coding sequences. In some examples, the heterologous coding sequences may have one or more non-native enzymes between one or more of the enzymes encoded by the plurality of heterologous coding sequences

The engineered host cells may also be modified to possess one or more genetic alterations to accommodate the heterologous coding sequences. Alterations of the native host genome include, but are not limited to, modifying the genome to reduce or ablate expression of a specific protein that may interfere with the desired pathway. The presence of such native proteins may rapidly convert one of the intermediates or final products of the pathway into a metabolite or other compound that is not usable in the desired pathway. Thus, if the activity of the native enzyme were reduced or altogether absent, the produced intermediates would be more readily available for incorporation into the desired product.

Heterologous coding sequences include but are not limited to sequences that encode enzymes, either wild-type or equivalent sequences, that are normally responsible for the production of BIAs of interest in plants. In some cases, the enzymes for which the heterologous sequences code may be any of the enzymes in the 1-benzylisoquinoline alkaloid pathway, and may be from any convenient source. The choice and number of enzymes encoded by the heterologous coding sequences for the particular synthetic pathway may be selected based upon the desired product. In certain embodiments, the host cells of the invention may include 1 or more. 2 or more. 3 or more, 4 or more, 5 or more, 6 or more, 7 or more, 8 or more, 9 or more, 10 or more. 11 or more. 12 or more, 13 or more, 14 or more, or even 15 or more heterologous coding sequences, such as 1, 2. 3. 4. 5. 6, 7, 8. 9. 10. 11, 12, 13, 14, or 15 heterologous coding sequences.

As used herein, the term "heterologous coding sequences" also includes the coding portion of the peptide or enzyme, i.e.. the cDNA or mRNA sequence, of the peptide or enzyme, as well as the coding portion of the full-length transcriptional unit, i.e., the gene including introns and exons, as well as "codon optimized" sequences, truncated sequences or other forms of altered sequences that code for the enzyme or code for its equivalent amino acid sequence, provided that the equivalent amino acid sequence produces a functional protein. Such equivalent amino acid sequences may have a deletion of one or more amino acids, with the deletion being N-terminal, C-terminal, or internal. Truncated forms are envisioned as long as they have the catalytic capability indicated herein. Fusions of two or more enzymes are also envisioned to facilitate the transfer of metabolites in the pathway, provided that catalytic activities are maintained.

Operable fragments, mutants, or truncated forms may be identified by modeling and/or screening. In some cases, this is achieved by deletion of, for example. N-terminal, C-terminal, or internal regions of the protein in a step-wise fashion, followed by analysis of the resulting derivative with regard to its activity for the desired reaction compared to the original sequence. If the derivative in question operates in this capacity, it is considered to constitute an equivalent derivative of the enzyme proper.

In some examples, some heterologous proteins may show occurrences where they are incorrectly processed when expressed in a recombinant host. For example, plant proteins such as cytochrome P450 enzymes expressed in microbial production hosts may have occurrences of incorrect processing. In particular, salutaridine synthase may undergo N-linked glycosylation when heterologously expressed in yeast. This N-linked glycosylation may not be observed in plants, which may be indicative of incorrect N-terminal sorting of the nascent SalSyn transcript so as to reduce the activity of the enzyme in the heterologous microbial host. In such examples, protein engineering directed at correcting N-terminal sorting of the nascent transcript so as to remove the N-linked glycosylation pattern may result in improved activity of the salutaridine synthase enzyme in the recombinant production host.

Aspects of the invention also relate to heterologous coding sequences that code for amino acid sequences that are equivalent to the native amino acid sequences for the various enzymes. An amino acid sequence that is "equivalent" is defined as an amino acid sequence that is not identical to the specific amino acid sequence, but rather contains at least some amino acid changes (deletions, substitutions, inversions, insertions, etc.) that do not essentially affect the biological activity of the protein as compared to a similar activity of the specific amino acid sequence, when used for a desired purpose. The biological activity refers to, in the example of an epimerase, its catalytic activity. Equivalent sequences are also meant to include those which have been engineered and/or evolved to have properties different from the original amino acid sequence. Mutable properties of interest include catalytic activity, substrate specificity, selectivity, stability, solubility, localization, etc

In some instances, the expression of each type of enzyme is increased through additional gene copies (i.e., multiple copies), which increases intermediate accumulation and/or BIA of interest production. Some embodiments of the invention include increased BIA of interest production in a host cell through simultaneous expression of multiple species variants of a single or multiple enzymes. In some cases, additional gene copies of a single or multiple enzymes are included in the host cell. Any convenient methods may be utilized including multiple copies of a heterologous coding sequence for an enzyme in the host cell.

In some examples, the engineered host cell includes multiple copies of a heterologous coding sequence for an enzyme, such as 2 or more. 3 or more, 4 or more, 5 or more, or even 10 or more copies. In certain embodiments, the engineered host cell includes multiple copies of heterologous coding sequences for one or more enzymes, such as multiple copies of two or more, three or more, four or more, etc. In some cases, the multiple copies of the heterologous coding sequence for an enzyme are derived from two or more different source organisms as compared to the host cell For example, the engineered host cell may include multiple copies of one heterologous coding sequence, where each of the copies is derived from a different source organism. As such, each copy may include some variations in explicit sequences based on inter-species differences of the enzy me of interest that is encoded by the heterologous coding sequence.

In certain embodiments, the engineered host cell includes multiple copies of heterologous coding sequences for one or more enzymes, such as multiple copies of two or more, three or more, four or more, etc. In some cases, the multiple copies of the heterologous coding sequence for an enzyme are derived from two or more different source organisms as compared to the host cell. For example, the engineered host cell may include multiple copies of one heterologous coding sequence, where each of the copies is derived from a different source organism. As such, each copy may include some variations in explicit sequences based on inter-species differences of the enzy me of interest that is encoded by the heterologous coding sequence.

The engineered host cell medium may be sampled and monitored for the production of BIAs of interest. The BIAs of interest may be observed and measured using any convenient methods. Methods of interest include, but are not limited to. LC-MS methods (e.g., as described herein) where a sample of interest is analyzed by comparison with a known amount of a standard compound. Additionally, there are other ways that BIAs of interest may be observed and/or measured. Examples of alternative ways of observing and/or measuring BIAs include GC-MS, UV-vis spectroscopy, NMR, LC-NMR, LC-UV, TLC, capillary electrophoresis, among others. Identity may be confirmed, e.g., by m/z and MS/MS fragmentation patterns, MRM transitions, and quantitation or measurement of the compound may be achieved via LC trace peaks of know retention time and/or EIC MS peak analysis by reference to corresponding LC-MS analysis of a known amount of a standard of the compound. In some cases, identity may be confirmed via multiple reaction monitoring using mass spectrometry.

Additionally, a culture of the engineered host cell may be sampled and monitored for the production of enzymes of interest, such as a neopinone isomerase enzyme. The enzymes of interest may be observed and measured using any convenient methods. Methods of interest include enzyme activity assays, polyacrylamide gel electrophoresis, carbon monoxide spectroscopy, and western blot analysis.

### METHODS

### Methods for Culturing Host Cells for BIA production

As summarized above, some aspects of the invention include methods of preparing beozylisoquinoline alkaloids (BIAs) of interest. Additionally, some aspects of the invention include methods of preparing enzymes of interest. As such, some aspects of the invention include culturing an engineered host cell under conditions in which the one or more host cell modifications (e.g.. as described herein) are functionally expressed such that the cell converts starting compounds of interest into product enzymes and/or BIAs of interest. Also provided are methods that include culturing an engineered host cell under conditions suitable for protein production such that one or more heterologous coding sequences are functionally expressed and convert starting compounds of interest into product enzymes or BIAs of interest. In some examples, the method is a method of preparing a benzylisoquinoline alkaloid (BIA) that includes culturing an engineered host cell (e.g.. as described herein); adding a starting compound to the cell culture: and recovering the BIA from the cell culture. In some examples, the method is a method of preparing an enzyme that includes culturing an engineered host cell (e.g., as described herein); adding a starting compound to the cell culture; and recovering the enzyme from the cell culture.

Fermentation media may contain suitable carbon substrates. The source of carbon suitable to perform the methods of this disclosure may encompass a wide variety of carbon containing substrates. Suitable substrates may include, without limitation, monosaccharides (e.g., glucose, fructose, galactose, xylose), oligosaccharides (e.g., lactose, sucrose, raffinose), polysaccharides (c.g., starch, cellulose), or a combination thereof. In some cases, unpurified mixtures from renewable feedstocks may be used (e.g.. cornsteep liquor, sugar beet molasses, barley malt). In some cases, the carbon substrate may be a one-carbon substrate (e.g., methanol, carbon dioxide) or a two-carbon substrate (e.g., ethanol). In other cases, other carbon containing compounds may be utilized, for example, methylamine, glucosamine, and amino acids.

Any convenient methods of culturing engineered host cells may be employed for producing the enzymes and/or BIAs of interest. The particular protocol that is employed may vary, e.g.. depending on the engineered host cell, the heterologous coding sequences, the enzymes of interest, the BIAs of interest, etc. The engineered host cells may be present in any convenient environment, such as an environment in which the engineered host cells are capable of expressing one or more functional heterologous enzymes. In some embodiments, the engineered host cells are cultured under conditions that are conducive to enzyme expression and with appropriate substrates available to allow production of enzymes and/or BIAs of interest in vivo. In some embodiments, the functional enzymes are extracted from the engineered host for production of enzymes and/or BIAs of interest under *in vitro* conditions. In some instances, the engineered host cells are placed back into a multicellular host organism. The engineered host cells are in any phase of growth, including, but not limited to, stationary phase and log-growth phase, etc. In addition, the cultures themselves may be continuous cultures or they may be batch cultures.

Cells may be grown in an appropriate fermentation medium at a temperature between 14-40°C. Cells may be grown with shaking at any convenient speed (e.g., 200 rpm). Cells may be grown at a suitable pH. Suitable pH ranges for the fennentation may be between pH 5-9. Fermentations may be performed under aerobic, anaerobic, or microaerobic conditions. Any suitable growth medium may be used. Suitable growth media may include, without limitation, common commercially prepared media such as synthetic defined (SD) minimal media or yeast extract peptone dextrose (YEPD) nch media. Any other rich, defined, or synthetic growth media appropriate to the microorganism may be used.

Cells may be cultured in a vessel of essentially any size and shape. Examples of vessels suitable to perform the methods of this disclosure may include, without limitation, multi-well shake plates, test tubes, flasks (baffled and non-baffled), and bioreactors. The volume of the culture may range from 10 microliters to greater than 10,000 liters.

The addition of agents to the growth media that are known to modulate metabolism in a manner desirable for the production of alkaloids may be included. In a non-limiting example, cyclic adenosine 2'3'-monophosphate may be added to the growth media to modulate catabolite repression.

Any convenient cell culture conditions for a particular cell type may be utilized. In certain embodiments, the host cells that include one or more modifications are cultured under standard or readily optimized conditions, with standard cell culture media and supplements. As one example, standard growth media when selective pressure for plasmid maintenance is not required may contain 20 g/L yeast extract, 10 g/L peptone, and 20 g/L dextrose (YPD). Host cells containing plasmids are grown in synthetic complete (SC) media containing 1.7 g/L, yeast nitrogen base. 5 g/L ammonium sulfate, and 20 g/L dextrose supplemented with the appropriate amino acids required for growth and selection. Alternative carbon sources which may be useful for inducible enzyme expression include, but are not limited to, sucrose, raffinose, and galactose. Cells are grown at any convenient temperature (e.g., 30°C) with shaking at any convenient rate (e.g., 200 rpm) in a vessel. e.g., in test tubes or flasks in volumes ranging from 1-1000 mL, or larger, in the laboratory.

Culture volumes may be scaled up for growth in larger fermentation vessels, for example, as part of an industrial process. The industrial fermentation process may be carried out under closed-batch, fed-batch, or continuous chemostat conditions, or any suitable mode of fermentation. In some cases, the engineered host cells may be immobilized on a substrate as whole cell catalysts and subjected to fermentation conditions for alkaloid production.

A batch fermentation is a closed system, in which the composition of the medium is set at the beginning of the fermentation and not altered during the fermentation process. The desired organism(s) are inoculated into the medium at the beginning of the fermentation. In some instances, the batch fermentation is run with alterations made to the system to control factors such as pH and oxygen concentration (but not carbon). In this type of fermentation system, the biomass and metabolite compositions of the system change continuously over the course of the fermentation. Cells typically proceed through a lag phase, then to a log phase (high growth rate), then to a stationary phase (growth rate reduced or halted), and eventually to a death phase (if left untreated). In additional cases, the batch fermentation system may be opened at certain times to add additional substrates for fennentating the desired organism. In particular, in some cases, a fermentation system may include a fed batch reactor.

A continuous fermentation is an open system, in which a defined fermentation medium is added continuously to the bioreactor and an equal amount of fermentation media is continuously removed from the vessel for processing. Continuous fennentation systems are generally operated to maintain steady state growth conditions, such that cell loss due to medium being removed must be balanced by the growth rate in the fermentation. Continuous fermentations are generally operated at conditions where cells are at a constant high cell density. Continuous fermentations allow for the modulation of one or more factors that affect target product concentration and/or cell growth.

The liquid medium may include, but is not limited to, a rich or synthetic defined medium having an additive component described above. Media components may be dissolved in water and sterilized by heat, pressure, filtration, radiation, chemicals, or any combination thereof. Several media components may be prepared separately and sterilized, and then combined in the fermentation vessel. The culture medium may be buffered to aid in maintaining a constant pH throughout the fermentation.

Process parameters including temperature, dissolved oxygen, pH. stirring, aeration rate, and cell density may be monitored or controlled over the course of the fermentation. For example, temperature of a fermentation process may be monitored by a temperature probe immersed in the culture medium. The culture temperature may be controlled at the set point by regulating the jacket temperature. Water may be cooled in an external chiller and then flowed into the bioreactor control tower and circulated to the jacket at the temperature required to maintain the set point temperature in the vessel.

Additionally, a gas flow parameter may be monitored in a fermentation process. For example, gases may be flowed into the medium through a sparger. Gases suitable for the methods of this disclosure may include compressed air, oxygen, and nitrogen. Gas flow may be at a fixed rate or regulated to maintain a dissolved oxygen set point.

The pH of a culture medium may also be monitored. In some examples, the pH may be monitored by a pH probe that is immersed in the culture medium inside the vessel. If pH control is in effect, the pH may be adjusted by acid and base pumps which add each solution to the medium at the required rate. The acid solutions used to control pH may be sulfuric acid or hydrochloric acid. The base solutions used to control pH may be sodium hydroxide, potassium hydroxide, or ammonium hydroxide.

Further, dissolved oxygen may be monitored in a culture medium by a dissolved oxygen probe immersed in the culture medium. If dissolved oxygen regulation is in effect, the oxygen level may be adjusted by increasing or decreasing the stirring speed. The dissolved oxygen level may also be adjusted by increasing or decreasing the gas flow rate. The gas may be compressed air, oxygen, or nitrogen.

Stir speed may also be monitored in a fermentation process. In some examples, the stirrer motor may drive an agitator. The stirrer speed may be set at a consistent rpm throughout the fermentation or may be regulated dynamically to maintain a set dissolved oxygen level.

Additionally, turbidity may be monitored in a fermentation process. In some examples, cell density may be measured using a turbidity probe. Alternatively, cell density may be measured by taking samples from the bioreactor and analyzing them in a spectrophotometer. Further, samples may be removed from the bioreactor at time intervals through a sterile sampling apparatus. The samples may be analyzed for alkaloids produced by the host cells. The samples may also be analyzed for other metabolites and sugars, the depletion of culture medium components, or the density of cells.

In another example, a feed stock parameter may be monitored during a fermentation process. In particular, feed stocks including sugars and other carbon sources, nutrients, and cofactors that may be added into the fermentation using an external pump. Other components may also be added during the fermentation including, without limitation, anti-foam, salts, chelating agents, surfactants, and organic liquids.

Any convenient codon optimization techniques for optimizing the expression of heterologous polynucleotides in host cells may be adapted for use in the subject host cells and methods, see e.g., Gustafsson, C. et al. (2004) Trends Biotechnol, 22, 346-353, which is incorporated by reference in its entirety.

The subject method may also include adding a starting compound to the cell culture. Any convenient methods of addition may be adapted for use in the subject methods. The cell culture may be supplemented with a sufficient amount of the starting materials of interest (e.g.. as described herein). e.g.. a mM to µM amount such as between about 1-5 mM of a starting compound. It is understood that the amount of starting material added, the timing and rate of addition, the form of material added, etc., may vary according to a variety of factors. The starting material may be added neat or pre-dissolved in a suitable solvent (e.g., cell culture media, water, or an organic solvent). The starting material may be added in concentrated form (e.g., 10x over desired concentration) to minimize dilution of the cell culture medium upon addition. The starting material may be added in one or more batches, or by continuous addition over an extended period of time (e.g., hours or days).

### Methods for Isolating Products from the Fermentation Medium

The subject methods may also include recovering the enzymes and/or BIAs of interest from the cell culture. Any convenient methods of separation and isolation (e.g., chromatography methods or precipitation methods) may be adapted for use in the subject methods to recover the enzymes and/or BIAs of interest from the cell culture. Filtration methods may be used to separate soluble from insoluble fractions of the cell culture. In some cases, liquid chromatography methods (e.g., reverse phase HPLC, size exclusion, normal phase chromatography) may be used to separate the BIA of interest from other soluble components of the cell culture. In some cases, extraction methods (e.g.. liquid extraction, pH based purification, solid phase extraction, affinity chromatography, ion exchange, etc.) may be used to separate the enzymes and/or BIAs of interest from other components of the cell culture.

The produced alkaloids may be isolated from the fermentation medium using methods known in the art. A number of recovery steps may be performed immediately after (or in some instances, during) the fermentation for initial recovery of the desired product. Through these steps, the alkaloids (e.g., BIAs) may be separated from the engineered host cells, cellular debris and waste, and other nutrients, sugars, and organic molecules may remain in the spent culture medium. This process may be used to yield a BIA-enriched product.

In an example, a product stream having a benzylisoquinoline alkaloid (BIA) product is formed by providing engineered yeast cells and a feedstock including nutrients and water to a batch reactor. In particular, the engineered yeast cells may be subjected to fermentation by incubating the engineered yeast cells for a time period of at least about 5 minutes to produce a solution comprising the BIA product and cellular material. Once the engineered yeast cells have been subjected to fermentation, at least one separation unit may be used to separate the BIA product from the cellular material to provide the product stream comprising the BIA product. In particular, the product stream may include the BIA product as well as additional components, such as a clarified yeast culture medium Additionally, a BIA product may comprise one or more BIAs of interest, such as one or more BIA compounds.

Different methods may be used to remove cells from a bioreactor medium that include an enzyme and/or BIA of interest. In some examples, cells may be removed by sedimentation over time. This process of sedimentation may be accelerated by chilling or by the addition of fining agents such as silica. The spent culture medium may then be siphoned from the top of the reactor or the cells may be decanted from the base of the reactor. Alternatively, cells may be removed by filtration through a filter, a membrane, or other porous material. Cells may also be removed by centrifugation, for example, by continuous flow centrifugation or by using a continuous extractor.

If some valuable enzymes and/or BIAs of interest are present inside the engineered host cells, the engineered host cells may be permeabilized or lysed and the cell debris may be removed by any of the methods described above. Agents used to permeabilize the engineered host cells may include, without limitation, organic solvents (e.g.. DMSO) or salts (e.g., lithium acetate). Methods to lyse the engineered host cells may include the addition of surfactants such as sodium dodecyl sulfate, or mechanical disruption by bead milling or sonication.

Enzymes and/or BIAs of interest may be extracted from the clarified spent culture medium through liquid-liquid extraction by the addition of an organic liquid that is immiscible with the aqueous culture medium. In some examples, the use of liquid-liquid extraction may be used in addition to other processing steps. Examples of suitable organic liquids include, but are not limited to, isopropyl myristate, ethyl acetate, chloroform, butyl acetate, methylisobutyl ketone, methyl oleate, toluene, oleyl alcohol, ethyl butyrate. The organic liquid may be added to as little as 10% or as much as 100% of the aqueous medium. The organic liquid may be as little as 10%, may be 100%, may be 200%, may be 300%, may be 400% may be 500%, may be 600%, may be 700%, may be 800%, may be 900%, may be 1000%, may be more than 1000% or may be a percentage in between those listed herein of the volume of the aqueous liquid.

In some cases, the organic liquid may be added at the start of the fermentation or at any time during the fermentation. This process of extractive fermentation may increase the yield of enzymes and/or BIAs of interest from the host cells by continuously removing enzymes and/or BIAs to the organic phase.

Agitation may cause the organic phase to form an emulsion with the aqueous culture medium. Methods to encourage the separation of the two phases into distinct layers may include, without limitation, the addition of a demulsifier or a nucleating agent, or an adjustment of the pH. The emulsion may also be centrifuged to separate the two phases, for example, by continuous conical plate centrifugation.

Alternatively, the organic phase may be isolated from the aqueous culture medium so that it may be physically removed after extraction. For example, the solvent may be encapsulated in a membrane.

In some examples, enzymes and/or BIAs of interest may be extracted from a fermentation medium using adsorption methods. In some examples, BIAs of interest may be extracted from clarified spent culture medium by the addition of a resin such as Amberlite^{®} XAD4 or another agent that removes BIAs by adsorption. The BIAs of interest may then be released from the resin using an organic solvent. Examples of suitable organic solvents include, but are not limited to, methanol, ethanol, ethyl acetate, or acetone.

BIAs of interest may also be extracted from a fermentation medium using filtration. At high pH, the BIAs of interest may form a crystalline-like precipitate in the bioreactor. This precipitate may be removed directly by filtration through a filter, membrane, or other porous material. The precipitate may also be collected by centrifugation and/or decantation.

The extraction methods described above may be carried out either *in situ* (in the bioreactor) or *ex situ* (e.g.. in an external loop through which media flows out of the bioreactor and contacts the extraction agent, then is recirculated back into the vessel). Alternatively, the extraction methods may be performed after the fermentation is terminated using the clarified medium removed from the bioreactor vessel.

### Methods for Purifying Products from Alkaloid-Enriched Solutions

Subsequent purification steps may involve treating the post-fermentation solution enriched with BIA product(s) of interest using methods known in the art to recover individual product species of interest to high purity.

In one example, BIAs of interest extracted in an organic phase may be transferred to an aqueous solution. In some cases, the organic solvent may be evaporated by heat and/or vacuum, and the resulting powder may be dissolved in an aqueous solution of suitable pH. In a further example, the BIAs of interest may be extracted from the organic phase by addition of an aqueous solution at a suitable pH that promotes extraction of the BIAs of interest into the aqueous phase. The aqueous phase may then be removed by decantation, centrifugation, or another method.

The BIA-containing solution may be further treated to remove metals, for example, by treating with a suitable chelating agent. The BIA of interest-containing solution may be further treated to remove other impurities, such as proteins and DNA. by precipitation. In one example, the BIA of interest-containing solution is treated with an appropriate precipitation agent such as ethanol, methanol, acetone, or isopropanol. In an alternative example. DNA and protein may be removed by dialysis or by other methods of size exclusion that separate the smaller alkaloids from contaminating biological macromolecules.

In further examples, the solution containing BIAs of interest may be extracted to high purity by continuous cross-flow filtration using methods known in the art.

If the solution contains a mixture of BIAs of interest, it may be subjected to acid-base treatment to yield individual BIA of interest species using methods known in the art. In this process, the pH of the aqueous solution is adjusted to precipitate individual BIAs.

For high purity, small-scale preparations, the BIAs may be purified in a single step by liquid chromatography.

### Liquid Chromatography Mass Spectrometry (LCMS) Method

The BIA compounds of interest, including 1-benzylisoquinoline alkaloids, bisbenzylisoquinoline alkaloids, promorphinan alkaloids, morphinan alkaloids, nal-opioids, and nor-opioids, may be separated using liquid chromatography, and detected and quantified using mass spectrometry. Compound identity may be confirmed by characteristic elution time, mass-to-charge ratio (m/z) and fragmentation patterns (MS/MS). Quantitation may be performed by comparison of compound peak area to a standard curve of a known reference standard compound. Additionally, BIAs of interest may be detected by alternative methods such as GC-MS, UV-vis spectroscopy, NMR, LC-NMR, LC-UV. TLC, and capillary electrophoresis.

### Purpald Assay Method

For high throughput screening of demethylation reactions a purpald assay may be used. For example, demethylation catalyzed by 2-oxoglutarate dependent dioxygenases produces formaldehyde a as product as shown in the generalized chemical equation: [substrate] + 2-oxoglutarate + O₂ 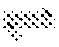 [product] + formaldehyde + succinate + CO₂. Purpald reagent in alkaline conditions undergoes a color change in the presence of formaldehyde that can be quantified to concentrations as low as 1 nM with a spectrophotometer at 510 nm.

### Yeast-Derived Alkaloid APIs Versus Plant-Derived APIs

The clarified yeast culture medium (CYCM) may contain a plurality of impurities. The clarified yeast culture medium may be dehydrated by vacuum and/or heat to yield an alkaloid-rich powder. This product is analogous to the concentrate of poppy straw (CPS) or opium, which is exported from poppy-growing countries and purchased by API manufacturers. For the purposes of this invention, CPS is a representative example of any type of purified plant extract from which the desired alkaloids product(s) may ultimately be further purified. Tables 17 and 18 highlight the impurities in these two products that may be specific to either CYCM or CPS or may be present in both. While some BIAs may have a pigment as an impurity, other BIAs may be categorized as pigments themselves. Accordingly, these BIAs may be assessed for impurities based on non-pigment impurities. By analyzing a product of unknown origin for a subset of these impurities, a person of skill in the art could determine whether the product originated from a yeast or plant production host.

APl-grade phannaceutical ingredients are highly purified molecules. As such, impurities that could indicate the plant- or yeast-onigin of an API (such as those listed in **Tables 17 and 18**) may not be present at the API stage of the product. Indeed, many of the API products derived from yeast strains of some embodiments of the present invention may be largely indistinguishable from the traditional plant-derived APIs. In some cases, however, conventional alkaloid compounds may be subjected to chemical modification using chemical synthesis approaches, which may show up as chemical impurities in plant-based products that require such chemical modifications. For example, chemical derivatization may often result in a set of impurities related to the chemical synthesis processes. In certain situations, these modifications may be performed biologically in the yeast production platform, thereby avoiding some of the impurities associated with chemical derivation from being present in the yeast-derived product. In particular, these impurities from the chemical derivation product may be present in an API product that is produced using chemical synthesis processes but may be absent from an API product that is produced using a yeast-derived product. Alternatively, if a yeast-derived product is mixed with a chemically - derived product, the resulting impurities may be present but in a lesser amount than would be expected in an API that only or primarily contains chemically-derived products. In this example, by analyzing the API product for a subset of these impurities, a person of skill in the art could detennine whether the product originated from a yeast production host or the traditional chemical derivatization route.

Non-limiting examples of impurities that may be present in chemically-derivatized morphinan APIs but not in biosynthesized APIs include a codeine-*O*(6)-methyl ether impurity in API codeine: 8,14-dihydroxy-7,8-dihydrocodeinone in API oxycodone; and tetrahydrothebaine in API hydrocodone. The codeine-*O*(6)-methyl ether may be formed by chemical over-methylation of morphine. The 8,14-dihydroxy-7,8-dihydrocodeinone in API oxycodone may be formed by chemical over-oxidation of thebaine. Additionally, the tetrahydrothcbaine in API hydrocodone may be formed by chemical over-reduction of thebaine.

However, in the case where the yeast-derived compound and the plant-derived compound are both subjected to chemical modification through chemical synthesis approaches, the same impurities associated with the chemical synthesis process may be expected in the products. In such a situation, the starting material (e.g., CYCM or CPS) may be analyzed as described above.

### Host Cell Derived Nal-opioids vs Chemically Derived Nal-opioids

Nal-opioids produced by chemical synthesis may contain a plurality of impurities. These impurities may arise from many different causes, for example, unreacted starting materials, incomplete reactions, the formation of byproducts, persistence of intermediates, dimerization, or degradation. An example of an unreacted starting material could be oxymorphone remaining in a preparation of naltrexone. An example of an impurity arising from an incomplete reaction could be 3-*O-*Methylbuprenorphine resulting from the incomplete 3-*O*-demethylation of thebaine. Chemical modification can result in the addition or removal of functional groups at off-target sites. For example, the oxidation of C10 to create 10-hydroxynaltrexone and 10-ketonaltrexone during naltrexone synthesis, or the removal of the 6-*O*-methyl group to give 6-*O*-desmetylbuprenorphine during buprenorphine synthesis. Impurites may arise from the persistence of reaction intermediates, for example the persistence of *N*-oxides like oxymorphone *N*-oxide formed during the *N*-demethylation process. Another source of impurities is dimerization, the conjugation of two opioid molecules, for example two buprenorphine molecules (2,2'-bisbuprenorphine), two naltrexone molecules (2,2'-bisnaltrexone), or two naloxone molecules (2,2'-bisnaloxone). Impurities may arise from degradation of starting materials, reaction intermediates, or reaction products. The extreme physical conditions used in chemical syntheses may make the presence of degradation more likely. An example of an impurity that may arise from degradation is dehydrobuprenorphine produced by oxidizing conditions during buprenorphine synthesis.

Nal-opioids produced by enzyme catalysis in a host cell may contain different impurities than nal-opioids produced by chemical synthesis. Nal-opioids produced by enzy me catalysis in a host cell may contain fewer impurities than nal-opioids produced by chemical synthesis. Nal-opioids produced by enzyme catalysis in a host cell may lack certain impurities that are found in nal-opioids produced by chemical synthesis. In some examples, key features of enzyme synthesis may include. (1) enzymes target a specific substrate and residue with high fidelity: (2) enzymes perform reactions in the mild physiological conditions within the cell which do not compromise the stability of the molecules: and (3) enzymes are engineered to be efficient catalysts that drive reactions to completion.

**Table 19** highlights some of the impurities that may be specific to chemically produced nal-opioids. Accordingly, nal-opioids may be assessed for impurities to determine the presence or absence of any impurity from **Table 19.** By analyzing a product of unknown origin for a subset of these impurities, a person of skill in the art could determine whether the product originated from a chemical or enzymatic synthesis.

### Methods of Engingering Host Cells

Also included are methods of engineering host cells for the purpose of producing enzymes and/or BIAs of interest. Inserting DNA into host cells may be achieved using any convenient methods. The methods are used to insert the heterologous coding sequences into the engineered host cells such that the host cells functionally express the enzymes and convert starting compounds of interest into product enzymes and/or BIAs of interest.

Any convenient promoters may be utilized in the subject engineered host cells and methods. The promoters driving expression of the heterologous coding sequences may be constitutive promoters or inducible promoters, provided that the promoters are active in the engineered host cells. The heterologous coding sequences may be expressed from their native promoters, or non-native promoters may be used. Such promoters may be low to high strength in the host in which they are used. Promoters may be regulated or constitutive. In certain embodiments, promoters that are not glucose repressed, or repressed only mildly by the presence of glucose in the culture medium, are used. Promoters of interest include but are not limited to, promoters of glycolytic genes such as the promoter of the *B. subtilis* tsr gene (encoding the promoter region of the fructose bisphosphate aldolase gene) or the promoter from yeast *S. cerevisiae* gene coding for glyceraldehyde 3-phosphate dehydrogenase (GPD, GAPDH, or TDH3), the ADH1 promoter of baker's yeast, the phosphate-stairation induced promoters such as the PHOS promoter of yeast, the alkaline phosphatase promoter from *B*. *licheniformis,* yeast inducible promoters such as Gal1-10. Gall. GalL. GalS. repressible promoter Met25, tetO, and constitutive promoters such as glyceraldehyde 3-phosphate dehydrogenase promoter (GPD), alcohol dehydrogenase promoter (ADH), translation-elongation factor-1-α promoter (TEF), cytochrome c-oxidase promoter (CYC1), MRP7 promoter, etc. Autonomously replicating yeast expression vectors containing promoters inducible by hormones such as glucocorticoids, steroids, and thyroid hormones may also be used and include, but are not limited to, the glucorticoid responsive element (GRE) and thyroid hormone responsive element (TRE). These and other examples are described in U.S. Pat. No. 7,045,290, which is incorporated by reference, including the references cited therein. Additional vectors containing constitutive or inducible promoters such as a factor, alcohol oxidase, and PGH may be used. Additionally any promoter/enhancer combination (as per the Eukaryotic Promoter Data Base EPDB) could also be used to drive expression of genes. Any convenient appropriate promoters may be selected for the host cell. e.g., *E. coli.* One may also use promoter selection to optimize transcript, and hence, enzyme levels to maximize production while minimizing energy resources.

Any convenient vectors may be utilized in the subject engineered host cells and methods. Vectors of interest include vectors for use in yeast and other cells. The types of yeast vectors may be broken up into 4 general categories: integrative vectors (YIp), autonomously replicating high copy-number vectors (YEp or 2µ plasmids), autonomously replicating low copy-number vectors (YCp or ccntromeric plasmids) and vectors for cloning large fragments (YACs). Vector DNA is introduced into prokaryotic or eukaryotic cells via any convenient transformation or transfection techniques. DNA of another source (e.g. PCR-generated double stranded DNA product, or synthesized double stranded or single stranded oligonucleotides) may be used to engineer the yeast by integration into the genome. Any single transformation event may include one or several nucleic acids (vectors, double stranded or single stranded DNA fragments) to genetically modify the host cell. **Table 10** illustrates examples of convenient vectors.

### UTILITY

The engineered host cells and methods of the invention. e.g., as described above, find use in a variety of applications. Applications of interest include, but are not limited to: research applications and therapeutic applications. Methods of the invention find use in a variety of different applications including any convenient application where the production of enzymes and/or BIAs is of interest.

The subject engineered host cells and methods find use in a variety of therapeutic applications. Therapeutic applications of interest include those applications in which the preparation of pharmaceutical products that include BIAs is of interest. The engineered host cells described herein produce BIAs of interest and enzymes of interest. Reticuline is a major branch point intermediate of interest in the synthesis of BIAs including engineering efforts to produce end products such as opioid products. The subject host cells may be utilized to produce BIAs of interest from simple and inexpensive starting materials that may find use in the production of BIAs of interest, including reticuline, and BIA end products. As such, the subject host cells find use in the supply of therapeutically active BIAs of interest.

In some instances, the engineered host cells and methods find use in the production of commercial scale amounts of BIAs thereof where chemical synthesis of these compounds is low yielding and not a viable means for large-scale production. In certain cases, the host cells and methods are utilized in a fermentation facility that would include bioreactors (fermenters) of e.g., 5,000-200,000 liter capacity allowing for rapid production of BIAs of interest thereof for therapeutic products. Such applications may include the industrial-scale production of BIAs of interest from fermentable carbon sources such as cellulose, starch, and free sugars.

The subject engineered host cells and methods find use in a variety of research applications. The subject host cells and methods may be used to analyze the effects of a variety of enzymes on the biosynthetic pathways of a variety of enzymes and/or BIAs of interest. In addition, the engineered host cells may be engineered to produce enzymes and/or BIAs of interest that find use in testing for bioactivity of interest in as yet unproven therapeutic functions. In some cases, the engineering of host cells to include a variety of heterologous coding sequences that encode for a variety of enzymes elucidates the high yielding biosynthetic pathways towards enzymes and/or BIAs of interest. In certain cases, research applications include the production of enzymes and/or BIAs of interest for therapeutic molecules of interest that may then be further chemically modified or derivatized to desired products or for screening for increased therapeutic activities of interest. In some instances, host cell strains are used to screen for enzyme activities that are of interest in such pathways, which may lead to enzyme discovery via conversion of BIA metabolites produced in these strains

The subject engineered host cells and methods may be used as a production platform for plant specialized metabolites. The subject host cells and methods may be used as a platform for drug library development as well as plant enzyme discovery. For example, the subject engineered host cells and methods may find use in the development of natural product based drug libraries by taking yeast strains producing interesting scaffold molecules, such as guattegaumerine, and further functionalizing the compound structure through combinatorial biosynthesis or by chemical means. By producing drug libraries in this way, any potential drug hits are already associated with a production host that is amenable to large-scale culture and production. As another example, these subject engineered host cells and methods may find use in plant enzyme discovery. The subject host cells provide a clean background of defined metabolites to express plant EST libraries to identify, new enzyme activities. The subject host cells and methods provide expression methods and culture conditions for the functional expression and increased activity of plant enzymes in yeast.

### KITS AND SYSTEMS

Aspects of the invention further include kits and systems, where the kits and systems may include one or more components employed in methods of the invention, e.g., engineered host cells, starting compounds, heterologous coding sequences, vectors, culture medium, etc., as described herein. In some embodiments, the subject kit includes an engineered host cell (e.g., as described herein), and one or more components selected from the following: starting compounds, a heterologous coding sequence and/or a vector including the same, vectors, growth feedstock, components suitable for use in expression systems (e.g., cells, cloning vectors, multiple cloning sites (MCS), bi-directional promoters, an internal ribosome entry site (IRES), etc.), and a culture medium.

Any of the components described herein may be provided in the kits. e.g., host cells including one or more modifications, starting compounds, culture medium, etc. A variety of components suitable for use in making and using heterologous coding sequences, cloning vectors and expression systems may find use in the subject kits. Kits may also include tubes, buffers, etc., and instructions for use. The vanous reagent components of the kits may be present in sepamte containers, or some or all of them may be pre-combined into a reagent mixture in a single container, as desired.

Also provided are systems for producing enzymes and/or BIAs of interest, where the systems may include engineered host cells including one or more modifications (e.g., as described herein), starting compounds, culture medium, a fermenter and fermentation equipment, e.g.. an apparatus suitable for maintaining growth conditions for the host cells, sampling and monitoring equipment and components, and the like. A variety of components suitable for use in large scale fermentation of yeast cells may find use in the subject systems.

In some cases, the system includes components for the large scale fermentation of engineered host cells, and the monitoring and purification of enzymes and/or BIA compounds produced by the fermented host cells. In certain embodiments, one or more starting compounds (e.g., as described herein) are added to the system, under conditions by which the engineered host cells in the fermenter produce one or more desired BIA products of interest. In some instances, the host cells produce a BIA of interest (e.g., as described herein). In certain cases, the BIA products of interest are opioid products, such as thebaine, codeine, neopine, morphine, neomorphinc, hydrocodone, oxycodone, hydromorphone, dihydrocodeine, 14-hydroxycodeine, dihydromorphine, and oxymorphone. In some cases, the BIA products of interest are nal-opioids, such as naltrexone, naloxone, malmefene, nalorphine, nalorphine, nalodeine, naldemedine, naloxegol, 6β-naltrexol, naltrindole, methylnaltrexone, methylsamidorphan, alvimopan, axelopran, bevenpran, dinicotinate, levallorphan, samidorphan, buprenorphine, dezocine, eptazocine, butorphanol, levorphanol, nalbuphine, pentazocine, phenazocine, norbinaltorphimine, and diprenorphine. In some cases, the BIA products of interest are nor-opioids, such as norcodeine, noroxycodone, northebaine, norhydrocodone, nordihydro-codeine, nor-14-hydroxy-codeine, norcodeinone, nor-14-hydroxy-codeinone, normorphine, noroxymorphone, nororipavine, norhydro-morphone, nordihydro-morphine, nor-14-hydroxy-morphine, normorphinone, and nor-14-hydroxy-morphinone. In some cases, the BIA products are bisbenzylisoquinoline products, such as berbamunine, guattegaumerine, dauricine, and liensinine.

In some cases, the system includes processes for monitoring and or analyzing one or more enzymes and/or BIAs of interest compounds produced by the subject host cells. For example, a LC-MS analysis system as described herein, a chromatography system, or any convenient system where the sample may be analyzed and compared to a standard, e.g.. as described herein. The fermentation medium may be monitored at any convenient times before and during fermentation by sampling and analysis. When the conversion of starting compounds to enzymes and/or BIA products of interest is complete, the fermentation may be halted and purification of the BIA products may be done. As such, in some cases, the subject system includes a purification component suitable for purifying the enzymes and/or BIA products of interest from the host cell medium into which it is produced. The purification component may include any convenient process that may be used to purify the enzymes and/or BIA products of interest produced by fermentation, including but not limited to, silica chromatography, reverse-phase chromatography, ion exchange chromatography. HIC chromatography, size exclusion chromatography, liquid extraction, and pH extraction methods. In some cases, the subject system provides for the production and isolation of enzyme and/or BIA fermentation products of interest following the input of one or more starting compounds to the system.

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.), but some experimental errors and deviations should be accounted for. Unless indicated otherwise, parts are parts by weight, molecular weight is weight average molecular weight, temperature is in degrees Centigrade, and pressure is at or near atmospheric.

### Discussion of Enzyme List

The host cells may be engineered to include one or more modifications (such as two or more, three or more, four or more, five or more, or even more modifications) that provide for the production of BIAs of interest and/or enzymes of interest. **Table 11** provides a list of exemplary genes that may be acted upon by one or more modifications so as to provide for the production of BIAs of interest and/or enzymes of interest in an engineered host cell.

Modifications of genes as provided in **Table 11** may be used to produce BIAs of interest from engineered host cells that are supplied with a medium containing the minimal nutrients required for growth. This minimal medium may contain a carbon source, a nitrogen source, amino acids, vitamins, and salts. For example, modifications of genes as provided in **Table 11** may be used to produce BIAs of interest from engineered host cells that are fed sugar. Additionally, modifications of one or more genes as provided in **Table 11** may be used to augment the biosynthetic processes of host cells that may be engineered for drug production.

Additionally, the use of these modifications to provide for the production of BIAs of interest and/or enzymes of interest in engineered host cells is not readily apparent from the mere identification of enzymes that may be produced by the genes. In particular, synthetic pathways that have been reconstructed in host cells, such as yeast cells, as described herein comprise a variety of enzymes that do not act together in nature within a single organism. Additionally, some of the enzymes discussed herein do not act for BIA biosynthesis in their natural context. Further, some of the enzymes described herein are not evolved to function in particular host cells, such as yeast cells, and are not evolved to function together. In these cases, it would not be obvious that the enzymes would exhibit sufficient activity in the context of the synthetic BIA pathway in a host cell, such as yeast, to have sufficient flux through the pathway to produce downstream BIA end products.

For example, plant enzymes are often difficult to functionally express in heterologous microbial hosts, such as yeast. In many cases the enzymes may be misfolded, not correctly localized within the host cell, and/or incorrectly processed. The differences in protein translation and processing between yeast and plants can lead to these enzymes exhibiting substantially reduced to no detectable activities in the yeast host. These challenges arise commonly for endomembrane localized enzymes, such as cytochrome P450s, which are strongly represented in the BIA pathways. Even reduced enzyme activities may pose a substantial challenge to engineering yeast to produce complex BIAs, which requires sufficient activity at each step to ensure high-level accumulation of the desired BIA products.

Additionally, there are endogenous enzymes/pathways in some host cells, such as yeast, that may act on many of the early precursors in the BIA pathway (i.e.. intermediates from tyrosine to norcoclaurine), and thus it may not be readily apparent that there would be sufficient flux through the heterologous pathway to achieve substantial BIA production given these competing endogenous pathways. For example, the Erlich pathway (Hazelwood, et al. 2008. Appl. Environ. Microbiol. 74: 2259-66; Larroy, et al. 2003. Chem. Biol. Interact. 143-144: 229-38: Larroy, et al. 2002. Eur. J. Biochem. 269: 5738-45) in yeast is the main endogenous pathway that would act to convert many of the intennediates in the early BIA pathway to undesired products and divert flux from the synthetic pathway.

Further, many of the enzymes as discussed herein, and as provided in **Table 11,** may function under very specific regulation strategies, including spatial regulation, in the native plant hosts, which may be lost upon transfer to the heterologous yeast host. In addition, plants present very different biochemical environments than yeast cells under which the enzymes are evolved to function, including pH, redox state, and substrate, cosubstrate, coenzy me, and cofactor availabilities. Given the differences in biochemical environments and regulatory strategies between the native hosts and the heterologous yeast hosts, it is not obvious that the enzymes would exhibit substantial activities when in the context of the yeast environment and further not obvious that they would work together to direct simple precursors such as sugar to complex BIA compounds. Maintaining the activities of the enzymes in the yeast host is particularly important as many of the pathways have many reaction steps (>10), such that if these steps are not efficient then one would not expect accumulation of desired downstream products.

In addition, in the native plant hosts, the associated metabolites in these pathways may be localized across different cell and tissue types. In several examples, there are cell types that may be specialized for biosynthesis and cell types that may be synthesized for metabolite accumulation. This type of cell specialization may be lost when expressing the pathways within a heterologous yeast host, and may play an important role in controlling the toxicity of these metabolites on the cells. Thus, it is not obvious that yeast could be successfully engineered to biosynthesize and accumulate these metabolites without being harmed by the toxicity of these compounds.

As one example, in the native plant hosts, the enzyme BBE is reported to have dynamic subcellular localization. In particular, the enzyme BBE initially starts in the ER and then is sorted to the vacuole (Bird and Facchini. 2001. Planta. 213: 888-97). It has been suggested that the ER-association of BBE in plants (Alcantara, et al. 2005. Plant Physiol. 138: 173-83) provides the optimal basic pH (pH ~8.8) for BBE activity (Ziegler and Facchini. 2008. Annu. Rev. Plant Biol. 59: 735-69). As another example, there is evidence that sanguinarine biosynthesis occurs in specialized vesicles within plant cells (Amann, et al. 1986. Planta. 167: 310-20), but only some of the intermediates accumulate in the vesicles. This may occur so as to sequester them from other enzyme activities and/or toxic effects.

As another example, the biosynthetic enzymes in the morphinan pathway branch are all localized to the phloem, winch is part of the vascular tissue in plants. In the phloem, the pathway enzymes may be further divided between two cell types: the sieve elements common to all plants, and the laticifer which is a specialized cell type present only in certain plants which make specialized secondary metabolites. The upstream enzymes (i.e., from NCS through to SalAT) are predominantly in the sieve elements, and the downstream enzymes (i.e., T6ODM, COR, CODM) are mostly in the laticifer (Onoyovwe, et al. 2013. Plant Cell. 25: 4110-22). Additionally, it was discovered that the final steps in the noscapine biosynthetic pathway take place in the laticifer (Chen and Facchini. 2014. Plant J. 77: 173-84). This compartmentalization is thought to be highly important for regulating biosynthesis by isolating or trafficking intermediates, providing optimal pH, enhancing supply of cofactors, although the nature of the poppy laticifer microenvironment is still under investigation (Ziegler and Facchini. 2008. Annu. Rev. Plant Biol. 59: 735-69). Further, it is predicted that several of the enzymes may function as multi-enzyme complexes or metabolic channels common to plant secondan metabolism (Kempe, et al. 2009. Phytochemistry. 70: 579-89: Allen, et al. 2004. Nat. Biotechnol. 22: 1559-66). When biosynthetic enzymes are combined from different hosts and/or expressed recombinantly in a heterologous yeast cell it is not clear that these complexes or channels will form as they would in the native host. In an additional example, in *Coptis japonica,* berberine is biosynthesized in root tissues and then accumulated within the rhizome via the action of specialized ATP-binding cassette transport proteins (Shitan, et al. 2013. Phytochemistry. 91: 109-16). In opium poppy, morphinan alkaloids are accumulated within the latex (cytoplasm of laticifer cells) (Martin, et al. 1967. Biochemistry. 6: 2355-63).

Further, even without these considerations, it is also the case that the plant enzymes for several of the steps in the pathways described herein have not yet been characterized. For example, the conversion of tyrosine to the early benzylisoquinoline alkaloid scaffold norcoclaurine has not yet been characterized. Thus, for several of the steps in the pathways described herein, alternative biosynthetic scheme were produced by bringing together enzyme activities that do not normally occur together in nature for the biosynthesis of BIAs or identifying new enzyme activities from genome sequence information to use in the reconstructed pathways.

For example, the two-step conversion of tyrosine to dopamine may be achieved by combining at least 5 mammalian enzymes and 1 bacterial enzyme, which do not naturally occur together and were not evolved to function in the context of this pathway or with plant enzymes. In these instances, it may not be obvious to utilize these enzymes for the biosynthesis of compounds they were not evolved for in nature and that they would function effectively in the context of a heterologous microbial host and this pathway.

As another example, until recent years the enzyme responsible for the conversion of *(S)-*reticuline to *(R)*-reticuline was unknown. Even when a fused epimerase enzyme was discovered, evolutionary analysis suggested that morphine-producing poppies evolved a fusion enzyme between the oxidase and reductase for an epimerase reaction, which was in contrast to non-morphine producing poppies where the epimerase enzymes were non-fused. Based on this analysis, some scholars believed the fusion of the oxidase and reductase portions was necessary to efficiently catalyze the com crsion of *(S)-*reticuline to *(R)*-reticuline. Novel methods of using engineered split epimemses as discussed herein may perform this epimerization reaction in yeast and in the context of the synthetic BIA pathway, and may perform this epimerization with greater efficiency than performing an epimerization with a wild-type epimerase.

Examples of the genes that are the object of modifications so as to produce BIAs of interest and/or enzymes of interest are discussed below. Additionally, the genes are discussed in the context of a series of Figures that illustrate pathways that are used in generating BIAs of interest and/or enzymes of interest.

**[TKL1]** In some examples, the engineered host cell may modify the expression of the enzyme transketolase. Transketolase is encoded by the TKL1 gene. In some examples, transketolase catalyzes the reaction of fructose-6-phosphate + glyceraldehyde-3-phosphate ↔ xylulose-5-phosphate + etythrose-4-phosphate, as referenced in **FIG. 1****.** An engineered host cell may be modified to include constitutive overexpression of the TKL1 gene in the engineered host cell Additionally or alternatively, the engineered host cell may be modified to synthetically regulate the expression of the TKL1 gene in the engineered host cell. In some examples, the engineered host cell may be modified to incorporate a copy, copies, or additional copies, of the TKL1 gene. Additionally or alternatively, the engineered host cell may be modified to incorporate the introduction of a strong promoter element for the overexpression of the TKL1 gene within the engineered host cell. The TKL1 gene may be derived from *Saccharomyces cerevisiae* or another species.

**[ZWF1]** In some examples, the engineered host cell may modify the expression of the enzyme glucose-6-phosphate dehydrogenase. Glucose-6-phosphate dehydrogenase is encoded by the ZWF1 gene. In some examples. glucose-6-phosphate dehydrogenase catalyzes the reaction of glucose-6-phosphate → 6-phosphogluconolactone, as referenced in **FIG. 1****.** An engineered host cell may be modified to delete the coding region of the ZWF1 gene in the engineered host cell. Alternatively, the engineered host cell may be modified to disable the functionality of the ZWF1 gene, such as by introducing an inactivating mutation.

**[ARO4]** In some examples, the engineered host cell may modify the expression of the enzyme 3-deoxy-D-arabino-heptulosonate-7-phosphate (DAHP) synthase. DAHP synthase is encoded by the ARO4 gene. In some examples. DAHP synthase catalyzes the reaction of erythrose-4-phosphate + phosphoenolpyruvic acid → DAHP. as referenced in **FIG. 1****.** An engineered host cell may modify the ARO4 gene to incorporate one or more feedback inhibition alleviating mutations. In particular, a feedback inhibition alleviating mutation (e.g.. ARO4^{FBR}) may be incorporated as a directed mutation to a native ARO4 gene at the original locus: as an additional copy introduced as a genetic integration at a separate locus: or as an additional copy on an episomal vector such as a 2-µm or centromeric plasmid. The identifier "FBR" in the mutation ARO4^{FBR} refers to feedback resistant mutants and mutations. The feedback inhibited copy of the DAHP synthase enzyme may be under a native yeast transcriptional regulation, such as when the engineered host cell is a yeast cell. Alternatively, the feedback inhibited copy of the DAHP synthase enzyme may be introduced to the engineered host cell with engineered constitutive or dynamic regulation of protein expression by placing it under the control of a synthetic promoter. In some cases, the ARO4 gene may be derived from *Saccharomyces cerevisiae.* Examples of modifications to the ARO4 gene include a feedback inhibition resistant mutation. K229L. or Q166K.

[ARO7] In sonic examples, the engineered host cell may modify the expression of the enzyme chorismate mutase. Chorismate mutase is encoded by the ARO7 gene. In some examples, chorismate mutase catalyzes the reaction of chorismate → prephenate, as referenced in **FIG. 1****.** An engineered host cell may modify the ARO7 gene to incorporate one or more feedback inhibition alleviating mutations. In particular, a feedback inhibition alleviating mutation (e.g., ARO7^{FBR}) may be incorporated as a directed mutation to a native ARO7 gene at the original locus: as an additional copy introduced as a genetic integration at a separate locus; or as an additional copy on an episomal vector such as a 2-µm or centromeric plasmid. The identifier "FBR" in the mutation ARO7^{FBR} refers to feedback resistant mutants and mutations. The feedback inhibited copy of the chorismate mutase enzyme may be under a native yeast transcriptional regulation, such as when the engineered host cell is a yeast cell. Alternatively, the feedback inhibited copy of the chorismate mutase enzyme may be introduced to the engineered host cell with engineered constitutive or dynamic regulation of protein expression by placing it under the control of a synthetic promoter. In some cases, the ARO7 gene may be derived from *Saccharomyces cerevisiae.* Examples of modifications to the ARO7 gene include a feedback inhibition resistant mutation or T226I.

[ARO10] In some examples, the engineered host cell may modify the expression of the enzyme phenylpyruvate decarboxylase. Phenylpyruvate decarboxylase is encoded by the ARO10 gene. In some examples, phenylpyruvate decarboxylase catalyzes the reaction of hydroxyphenylpyruvate → 4-hydroxyphenylacetate (4-HPAA), as referenced in **FIG. 1****.** An engineered host cell may be modified to include constitutive overexpression of the ARO10 gene in the engineered host cell. Additionally or alternatively, the engineered host cell may be modified to synthetically regulate the expression of the ARO10 gene in the engineered host cell. In some examples, the engineered host cell may be modified to incorporate a copy, copies, or additional copies, of the ARO10 gene. Additionally or alternatively, the engineered host cell may be modified to incorporate the introduction of a strong promoter element for the overexpression of the ARO10 gene within the engineered host cell. The ARO10 gene may be derived from *Saccharomyces cerevisiae* or another species.

**[ADH2-7, SFA1]** In some examples, the engineered host cell may modify the expression of alcohol dehydrogenase enzymes. Alcohol dehydrogenase enzymes may be encoded by one or more of the ADH2. ADH3. ADH4. ADH5. ADH6. ADH7. and SFA1 genes. In some examples, alcohol dehydrogenase catalyzes the reaction of 4-HPAA → tyrosol. An engineered host cell may be modified to delete the coding region of one or more of the ADH2. ADH3, ADH4. ADH5, ADH6. ADH7. and SFA1 genes in the engineered host cell. Alternatively, the engineered host cell may be modified to disable the functionality of one or more of the ADH2. ADH3, AOH4, ADH5. ADH6, ADH7. and SFA1 genes, such as by introducing an inactivating mutation.

**[ALD2-6]** In some examples, the engineered host cell may modify the expression of aldehyde oxidase enzymes. Aldehyde oxidase enzymes may be encoded by one or more of the ALD2. ALD3, ALD4, ALD5, and ALD6 genes. In some examples, aldehyde oxidase catalyzes the reaction of 4-HPAA → hydroxyphenylacetic acid. An engineered host cell may be modified to delete the coding region of one or more of the ALD2, ALD3, ALD4, ALD5, and ALD6 genes in the engineered host cell. Alternatively, the engineered host cell may be modified to disable the functionality of one or more of the ALD2. ALD3. ALD4, ALD5, and ALD6 genes, such as by introducing an inactivating mutation.

**[AAD4], [AAD6], [AAD10]], [AAD14], [AAD15], [AAD16]** In some examples, the engineered host cell may modify the expression of aryl-alcohol dehydrogenase enzymes. Aryl-alcohol dehydrogenase enzymes may be encoded by one or more of AAD4, AAD6. AAD 10, AAD14, AAD15, and AAD16 genes. In some examples, aryl-alcohol dehydrogenase catalyzes the reaction of aromatic aldehyde + NAD⁺ →aromatic alcohol + NAOH.

**[ARI1]** In some examples, the engineered host cell may modify the expression of an aldehyde reductase. The aldehyde reductase enzy me may be encoded by the ARI1 gene. In some examples, aldehyde reductase catalyzes the reduction of aromatic aldehyde substrates. In some examples, aldehyde reductase catalyzes the reduction of alophatic aldehyde substrates. In some examples the substrate of the aldehyde reductase ARI1 is 4-hydroxyphenylacetaldehyde (4-HPAA). An engineered host cell may be modified to delete the coding region of ARI. Alternatively, the engineered host cell may be modified to functionally disable ARI1, such as by introducing an inactivating mutation.

**[OPI]** In some examples, the engineered host cell may modify the expression of a transcriptional regulator of phospholipid biosynthetic genes. The transcriptional regulator may be encoded by the OPI1 gene. In some examples, the transcriptional regulator represses phospholipid biosynthetic genes. An engineered host cell may be modified to delete the coding region of OPI1. Alternatively, the engineered host cell may be modified to functionally disable OPI1, such as by introducing an inactivating mutation.

**[ARO9]** In some examples, the engineered host cell may modify the expression of the enzyme aromatic aminotransferase. Aromatic aminotransferase is encoded by the ARO9 gene. In some examples, aromatic aminotransferase catalyzes the reaction of hydroxyphenylpyruvate + L-alanine ↔ tyrosine + pyruvate, as referenced in **FIG. 1****.** An engineered host cell may be modified to include constitutive overexpression of the ARO9 gene in the engineered host cell. Additionally or alternatively, the engineered host cell may be modified to synthetically regulate the expression of the ARO9 gene in the engineered host cell. In some examples, the engineered host cell may be modified to incorporate a copy, copies, or additional copies, of the ARO9 gene. Additionally or alternatively, the engineered host cell may be modified to incorporate the introduction of a strong promoter element for the overexpression of the ARO9 gene within the engineered host cell. The ARO9 gene may be derived from *Saccharomyces cerevisiae* or another species.

**[ARO8]** In some examples, the engineered host cell may modify the expression of the enzyme aromatic aminotransferase. Aromatic aminotransferase is encoded by the ARO8 gene In some examples, aromatic aminotransferase catalyzes the reaction of hydroxyphenylpyruvate + glutamate ↔ tyrosine + alpha-ketogluterate, as referenced in **FIG. 1****.** An engineered host cell may be modified to include constitutive overexpression of the AROS gene in the engineered host cell. Additionally or alternatively, the engineered host cell may be modified to synthetically regulate the expression of the ARO8 gene in the engineered host cell. In some examples, the engineered host cell may be modified to incorporate a copy, copies, or additional copies, of the ARO8 gene, Additionally or alternatively, the engineered host cell may be modified to incorporate the introduction of a strong promoter element for the overexpression of the AROS gene within the engineered host cell. The ARO8 gene may be derived from *Saccharomyces cerevisiae* or another species.

**[TYR1]** In some examples, the engineered host cell may modify the expression of the enzyme prephenate dehydrogenase. Prephenate dehydrogenase is encoded by the TYR1 gene. In some examples. prephenate dehydrogenase catalyzes the reaction of prephenate + NADP⁻ → 4-hydroxvphenylpyruvate + CO₂ + NADPH, as referenced in **FIG. 1****.** An engineered host cell may be modified to include constitutive overexpression of the TYR1 gene in the engineered host cell. Additionally or alternatively, the engineered host cell may be modified to synthetically regulate the expression of the TYR1 gene in the engineered host cell. In some examples, the engineered host cell may be modified to incorporate a copy, copies, or additional copies, of the TYR 1 gene. Additionally or alternatively, the engineered host cell may be modified to incorporate the introduction of a strong promoter element for the overexpression of the TYR1 gene within the engineered host cell. The TYR1 gene may be derived from *Saccharomyces cerevisiae* or another species.

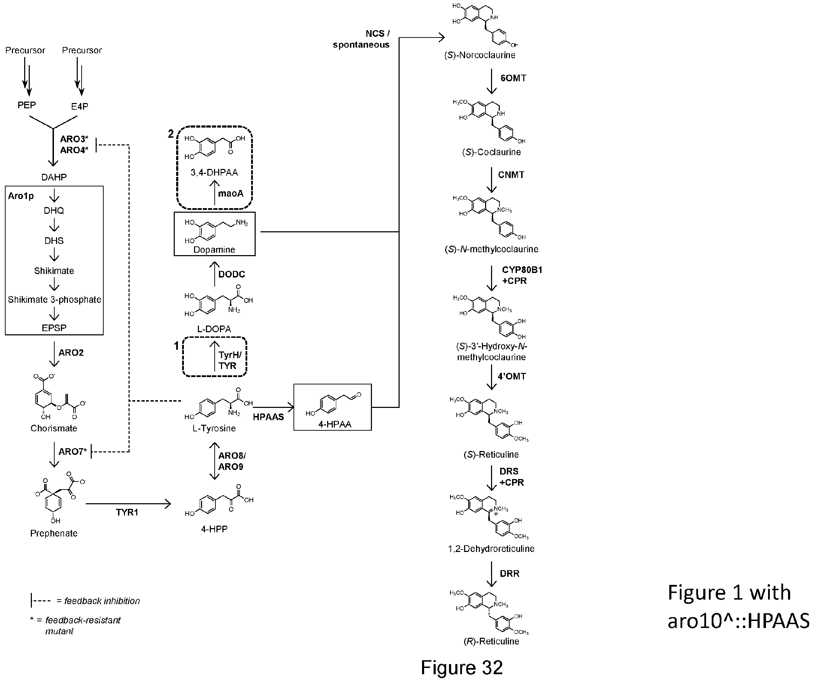

**[HPAAS]** In some examples, the engineered host cell may modify the expression of the enzyme 4-hydroxyphenylacctaldehyde synthase. 4-Hydroxyphenylacetaldehyde synthase is encoded by the 4HPAAS gene. In some examples. 4-hydroxyphenylacctaldehyde synthase catalyzes the reaction of L-tyrosine → 4-hydroxyphenylacctaldehyde as referenced in **FIG. 32****.** The engineered host cell may be modified to include constitutive expression of the 4HPAAS gene in the engineered host cell. Additionally or alternatively, the engineered host cell may be modified to synthetically regulate the expression of the 4HPAAS gene in the engineered host cell. In some examples, the engineered host cell may be modified to incorporate a copy, copies, or additional copies, of the 4HPAAS gene. Additionally or alternatively, the engineered host cell may be modified to incorporate the introduction of a strong promoter element for the overexpression of the 4HPAAS gene within the engineered host cell. In some cases, the 4HPAAS gene may be codon optimized for expression in *Saccharomyces cerevisiae.* The 4HPAAS gene may be derived from *Petroselinum crispum, Rhodiola rosea,* or another species.

**[SAH]** In some examples, the engineered host cell may modify the expression of the enzyme S-adenosyl-L-homocysteine hydrolase. S-adenosyl-L-homocysteine hydrolase is encoded by the SAH1 gene. In some examples. S-adenosyl-L-homocysteine catalyzes the reaction of S-adenosyl-L-homocysteine → L-homocysteine + adenosine as referenced in **FIG. 39** The engineered host cell may be modified to include constitutive expression of the SAH1 gene in the engineered host cell. Additionally or alternatively, the engineered host cell may be modified to synthetically regulate the expression of the SAH1 gene in the engineered host cell. In some examples, the engineered host cell may be modified to incorporate a copy, copies, or additional copies, of the SAH1 gene, Additionally or alternatively, the engineered host cell may be modified to incorporate the introduction of a strong promoter element for the overexpression of the SAH 1 gene within the engineered host cell. In some cases, the SAH1 gene may be codon optimized for expression in *Saccharomyces cerevisiae.* The SAH1 gene may be derived from *Saccharomyces cerevisiae* or another species.

**[SAM]** In some examples, the engineered host cell may modify the expression of the enzyme S-adenosylmethionine synthetase. S-adenosylmethionine synthetase is encoded by the SAM1 and SAM2 genes. In some examples, S-adenosylmethionine synthetase catalyzes the reaction of ATP + methionine → S-adenosylmethionine as referenced in **FIG. 39****.** The engineered host cell may be modified to include constitutive expression of the SAM1 or SAM2 gene in the engineered host cell. Additionally or alternatively, the engineered host cell may be modified to synthetically regulate the expression of the SAM1 or SAM2 gene in the engineered host cell. In some examples, the engineered host cell may be modified to incorporate a copy, copies, or additional copies, of the SAM1 or SAM2 gene, Additionally or alternatively, the engineered host cell may be modified to incorporate the introduction of a strong promoter element for the overexpression of the SAM1 or SAM2 gene within the engineered host cell. In some cases, the SAM1 or SAM2 gene may be codon optimized for expression in *Saccharomyces cerevisiae.* The SAM1 or SAM2 gene may be derived from *Saccharomyces cerevisiae* or another species.

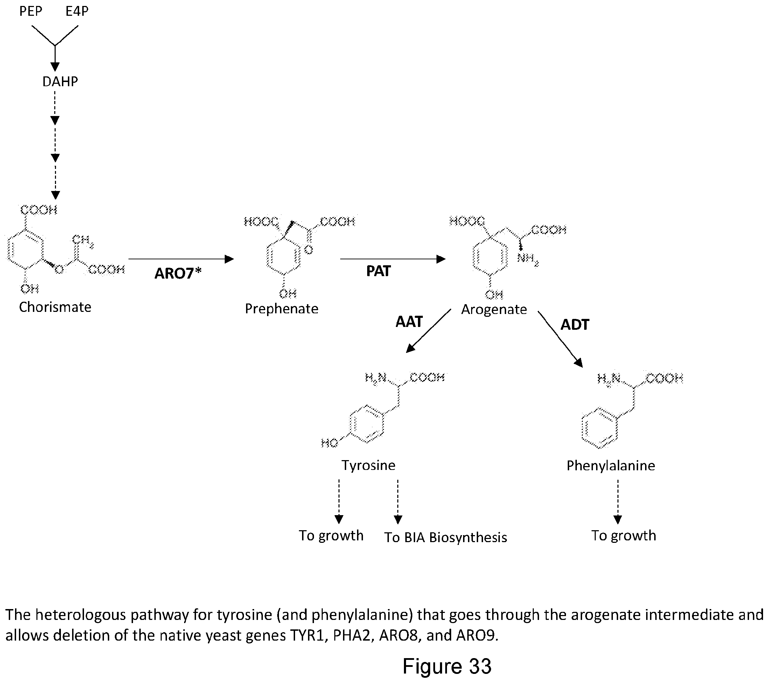

**[PAT]** In some examples, the engineered host cell may modify the expression of the enzyme prephenate aminotransferase. Prephenate aminotransferase is encoded by the PAT gene. In some examples, prephenate aminotransferase catalyzes the reaction of prephenate → arogenate as referenced in **FIG. 33****.** The engineered host cell may be modified to include constitutive expression of the PAT gene in the engineered host cell. Additionally or alternatively, the engineered host cell may be modified to synthetically regulate the expression of the PAT gene in the engineered host cell. In some examples, the engineered host cell may be modified to incorporate a copy, copies, or additional copies, of the PAT gene. Additionally or alternatively, the engineered host cell may be modified to incorporate the introduction of a strong promoter element for the overexpression of the PAT gene within the engineered host cell. In some cases, the PAT gene may be codon optimized for expression in *Saccharomyces cerevisiae.* The PAT gene may be derived from *Arabidopsis thailiona* or another species.

**[AAT]** In some examples, the engineered host cell may modify the expression of the enzyme arogenate dehydrogenase. Arogenate dehydrogenase is encoded by the AAT gene, In some examples, arogenate dehydrogenase catalyzes the reaction of arogenate → tyrosine as referenced in **FIG. 33****.** The engineered host cell may be modified to include constitutive expression of the AAT gene in the engineered host cell. Additionally or alternatively, the engineered host cell may be modified to synthetically regulate the expression of the AAT gene in the engineered host cell. In some examples, the engineered host cell may be modified to incorporate a copy, copies, or additional copies, of the AAT gene. Additionally or alternatively, the engineered host cell may be modified to incorporate the introduction of a strong promoter element for the overexpression of the AAT gene within the engineered host cell. In some cases, the AAT gene may be codon optimized for expression in *Saccharomyces cerevisiae.* The AAT gene may be derived from *Arabidopsis thaliana* or another species.

**[ADT]** In some examples, the engineered host cell may modify the expression of the enzyme arogenate dehydrogenase. Arogenate dehydrogenase is encoded by the ADT gene. In some examples, arogenate dehydrogenase catalyzes the reaction of arogenate → phenylalanine as referenced in **FIG. 33****.** The engineered host cell may be modified to include constitutive expression of the ADT gene in the engineered host cell. Additionally or alternatively, the engineered host cell may be modified to synthetically regulate the expression of the ADT gene in the engineered host cell. In some examples, the engineered host cell may be modified to incorporate a copy, copies, or additional copies, of the ADT gene. Additionally or alternatively, the engineered host cell may be modified to incorporate the introduction of a strong promoter element for the overexpression of the ADT gene within the engineered host cell. In some cases, the ADT gene may be codon optimized for expression in *Saccharomyces cerevisiae.* The ADT gene may be derived from *Papaver* somniferum, *Arabidopsis thaliana* or another species.

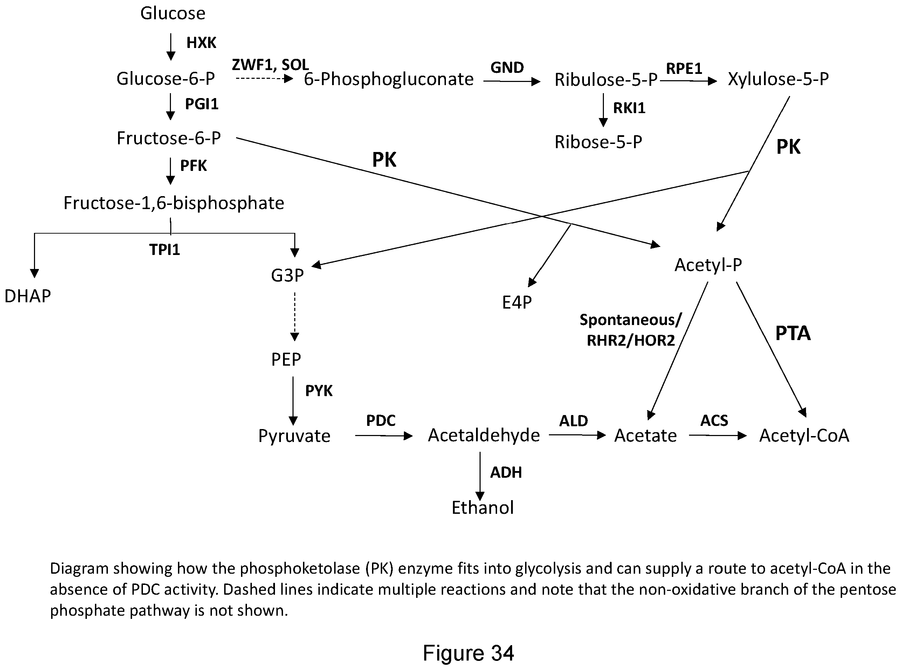

**[PK]** In some examples, the engineered host cell may modify the expression of the enzyme phosphoketolase. Phosphoketolase is encoded by the PK gene, In some examples, phosphoketolase catalyzes the reaction of fructose-6-phosphate → erythrose-4-phosphate + acetyl-phosphate as referenced in **FIG. 34****.** In some examples, phosphoketolase catalyzes the reaction of xylulose-5-phosphate → glyceraldehyde-3-phosphate + acetyl-phosphate as referenced in FIG. 34. The engineered host cell may be modified to include constitutive expression of the PK gene in the engineered host cell. Additionally or alternatively, the engineered host cell may be modified to synthetically regulate the expression of the PK gene in the engineered host cell. In some examples, the engineered host cell may be modified to incorporate a copy, copies, or additional copies, of the PK gene. Additionally or alternatively, the engineered host cell may be modified to incorporate the introduction of a strong promoter element for the overexpression of the PK gene within the engineered host cell. In some cases, the PK gene may be codon optimized for expression in *Saccharomyces cerevisiae.* The PK gene may be derived from *Bifidobacterium breve, Bifidobacterium animalis, Leuconostoc mesenteroides, Clostridium acetobutylicum,* or another species.

**[PTA]** In some examples, the engineered host cell may modify the expression of the enzyme phosphate acetyltransferase. Phosphate acetyltransferase is encoded by the PTA gene. In some examples, phosphate acetyltransferase catalyzes the reaction of acetyl-CoA + phosphate → acetyl-phosphate + CoA as referenced in **FIG. 34****.** The engineered host cell may be modified to include constitutive expression of the PTA gene in the engineered host cell. Additionally or alternatively, the engineered host cell may be modified to synthetically regulate the expression of the PTA gene in the engineered host cell. In some examples, the engineered host cell may be modified to incorporate a copy, copies, or additional copies, of the PTA gene. Additionally or alternatively, the engineered host cell may be modified to incorporate the introduction of a strong promoter element for the overexpression of the PTA gene within the engineered host cell. In some cases, the PTA gene may be codon optimized for expression in *Saccharomyces cerevisiae.* The PTA gene may be derived from *Escherichia coli, Clostridium kluyveri, Methanosarcina thermophila, Salmonella enterica, Bacillus subtilis* or another species.

**[UGT]** In some examples, the engineered host cell may modify the expression of the enzyme uridine 5'-diphospho-glucosy transferase. Uridine 5'-diphospho-glucosyltransferase activity is encoded by the UGT gene, In some examples, uridine 5'-diphospho-glucosyltnmsferase catalyzes the reaction of UDP-glucose + a phenol → UDP + an aryl beta-D-glucoside. The engineered host cell may be modified to include constitutive expression of the UGT gene in the engineered host cell. Additionally or alternatively, the engineered host cell may be modified to synthetically regulate the expression of the UGT gene in the engineered host cell. In some examples, the engineered host cell may be modified to incorporate a copy, copies, or additional copies, of the UGT gene, Additionally or alternatively, the engineered host cell may be modified to incorporate the introduction of a strong promoter element for the overexpression of the UGT gene within the engineered host cell. In some cases, the UGT gene may be codon optimized for expression in *Saccharomyces cerevisiae.* The UGT gene may be derived from *Rhodiola rosea* or another species.

**[TYR]** In some examples, the engineered host cell may modify the expression of the enzyme tyrosinase. Tyrosinase is encoded by the TYR gene. In some examples, tyrosinase catalyzes the reaction of tyrosine → L-DOPA, as referenced in **FIGs. 1** **and** **2****.** In other examples, tyrosinase catalyzes the reaction of L-DOPA → dopaquinone. An engineered host cell may be modified to include constitutive expression of the TYR gene in the engineered host cell. Additionally or alternatively, the engineered host cell may be modified to synthetically regulate the expression of the TYR gene in the engineered host cell. In some examples, the engineered host cell may be modified to incorporate a copy, copies, or additional copies, of the TYR gene. Additionally or alternatively, the engineered host cell may be modified to incorporate the introduction of a strong promoter element for the overexpression of the TYR gene within the engineered host cell. The TYR gene may be derived from *Ralstonia solanacearum, Agaricus bisporus, Escherichia coli* or another species.

**[TyrH]** In some examples, the engineered host cell may modify the expression of the enzyme tyrosine hydroxylase. Tyrosine hydroxylase is encoded by the TyrH gene. In some examples, tyrosine hydroxylase catalyzes the reaction of tyrosine → L-DOPA, as referenced in **FIGs. 1** **and** **2****.** An engineered host cell may be modified to include constitutive expression of the Ty rH gene in the engineered host cell. Additionally or alternatively, the engineered host cell may be modified to synthetically regulate the expression of the TyrH gene in the engineered host cell. In some examples, the engineered host cell may be modified to incorporate a copy, copies, or additional copies, of the TyrH gene, Additionally or alternatively, the engineered host cell may be modified to incorporate the introduction of a strong promoter element for the overexpression of the TyrH gene within the engineered host cell. The TyrH gene may be derived from *Homo sapiens, Rattus norvegicus, Mus musculus, Drosophilia melanogaster, Apis mellifera,* or another species.

**[DODC]** In some examples, the engineered host cell may modify the expression of the enzyme L-DOPA decarboxylase. L-DOPA decarboxylase is encoded by the DODC gene. In some examples, L-DOPA decarboxylase catalyzes the reaction of L-DOPA → dopamine, as referenced in **FIGs. 1** An engineered host cell may be modified to include constitutive expression of the DODC gene in the engineered host cell. Additionally or alternatively, the engineered host cell may be modified to synthetically regulate the expression of the DODC gene in the engineered host cell. In some examples, the engineered host cell may be modified to incorporate a copy, copies, or additional copies, of the DODC gene. Additionally or attematively, the engineered host cell may be modified to incorporate the introduction of a strong promoter element for the overexpression of the DODC gene within the engineered host cell. The DODC gene may be derived from *Pseudomonas putida, Rattus norvegicus,* or another species.

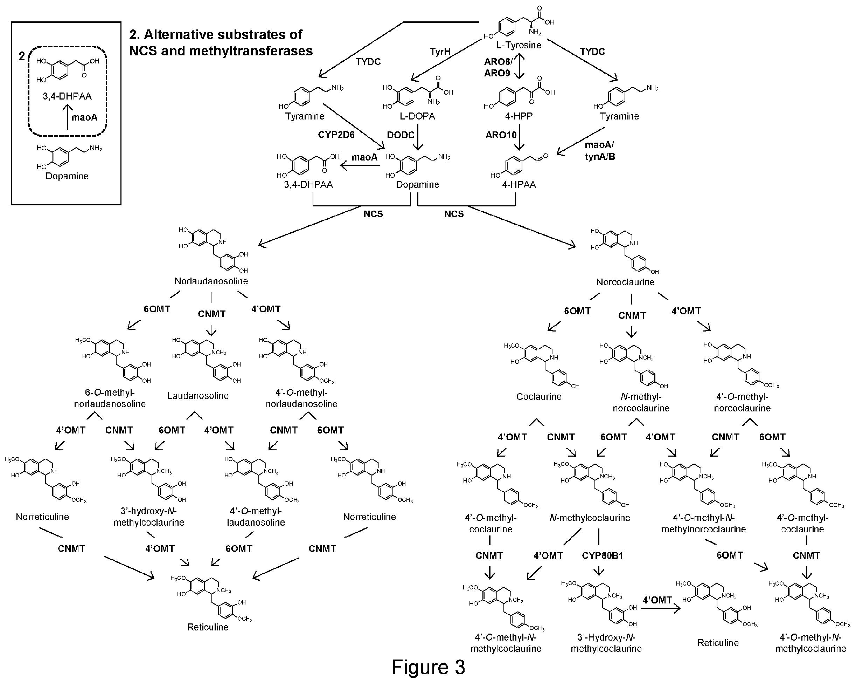

**[TYDC]** In some examples, the engineered host cell may modify the expression of the enzyme tyrosine/DOPA decarboxylase. Tyrosine/DOPA decarboxylase is encoded by the TYDC gene. In some examples, tyrosine/DOPA decarboxylase catalyzes the reaction of L-DOPA → dopamine, as referenced in **FIG. 3****.** An engineered host cell may be modified to include constitutive expression of the TYDC gene in the engineered host cell. Additionally or alternatively, the engineered host cell may be modified to synthetically regulate the expression of the TYDC gene in the engineered host cell. In some examples, the engineered host cell may be modified to incorporate a copy, copies, or additional copies, of the TYDC gene. Additionally or alternatively, the engineered host cell may be modified to incorporate the introduction of a strong promoter element for the overexpression of the TYDC gene within the engineered host cell. The TYDC gene may be derived from *Papaver somniferum* or another species.

**[MAO]** In some examples, the engineered host cell may modify the expression of the enzyme monoamine oxidase. Monoamine oxidase is encoded by the MAO gene. In some examples, monoamine oxidase catalyzes the reaction of dopamine → 3.4-DHPA, as referenced in **FIGs. 1** and **3****.** An engineered host cell may be modified to include constitutive expression of the MAO gene in the engineered host cell. Additionally or alternatively, the engineered host cell may be modified to synthetically regulate the expression of the MAO gene in the engineered host cell. In some examples, the engineered host cell may be modified to incorporate a copy, copies, or additional copies, of the MAO gene. Additionally or alternatively, the engineered host cell may be modified to incorporate the introduction of a strong promoter element for the overexpression of the MAO gene within the engineered host cell. In some cases, the MAO gene may be codon optimized for expression in *Saccharomyces cerevisiae.* The MAO gene may be derived from *Escherichia coli, Homo sapiens. Micrococcus luteus,* or another species.

**[NCS]** In some examples, the engineered host cell may modify the expression of the enzyme norcoclaurine synthase. Norcoclaunne synthase is encoded by the NCS gene. In some examples. norcoclaurine synthase catalyzes the reaction of 4-HPAA + dopamine → (S)-norcoclaurine, as referenced in **FIGs. 1** and **3****.** In particular. **FIG. 1** illustrates a biosynthetic scheme for conversion of L-tyrosine to reticuline via norcoclaurine, in accordance with some embodiments of the invention. **FIG. 1** provides the use of the enzymes TyrH. tyrosine hydroxylase; DODC, DOPA decarboxylase; NCS, norcoclaurine synthase, as discussed herein; 6OMT, 6-*O*-methyltransferase; CNMT, coclaurine N-methyltransferase: CYP80B1, cytochrome P4350 80B1; CPR. cytochrome P450 NADPH reductase; 4 OMT, 3'hydroxy-*N-*methylcoclaunne 4'-*O*-methyltransferase. L-DOPA, L-3,4-dihydroxyphenylalanine: and 4-HPAA, 4-hydroxyphenylacetylaldehyde. Of the enzymes that are illustrated in **FIG. 1****,** 4-HPAA and L-tyrosine are naturally synthesized in yeast. All other listed metabolites are not naturally produced in yeast. Additionally, although TyrH may catalyze the conversion of L-tyrosine to L-DOPA, other enzymes may also be used to perform this step as described in the specification. For example, tyrosinases may also be used to perform the conversion of L-tyrosine to L-DOPA. In addition, other enzymes such as cytochrome P450 oxidases may also be used to perform the conversion of L-tyrosine to L-DOPA. Such enzymes may exhibit oxidase activity on related BIA precursor compounds including L-DOPA and L-tyrosine.

Additionally, norcoclaurine synthase catalyzes the reaction of 3,4-DHPAA + dopamine → (*S*)-norlaudanosoline. as referenced in **FIGs. 1** and **3****.** In particular. **FIG. 3** illustrates a biosynthetic scheme for conversion of L-tyrosine to reticuline via notlaudanosoline. in accordance with some embodiments of the invention. **FIG. 3** provides the use of the enzymes TyrH, tyrosine hydroxylase; DODC. DOPA decarboxylase; maoA, monoamine oxidase; NCS. norcoclaurine synthase; 6OMT, 6-*O*-methyltransferase; CNMT, coclaurine *N*-methyltransferase; 4'OMT, 3hydroxy-*N*-methylcoclaunine 4'-*O*-methyltransferase. L-DOPA, L-3,4-dihydroxyphenylalanine; and 3,4-DHPAA. 3,4-dihydroxyphenylacetaldehyde. Of the enzymes that are illustrated in **FIG. 3****,** L-tyrosine is naturally synthesized in yeast.

An engineered host cell may be modified to include constitutive expression of the NCS gene in the engineered host cell. Additionally or alternatively, the engineered host cell may be modified to synthetically regulate the expression of the NCS gene in the engineered host cell. In some examples, the engineered host cell may be modified to incorporate a copy, copies, or additional copies, of the NCS gene. Additionally or alternatively, the engineered host cell may be modified to incorporate the introduction of a strong promoter element for the overexpression of the NCS gene within the engineered host cell. Additionally, the norcoclaurine synthase may have an N-terminal truncation. In some cases, the NCS gene may be codon optimized for expression in *Saccharomyces cerevisiae* The NCS gene may be derived from *Coptis japonica, Papaver somniferum, Papver bracteatum, Thalicuum flavum, Corydalis saxicola,* or another species.

[6OMT] In some examples, the engineered host cell may modify the expression of the enzyme norcoclaurine 6-*O*-methyltransferase. Norcoclaurine 6-*O*-methyltransferase is encoded by the 6OMT gene, In some examples. norcocLaurine 6-O-methyltransferase catalyzes the reaction of norcoclaurine → coclaurine, as referenced in **FIG. 1****.** In other examples, norcoclaurine 6-*O*-methyltransferase catalyzes the reaction of norlaudanosoline → 3'hydroxycoclaurine, as well as other reactions detailed herein, such as those provided in **FIG. 3****.** Additionally, the engineered host cell may be modified to include constitutive expression of the 6OMT gene in the engineered host cell. Additionally or alternatively, the engineered host cell may be modified to synthetically regulate the expression of the 6OMT gene in the engineered host cell. In some examples, the engineered host cell may be modified to incorporate a copy, copies, or additional copies, of the 6OMT gene, Additionally or alternatively, the engineered host cell may be modified to incorporate the introduction of a strong promoter element for the overexpression of the 6OMT gene within the engineered host cell. The 6OMT gene may be derived from *P. sommiferum, T. flavum. Coptis japonica,* or another species.

**[CNMT]** In some examples, the engineered host cell may modify the expression of the enzyme coclaurine-*N*-methyltransfemse. Coclaurine-*N*-methyltransferase is encoded by the CNMT gene. In some examples, coclaurine-*N*-methyltransferase catalyzes the reaction of coclaurine → *N*-methylcoclaurine, as referenced in **FIG. 1****.** In other examples, the coclaurine-*N*-methyltransferase enzyme may catalyze the reaction of 3'hydroxycoclaunne → 3'hydroxy-*N*-methylcoclaurine. In other examples, coclaurine-*N-*methyltransferase may catalyze other reactions detailed herein, such as those provided in **FIG. 3****.** Additionally, the engineered host cell may be modified to include constitutive expression of the CNMT gene in the engineered host cell. Additionally or alternatively, the engineered host cell may be modified to synthetically regulate the expression of the CNMT gene in the engineered host cell. In some examples, the engineered host cell may be modified to incorporate a copy, copies, or additional copies, of the CNMT gene. Additionally or alternatively, the engineered host cell may be modified to incorporate the introduction of a strong promoter element for the overexpression of the CNMT gene within the engineered host cell. The CNMT gene may be derived from *P. somniferum, T. flavum, Coptisjaponica,* or another species.

**[4'OMT]** In some examples, the engineered host cell may modify the expression of the enzyme 4'-*O*-methyltransferase. 4'-*O*-methyltransferase is encoded by the 4'OMT gene. In some examples, 4'-*O*-methyltransferase catalyzes the reaction of 3'-hydroxy-*N*-methylcoclaurine → reticuline, as referenced in **FIG. 1****.** In other examples, 4'-*O*-methyltransferase catalyzes other reactions detailed herein, such as those provided in **FIG. 3****.** Additionally, the engineered host cell may be modified to include constitutive expression of the 4'OMT gene in the engineered host cell. Additionally or alternatively, the engineered host cell may be modified to synthetically regulate the expression of the 4'OMT gene in the engineered host cell. In some examples, the engineered host cell may be modified to incorporate a copy, copies, or additional copies, of the 4'OMT gene. Additionally or alternatively, the engineered host cell may be modified to incorporate the introduction of a strong promoter element for the overexpression of the 4'OMT gene within the engineered host cell. The 4'OMT gene may be derived from *P*. *somniferum, T*. *flavum*, *Coptisjaponica.* or another species.

**[CYP80B1]** In some examples, the engineered host cell may modify the expression of the enzyme cytochrome P450 80B1. Cytochrome P450 80B1 is encoded by the CYP80B1 gene. In some examples, cytochrome P450 80B1 catalyzes the reaction of *N*-methylcoclaurine → 3'-hydroxy-*N-*methylcoclaurine, as referenced in **FIG. 1****.** An engineered host cell may be modified to include constitutive expression of the cytochrome P450 80B1 gene in the engineered host cell. Additionally or alternatively, the engineered host cell may be modified to synthetically regulate the expression of the cytochrome P450 80B1 gene in the engineered host cell. In some examples, the engineered host cell may be modified to incorporate a copy, copies, or additional copies, of the cytochrome P450 80B1 gene. Additionally or alternatively, the engineered host cell may be modified to incorporate the introduction of a strong promoter element for the overexpression of the cytochrome P450 80B1 gene within the engineered host cell. In some cases, the CYP80B1 gene may be codon optimized for expression in *Saccharomyces cerevisiae*. The cytochrome P450 80B1 gene may be derived from *P*. *somniferum, E. californica, T. flavum,* or another species.

**[FOL2]** In some examples, the engineered host cell may modify the expression of the enzyme GTP cyclohydrolase. GTP cyclohydrolase is encoded by the FOL2 gene. In some examples, GTP cyclohydrolase catalyzes the reaction of GTP → dihydroneopterin triphosphate, as referenced in **FIG. 2****.** The engineered host cell may be modified to include constitutive overexpression of the FOL2 gene in the engineered host cell. The engineered host cell may also be modified to include native regulation. Additionally or alternatively, the engineered host cell may be modified to synthetically regulate the expression of the FOL2 gene in the engineered host cell. In some examples, the engineered host cell may be modified to incorporate a copy, copies, or additional copies, of the FOL2 gene. Additionally or alternatively, the engineered host cell may be modified to incorporate the introduction of a strong promoter element for the overexpression of the FOL2 gene within the engineered host cell. The FOL2 gene may be derived from *Saccharomyces cerevisiae, Homo sapiens, Mus musculus,* or another species.

**(PTPS)** In some examples, the engineered host cell may modify the expression of the enzyme 6-pyruvoyl tetrahydrobiopterin (PTP) synthase. Pynwoyl tetrahydrobiopterin synthase is encoded by the PTPS gene. In some examples, 6-pyruvoyl tetrahydrobiopterin synthase catalyzes the reaction of dihydroneopterin triphosphate → PTP. as referenced in **FIG. 2****.** The engineered host cell may be modified to include constitutive expression of the PTPS gene in the engineered host cell. Additionally or alternatively, the engineered host cell may be modified to synthetically regulate the expression of the PTPS gene in the engineered host cell. In some examples, the engineered host cell may be modified to incorporate a copy, copies, or additional copies, of the PTPS gene. Additionally or alternatively, the engineered host cell may be modified to incorporate the introduction of a strong promoter element for the overexpression of the PTPS gene within the engineered host cell. In some cases, the PTPS gene may be codon optimized for expression in *Saccharomyces cerevisiae*. The PTPS gene may be derived from *Rattus norvegicus, Homo sapiens, Mus musculus*, or another species.

**[SepR]** In some examples, the engineered host cell may modify the expression of the enzyme sepiapterin reductase. Sepiapterin reductase is encoded by the SepR gene. In some examples, sepiapterin reductase catalyzes the reaction of PTP → BH₄, as referenced in **FIG. 2****.** The engineered host cell may be modified to include constitutive expression of the SepR gene in the engineered host cell. Additionally or alternatively, the engineered host cell may be modified to synthetically regulate the expression of the SepR gene in the engineered host cell. In some examples, the engineered host cell may be modified to incorporate a copy, copies, or additional copies, of the ScpR gene. Additionally or alternatively, the engineered host cell may be modified to incorporate the introduction of a strong promoter element for the overexpression of the SepR gene within the engineered host cell. In some cases, the SepR gene may be codon optimized for expression in *Saccharomyces cerevisiae*. The SepR gene may be derived from *Rattus norvegicus, Homo sapiens, Mus musculus*, or another species.

**[PCD]** In some examples, the engineered host cell may modify the expression of the enzyme 4a-hydroxytetrahydrobiopterin (pterin-4α-carbinolamine) dehydratase. 4a-hydroxytetrahydrobiopterin dehydratase is encoded by the PCD gene. In some examples, 4a-hydroxytetrahydrobiopterin dehydratase catalyzes the reaction of 4a-hydroxytetrahydrobiopterin → H₂O + quinonoid dihydropteridine, as referenced in **FIG. 2****.** The engineered host cell may be modified to include constitutive expression of the PCD gene in the engineered host cell. Additionally or alternatively, the engineered host cell may be modified to synthetically regulate the expression of the PCD gene in the engineered host cell. In some examples, the engineered host cell may be modified to incorporate a copy, copies, or additional copies, of the PCD gene. Additionally or alternatively, the engineered host cell may be modified to incorporate the introduction of a strong promoter element for the overexpression of the PCD gene within the engineered host cell. In some cases, the PCD gene may be codon optimized for expression in *Saccharomyces cerevisiae*. The PCD gene may be derived from *Rattus norvegicus, Homo sapiens, Mus musculus,* or another species.

**[QDHPR]** In some examples, the engineered host cell may modify the expression of the enzyme quinonoid dihydropteridine reductase. Quinonoid dihydropteridine reductase is encoded by the QDHPR gene. In some examples, quinonoid dihydropteridine reductase catalyzes the reaction of quinonoid dihydropteridine → BH₄, as referenced in **FIG. 2****.** The engineered host cell may be modified to include constitutive expression of the QDHPR gene in the engineered host cell. Additionally or alternatively, the engineered host cell may be modified to synthetically regulate the expression of the QDHPR gene in the engineered host cell. In some examples, the engineered host cell may be modified to incorporate a copy, copies, or additional copies, of the QDHPR gene. Additionally or alternatively, the engineered host cell may be modified to incorporate the introduction of a strong promoter element for the overexpression of the QDHPR gene within the engineered host cell. In some cases, the QDHPR gene may be codon optimized for expression in *Saccharomyces cerevisiae*. The QDHPR gene may be derived from *Rattus norvegicus, Homo sapiens, Mus musculus*, or another species.

**[DHFR]** In some examples, the engineered host cell may modify the expression of the enzyme dihydrofolate reductase. Dihydrofolate reductase is encoded by the DHFR gene. In some examples, dihydrofolate reductase catalyzes the reaction of 7,8-dihydrobiopterin (BH₂) → 5,6,7,8-tetrahydrobiopterin (BH₄), as referenced in **FIG. 2****.** This reaction may be useful in recovering BH₄ as a co-substrate for the converstion of tyrosine to L-DOPA. as illustrated in **FIG. 2****.** The engineered host cell may be modified to include constitutive expression of the DHFR gene in the engineered host cell. Additionally or alternatively, the engineered host cell may be modified to synthetically regulate the expression of the DHFR gene in the engineered host cell. In some examples, the engineered host cell may be modified to incorporate a copy, copies, or additional copies, of the DHFR gene. Additionally or alternatively, the engineered host cell may be modified to incorporate the introduction of a strong promoter element for the overexpression of the DHFR gene within the engineered host cell. In some cases, the DHFR gene may be codon optimized for expression in *Saccharomyces cerevisiae*. The DHFR gene may be derived from *Rattus norvegicus*, *Homo sapiens,* or another species.

**[DRS-DRR]** As discussed above with regard to epimerizing 1-BIAs, the engineered host cell may modify the expression of a BIA epimerase. The BIA epimerase is encoded by the DRS-DRR gene. In some examples, DRS-DRR may also be referred to as CYP-COR. In some examples, an engineered split version, or an engineered fused version, of a BIA epimerase catalyzes the conversion of (*S*)-1-BIA → (*R*)-1-BIA, as referenced in **FIG. 4**. In particular. **FIG. 4** illustrates a biosynthetic scheme for conversion of L-tyrosine to morphinan alkaloids, in accordance with some embodiments of the invention. **FIG. 4** provides the use of the enzymes CPR. cytochrome P450 reductase: DRS-DRR. dehydroreticuline synthase and dehydroreticuline reductase: SalSyn, salutaridine synthase; SalR, salutaridine reductase; SalAT, salutaridinol 7-*O*-acetyltransferase; T6ODM, thebaine 6-O-demethylase: COR, codeinone reductase: and CODM, codeine-*O*-demethylase.

The engineered host cell may be modified to include constitutive expression of the engineered DRS-DRR gene in the engineered host cell. In some cases, the engineered DRS-DRR gene may encode an engineered fusion epimerase. In some cases, the engineered DRS-DRR gene may encode an engineered split epimerase. Additionally or alternatively, the engineered host cell may be modified to synthetically regulate the expression of the DRS-DRR gene in the engineered host cell. In some examples, the engineered host cell may be modified to incorporate a copy, copies, or additional copies, of the DRS-DRR gene. Additionally or alternatively, the engineered host cell may be modified to incorporate the introduction of a strong promoter element for the overexpression of the DRS-DRR gene within the engineered host cell. The DRS-DRR gene may be derived from *Papaver bracteatum, Papaver somniferum, Papaver setigerum, Chelidonium majus*, or another species.

**[CPR]** In some examples, the engineered host cell may modify the expression of the enzyme cytochrome P450 reductase. The cytochrome P450 reductase is encoded by the CPR gene. In some examples, the cytochrome P450 reductase catalyzes the reaction of *(R)*-reticuline → salutaridine, as referenced in **FIG. 4****.** Additionally, the cytochrome P450 reductase catalyzes other reactions such as those described in FIGs. throughout the application. The engineered host cell may be modified to include constitutive expression of the CPR gene in the engineered host cell. Additionally or alternatively, the engineered host cell may be modified to synthetically regulate the expression of the CPR gene in the engineered host cell. In some examples, the engineered host cell may be modified to incorporate a copy, copies, or additional copies, of the CPR gene. Additionally or alternatively, the engineered host cell may be modified to incorporate the introduction of a strong promoter element for the overexpression of the CPR gene within the engineered host cell. The CPR gene may be derived from *E*. *californica, P. somniferum, H*. *sapiens, S*. *cerevisiae*, *A*. *thaliana,* or another species.

**[SalSyn]** In some examples, the engineered host cell may modify the expression of the enzyme salutaridine synthase. The salutaridine synthase is encoded by the SalSyn gene. In some examples, the salutaridine synthase catalyzes the reaction of *(R)*-reticuline → salutaridine, as referenced in **FIG. 4****.** The engineered host cell may be modified to include constitutive expression of the SalSyn gene in the engineered host cell. Additionally or alternatively, the engineered host cell may be modified to synthetically regulate the expression of the SalSy n gene in the engineered host cell. In some examples, the engineered host cell may be modified to incorporate a copy, copies, or additional copies, of the SalSy n gene. Additionally or alternatively, the engineered host cell may be modified to incorporate the introduction of a strong promoter element for the overexpression of the SalSyn gene within the engineered host cell. In some cases, the SalSyn gene may be codon optimized for expression in *Saccharomyces cerevisiae.* In some examples the SalSyn may be modified at the N-terminus. The SalSyn gene may be derived from *Papaver somniferum, Papaver spp, Chelidonium majus,* or another species.

**[SaIR]** In some examples, the engineered host cell may modify the expression of the enzyme salutaridine reductase. Salutaridine reductase is encoded by the SalR gene. In some examples, salutaridine reductase reversibly catalyzes the reaction of salutaridinol → salutaridine, as referenced in **FIG. 4****.** The engineered host cell may be modified to include constitutive expression of the SalR gene in the engineered host cell. Additionally or alternatively, the engineered host cell may be modified to synthetically regulate the expression of the SalR gene in the engineered host cell. In some examples, the engineered host cell may be modified to incorporate a copy, copies, or additional copies, of the SalR gene. Additionally or alternatively, the engineered host cell may be modified to incorporate the introduction of a strong promoter element for the overexpression of the SalR gene within the engineered host cell. In some cases, the SalR gene may be codon optimized for expression in *Saccharomyces cerevisiae.* The SalR gene may be derived from *Papaver somniferum. Papaver bracteatum, Papaver spp., Chelidonium majus,* or another species.

**[SalAT]** In some examples, the engineered host cell may modify the expression of the enzyme acetyl-CoA:salutaridinol 7-*O*-acetyltransferase. Acetyl-CoA:salutaridinol 7-*O*-acetyltransferase is encoded by the SalAT gene. In some examples, acetyl-CoA:salutaridinol 7-*O*-acetyltransferase catalyzes the reaction of acetyl-CoA + salutaridinol → CoA + 7-*O*-acetylsalutaridinol, as referenced in **FIG. 4****.** The engineered host cell may be modified to include constitutive expression of the SalAT gene in the engineered host cell. Additionally or alternatively, the engineered host cell may be modified to synthetically regulate the expression of the SalAT gene in the engineered host cell. In some examples, the engineered host cell may be modified to incorporate a copy, copies, or additional copies, of the SalAT gene. Additionally or alternatively, the engineered host cell may be modified to incorporate the introduction of a strong promoter element for the overexpression of the SalAT gene within the engineered host cell. In some cases, the SalAT gene may be codon optimized for expression in *Saccharomyces cerevisiae.* The SalAT gene may be derived from *Papaver somniferum*, *Papaver bracteatum, Papaver orientale, Papaver spp.,* or another species.

**[TS]** In some examples, the engineered host cell may modify the expression of the enzyme thebaine synthase. Thebaine synthase is encoded by the TS gene. In some examples. a thebaine synthase or an engineered thebaine synthase catalyzes the reaction of 7-*O*-acetylsalutaridinol → thebaine + acetate, as referenced in **FIG. 4****.** In some examples. the reaction of 7-*O*-acetylsatutaridinol → thebaine + acetate occurs spontaneously, but thebaine synthase catalyzes some portion of this reaction. In particular, **FIG. 4** illustrates a biosynthetic scheme for conversion of L-tyrosine to morphinan alkaloids, in accordance with some embodiments of the invention. **FIG. 4** provides the use of the enzymes CPR, cytochrome P450 reductase; DRS-DRR. dehydroreticuline synthase and dehydroreticuline reductase: SalSy n. salutaridine synthase; SalR, salutaridine seductase; SalAT, salutaridinol 7-*O*-acetyltransferase; TS, thebaine synthase; T6ODM, thebaine 6-*O*-demethylase; COR. codeinone reductase: and CODM, codeine-*O*-demethylase.

The engineered host cell may be modified to include constitutive expression of the TS gene or the engineered TS gene in the engineered host cell. In some cases, the engineered TS gene may encode an engineered fusion enzyme. Additionally or altematively, the engineered host cell may be modified to synthetically regulate the expression of the TS gene in the engineered host cell. In some examples, the engineered host cell may be modified to incorporate a copy, copies, or additional copies, of the TS gene. Additionally or alternatively, the engineered host cell may be modified to incorporate the introduction of a strong promoter element for the overexpression of the TS gene within the engineered host cell. In some cases, the TS gene may be codon optimized for expression in *Saccharomyces cerevisiae*. The TS gene may be derived from *Papaver somniferum, Papaver bracteatum*, *Papaver orientale*, *Papaver spp.,* or another species.

**[T6ODM]** In some examples, the engineered host cell may modify the expression of the enzyme thebaine 6-*O*-demethylase. Thebaine 6-*O* demethylase is encoded by the T60DM gene. In some examples, thebaine 6-*O*-demethylase catalyzes the reaction of thebaine→ neopinone, as referenced in **FIG. 4****.** Once the neopinone has been produced, the neopinone may be converted to codeinone. The conversion of neopinone → codeinone may occur spontaneously. Alternatively, the conversion of neopinone → codeinone may occur as a result of a catalyzed reaction. In other examples, the T6ODM enzyme may catalyze the *O*-demethylation of substrates other than thebaine. For example, T6ODM may *O*-demethylate oripavine to produce morphinone. Alternatively, T60DM may catalyze the *O-*demethylation of BIAs within the 1-benzylisoquinoline, protoberberine, or protopine classes such as papaverine, canadine, and allocryptopine, respectively. The engineered host cell may be modified to include constitutive expression of the T60DM gene in the engineered host cell. Additionally or alternatively, the engineered host cell may be modified to synthetically regulate the expression of the T60DM gene in the engineered host cell. In some examples, the engineered host cell may be modified to incorporate a copy, copies, or additional copies, of the T6ODM gene. Additionally or alternatively, the engineered host cell may be modified to incorporate the introduction of a strong promoter element for the overexpression of the T6ODM gene within the engineered host cell. In some cases, the T6ODM gene may be codon optimized for expression in *Saccharomyces cerevisiae*. The T6ODM gene may be derived from *Papaver somniferum*, or another species.

**[NPI]** In some examples, the engineered host cell may modify the expression of the enzyme neopinone isomerase. Neopinone isomerase is encoded by the NPI gene. In some examples, a neopinone isomerase or an engineered neopinone isomerase catalyzes the reaction of neopinone → codeinone, as referenced in **FIG. 4****.** In some examples, the reaction of neopinone → codeinone occurs spontaneously. but neopinone isomerase catalyzes some portion of this reaction. In particular. **FIG. 4** illustrates a biosynthetic scheme for conversion of L-tycosine to morphinan alkaloids, in accordance with some embodiments of the invention. **FIG. 4** provides the use of the enzymes CPR, cytochrome P450 reductase: DRS-DRR, dehydroreticuline synthase and dehydroreticuline reductase: SalSyn, salutandine synthase: SalR, salutaridine reductase: SalAT, salutaridinol 7-*O*-acety transferase; TS, thebaine synthase; T6ODM, thebaine 6-*O*-demethylase; NPI, neopinone isomerase: COR, codeinone reductase: and CODM. codeine-*O*-demelhylase.

The engineered host cell may be modified to include constitutive expression of the NPI gene or the engineered NPI gene in the engineered host cell. In some cases, the engineered NPI gene may encode an engineered fusion enzyme. Additionally or alternatively, the engineered host cell may be modified to synthetically regulate the expression of the NPI gene in the engineered host cell. In some examples, the engineered host cell may be modified to incorporate a copy, copies, or additional copies, of the NPI gene. Additionally or alternatively, the engineered host cell may be modified to incorporate the introduction of a strong promoter element for the overexpression of the NPI gene within the engineered host cell. In some cases, the NPI gene may be codon optimized for expression in *Saccharomyces cerevisiae*. The NPI gene may be derived from *Papaver somniferum, Papaver bracteatum*, *Papaver orientale, Papaver spp.,* or another species.

**[COR]** In some examples, the engineered host cell may modify the expression of the enzyme codeinone reductase. Codeinone reductase is encoded by the COR gene. In some examples, codeinone reductase catalyzes the reaction of codeinone to codeine, as referenced in **FIG. 4****.** In some cases, codeinone reductase can catalyze the reaction of neopinone to neopine. In other examples. COR can catalyze the reduction of other morphinans including hydrocodone → dihydrocodeine. 14-hydroxycodeinone → 14-hydroxycodeine, and hydromorphone → dihydromorphine. The engineered host cell may be modified to include constitutive expression of the COR gene in the engineered host cell. Additionally or alternatively, the engineered host cell may be modified to synthetically regulate the expression of the COR gene in the engineered host cell. In some examples, the engineered host cell may be modified to incorporate a copy, copies, or additional copies, of the COR gene. Additionally or alternatively, the engineered host cell may be modified to incorporate the introduction of a strong promoter element for the overexpression of the COR gene within the engineered host cell. In some cases, the COR gene may be codon optimized for expression in *Saccharomyces cerevisiae*. Additionally or alternatively, the COR gene may be modified with the addition of targeting sequences for mitochondria, vacuole, endoplasmic reticulum, or a combination thereof. The COR gene may be derived from *Papaver somniferum,* or another species.

**[CODM]** In some examples, the engineered host cell may modify the expression of the enzyme codeine *O*-demethylase. Codeine O-demethylase is encoded by the CODM gene. In some examples, codeine *O*-demethylase catalyzes the reaction of codeine to morphine, as referenced in **FIG. 4****.** Codeine O-demethylase can also catalyze the reaction of neopine to neomorphine. Codeine *O*-demethylase can also catalyze the reaction of thebaine to oripavine. In other examples, CODM may catalyze the O-demethylation of BIAs within the 1-benzylisoquinoline, aporphine, and protoberberine classes such as reticuline, isocorydine, and scoulerine, respectively. In other examples, the CODM enzyme may catalyze an *O,O*-demethylenation reaction to cleave the methylenedioxy bridge structures in protopines. The engineered host cell may be modified to include constitutive expression of the CODM gene in the engineered host cell. Additionally or alternatively, the engineered host cell may be modified to synthetically regulate the expression of the CODM gene in the engineered host cell. In some examples. the engineered host cell may be modified to incorporate a copy, copies, or additional copies, of the CODM gene. Additionally or altematively, the engineered host cell may be modified to incorporate the introduction of a strong promoter element for the overexpression of the CODM gene within the engineered host cell. In some cases, the CODM gene may be codon optimized for expression in *Saccharomyces cerevisiae*. Additionally or alternatively, the CODM gene may be modified with the addition of targeting sequences for mitochondria. The CODM gene may be derived from *Papaver somniferum, Papaver spp*., or another species.

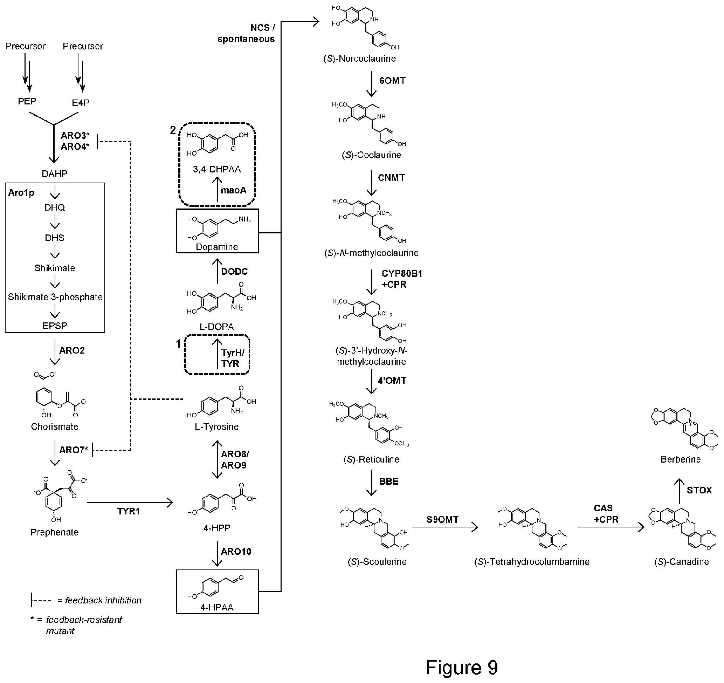

**[BBE]** In some examples, the engineered host cell may modify the expression of the enzyme berberine bridge enzyme. The berberine bridge enzyme is encoded by the BBE gene. In some examples, berberine bridge enzyme catalyzes the reaction of *(S)*-reticuline → *(S)*-scoulerine., as referenced in **FIG. 9. FIG. 9** illustrates a biosynthetic scheme for conversion of L-tyrosine to protoberberine alkaloids, in accordance with some embodiments of the invention. In particular, **FIG. 9** provides the use of the enzymes BBE. berberine bridge enzyme; S9OMT, scoulerine 9-*O*-methyltransferase; CAS, canadine synthase: CPR, cytochrome P450 reductase: and STOX, tetrahydroprotoberberine oxidase. The engineered host cell may be modified to include constitutive expression of the BBE gene in the engineered host cell. Additionally or alternatively, the engineered host cell may be modified to synthetically regulate the expression of the BBE gene in the engineered host cell. In some examples, the engineered host cell may be modified to incorporate a copy, copies, or additional copies, of the BBE gene. Additionally or alternatively, the engineered host cell may be modified to incorporate the introduction of a strong promoter element for the overexpression of the BBE gene within the engineered host cell. The BBE gene may be derived from *Papaver somniferum*, *Argemone mexicana, Eschscholzia californica, Berberis stolonifera, Thalictrum flavum subsp. glaucum, Coptis japonica,Papaver spp.,* or another species.

**[CYP2D6]** In some examples, the engineered host cell may modify the expression of cytochrome P450. family 2. subfamily D. polypeptide 6. This particular cytochrome P450 is encoded by the CYP2D6 gene. This particular cytochrome P450 enzyme may be charactenzed as a promiscuous oxidase. In some examples, this particular cytochrome P450 enzyme may catalyze the reaction of (R)-reticuline + NADPH + H⁺ + O₂ → salutaridine + NADP⁺ + 2 H₂O, among other reactions.

**[S9OMT]** In some examples, the engineered host cell may modify the expression of the enzyme *S*-adenosyl-L-methionine:*(S)*-scoulerine 9-*O*-methyltransferase. *S*-adenosyl-L-methionine:*(S)*-scoulerine 9-*O*-methyltransferase is encoded by the S9OMT gene. In some examples. *S*-adenosyl-L-methionine:*(S)-*scoulerine 9-*O*-methyltransferase catalyzes the reaction of *S*-adenosyl-L-methionine + *(S)*-scoulerine → *S*-adenosyl-L-homocysteine + *(S)*-tetrahydrocolumbamine, as referenced in **FIG. 9****.** The engineered host cell may be modified to include constitutive expression of the S9OMT gene in the engineered host cell. Additionally or alternatively, the engineered host cell may be modified to synthetically regulate the expression of the S9OMT gene in the engineered host cell. In some examples, the engineered host cell may be modified to incorporate a copy, copies, or additional copies, of the S9OMT gene. Additionally or alternatively, the engineered host cell may be modified to incorporate the introduction of a strong promoter element for the overexpression of the S9OMT gene within the engineered host cell. In some cases, the S9OMT gene may be codon optimized for expression in *Saccharomyces cerevisiae*. The S9OMT gene may be derived from *Thalictrum flavum subsp. glaucum, Coptis japonica, Coptis chinensis, Papaver somniferum, Thalictrum spp*., *Coptis spp*., *Papaver spp*., or another species. In some examples, the S9OMT gene may be 100% similar to the naturally occurring gene.

**[CAS]** In some examples, the engineered host cell may modify the expression of the enzyme *(S)-*canadine synthase. *(S)*-canadine synthase is encoded by the CAS gene. In some examples, *(S)-*canadine synthase catalyzes the reaction of *(S)*-tetrahydrocolumbamine → *(S)*-canadine, as referenced in **FIG. 9****.** The engineered host cell may be modified to express the CAS gene in the engineered host cell. The engineered host cell may be modified to include constitutive expression of the CAS gene in the engineered host cell. Additionally or alternatively, the engineered host cell may be modified to synthetically regulate the expression of the CAS gene in the engineered host cell. In some examples, the engineered host cell may be modified to incorporate a copy, copies, or additional copies, of the CAS gene. Additionally or alternatively, the engineered host cell may be modified to incorporate the introduction of a strong promoter element for the overexpression of the CAS gene within the engineered host cell. The CAS gene may be derived from *Thalictrum flavum subsp. glaucum, Coptis japonica, Thalictrum spp., Coptis spp.,* or another species.

**[STOX]** In some examples, the engineered host cell may modify the expression of the enzyme *(S)*-tetrahydroprotoberberine oxidase. *(S)*-tetrahydroprotoberberine oxidase is encoded by the STOX gene. In some examples, *(S)*-tetrahydroprotoberberine oxidase catalyzes the reaction of *(S)-*tetrahydroberberine + 2 O₂ → berberine + 2 H₂O₂, as referenced in **FIG. 9** The engineered host cell may be modified to include constitutive expression of the STOX gene in the engineered host cell. Additionally or alternatively, the engineered host cell may be modified to synthetically regulate the expression of the STOX gene in the engineered host cell. In some examples, the engineered host cell may be modified to incorporate a copy, copies, or additional copies, of the STOX gene. Additionally or alternatively, the engineered host cell may be modified to incorporate the introduction of a strong promoter element for the overexpression of the STOX gene within the engineered host cell. In some examples the STOX may be modified at the N-terminus. In some cases, the STOX gene may be codon optimized for expression in *Saccharomyces cerevisiae*. The STOX gene may be derived from *Berberis wilsonae, Coptis japonica, Berberis spp., Coptis spp*., or another species.

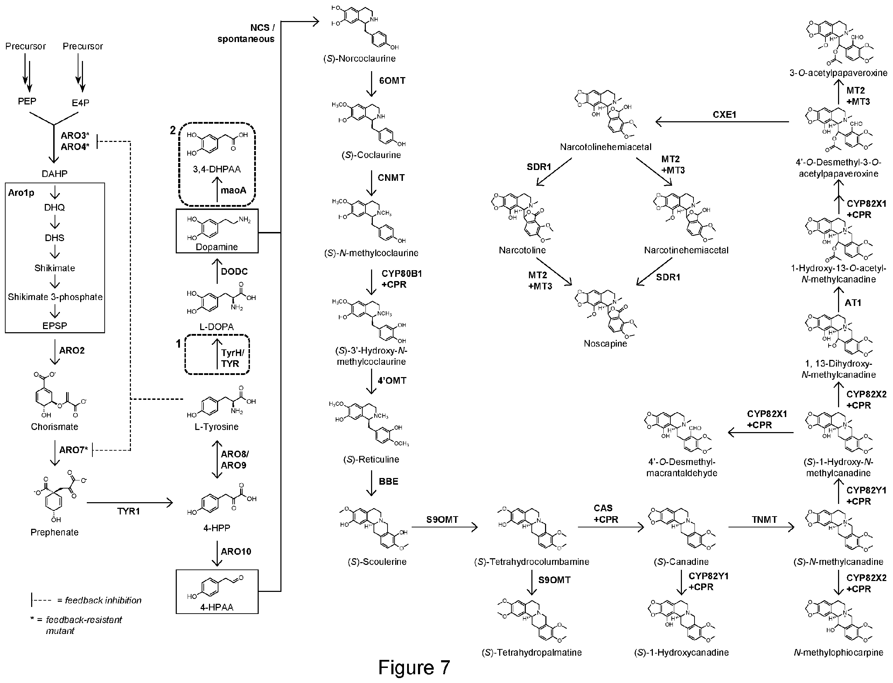

**[TNMT]** In some examples, the engineered host cell may modify the expression of the enzyme tetrahydroprotoberberine-*N*-methyltransferase. Tetrahydroprotoberberine-*N*-methyltransferase is encoded by the TNMT gene. In some examples, tetrahydroprotoberberine-*N*-methyltransferase catalyzes the reaction of canadine → *N*-methylcanadine, as referenced in **FIG. 7 FIG. 7** illustrates a biosynthetic scheme for conversion of L-tyrosine to noscapine, noscapinoid, and phthalideisoquinoline, in accordance with some embodiments of the invention. In particular. **FIG. 7** provides the use of the enzymes BBE. berberine bridge enzyme: S9OMT, scoulerine 9-*O*-methyltransferase; CAS. canadine synthase. CPR, cytochrome P450 reductase: TNMT, tetrahydroprotoberberine cas-*N*-methyltransferase; CYP82Y 1, *N-*methylcanadine 1-hydroxylase; CYP82X2. 1-hydroxy-*N*-methylcanadine 13-hydroxylase; AT1, 1,13-dihydroxy-*N*-methylcandine 13-*O*-acetyltransferase; CYP82X1, 4'-*O*-desmethyl-3-*O*-acetylpapaveroxine synthase: CXE1, narcotine hemiacetal synthase; NOS (or SDR1), noscapine synthase; MT2. narcotoline-4'-*O*-methyltrasnferase 1; MT3, narcotoline-4'-*O*-methyltransferase 2: and 6OMT. 6-*O*-methyltransferase.

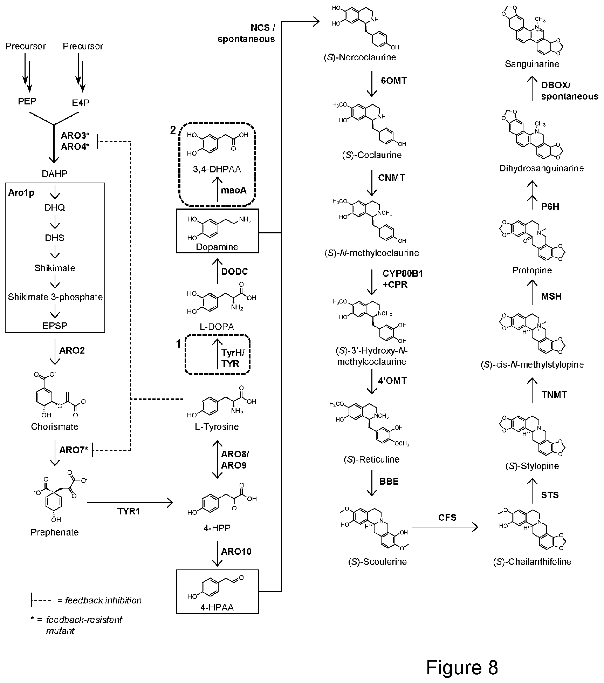

In other examples. tetrahydroprotobeiberine-*N*-methyltransferase catalyzes the reaction of stylopine → cis-*N*-methylstylopine, as referenced in **FIG. 8. FIG. 8** illustrates a biosynthetic scheme for conversion of L-tyrosine to sanguinarine and benzophenanthridine alkaloids, in accordance with some embodiments of the invention In particular, **FIG. 8** provides the use of the enzymes BBE, berberine bridge enzyme; CFS. cheilanthifoline synthase; STS, stylopine synthase; TNMT, tetrahydroberberine *N-*methyltransferase; MSH, cis-*N*-methylstylopine 14-hydroxylase: P6H, protopine 6-hydroxylase; and DBOX, dihydrobenzophenanthride oxidase. The engineered host cell may be modified to include constitutive expression of the TNMT gene in the engineered host cell. Additionally or alternatively, the engineered host cell may be modified to synthetically regulate the expression of the TNMT gene in the engineered host cell. In some examples, the engineered host cell may be modified to incorporate a copy, copies, or additional copies, of the TNMT gene. Additionally or alternatively, the engineered host cell may be modified to incorporate the introduction of a strong promoter element for the overexpression of the TNMT gene within the engineered host cell. In some cases, the TNMT gene may be codon optimized for expression in *Saccharomyces cerevisiae*. The TNMT gene may be derived from *Papaver somniferum*, *Eschscholzia califormca, Papaver bracteatum, Argemone mexicana*, or another species.

**[CYP82Y1]** In some examples, the engineered host cell may modify the expression of the enzyme *N*-methylcanadine 1-hydroxylase. *N*-methylcanadine 1-hydroxylase is encoded by the CYP82Y1 gene. In some examples. *N*-methylcanadine 1-hydroxylase catalyzes the reaction of *N*-methylcanadine → 1-hydroxy-*N*-methylcanadine, as referenced in **FIG. 7****.** The engineered host cell may be modified to include constitutive expression of the CYP82Y 1 gene in the engineered host cell. Additionally or alternatively, the engineered host cell may be modified to synthetically regulate the expression of the CYP82Y1 gene in the engineered host cell. In some examples, the engineered host cell may be modified to incorporate a copy, copies, or additional copies, of the CYP82Y 1 gene. Additionally or alternatively, the engineered host cell may be modified to incorporate the introduction of a strong promoter element for the overexpression of the CYP82Y1 gene within the engineered host cell. In some cases, the CYP82Y1 gene may be codon optimized for expression in *Saccharomyces cerevisiae*. In some examples the CYP82Y1 may be modified at the N-terminus. The CYP82Y1 gene may be derived from *Papaver somniferum*, *Papaver spp., Plantago arenaria, Rauwolfia heterophylla, Adlumia fungosa, Hydrastis canadensis, Stylomecon heterophylla, Hypecoum,* or another species.

**[CYP82X2]** In some examples, the engineered host cell may modify the expression of the enzyme 1-hydroxy-*N*-methylcanadine 13-hydroxylase. 1-hydroxy-*N*-methylcanadine 13-hydroxylase is encoded by the CYP82X2 gene. In some examples, 1-hydroxy-*N*-methylcanadine 13-hydroxylase catalyzes the reaction of 1-hydroxy-*N*-methylcanadine → 1-hydroxy-*N*-methylopinocarpine (i.e. 1,13-dihydroxy-*N*-methylcanadine), as referenced in **FIG. 7****.** The engineered host cell may be modified to include constitutive expression of the CYP82X2 gene in the engineered host cell. Additionally or alternatively, the engineered host cell may be modified to synthetically regulate the expression of the CYP82X2 gene in the engineered host cell. In some examples, the engineered host cell may be modified to incorporate a copy, copies, or additional copies, of the CYP82X2 gene. Additionally or alternatively, the engineered host cell may be modified to incorporate the introduction of a strong promoter element for the overexpression of the CYP82X2 gene within the engineered host cell. In some cases. the CYP82X2 gene may be codon optimized for expression in *Saccharomyces cerevisiae* In some examples the CYP82X2 may be modified at the N-terminus. The CYP82X2 gene may be derived from *P. somniferum, Papaver spp, Plantago arenaria, Rauwolfia heterophylla, Adlumia fungosa, Hydrastis Canadensis, Stylomecon heterophylla, Dactylicapnos torulosa, Glaucium flavum, Berberis laurina, B. Vulgaris, Corydalis spp, Fumaria spp, Dactylicapnos spp*., or another species. In some examples, the CYP82X2 gene may undergo N-terminus engineering. In some examples, N-terminus engineering may include N-terminal truncation.

**[CYP82X1]** In some examples, the engineered host cell may modify the expression of the enzyme 4'-*O*-desmethyl-3-*O*-acetylpapaveroxine synthase. 4'-*O*-desmethyl-3-*O*-acetylpapaveroxine synthase is encoded by the CYP82XI gene. In some examples, 4'-*O*-desmethyl-3-*O*-acetylpapaveroxine synthase catalyzes the reaction of 1-hydroxy-13-*O*-acetyl-*N*-methylcanadine → 4'-*O*-desmethyl-3-*O-*acetylpapaveroxine, as referenced in FIG. 7. Additionally, CYP82X1 catalyzes the reaction of 1-hydroxy-*N*-methylcanadine → 4'-*O*-desmethylmacrantaldehyde. The engineered host cell may be modified to include constitutive expression of the CYP82X1 gene in the engineered host cell. Additionally or alternatively, the engineered host cell may be modified to synthetically regulate the expression of the CYP82X1 gene in the engineered host cell. In some examples, the engineered host cell may be modified to incorporate a copy. copies. or additional copies, of the CYP82XI gene. Additionally or alternatively, the engineered host cell may be modified to incorporate the introduction of a strong promoter element for the overexpression of the CYP82X1 gene within the engineered host cell. In some cases, the CYP82X1 gene may be codon optimized for expression in *Saccharomyces cerevisiae.* In some examples the CYP82X1 may be modified at the N-terminus. The CYP82X1 gene may be derived from *Papaver somniferum, Papaver spp.. Plantago arenaria, Rauwolfia heterophylla, Adlumia fungosa*, *Hydrastis canadensis, Stylomecon heterophylla, Hypecoum,* or another species. In other examples. the CYP82X1 gene may undergo N-terminus engineering. In some examples, N-terminus engineering may include N-terminal truncation

**[CFS]** In some examples, the engineered host cell may modify the expression of the enzyme cheilanthifoline synthase. Cheilanthifoline synthase is encoded by the CFS gene. In some examples, cheilanthifoline synthase catalyzes the reaction of scoulerine → cheilaothifoline, as referenced in FIG. 8. An engineered host cell may be modified to include constitutive expression of the CFS gene in the engineered host cell. Additionally or alternatively, the engineered host cell may be modified to synthetically regulate the expression of the CFS gene in the engineered host cell. In some examples, the engineered host cell may be modified to incorporate a copy. copies. or additional copies. of the CFS gene. Additionally or alternatively, the engineered host cell may be modified to incorporate the introduction of a strong promotor element for the overexpression of the CFS gene within the engineered host cell. The CFS gene may be derived from *P. somniferum, E. californica, A. mexicana,* or another species.

**[STS]** In some examples, the engineered host cell may modify the expression of the enzyme stylopine synthase. Stylopine synthase is encoded by the STS gene. In some examples, stylopine synthase catalyzes the reaction of cheilanthifoline → stylopine, among other reactions, as referenced in **FIG. 8****.** An engineered host cell may be modified to include constitutive expression of the STS gene in the engineered host cell. Additionally or alternatively, the engineered host cell may be modified to synthetically regulate the expression of the STS gene in the engineered host cell. In some examples, the engineered host cell may be modified to incorporate a copy. copies, or additional copies, of the STS gene. Additionally or alternatively, the engineered host cell may be modified to incorporate the introduction of a strong promotor element for the overexpression of the STS gene within the engineered host cell. The STS gene may be derived from *P. somniferum, E. californica. A. mexicana,* or another species.

**[MSH]** In some examples, the engineered host cell may modify the expression of the enzyme cis-*N*-methylstylopine 14-hydroxylase. Cis-*N*-methylstylopine 14-hydroxylase is encoded by the MSH gene. In some examples, cis-*N*-methylstylopine 14-hydroxylase catalyzes the reaction of cis-*N-*methylstylopine → protopine, as referenced in **FIG. 8****.** An engineered host cell may be modified to include constitutive expression of the MSH gene in the engineered host cell. Additionally or alternatively. the engineered host cell may be modified to synthetically regulate the expression of the MSH gene in the engineered host cell. In some examples, the engineered host cell may be modified to incorporate a copy, copies, or additional copies, of the MSH gene. Additionally or alternatively, the engineered host cell may be modified to incorporate the introduction of a strong promotor element for the overexpression of the MSH gene within the engineered host cell. The MSH gene may be derived from *P. somniferum* or another species.

**[P6H]** In some examples. the engineered host cell may modify the expression of the enzyme protopine-6-hydroxylase. Protopine-6-hydroxylase is encoded by the P6H gene. In some examples, protopine-6-hydroxylase catalyzes the reaction of Protopine → 6-hydroxyprotopine, as referenced in **FIG. 8****.** An engineered host cell may be modified to include constitutive expression of the P6H gene in the engineered host cell. Additionally or alternatively, the engineered host cell may be modified to synthetically regulate the expression of the P6H gene in the engineered host cell. In some examples, the engineered host cell may be modified to incorporate a copy, copies, or additional copies, of the P6H gene. Additionally or alternatively, the engineered host cell may be modified to incorporate the introduction of a strong promotor element for the overexpression of the CFS gene within the engineered host cell. The P6H gene may be derived from *P. somniferum, E. californica*, or another species.

**[DBOX]** In some examples, the engineered host cell may modify the expression of the enzyme dihydrobenzophenanthridine oxidase. Dihydrobenzophenanthridine oxidase is encoded by the DBOX gene. In some examples, dihydrobenzophenanthridine oxidase catalyzes the reaction of dihydrosanguinarine → sanguinarine, as referenced in **FIG. 8****.** An engineered host cell may be modified to include constitutive expression of the DBOX gene in the engineered host cell. Additionally or alternatively. the engineered host cell may be modified to synthetically regulate the expression of the DBOX gene in the engineered host cell. In some examples. the engineered host cell may be modified to incorporate a copy, copies, or additional copies, of the DBOX gene. Additionally or alternatively, the engineered host cell may be modified to incorporate the introduction of a strong promotor element for the overexpression of the DBOX gene within the engineered host cell. The DBOX gene may be derived from *P. somniferum* or another species.

**[AT1]** In some examples, the engineered host cell may modify the expression of the enzyme 1, 13-dihydroxy-*N*-methylcanadine 13-*O* acetyl transferase. 1,13-dihydroxy-N-methylcanadine 13-*O* acetyltransferase is encoded by the AT1 gene. In some examples. 1, 13-dihydroxy-N-methylcanadine 13-*O* acetyltransferase catalyzes the reaction of 1, 13-dihydroxy-N-methylcanadine → 1-hydroxy-13-*O-*acetyl-*N*-methylcanadine, as referenced in **FIG. 7. FIG. 7** illustrates a biosynthetic scheme for conversion of canadine to noscapine, in accordance with some embodiments of the invention. The engineered host cell may be modified to include constitutive expression of the AT1 gene in the engineered host cell. Additionally or alternatively, the engineered host cell may be modified to synthetically regulate the expression of the AT1 gene in the engineered host cell. In some examples, the engineered host cell may be modified to incorporate a copy, copies, or additional copies, of the AT1 gene. Additionally or alternatively, the engineered host cell may be modified to incorporate the introduction of a strong promoter clement for the overexpression of the AT1 gene within the engineered host cell. In some cases, the AT1 gene may be codon optimized for expression *in Saccharomyces cerevisiae.* The AT1 gene may be derived from *P. somniferum, Papaver spp, Plantago arenaria, Rauwolfia heterophylla, Adlumia fungosa, Hydrastis Canadensis, Stylomecon heterophylla, Hypecoum leptocarpum, Dactylicapnos torulosa, Glaucium flavum, Berberis laurina, B. Vulgaris, Corydalis spp, Fumaria spp, Dactylicapnos spp,* or another species.

**[CXE1 or CXE2]** In sonic examples, the engineered host cell may modify the expression of the enzyme narcotinehemiacetal synthase. Narcotinehemiacetal synthase is encoded by the CXE1 gene. The enzyme encoded by the CXE2 gene can also function as a narcotinehemiacetal synthase. In some examples, narcotinehemiacetal synthase catalyzes the reaction of 4'-*O*-desmethyl-3-*O*-acetylpapaveroxine → narcotolinehemiacetal and 3-*O*-acetylpapaveroxine → narcotinchemiacetal, as referenced in **FIG. 7****.** The engineered host cell may be modified to include constitutive expression of the CXE1 or CXE2 gene in the engineered host cell. Additionally or alternatively, the engineered host cell may be modified to synthetically regulate the expression of the CXE1 or CXE2 gene in the engineered host cell. In some examples, the engineered host cell may be modified to incorporate a copy, copies, or additional copies, of the CXE1 or CXE2 gene. Additionally or alternatively, the engineered host cell may be modified to incorporate the introduction of a strong promoter element for the overexpression of the CXE1 or CXE2 gene within the engineered host cell. In some cases, the CXEl or CXE2 gene may be codon optimized for expression in *Saccharomyces cerevisiae.* The CXE1 or CXE2 gene may be derived from *P. somniferum, Papaver spp, Plantago arenaria, Rauwolfia heterophylla, Adlumia fungosa, Hydrastis Canadensis, Stylomecon heterophylla, Hypecoum leptocarpum, Dactylicapnos torulosa, Glaucium flavum, Berberis laurina, B. Vulgaris, Corydalis spp, Fumaria spp. Dactylicapnos spp.* or another species.

**[SDR1]** In some examples, the engineered host cell may modify the expression of the enzyme noscapine synthase. Noscapine synthase is encoded by the SDR1 gene. In some examples, noscapine synthase catalyzes the reaction of narcotolinchemiacetal → narcotoline, as referenced in **FIG. 7****.** Additionally, noscapine synthase catalyzes the reaction of narcotinehemiacetal → noscapine. The engineered host cell may be modified to include constitutive expression of the SDR1 gene in the engineered host cell. Additionally or alternatively, the engineered host cell may be modified to synthetically regulate the expression of the SDR1 gene in the engineered host cell. In some examples, the engineered host cell may be modified to incorporate a copy. copies, or additional copies, of the SDR1 gene. Additionally or atternatively, the engineered host cell may be modified to incorporate the introduction of a strong promoter element for the overexpression of the SDR1 gene within the engineered host cell. In some cases, the SDR1 gene may be codon optimized for expression in *Saccharomyces cerevisiae.* The SDR1 gene may be derived from *P. somniferum, Papaver spp, Plantago arenaria, Rauwolfia heterophylla, Adlumia fungosa, Hyvdrastis Canadensis, Stylomecon heterophylla, Hypecoum leptocarpum, Dactylicapnos torulosa, Glaucium flavum, Berberis laurina, B. Vulgaris, Corydalis spp, Fumaria spp, Dactylicapnos spp,* or another species.

**[MT2 and MT3]** In some examples. the engineered host cell may modify the expression of the enzyme narcotoline 4'-*O*-methylase. Narcotoline 4'-*O*-methylase is a heterodimer formed by the O-methyltransferase monomer encoded by the MT2 and MT3 genes. In some examples, narcotoline 4'-*O-*methylase catalyzes the reaction of narcotoline → noscapine. as referenced in FIG. 7. Additionally, narcotoline 4'-*O*-methylase catalyzes the reaction of narcotolinenchemiacetal → narcotinchemiacetal and 4'-*O*-desmethyl-3-*O*-acetylpapaveroxine → 3-*O*-acetylpapaveroxine. The engineered host cell may be modified to include constitutive expression of the MT2 and MT3 genes in the engineered host cell. Additionally or alternatively, the engineered host cell may be modified to synthetically regulate the expression of the MT2 and MT3 genes in the engineered host cell. In some examples. the engineered host cell may be modified to incorporate a copy. copies, or additional copies, of the MT2 and MT3 genes. Additionally or alternatively, the engineered host cell may be modified to incorporate the introduction of a strong promoter element for the overexpression of the MT2 and MT3 genes within the engineered host cell. In some cases. the MT2 and MT3 genes may be codon optimized for expression in *Saccharomyces cerevisiae.* The MT2 and MT3 genes may be derived from *P. sammniferum, Papaver spp, Fumaria parviflora, Plantago arenaria, Rauwolfia heterophylla,* or another species.

**[morA]** In some examples, the engineered host cell may modify the expression of the enzyme morphine dehydrogenase. Morphine dehydrogenase is encoded by the morA gene. In some examples, morphine dehydrogenase catalyzes the reaction of morphine → morphinone, as referenced in **FIG. 4****.** In other examples, morphine dehydrogenase catalyzes the reaction of codeinone → codeine, also as referenced in **FIG. 4. FIG. 4** illustrates a biosynthetic scheme for production of semi-synthetic opiods, in accordance with some embodiments of the invention. In particular. **FIG. 4** illustrates extended transformations of thebaine in yeast by incorporating morA, morphine dehydrogenase: and morB, morphine reductase.

The engineered host cell may be modified to include constitutive expression of the morA gene in the engineered host cell. Additionally or alternatively. the engineered host cell may be modified to synthetically regulate the expression of the morA gene in the engineered host cell. In some examples, the engineered host cell may be modified to incorporate a copy, copies, or additional copies, of the morA gene. Additionally or altematively, the engineered host cell may be modified to incorporate the introduction of a strong promoter element for the overexpression of the morA gene within the engineered host cell. In some cases, the morA gene may be codon optimized for expression in *Saccharomyces cerevisiae.* The morA gene may be derived from *Pseudomonas putida* or another species.

**[morB]** In some examples, the engineered host cell may modify the expression of the enzyme morphinone reductase. Morphinone reductase is encoded by the morB gene. In some examples, morphinone reductase catalyzes the reaction of codeinone → hydrocodone, as referenced in **FIG. 4****.** In other examples, morphinone reductase catalyzes the reaction of morphinone → hydromorphone , also as referenced in **FIG. 4****.** In other examples, morphinone reductase catalyzes the reaction 14-hydroxycodeinone → oxycodone. The engineered host cell may be modified to include constitutive expression of the morB gene in the engineered host cell. Additionally or alternatively, the engineered host cell may be modified to synthetically regulate the expression of the morB gene in the engineered host cell. In some examples, the engineered host cell may be modified to incorporate a copy, copies, or additional copies, of the morB gene. Additionally or alternatively, the engineered host cell may be modified to incorporate the introduction of a strong promoter element for the overexpression of the morB gene within the engineered host cell. In some cases. the morB gene may be codon optimized for expression in *Saccharomyces cerevisiae.* The morB gene may be derived from *Pseudomonas putida* or another species.

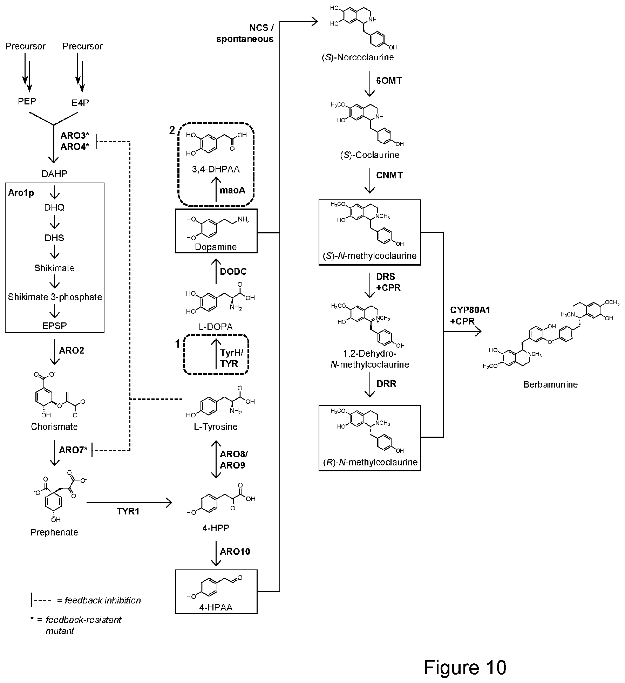

**[CYP80A1]** In some examples, the engineered host cell may express the enzyme berbamunine synthase. Berbamunine synthase is encoded by the gene for cytochrome P450 enzyme 80A1 (CYP80A1). In some examples. CYP80Al catalyzes the reaction *(S)-N*-methylcoclaurine + *(R)-N-*methylcoclaurine → berbamunine, as referenced in **FIG. 10****.** In other examples. CYP80A1 catalyzes the reaction *(R)-N-*methylcoclaurine + *(R)*-*N*-methylcoclaunne → guattegaumerine, as referenced in **FIG. 10****.** In other examples. CYP80A1 catalyzes the reaction *(R)-N*-methylcoclaurine + *(S)*-coclaurine → 2' nonberbamunine. The engineered host cell may be modified to include constitutive expression of the CYP80A1 gene in the engineered host cell. Additionally or alternatively, the engineered host cell may be modified to synthetically regulate the expression of the CYP80A1 gene in the engineered host cell. In some examples, the engineered host cell may be modified to incorporate a copy, copies, or additional copies. of the CYP80A1 gene. Additionally or alternatively, the engineered host cell may be modified to incorporate the introduction of a strong promoter element for the overexpression of the CYP80A1 gene within the engineered host cell. In some cases. the CYP80A1 gene may be codon optimized for expression in *Saccharomyces cerevisiae.* The CYP80A I gene may be derived from *Berberis stolonifera* or another species.

**[PODA]** In some example, the engineered host cell may express the enzyme protopine O-dealkylase. Protopine O-dealkylase is encoded by the gene PODA. In some examples. PODA catalyzes the *O*,*O*-demethylation of protoberberines and protopines such as canadine. stylopine, berberine, cryptopinc, allocryptopine, and protopine. In some examples. PODA catalyzes the O-demethylation of BIAs including tetrahydropapaverine. tetrahydropalmatine, and cryptopine. The engineered host cell may be modified to include constitutive expression of the PODA gene in the engineered host cell. Additionally or alternatively, the engineered host cell may be modified to synthetically regulate the expression of the PODA gene in the engineered host cell. In some examples, the engineered host cell may be modified to incorporate a copy. copies. or additional copies. of the PODA gene. Additionally or alternatively, the engineered host cell may be modified to incorporate the introduction of a strong promoter element for the overexpression of the PODA gene within the engineered host cell. In some cases, the PODA gene may be codon optimized for expression in *Saccharomyces cerevisiae.* The PODA gene may be derived from *Papaver somniferum* or other species.

**[RNMT]** In some examples. the engineered host cell may modify the expression of the enzyme Reticuline N-methyltransferase. Reticuline N-methyltransferase is encoded by the RNMT gene. In some examples, Reticuline N-methyltransferase may catalyze reactions such as reticuline→tembetarine, among other reactions.

**[P7OMT]** In some examples. the engineered host cell may modify the expression of the enzyme Papaverine 7-O-demethylase. Papaverine 7-O-demethylase is encoded by the P70MT gene. In some examples, Papaverine 7-O-demethylase may catalyze reactions such as papaverine→pacodine, among other reactions.

**[3ODM]** In some examples. the engineered host cell may modify the expression of the enzyme 3-O-demethylase. 3-O-demethylase is encoded by the 3ODM gene. In some examples. 3-O-demethylase may cataly ze reactions such as oxycodone-oxymorphone: hydrocodone→hydromorphone; dihydrocodeine→dihydromorphine; 14-hydroxycodeine→14-hydroxymorphine; codeinone→morphinone; and 14-hydroxycodeinone→14-hydroxymorphinone, among other reactions.

**[NDM]** In some examples. the engineered host cell may modify the expression of the enzyme *N*-demethylase. *N*-demethylase is encoded by the NDM gene. In some examples, *N*-demethylase may catalyze reactions. such as Codeine→Norcodeine; Morphine→Nomorphine; Oxycodone→Noroxycodone; Oxymorphone→Noroxymorphone; Thebaine→Northebaine; Oripavine→Nororipavine; Hydrocodone→Norhydrocodone; Hydromorphone→Norhydromorphone; Dihydrocodeine→Nordihydrocodeine; Dihydromorphine→Nordihydromorphine; 14-hydroxycodeine→Nor-14-hydroxycodeine; 14-hydroxymorphine→Nor-14-hydroxymorphine; Codeinone→Norcodeinone; Morphinone→Normrphinone; 14-hydroxycodeinone→Nor-14-hydroxycodeinone; and 14-hydroxymorphinone→Nor-14-hyvdroxymorphinone, among other reactions.

**[NMT]** In some examples, the engineered host cell may modify the expression of the enzyme *N*-methyltransferase. *N*-methyltmnsferase is encoded by the NMT gene. In some examples. *N-*methyltransferase may catalyze reactions. such as Norcodeine-codeine; Normorphine→morphine; Noroxycodone→oxycodone; Noroxymorphone→noroxymorphone; Northebaine→thebaine; Nororipavine→oripavine; Norhydrocodone→hydrocodone; Norhydromorphone→ Hydromorphone; Nordihydrocodeine→ Dihydrocodeine; Nordihydromorphine→ Dihydromorphine; Nor-14-hydroxycodeine→ 14-hydroxycodeine; Nor-14-hydroxymorphine→ 14-hydroxymorphine; Norcodeineone→ Codeineone; Normorphinone→ Morphinonc: Nor-14-hydroxy-codeinone→ 14-hydroxycodeinone; Nor-14-hydroxy-morphinone→ 14-hydroxymorphinone.

**[NAT]** In some examples. the engineered host cell may modify the expression of the enzyme *N-*allyltransferase. *N*-allyltransferase is encoded by the NAT gene. In some examples, *N*-allyltransferase may catalyze reactions, such as Norcodeine→N-allyl-norcodeine; Norrnorphine-N-allyl-nonnorphine; Noroxycodone-N-allyl-noroxycodone; Noroxymorphone→N-allyl-nomoroxymorphone; Northebaine→N-allyl-northebaine; Nororipavine→N-allyl-nororipavine; Norhydrocodone-N-allyl-norhydrocodone; Norhydromorphone- N-allyl-norhydromorphone; Nordihydrocodeine→ N-allyl-nordihydrocodeine: Nordihydromorphine→ N-allyl-nordihydromorphine; Nor-14-hydroxycodeine→ N-allyl-nor-14-hydroxycodeine; Nor-14-hydroxymorphine→N-allyl-nor-14-hydroxymorphine; Norcodeineone→ N-allyl-norcodeineone: Normorphinone→ N-allyl-normorphinone; Nor-14-hydroxy-codeinone→ N-allyl-nor-14-hydroxycodeinone; Nor-14-hydroxy-morphinone→ N-allyl-nor-14-hydroxymorphinone, among other reactions.

**[CPMT]** In some examples. the engineered host cell may modify the expression of the enzyme *N*-cyclopropylmethyltranserase. *N*-cyclopropylmethyltranserase is encoded by the CPMT gene. In some examples. N-cyclopropylmethyltransferase may catalyze reactions, such as Norcodeine→N(cyclopropylmethyl)notcodeine; Normorphine→N(cyclopropylmethyl) normorphine; Noroxycodone→-N(cyclopropylmethyl) noroxycodone; Noroxymorphone→N(cyclopropylmethyl) nornoroxymorphone; Northebaine→N(cyclopropylmethyl) northebaine; Nororipavine→N(cyclopropylmethyl) nororipavine; Norhydrocodone-N(cyclopropylmethyl) norhydrocodone: Norhydromophone→ N(cyclopropylmethyl)norhydromophone; Nordihydrocodeine→ N(cyclopropylmethyl)nordihydrocodeine; Nordihydromorphine→ N(cyclopropylmethyl)nordihydromrphine; Nor-14-hydroxycodeine→ N(cyclopropylmethyl)nor-14-hydroxycodeine; Nor-14-hydroxymorphine→ N(cyclopropylmethyl)nor-14-hydroxymorphine; Norcodeineone→ N(cyclopropylmethyl)norcodeineone; Normorphinone→ N(cyclopropylmethyl)normorphinone; Nor-14-hydroxy-codeinone→ N(cyclopropylmethyl)nor-14-hydroxycodeinone; and Nor-14-hydroxy-morphinone→ N(cyclopropylmethyl)nor-14-hydroxy morphinone. among other reactions.

**[BM3]** In some examples, the engineered host cell may express the enzyme BM3. BM3 is a *Bacillus megaterium* cytochrome P450) involved in fatty acid monooxygenation in its native host. In some cases BM3 *N*-demethylates an opioid to produce a nor-opioid. In some cases the host cell is modified to express BM3 in addition to other heterologous enzymes for the production of a nal-opioid or nor-opioid. The engineered host cell may be modified to include constitutive expression of the BM3 gene in the engineered host cell. Additionally or alternatively, the engineered host cell may be modified to synthetically regulate the expression of the BM3 gene in the engineered host cell. In some examples. the engineered host cell may be modified to incorporate a copy. copies. or additional copies. of the BM3 gene. Additionally or alternatively, the engineered host cell may be modified to incorporate the introduction of a strong promoter element for the overexpression of the BM3 gene within the engineered host cell. BM3 has several advantages as a biosynthetic enzyme including that it is soluble, comes with a fused reductase partner protein, and can readily be engineered to accept new substrates. Additionally, **Table 9** illustrates variants of BM3 *N*-demethylases.

Examples of the aforementioned genes can be expressed from a number of different platforms in the host cell, including plasmid (2µ, ARS/CEN). YAC, or genome In addition, examples of the aforementioned gene sequences can either be native or codon optimized for expression in the desired heterologous host (e.g., *Saccharomyces cerevisiae*).

### EXAMPLES

The following examples are given for the purpose of illustrating various some embodiments of the invention and are not meant to limit the invention in any fashion. Where indicated. expression constructs are understood to incorporate a suitable promoter. gene, and terminator, even if the exact terminator sequence used is not specified. The present examples, along with the methods described herein are presently representative of preferred embodiments, are exemplary, and are not intended as limitations on the scope of the invention. Changes therein and other uses which are encompassed within the spirit of the invention as defined by the scope of the claims will occur to those skilled in the art.

### Example 1: Bioinformatic identification of enzymes for morphinan alkaloid production

The OncKP (Matasci N ct al. 2014. Data access for the 1,000 Plants (1KP) project. Gigascience 3:17) and plant transcriptome database was queried with amino acid sequences of representative variants from each of the hy pothesized classes of enzymes. In particular. the *Papaver* genus, which includes many plant species that produce benzylisoquinoline alkaloids of interest, were searched. The list of candidate sequences from these plants were narrowed down using an e-value cutoff of 10⁻⁵⁰ to the representative sequence. For some candidates. the complete sequence was not present in the assembled transcriptome. In these cases. the sequence was completed using raw sequencing reads.

### Example 2: Platform yeast strains engineered to produce (S)-reticuline from glucose and simple nitrogen sources

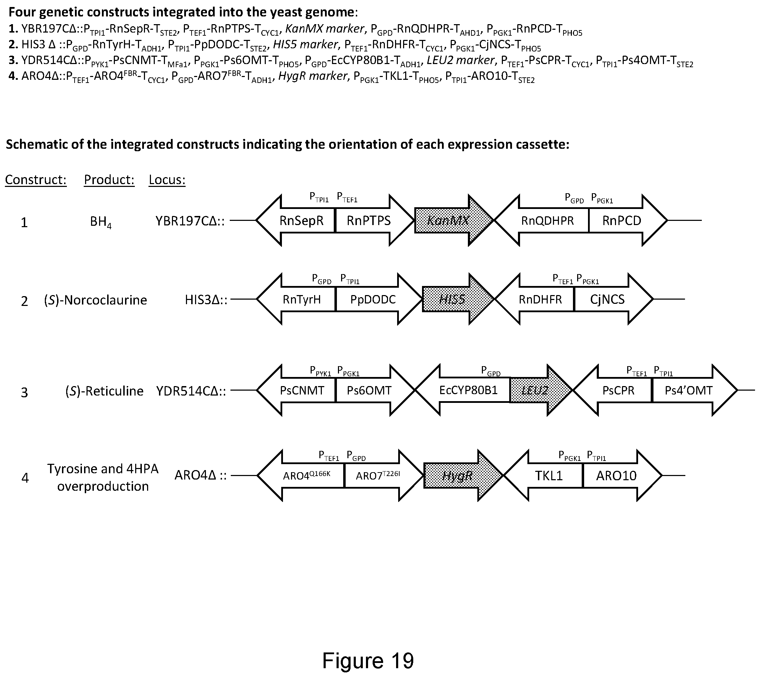

A platform yeast strain that produces the significant branch point BIA intermediate *(S)-*reticuline from L-tyrosine was constructed **(****FIG. 19****).** Specifically, four multi-gene expression constructs were integrated into the genome of a yeast strain. The composition of the four constructs is indicated in **FIG. 19****.** Each construct is compnsed of 4 or 5 genes expressed from yeast promoters. Genes are positioned at each locus as complete expression cassettes comprising a promoter, gene open reading frame, and terminator as specified in the annotations above the schematic. The schematic shows the orientation of each expression cassette by the direction of the arrow representing a given gene. Selectable markers are italicized in the annotation and represented by grey arrows in the schematic. Each selection marker is flanked by loxP sites to allow removal of the marker from the locus. Additionally, each construct has a selectable marker flanked by loxP sites so that it can be removed by Cre recombinase,

In the first integration construct, four heterologous genes from *Rattus norvegicus* are integrated into the YBR197C locus together with a G418 selection marker (*KanMX*). *RnPTPS, RnSepR, RnPCD,* and *RnQDHPR* are required to synthesize and regenerate tetrahydrobiopterin (BH₄) from the yeast endogenous folate synthesis pathway as indicated in **FIG. 2****.** Each gene is codon optimized for expression in yeast.

In the second integration construct, four heterologous genes are integrated into the *HIS3* locus together with the HIS5 selection marker. *Rattus norvegicus* tyrosine hydroxylase (*RnTvrH*) converts tyrosine to L-DOPA using the cosubstrate BH₄ generated by the preceding integration construct. The *RnTyrH* gene can be any of the wild-type or improved mutants which confer enhanced activity (e.g.. W166Y, R37E. and R38E). A second *Rattus norvegicus* gene, *RnDHFR*, encodes an enzyme that reduces dihydrobiopterin (an oxidation product of BH₄) to BH₄, in this way increasing the availability of this cosubstrate. Also included in the third construct is *PpDODC* from *Pseudomonas putida*, an enzyme that converts L-DOPA to dopamine. The fourth enzyme is CiNCS from *Coptis japonica,* which condenses 4-HPA and dopamine to make norcoclaurine. Each gene is codon optimized for expression in yeast.

In the third integration construct five heterologous genes from plants and the *LEU2* selection marker are integrated into the locus YDR514C. *Ps6OMT*, *Ps4'OMT*, and *PsCNMT* are methyltransferases from *Papaver somniferum* and are expressed as native plant nucleotide sequences. A fourth *P. somniferum* gene, y*PsCPRv2*, is codon optimized for yeast and encodes a reductase that supports the activity of a cytochrome P450 from *Eschscholzin californica*, *EcCYP80A1.* The enzymes encoded in this construct perform two *O*-methylations, an *N*-methylation, and a hydroxylation to produce reticuline from the norcoclaurine produced by the preceding integration construct. Each gene is codon optimized for expression in yeast.

In the final integration construct. additional copies of *Sacc haromyces cerevisiae* endogenous genes *ARO4^{Q166K}*, *4RO7^{T226I}*, *TYR1*, and *ARO10* are integrated into the *ARO4* locus together with a hygromycin resistance selection marker. *ARO4^{Q166K}* and *ARO7^{T226I}* are feedback-resistant mutants of *ARO4* and *ARO7* which each encode a single base pair substitution relative to the wild-type sequence. *TIR1* and *ARO10* are identical to the native yeast genes. but are expressed behind strong promoters. Aro4p and Aro7p are enzymes in the biosynthesis of aromatic amino acids including tyrosine. Removing feedback inhibition from these enzymes results in upregulation of endogenous ty rosine biosynthesis. Overexpression of Tyr1p upregulates tyrosine biosynthesis and thus production of tyrosine. Overexpression of Aro10p increases the production of 4-HPA.

Platform yeast strains can be constructed with any number of the four expression cassettes. Specifically, platfonn yeast strains were constructed with integration constructs 1-4 and integration constructs 1-3. In the latter strain in which the tyrosine over-production construct (construct 4) is excluded, additional tyrosine may be supplied in the culture medium to support the biosynthesis of reticuline. Additional genetic modifications may be incorporated into the platform strains to support production of downstream BIAs and increased flux to BIA biosynthesis.

The yeast strains were grown in synthetic complete media with the appropriated amino acid drop out solution at 28 °C. BIA metabolites in the media supernatant were analyzed after 48 and 96 hours of growth by LC-MS/MS analysis.

### Example 3: Platform yeast strains engineered to produce thebaine from glucose and simple nitrogen sources

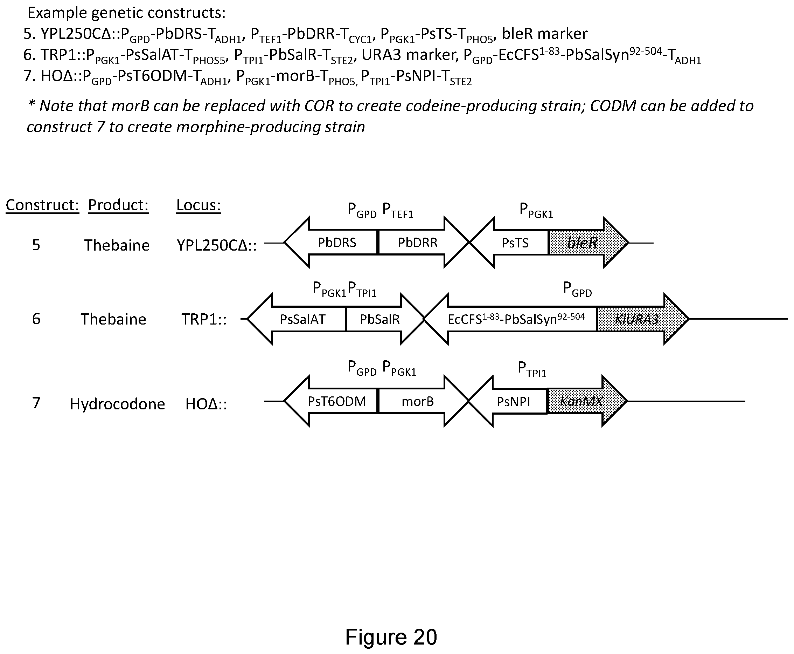

Yeast strains can be engineered for the production of the morphinan alkaloid thebaine from early precursors such as tyrosine. As an example, the platform yeast strains described in **Example** 2 can be further engineered to produce the morphinan alkaloid products from L-tyrosine (FIG. 20).

The platform yeast strain producing *(S)*-reliculine from L-tyrosine (see description in **Example 2)** was further engineered to incorporate an engineered split epimerase DRS-DRR, an engineered salutaridine synthase, salutaridine reductase, salutaridinol acetyltransferase, and thebaine synthase to convert the biosynthesized *(S)*-reticuline to the first morphinan alkaloid thebaine **(****FIG. 4****).** Three expression cassettes (P*_{TDH3-}yEcCFS¹⁻²⁶-yPbSS³³⁻⁵⁰⁴*, P*_{TPH}-yPbSalR*, P*_{TEF1}-yPsSalAT*) were assembled into an integration construct with a URA3 selective marker and integrated into the locus TRP1 in the platform yeast strain. An additional three expression cassettes (P*_{TDH3-}yPbDRS*, P*_{TEF1}-yPbDRR*, P*_{PGK1}-yPsTS*) were assembled into an integration construct with a *bleR* selective marker and integrated into the locus YPL250CΔ in the platform yeast strain. The composition of the two constructs is indicated in **FIG. 20****.**

The yeast strains harboring the integrated cassettes were grown in synthetic complete media with the appropriated drop out solution at 28 °C. After 96 hours of growth, the media was analyzed for BIA metabolites by LC-MS/MS analysis.

### Example 4: Yeast strains engineered to produce downstream morphinan alkaloids from glucose and simple nitrogen sources

Yeast strains can be engineered for the production of the downstream morphinan alkaloids from early precursors such as tyrosine. As an example. the platfonn yeast strains described in **Example 3** can be further engineered to produce the downstream morphinan alkaloid products from L-tyrosine **(****FIG. 4****).**

The platform yeast strain producing thebaine from L-tyrosine (see description in **Example 3)** was further engineered to incorporate thebaine 6-*O*-demethylase, neopinone isomerase, codeinone reductase, and codeinone-O-dcmethylase to convert the biosynthesized thebaine to the ***downstream*** morphinan alkaloids including morphine **(****FIG. 20****).** Four expression cassettes (P*_{GPD}*-*T6ODM*, P*_{PGK1}-COR*, P*_{ADH1}-CODM*, P*_{TPH}-yPsNPI*) were directly assembled with a *KanMX* selective marker and integrated into the *HOΔ* locus in the thebaine platform yeast strain to create a morphine-producing yeast strain (Thodey et al.. 2014). Three expression cassettes (P*_{GPD}-T6ODM*, P*_{PGK1}-COR*, P*_{TPH}-yPsNPI*) were directly assembled with a *KanMX* selective marker and integrated into the *HOΔ* locus in the thebaine platform yeast strain to create a codeine-producing yeast strain.

The yeast strains harboring the integrated cassettes were grown in synthetic complete media with the appropriated drop out solution at 28 °C. After 96 hours of growth, the media was analyzed for BIA metabolites by LC-MS/MS analysis.

### Example 5: Yeast strains engineered to produce semi-synthetic opioids from glucose and simple nitrogen sources

Yeast strains can be engineered for the production of the ***downstream semi-synthetic*** morphinan alkaloids from early precursors such as tyrosine. As an example, the yeast strains described in **Examples 3** and **4** can be further engineered to produce the semi-synthetic opioid products from L-tyrosine **(****FIG. 4****).**

The yeast strains producing thebaine from L-ty rosine (see description in **Examples 3 and 4)** were further engineered to incorporate thebaine 6-*O*-demethylase, neopinone isomerase. and morphinone reductase to convert the biosynthesized thebaine to the semi-synthetic morphinan alkaloid hydrocodone **(****FIG. 20****).** Three expression cassettes (P*_{GPD}-T6ODM*, P*_{PGK1}-morB*, P*_{TPH}-yPsNPI*) were directly assembled with a *KanMX* selective marker and integrated into the *HOΔ* locus in the thebaine platform yeast strain to create a hydrocodone-producing yeast strain (Thodey et al., 2014).

The yeast strains harboring the integrated cassettes were grown in synthetic complete media with the appropriated drop out solution at 28 °C. After 96 hours of growth, the media was analyzed for BIA metabolites by LC-MS/MS analysis.

### Example 6: Production of downstream morphinan alkaloids from glucose and simple nitrogen sources via engineered yeast strains

Yeast strains were engineered as described in **Examples 2, 3,** and **4** to produce the downstream morphinan alkaloids codeine and morphine directly from simple sugars (e.g., glucose) and nitrogen sources present in standard growth media. Specifically, a CEN.PK strain of *Saccharomyces cerevisiae* was engineered to express the following heterologous enzymes via integration into the yeast chromosome: TyrH, DODC. PTPS, SepR. PCD. QDHPR. NCS, 6OMT, CNMT, CYP80B1, CPR. 4OMT, DRS, DRR, SalSyn. SalR. SalAT, TS, T6ODM, COR (variant 1.3, SEQ ID NO. 87). A version of this yeast strain was also engineered to express CODM via integration into the yeast chromosome. In this example. the SalSyn enzyme is engineered to have its leader sequence replaced with 83 amino acids from the N-terminus of *Eschscholzia californica* chelanthifoline synthase (EcCFS). Additional modifications were made to the strain to increase BIA precursor accumulation, including: overexpression of ARO10, overexpression of TYR1, expression of a feedback resistant ARO4 (ARO4^{Q166K}), and expression of a feedback resistant ARO7 (ARO7^{T2261}) Separate engineered yeast strains were made as described, harboring different variants of enzymes encoding neopinone isomerase activity (NPI), including SEQ ID NO. 83, which is a variant of SEQ ID NO. 82 with a N-terminal truncation of the first 18 amino acids (i.e., NPI (truncated)), and no neopinone isomerase enzyme (codeine-producing strain: YA1033; morphine-producing strain: YA1022). The sequences of the enzyme vanants are provided in **Table 3.**

The described yeast strains were inoculated into 2 ml of synthetic complete media (yeast nitrogen base and amino acids) with 2% glucose and grown for approximately 4 hours at 28°C. Then, 10 uL of each culture was transferred to 400 uL of fresh media in a 96-well plate in replicates of 4 and grown for an additional 48 hours at 28°C. The production media contains 1x yeast nitrogen broth and amino acids. 20 mM ascorbic acid, 300 mg/L tyrosine. 40 g/L maltodextrin, and 2 units/L amylase. The amylase is used to mimic a fed-batch process and gradually releases glucose from maltodextrin polymer so that the yeast can use it as a carbon source. The cells were separated from the media by centrifugation, and thebaine concentration was measured directly in the supernatant by LC-MS/MS analysis.

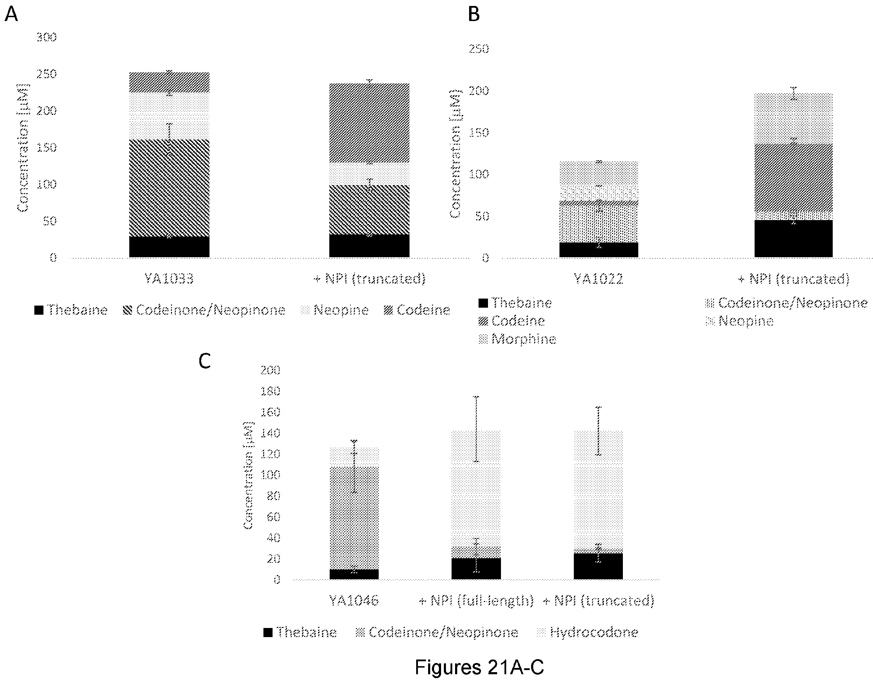

Engineered codeine-producing yeast strains produced thebaine, codeine, and other benzylisoquinoline alkaloids from glucose and simple nitrogen sources present in the growth media **(****FIG. 21A****).** Engineered morphine-producing yeast strains produced thebaine. codeine. morphine, and other benzylisoquinoline alkaloids from glucose and simple nitrogen sources present in the growth media **(****FIG. 21B****).**

### Example 7: Production of downstream semi-synthetic opioids from glucose and simple nitrogen sources via engineered yeast strains

Yeast strains were engineered as described in **Examples 2, 3, 4, and 5** to produce the downstream semi-synthetic opioid hydrocodone directly from simple sugars (e.g., glucose) and nitrogen sources present in standard growth media. Specifically, a CEN.PK strain of *Saccharomyces cerevisiae* was engineered to express the following heterologous enzymes via integration into the yeast chromosome: TyrH, DODC, PTPS, ScpR, PCD. QDHPR. NCS. 6OMT, CNMT. CYP80B1, CPR, 4OMT, DRS, DRR. SalSyn, SalR, SalAT, TS, T6ODM, morB. In this example. the SalSyn enzyme is engineered to have its leader sequence replaced with 83 amino acids from the N-terminus of *Eschscholzia californica* chelanthifoline synthase (EcCFS). Additional modifications were made to the strain to increase BIA precursor accumulation, including: overexpression of ARO10, overexpression of TYR1, expression of a feedback resistant ARO4 (ARO4^{Q166K}), and expression of a feedback resistant ARO7 (ARO7^{T226I}). Separate engineered yeast strains were made as described, harboring different variants of enzymes encoding neopinone isomerase activity (NPI). including SEQ ID NO. 54 (i.e.. NPI (full-length)) and SEQ ID NO. 55. which is a variant of SEQ ID NO. 56 with a N-terminal truncation of the first 18 amino acids (i.e.. NPI (truncated)), and no neopinone isomerase enzyme (YA1046). The sequences of the enzyme variants are provided in **Table 3.**

The described yeast strains were inoculated into 2 ml of synthetic complete media (yeast nitrogen base and amino acids) with 2% glucose and grown for approximately 4 hours at 28°C. Then, 10 uL of each culture was transferred to 400 uL of fresh media in a 96-well plate in replicates of 4 and grown for an additional 48 hours at 28°C. The production media contains 1x yeast nitrogen broth and amino acids, 20 mM ascorbic acid, 300 mg/L tyrosine, 40 g/L maltodextrin. and 2 units/L amylase. The amylase is used to mimic a fed-batch process and gradually releases glucose from maltodextrin polymer so that the yeast can use it as a carbon source The cells were separated from the media by centrifugation, and thebaine concentration was measured directly in the supernatant by LC-MS/MS analysis.

Engineered hydrocodone-producing yeast strains produced thebaine, hydrocodone, and other benzylisoquinoline alkaloids from glucose and simple nitrogen sources present in the growth media **(****FIG. 21C****).**

### Example 8: Microbial strains engineered to produce O-demethylated opioid compounds from glucose and simple nitrogen sources

Enzymes listed in **Table 11** that displayed *O*-demethylase activity on morphinan alkaloids, were incorporated into a microbial strain (either *Saccharomyces cerevisiae* or *Escherichia coli)* which biosynthesizes morphinan alkaloids de novo (as described in **Examples 3, 4, and 5).** The complete BIA biosynthetic pathway uses L-tyrosine produced by the host cell and/or supplemented in the culture medium. Two molecules of tyrosine are modified and condensed to form the first benzylisoquinoline structure, which may be either norcoclaurine or norlaudanosoline. The benzylisoquinoline is further modified to form (S)-reticuline and then stereochemically inverted by the activity of an epimerase enzyme to yield (*R*)-reticuline. (*R*)-reticuline undergoes a carbon-carbon coupling reaction to form the first promorphinan, salutaridine, and is further modified before undergoing an oxygen-carbon coupling reaction catalyzed by a thebaine synthase to arrive at the first morphinan alkaloid structure, thebaine (see **FIG. 4****). Table 11** lists enzymes and activities in the complete pathway.

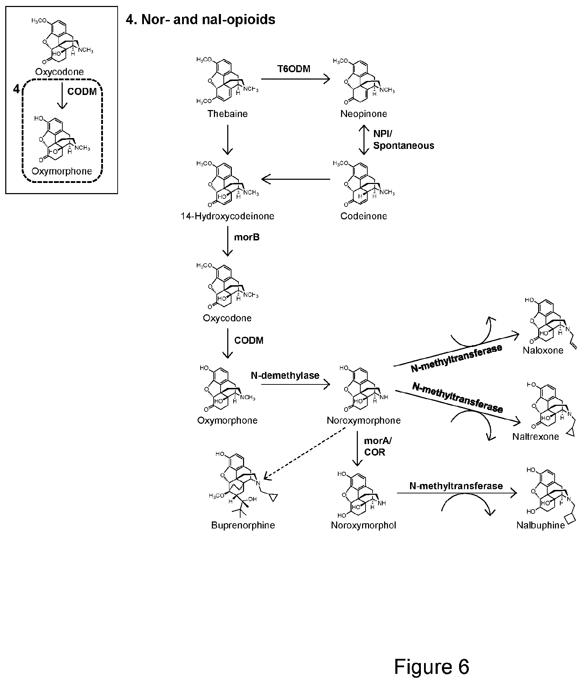

**FIG. 6** illustrates a biosynthesis scheme in a microbial cell, in accordance with some embodiments of the invention. Tyrosine produced endogenously by the cell and/or supplied in the culture medium is converted to oxycodone (broken arrows represent multiple enzymatic steps). The oxycodone is then 3-*O*-demethylated to oxymorphone and N-clemethylated to noroxymorphone. Finally, an *N-*methyltransferase accepts allyl and cyclopropylmethyl carbon moieties from SAM analogues to produce naloxone and naltrexone. respectively.

To detect *O*-demethylase activity in strains producing morphinan alkaloid molecules, cells expressing candidate enzymes, either from plasmid vectors or chromosomally-integrated cassettes, were propagated by fennentation and cell supernatants were collected to analyze the total opioid profile (as described above). *O*-demethylation of opioid molecules in strains harboring the complete BIA pathway was detected by LC-MS (as described above). Specifically, the conversion of oxycodone to oxymorphone was detected. To detect *O*-demethylation activity via biocatalysis, strains were cultured in selective medium and then lysed by glass bead disruption. Cell lysates were supplied exogenously with opioid substrates (see **FIG. 11** **and** **12****),** and other cofactors necessary for enzyme function. O-demethylation of opioid molecules was detected by LC-MS.

### Example 9: Microbial strains engineered to produce N-demethylated opioid compounds from glucose and simple nitrogen sources

Enzymes listed in **Table 13.** that displayed *N*-demethylase activity on morphinan alkaloids, were incorporated into a microbial strain (either *Saccharomyces cerevisiae* or *Escherichia coli*) which biosynthesizes morphinan alkaloids de novo (as described in **Examples 3, 4, and 5).** The complete BIA biosynthetic pathway uses L-tyrosine produced by the host cell and/or supplemented in the culture medium. Two molecules of tyrosine are modified and condensed to form the first benzylisoquinoline structure which may be either norcoclaurine or norlaudanosoline. The benzylisoquinoline is further modified to form (*S*)-reticuline and then stereochemically inverted by the activity of an epimerase enzyme to yield (*R*)-reticuline. (*R*)-reticuline undergoes a carbon-carbon coupling reaction to form the first promorphinan, salutaridine, and is further modified before undergoing an oxygen-carbon coupling reaction catalyzed by a thebaine synthase to arrive at the first morphinan alkaloid structure, thebaine (see **FIG. 4****). Table 11** lists enzymes and activities in the complete pathway.

To detect *N*-demethylase activity in strains producing morphinan alkaloid molecules, cells expressing candidate enzymes, either from plasmid vectors or chromosomally-integrated cassettes, were propagated by fermentation and cell supernatants were collected to analyze the total opioid profile (as described above). *N*-demethylation of opioid molecules in strains harboring the complete BIA pathway was detected by LC-MS (as described above). Specifically, the conversion of oxy morphone to noroxymorphone was detected. To detect *N*-demethylation activity via biocatalysis. strains were cultured in selective medium and then lysed by glass bead disruption. Cell lysates were supplied exogenously with opioid substrates (see **FIGs. 13****),** and other cofactors necessary for enzyme function. *N*-demethylation of opioid molecules was detected by LC-MS.

### Example 10: Microbial strains engineered to produce nal-opioid compounds from glucose and simple nitrogen sources

Enzymes listed in **Table 14.** that displayed *N*-methylase activity on morphinan alkaloids, were incorporated into a microbial strain (either *Saccharomyces cerevisiae* or *Escherichia coli)* which biosynthesizes morphinan alkaloids de novo (as described in **Examples 3, 4, and 5).** **FIG. 6** shows an example of the complete reaction scheme from the precursor molecule thebaine to the final nal-opioid compounds naloxone and naltrexone. These strains additionally express enzymes from **Examples 8** and **9** and **Table 11.** that are responsible for generating nor-opioid compounds from the complete BIA pathway. *N*-methylase enzymes were also expressed in a microbial strain (either Cen.PK2 for *S*. *cerevisiae* or BL21 for *E. coli.* for example) lacking the biosynthetic pathway, to generate a strain that is capable of biocatalysis of several different exogenously-supplied substrate molecules. The complete BIA biosynthetic pathway uses tyrosine produced by the host cell and/or supplemented in the culture medium. Two molecules of tyrosine are modified and condensed to form the first benzylisoquinoline structure which may be either norcoclaurine or norlaudanosoline. The benzylisoquinoline is further modified to form (*S*)-reticuline and then stereochemically inverted by the activity of an epimerase enzyme to yield (*R*)-reticuline. (*R*)-reticuline undergoes a carbon-carbon coupling reaction to form the first promorphinan, salutaridine, and is further modified before undergoing an oxygen-carbon coupling reaction catalyzed by a thebaine synthase to arrive at the first morphinan alkaloid structure, thebaine (see **FIG. 4**). **Table 11** lists enzymes and activities in the complete pathway,

To detect *N*-modifying activity in strains with the complete BIA pathway to nor-opioids (see **FIG. 6**). cells expressing candidate enzymes were propagated by fermentation (as described above) and incubated with SAM or SAM analogs. such as those listed in **FIG. 18****.** Enzymatic modification of nor-opioid or other BIA molecules in strains harboring the complete BIA pathway was detected in supernatants by LC-MS (as described above). To detect N-modifying activity via biocatalysis, strains were cultured in selective medium and then lysed by glass bead disruption. Cell lysates were supplied exogenously with SAM or SAM analogs. and other cofactors necessary for enzyme function. Specifically, the conversion of noroxymorphone to naloxone and naltrexone (using the SAM analogs allyl-SAM or cyclopropane-SAM, as shown in **FIG. 18****)** was detected. Modification of nor-opioid or other BIA molecules was detected by LC-MS. To detect *N*-modifying activity by biocatalysis in a strain that does not have the complete BIA pathway, Cen.PK2 strains expressing the described heterologous enzymes were grown in selective medium and lysed by glass bead disruption. Cell lysates were supplied exogenously with SAM or SAM analogs, cofactors necessary for enzyme function, and nor-opioid molecules such as those listed in **FIG. 18** and **Table 11.** Modification of these compounds was detected by LC-MS.

### Example 11: Norcoclaurine synthase activity in microbial strains

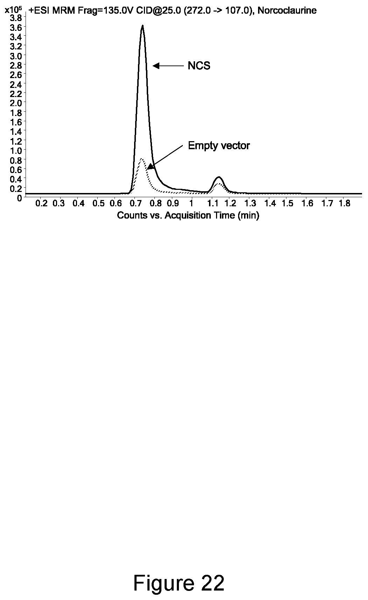

To engineer norcoclaurine production in yeast we: (1) introduced a heterologous NCS enzyme on a plasmid, (2) supplied tyrosine in the culture medium to promote 4-HPAA production by the host cell's native tyrosine catabolic pathway, and (3) supphed dopamine directly in the culture medium to provide the second substrate for NCS activity. CEN.PK2 was transformed with either a yeast shuttle plasmid containing NCS (SEQ ID NO: 70) cloned in an expression cassette with a P_{TDH3} promoter and T_{CYC1} terminator, or with an empty vector in which there was no gene inserted between the promoter and terminator. Cells were transformed using the standard lithium acetate technique, plated on selective solid medium, and cultured 2 days at 30°C. Colonies were picked at random and cultured first in standard synthetic complete (SC) dropout liquid medium for 48 hours at 30°C with 300 rpm agitation. The stationary phase cultures were then backdiluted 100x into SC media supplemented with 0 or 100 mM dopamine and 300 mg/L tyrosine and cultured a further 48 hours at 30°C, 300 rpm. When the cells were pelleted and the culture medium analyzed by LCMS we observed that the empty vector control strain (no-enzyme control) biosynthesized and exported 21.9 µM norcoclaurine into the culture medium when supplied with 100 mM dopamine **(****FIG. 22****).** This demonstrated that wild type yeast can take up dopamine from the culture medium to support a low-level of spontaneous norcoclaurine production by the condensation of endogenous 4-HPAA and exogenous dopamine. However, the engineered strain expressing an active NCS enzyme and supplied with 100 mM dopamine in the culture medium produced 72.1 µM norcoclaurine, more than 3-fold greater than observed in the no-enzyme control, demonstrating that NCS is active in yeast and catalyzes the formation of norcoclaurine **(****FIG. 22****).**

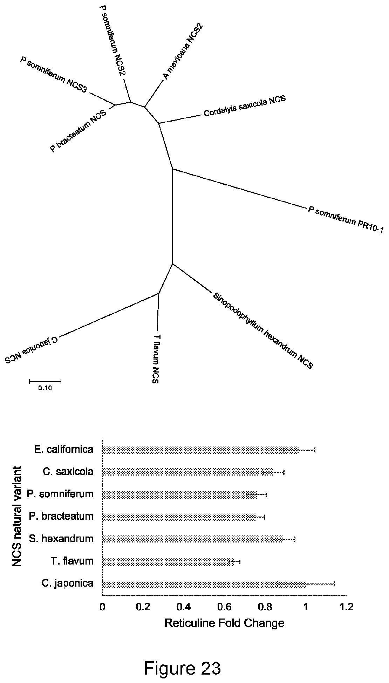

To investigate NSC natural diversity, plant Bet v I proteins from Coptis japonica, Thalictrum flavum, Argemone mexicana, Sinopodophyllum hexandrum, Papaver bracteatum, Papaver somniferum, and. Cordalyis saxicola **(Table 5)** were identified from the public transcriptomics databases and expressed in yeast. The open ready frames of each gene were first codon optimized for *Saccharomyces* cerevisiae and then synthesized with 30 base pair overlaps to the P_{TDH3} promoter and T_{CYC1} terminator of the yeast shuttle vector. The genes were then individually transformed into yeast strain YA139 (harboring a complete biosynthetic pathway to reticuline, but lacking NCS activity) together with linearized vector. On transformation, each NCS was incorporated into the plasmid by gap repair creating a construct in which NCS expression was driven by the P_{TDH3} promoter. After two days incubation at 30°C on solid selective medium individual colonies were picked and assayed for NCS activity. Strains were cultured first in standard SC medium for 48 hours at 30°C and then backdiluted 100x into SC media supplemented with 200 mg/L tyrosine and cultured a further 48 hours at 30°C. Because strain YA139 encodes a complete heterologous pathway to reticuline (with the exception of a functional NCS enzyme) die spent culture medium was analyzed by LCMS for the production of the final end product, reticuline. All tested NCS proteins were observed to be active and catalyzed the formation of norcoclaurine which was incorporated into reticuline and exported into the culture medium **(****FIG. 23****).**

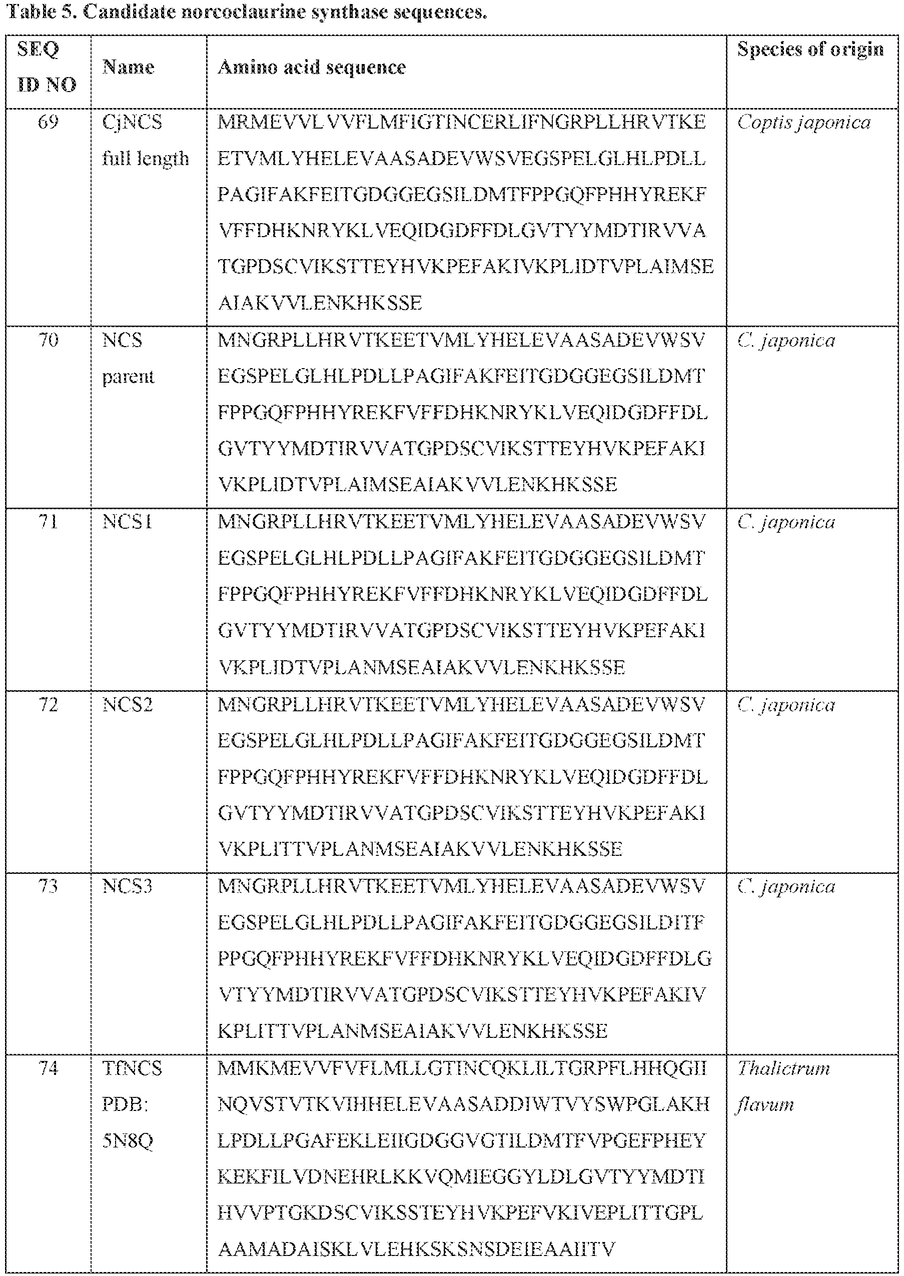

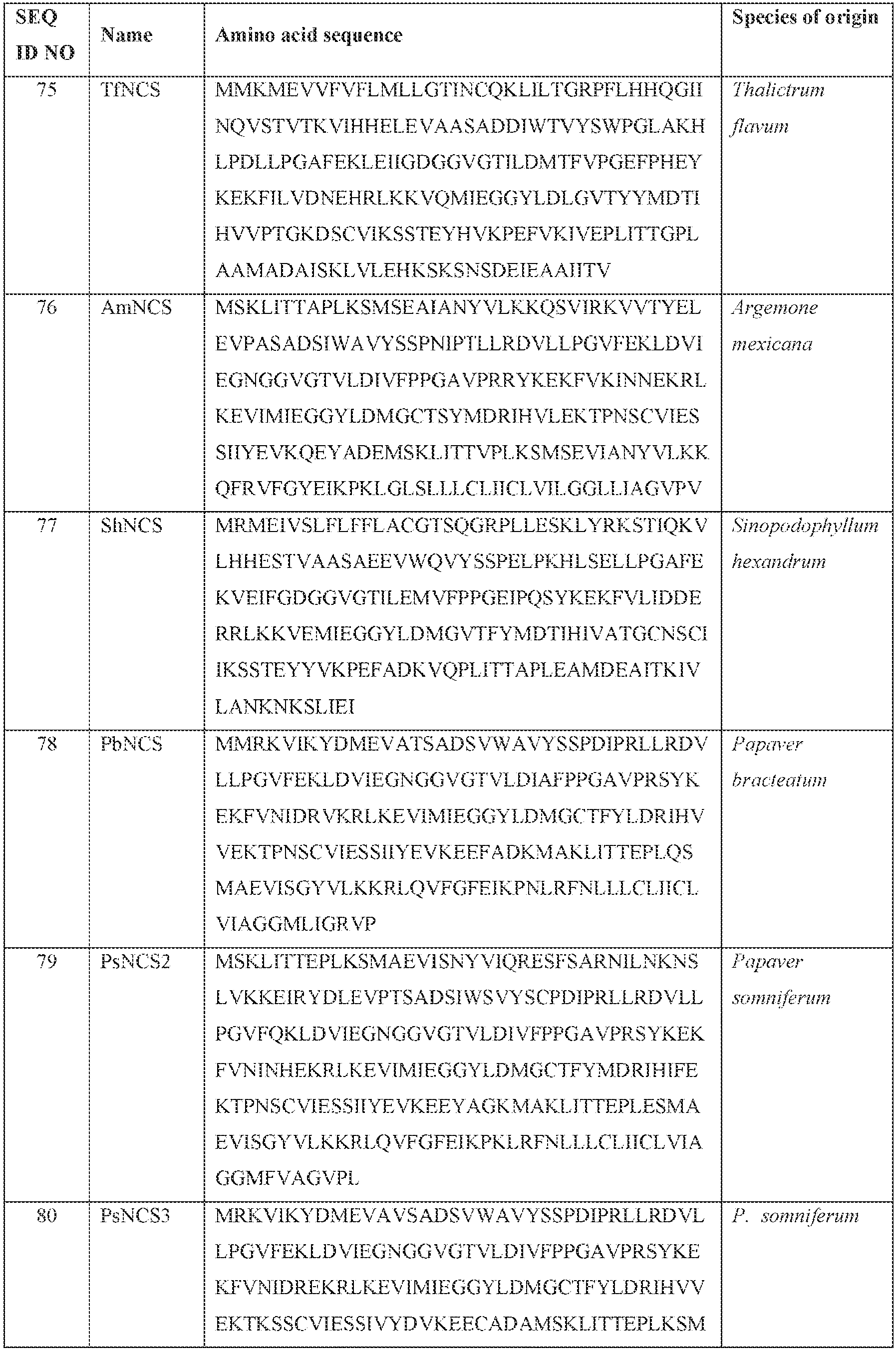

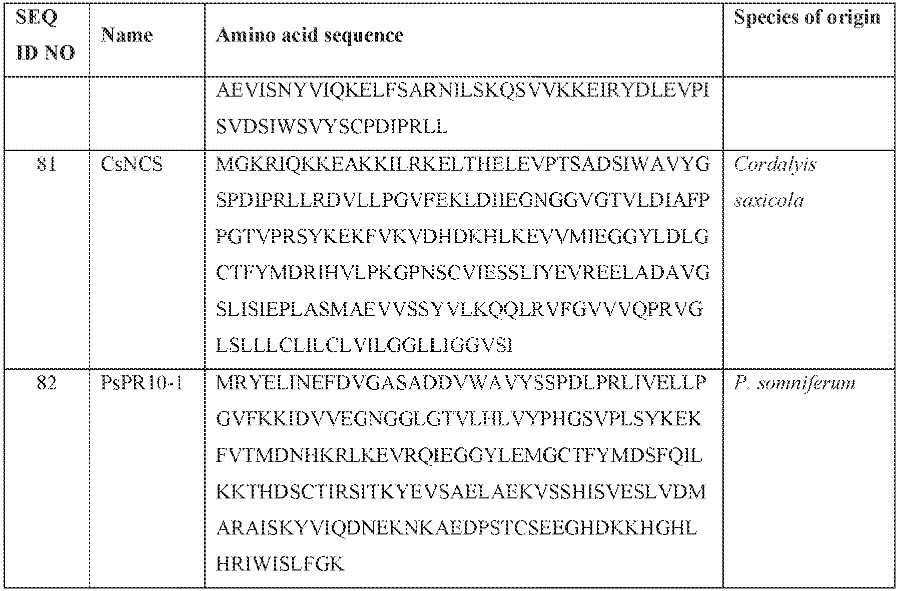

### Example 12: Microbial strains with enhanced production of HPAA

To support the production of norcoclaurine by yeast fermentation, strains were engineered to enhance the supply of 4-HPAA from the host cell metabolism. Yeast produce 4-HPAA from 4-hydroxyphenylpyruvate (4HPP) when tyrosine is catabolized in the Ehrlich pathway, Therefore, to enhance production of 4-HPAA we. (1) enhanced flux through the shikimate pathway to upregulate production of 4HPP and tyrosine, and (2) overexpressed ARO10, the enzyme responsible for the irreversible conversion of 4HPP to 4-HPAA.

A yeast strain was engineered with modifications to the chromosomal loci encoding shikimate pathway enzymes Aro4p. Aro7p, and Tyr1p, and Ehrlich pathway enzyme Aro10p (FIG. 1). Specifically, using homologous recombination we introduced mutations ARO4^{Q166K} and ARO7^{T2261} to relieve tyrosine feedback inhibition of these enzymes. We further modified the upstream regions of the TYR1 gene and ARO10 gene to replace the native promoters with the P_{TDH3} and P_{GAL7} promoters, respectively. These promoter swaps removed the native regulation and introduced promoters that drive constitutive expression when yeast are cultured in medium with select carbon sources. Additionally, the GAL80 gene was deleted to allow for constitutive expression of P_{GAL7}-ARO10 in the presence of glucose. The 4-HPAA-engineered strain and a control strain were transformed with a plasmid encoding NCS and the resulting colonies were cultured 48 hours in standard SC media. The stationary phase cultures were then backdiluted 100x into SC media supplemented with 0 or 100 mM dopamine and cultured a further 48 hours. When the culture medium was analyzed by LCMS the 4-HPAA-engineered strain was observed to produce 3- to 4-fold more norcoclaurine relative to the control strain.

### Example 13: Microbial strains that biosynthesizes dopamine

To support the production of norcoclaurine by yeast fermentation, strains were engineered to produce its second substrate, dopamine. In mammalian cells dopamine is synthesized from tyrosine, with either 3.4-dihydroxy-L-phenylalanine (L-DOPA) or tyramine as intermediates. To re-construct the mammalian biosynthetic pathway in yeast we first engineered a functional tyrosine hydroxylase (TyrH) system to convert tyrosine to L-DOPA. TyrH requires a cofactor, tetrahydrobiopterin (BH₄), that is not produced naturally in yeast. Therefore, we introduced a four-enzyme system into yeast to: (1) convert dihydroneopterin triphosphate from the native pathway for tetrahydrofolate synthesis into BH₄, and (2) return BH₄ to its reduced state after it is oxidized by TyrH. Rat BH₄ biosynthesis genes 6-pyruvoyl-tetrahydropterin synthase (RnPTPS) and sepiapterin reductase (RnSepR) were integrated into the yeast genome together with rat BH₄ recycling genes pterin-4alpha-carbinolamine dehydratase (RnPCD) and quinonoid dihydropteridine reductase (RnQDHPR). Next we integrated a human TyrH gene encoding mutations HsTyrH^{W166Y,R37E.R38E} to relieve substrate inhibition by tyrosine. Finally, we integrated a bacterial DOPA decarboxylase from *Pseudomonas putida.* PpDODC. to complete the pathway from tyrosine to dopamine. All heterologous enzymes were codon optimized for expression in *S. cerevisiae* and expressed from yeast high-expression or constitutive-expression promoters.

The dopamine engineering described above was combined with the 4-HPAA engineering from **Example 12** in a strain expressing an NCS variant **(****FIG. 1****).** This norcoclaurine total biosynthesis strain and a control strain were cultured 48 hours in standard SC media. The stationary phase cultures were then backdiluted 100x into SC media supplemented with 300 mg/L tyrosine and 20 mM ascorbic acid and cultured a further 48 hours. The cells were pelleted and the culture medium analy zed by LCMS. L-DOPA, dopamine, and norcoclaurine were all observed in the culture medium from the norcoclaurine total biosynthesis strain, but not in the culture medium from the control strain.

### Example 14: Improving NCS activity by N-terminaltruncation

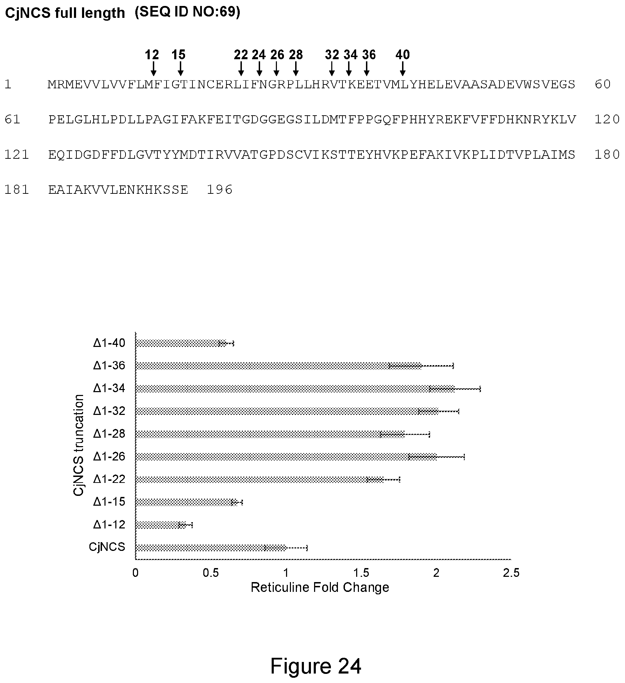

CjNCS (SEQ ID NO: 69) contains a hydrophobic domain in the first 24 amino acids of the N-terminus. This domain could represent a signal peptide, a transmembrane or membrane-interacting domain, or a protein-protein interaction domain. Alternatively. this region could be involved in the regulation or catalytic function of the enzyme. To determine if NCS activity could be enhanced by removal of the N-terminal region, we made deletions of the first 12 to 40 amino acid residues **(****FIG. 24****).** The CjNCS template was PCR amplified with oligos to remove the first 12, 15. 22, 26, 28, 32, 34, 36. or 40 amino acids (while replacing the methionine start codon) and introduce 30 base pair overlaps to the P_{TDH3} promoter and T_{CYC1} terminator of a yeast shuttle vector. The PCR products were each transformed together with linearized vector into yeast strains encoding the 4-HPAA and dopamine engineering described in **Examples 12 and 13 (****FIG. 1****).** The strains further encoded a heterologous pathway from norcoclaurine to reticuline comprised of *P. somniferum* norcoclaurine 6-O-methyltransferase (Ps6OMT), *P. somniferum* coclaurine N-methylt ransferase (PsCNMT), *P. bracteatum* N-methylcoclaurine-3'-hydroxylase (PbCYP80B1), *P. somniferum* cytochrome P450 reductase (PsCPR) and *P. somniferum 3'-*hydroxy-N-methylcoclaurine 4'-0- methyltransferase (Ps4OMT) **(****FIG. 1****).**

Strains expressing full-length and N-terminal truncated CjNCS variants were cultured 48 hours in standard SC dropout medium. 30°C. at 300 rpm. The stationary phase cultures were then backdiluted 100x into SC media supplemented with 200 mg/L tyrosine and 10 mM ascorbic acid and cultured a further 48 hours. Cells were pelleted and the culture medium was analyzed by LCMS. Strains expressing the truncated CjNCS variants were observed to produce different titers of reticuline relative to the full-length CjNCS control strain. Truncation of 12 or 15 amino acids from the N-terminus of CjNCS resulted in titers 3-fold and 1.5-fold lower than full-length CjNCS **(****FIG. 24****).** In contrast, truncation of 22, 26, 28. 32, 34, or 36 CjNCS N-terminal amino acids increased reticuline titers to 1.6- to 2.1-fold that of the full-length enzyme. Further truncation of 40 amino acid residues caused a drop in reticuline titers.

### Example 15: Improving NCS activity by mutagenesis

A directed evolution campaign was carried out using a 24 amino acid N-terminally deleted (Δ1-24) *Coptisjaponica* NCS sequence (SEQ ID NO: 70) as a starting template. The purpose of the screen was to identify residues in any Bet v1 fold protein that can be modified to enhance activity. A pool of randomly mutated NCS variants was generated by error-prone PCR to incorporate base pair mutations at a rate of 1-4 bp changes per gene (the NCS open reading frame is 522 bp including the start and stop codons). The oligos were designed to introduce 30 bp overlaps to the P_{TDH3} promoter and T_{CYC1} terminator of a yeast shuttle vector. The mutagenized PCR products were transformed into YA 139 together with linearized vector to generate a library of NCS variants by gap repair. A control PCR was also set up with the same NCS template and oligos but using a high-fidelity polymerase for amplification in place of the error-prone polymerase. The non-mutagenized PCR product and linear vector were transformed into YA139 in a second transformation to generate a control strain expressing the parent NCS sequence. Strain YA139 contains the 4-HPAA. dopamine, and reticuline engineering from Examples 2-4 above. but lacks a functional NCS enzyme. Individual colonies were picked and cultured in 96-well microtiter plates in 400 µL standard SC dropout medium at 30°C with 300 rpm shaking. After 48 hours the wells were backdiluted 100x mto 400 µL standard SC dropout medium without supplementation and incubated again at 30°C with 300 rpm agitation. 48 hours following dilution the plates were centrifuged to pellet the cells and the culture medium in each well analyzed by LCMS to determine the reticuline titer.

Final reticuline titers were compared between strains expressing the NCS mutagenesis library variants and the control strain expressing parent NCS (8 wells of every 96-well plate were reserved for "parent"). For every well determined to have enhanced reticuline titer. 100 µL of yeast cells was removed and used to prepare plasmid. The plasmids were then transferred to *Escherichia coli* for amplification and sequencing. Many NCS variants contained multiple mutations, some of them silent. To identify which mutations were causal, oligos were ordered to recreate the individual mutations in yeast by gap repair for further testing and validation. The results of the mutagenesis screen are provided in **Table 6.** Residues confirmed to positively impact reticuline titer (and hence norcoclaurine synthase activity) were then subjected to saturation mutagenesis. Specifically, oligos were ordered, each with an NNN codon at the position of the residue of interest. The new PCR products were transformed into YA139 to generate a library of NCS variants with every possible codon at the target residue. These new saturation mutagenesis libraries were then screened for enhanced reticuline titers. The results of the saturation mutagenesis screen are provided in **Table 7.** Finally, improved variants from the random mutagenesis and saturation mutagenesis screens were shuffled to identify combinations of mutations that gave the greatest increase in NCS activity. The results of the shuffling screen are provided in **Table 8.** The random mutagenesis, saturation mutagenesis, and shuffling rounds were later repeated with NCS2 (SEQ ID NO: 72) as a template.

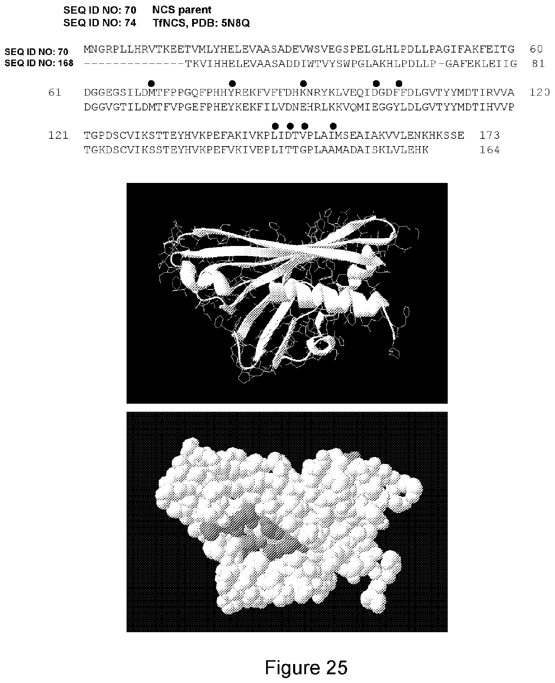

The residues identified as being important for catalytic function in the mutagenesis screen included amino acids 70. 81. 91. 101. 104, 147. 149, 151, and 155 of NCS parent, SEQ ID NO: 70. To identify the structural locations important for engineering Bet v I enzymes. NCS parent (SEQ ID NO: 70) was aligned with TfNCS (PDB: 5N8Q) (SEQ ID NO: 74), a natural norcoclaurine synthase enzyme with a solved crystal structure. Mapping of the residues from NCS parent to TfNCS (PDB: 5N8Q) revealed that residues M70, Y81, D101, F104, L147, D149, V151, I155 are located at the entry point to the active site **(****FIG. 25****).** One exception is K91 which is located at the opposite end of the catalytic tunnel.

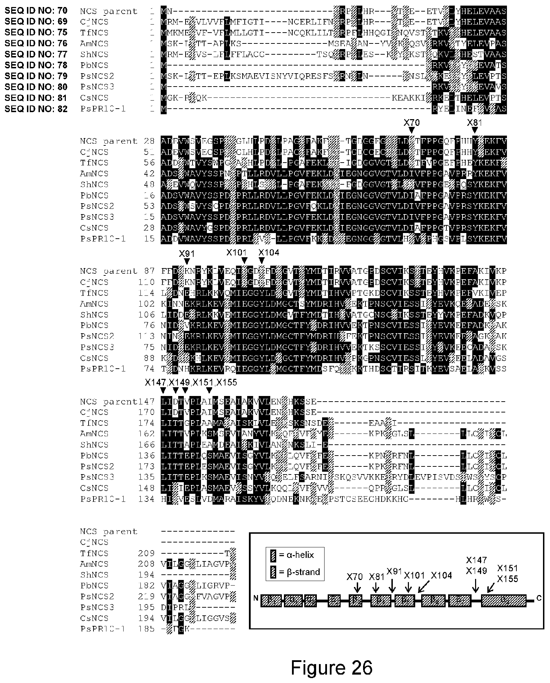

To further characterize the structural features of Bet v I enzymes that are important for engineering improved activity, NCS parent (SEQ ID NO: 70) was aligned with plant Bet v I proteins from *Coptis japonica, Thalictrum* flavum, *Argemone mexicana, Sinopodophyllum hexandrum, Papaver bracteatum, Papaver sonmniferum,* and, *Cordalyis saxicola* **(****FIG. 26****).** Given that Bet v I proteins are highly conserved at the structural level, the indicated residues of the NCS parent sequence point to the equivalent structural and sequence locations for engineering any one of these or other Bet v I proteins. The locations in the alpha helices and beta strands common to plant Bet v I proteins are shown in the inset of **FIG. 26** and described in **Table 9.**

In general, the norcoclaurine synthase natural and improved vanants are descnbed and recorded here as amino acid sequences using the standard one letter abbreviation for amino acid residues. These peptide sequences could be translated to nucleotide sequences using any of the codons in **Table 10.** In addition, any one amino acid could be substituted for another amino acid of similar properties as indicated in **Table 10.**

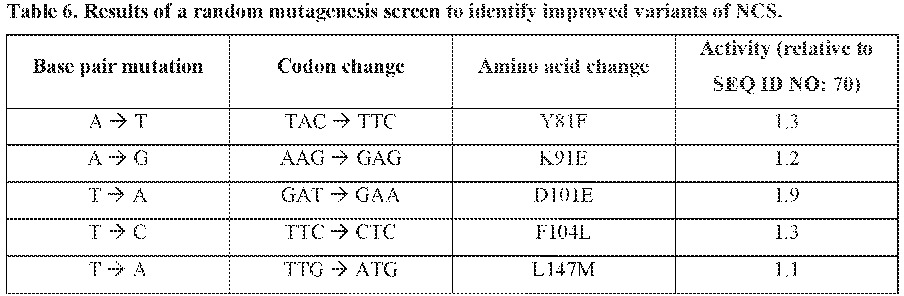

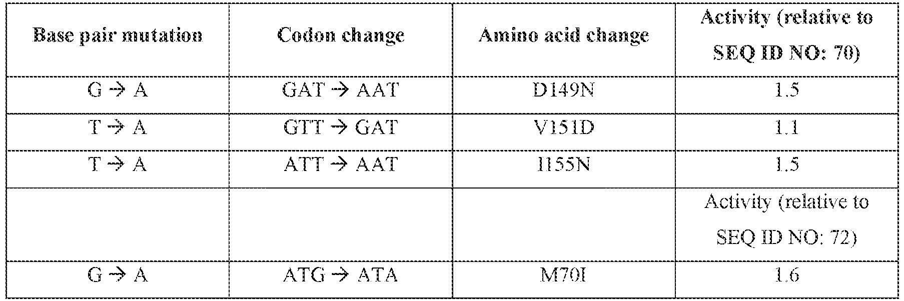

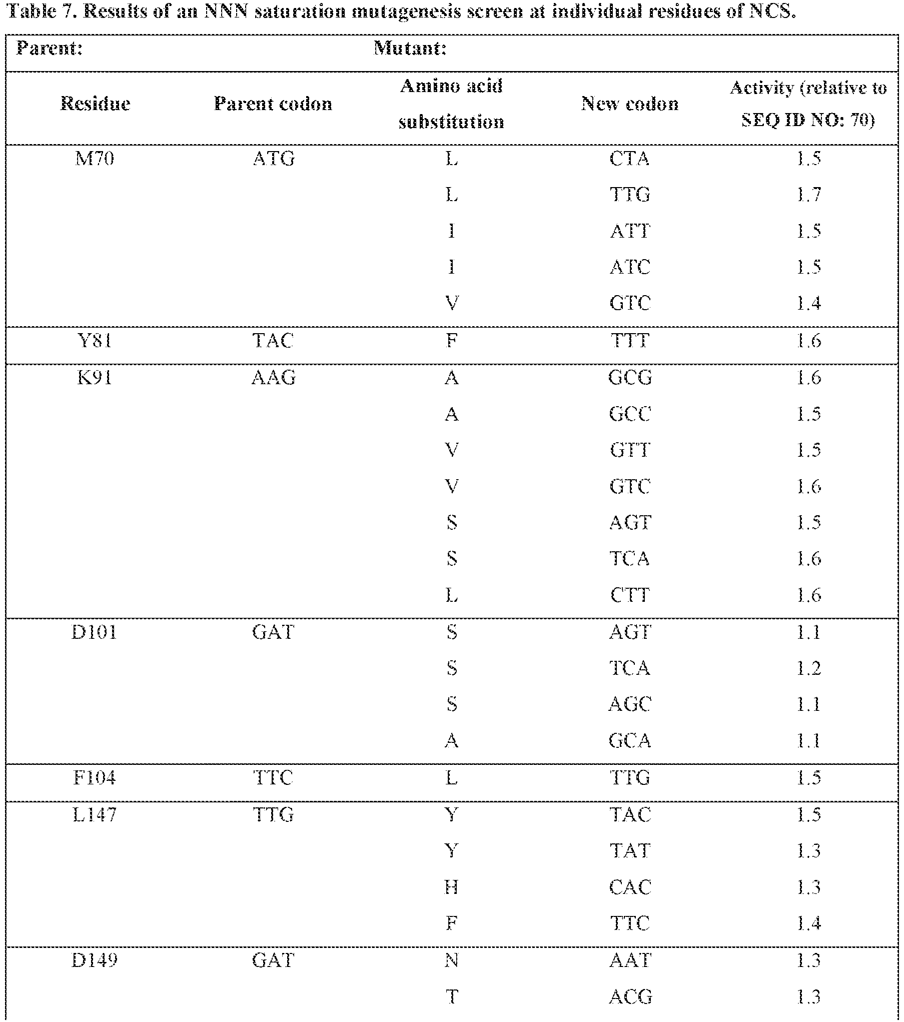

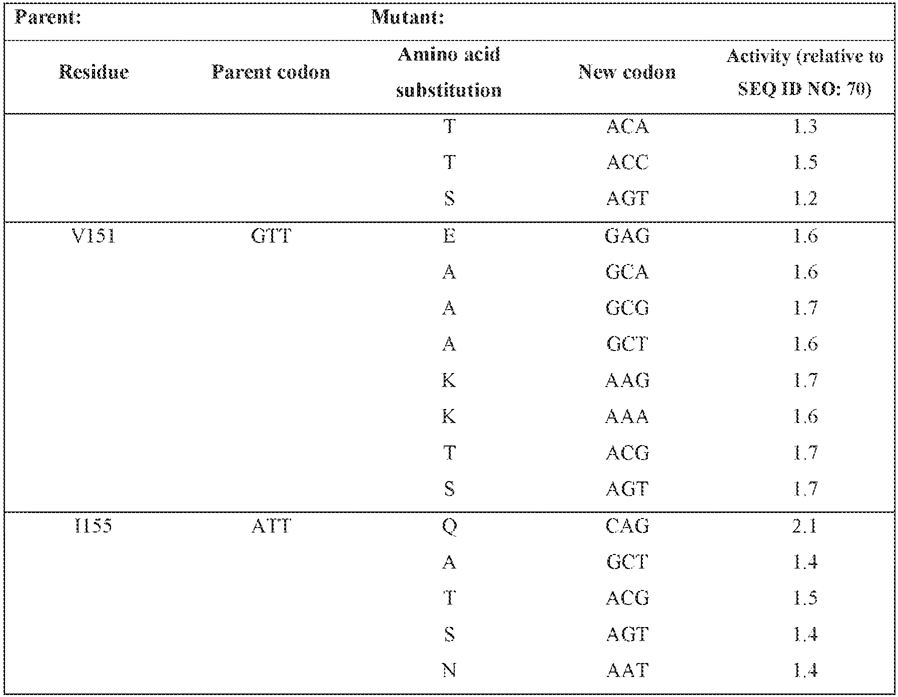

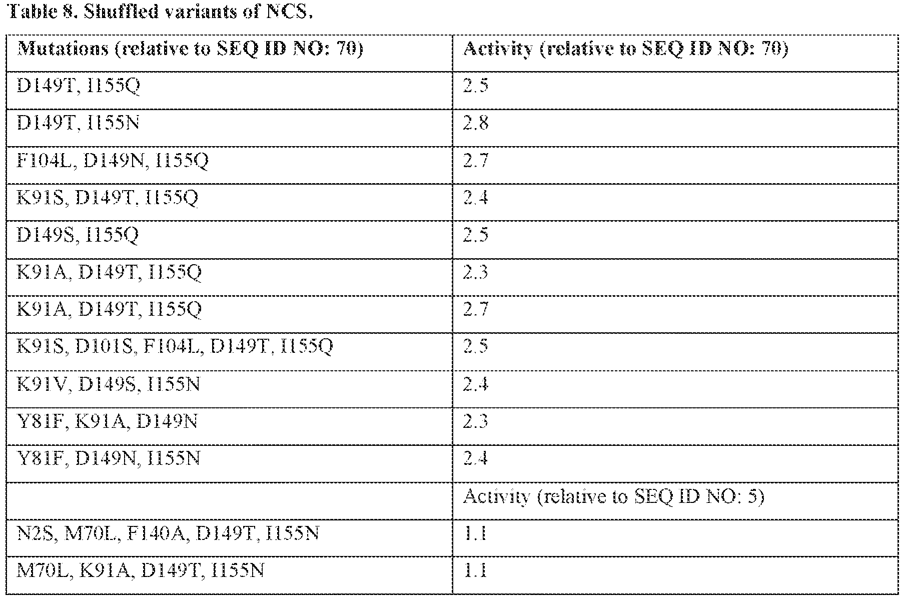

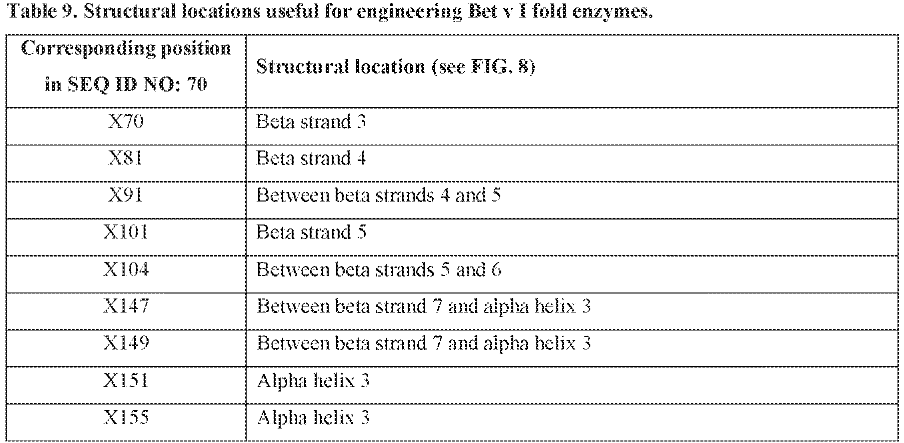

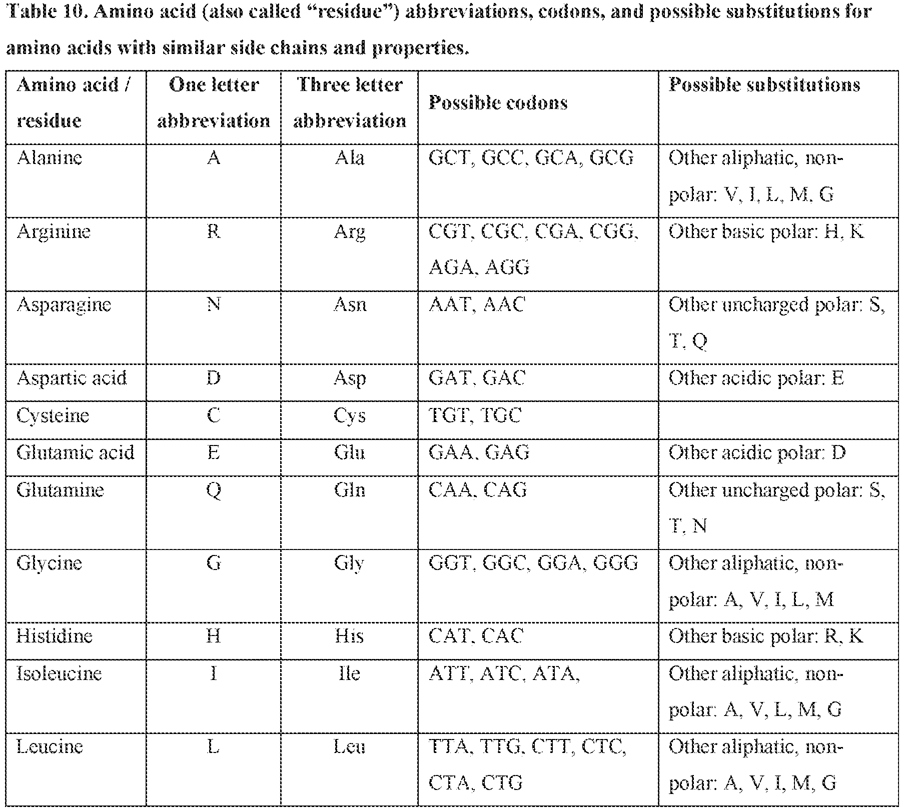

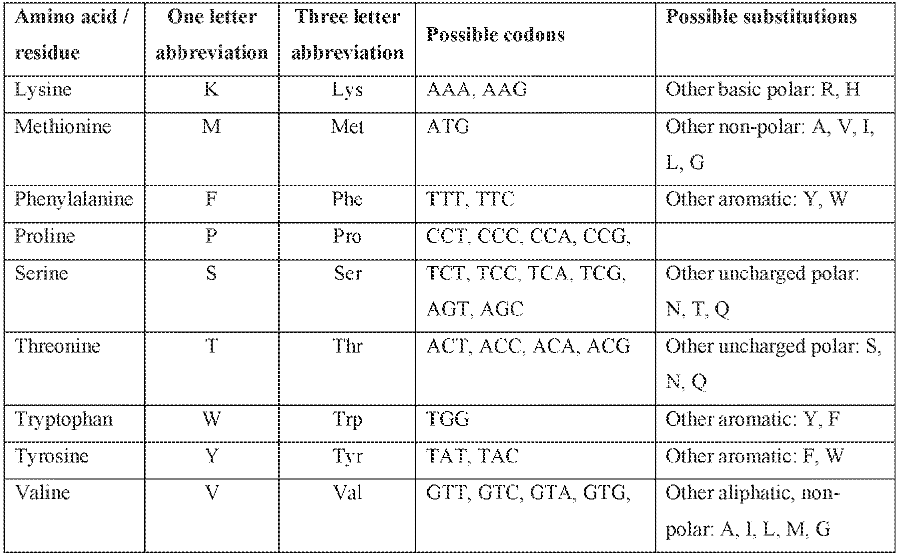

### Example 16: Production of BIAs in microbial strains with engineered NCS

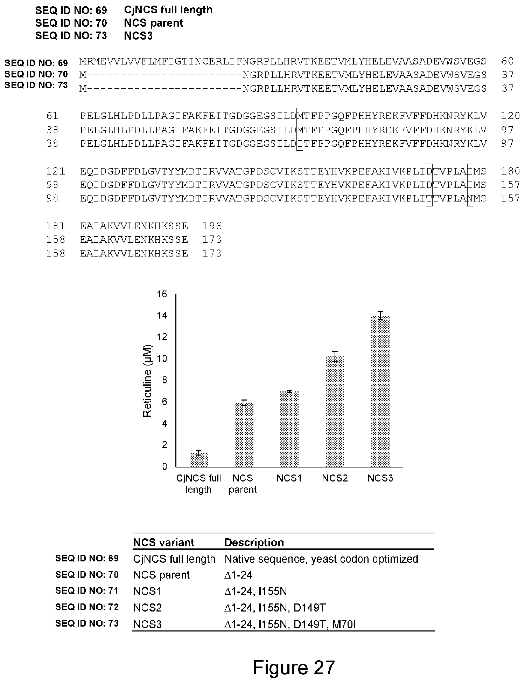

To demonstrate the utilization of the engineered NCS enzymes in enhancing the production of benzylisoquinoline alkaloids in yeast, both untruncated and truncated NCS variants with one, two, or three beneficial mutations were used to generate an enzyme ladder. Specifically, plasmids encoding CjNCS full length (SEQ ID NO: 69), NCS parent (SEQ ID NO: 70). NCS1 (SEQ ID NO: 71). NCS2 (SEQ ID NO: 72). and NCS3 (SEQ ID NO: 73) were transformed into YA139. When cultured under standard assay conditions in 96-well microtiter plates (and without tyrosine supplementation). NCS 3 with a 24 amino acid N-terminal truncation and mutations M701. D149T, and I155N, produced 14.02 µM reticuline (**FIG. 27**). This observed titer was more than 10-fold greater than the 1.31 µM inticuline produced by a strain expressing CiNCS full length.

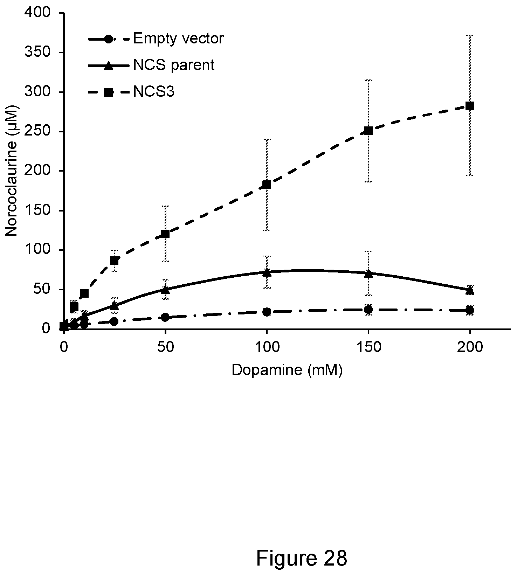

To further characterize the enhanced activity of NCS3 (SEQ ID NO: 73), a wild type CEN.PK2 strain was transformed with plasmids carry ing either NCS parent (SEQ ID NO: 70). NCS3 (SEQ ID NO: 73), or an empty plasmid (no-enzy me control). Strains were cultured in 96-well microtiter plates with medium supplemented with 300 mg/L tyrosine (to promote 4-HPAA production by the host cell's native metabolism) and 0, 5, 10, 25, 50, 100, 150, 200 mM dopamine. After 48 hours growth the plates were centrifuged to pellet the cells and the final norcoclaurine titer determined for each well by LCMS (**FIG. 28**). The strain expressing NCS parent produced 72.1 µM norcoclaurine when supplied with 100 mM dopamine in the culture medium. However. at higher concentrations of 150 and 200 mM dopamine, the norcoclaurine yield for this strain decreased, indicating the NCS parent suffered inhibition at dopamine concentrations over 100 mM. In contrast, the strain expressing NCS3 accumulated 182.6 µM norcoclaurine at 100 mM dopamine, and titers increased further to 250.8 and 283.0 µM norcoclaurine at dopamine supplementation of 150 and 200 mM. respectively.

### Example 17: Production of thebaine using engineered microbial strains

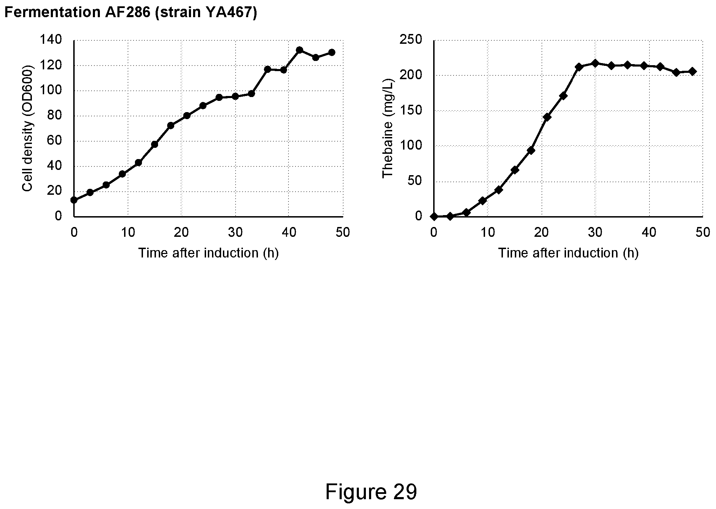

A thebaine production stain was constructed that included three copies of the improved norcoclaurine synthase variant, NCS3. and the complete biosynthetic pathway to thebaine (strain YA467, **FIG. 4**). Strain YA467 was cultured in a 1 L fermentor with fed-batch glucose (fermentation AF286). The synthetic complete medium was further supplemented with amino acids, vitamins, salts, and trace elements at the start of the culture and at time points during the fermentation. After 48 h the bioprocess reached OD₆₀₀ 130 and achieved a final titer of 206 mg/L. thebaine (**FIG. 29**). The thebaine strain and bioprocess could be further modified to produce any of the opioid molecules depicted in **FIGs. 4** **and** **6**.

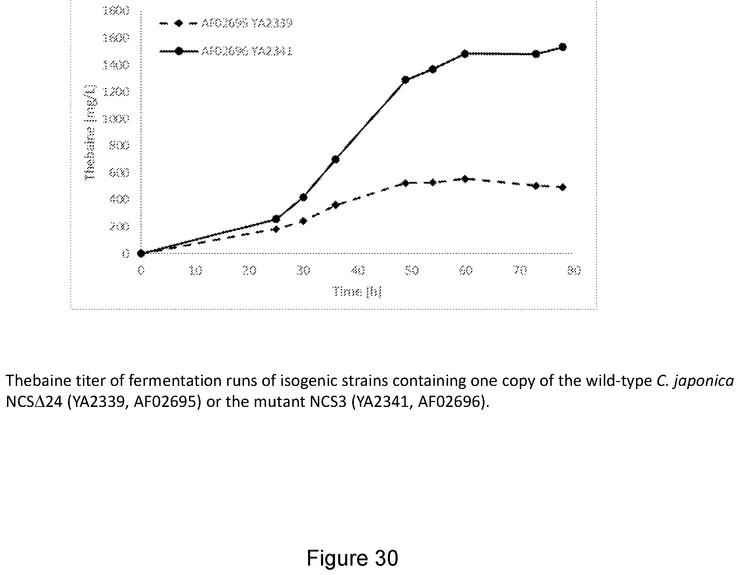

The improved variant NCS3 was also compared to the wild-type NCSA24 sequence in strains producing thebaine from glucose. Strains were nm in a fed-batch fermentation process supplemented with additional amino acids, vitamins, salts, and trace elements. The strain with NCS3 (YA2341, AF02696) produced 1.5 g/L thebaine compared to the wild-type sequence with an N-terminal tmncation (YA2339, AF02695) that produced only 0.5 g/L thebaine after 78 h (**FIG. 30**).

### Example 18: Production of noscapine using engineered microbial strains

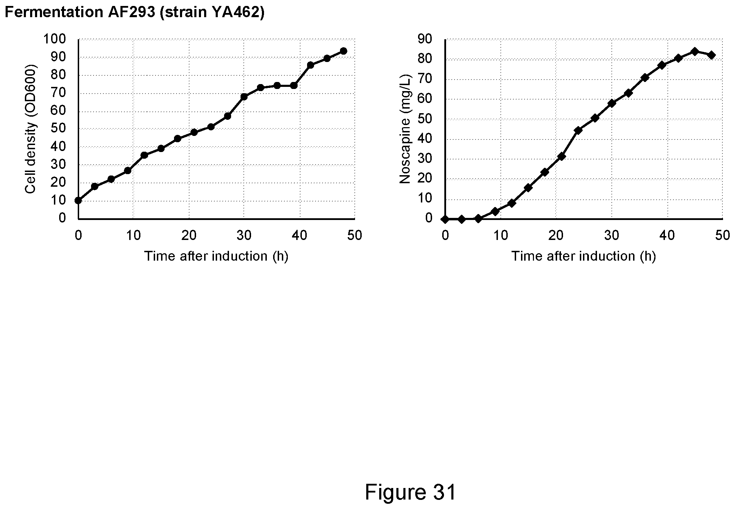

A noscapine production strain was constructed that included four copies of the improved norcoclaurine synthase variant NCS3. and the complete biosynthetic pathway to noscapine (strain YA462. **FIG. 7**). Strain YA462 was cultured in a 1 L fermentor with fed-batch glucose (fermentation AF293). The synthetic complete medium was further supplemented with amino acids, vitamins, salts, and trace elements at the start of the culture and at time points during the fermentation. After 48 h the bioprocess reached OD₆₀₀ 82 and achieved a final titer of 82 mg/L noscapine (**FIG. 31**).

### Example 19: Platform microbial strains engineered to product thebaine with reduced fusel alcohol production

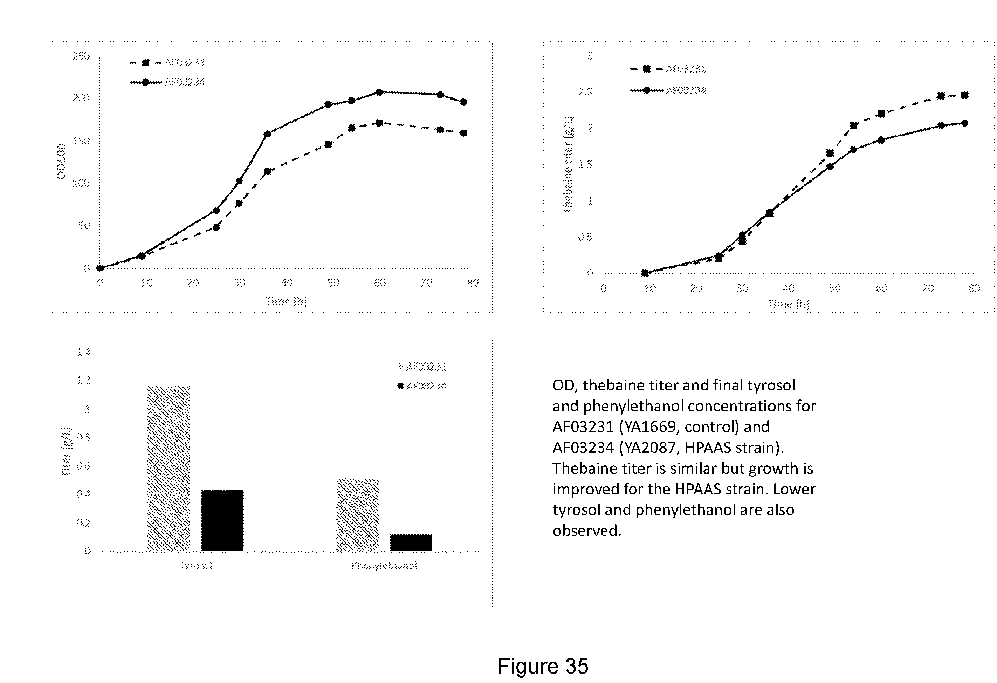

Yeast strains that produce thebaine can be further engineered to reduce accumulation of fusel alcohols that limit growth and titer. As an example, the platform yeast strains described in **Example 17** can be further engineered to reduce phenylethanol production while maintaining or increasing thebaine titer (**FIG. 35**). The platfonn yeast strain producing thebaine from glucose was further engineered to incorporate a 4-hydroxyphenylacetaldehyde synthase (4HPAAS). An expression cassette (P*_{GAL7}*-*PcHPAAS*) was assembled and integrated into the ARO10 locus, disrupting the gene. The strain with 4HPAAS was compared to an isogenic strain where the native ARO10 promoter was replaced with the GAL7 promoter.

Strain YA1669 with P*_{GAL7}-ARO10* was compared to YA2087 with *P_{GAL7}-PcHPAAS* disrupting the ARO10 locus in a 1 L fermentor with fed-batch glucose (fermentations AF03231 and AF03234). The medium was further supplemented with amino acids, vitamins, salts, and trace elements. After 73 h the titer of YA1669 was 2.4 g/L thebaine, and the titer of YA2087 was 2.0 g/L thebaine. The phenylethanol level was 0.51 g/L for YA1669 compared to 0.12 g/L for YA2087. The tyrosol level was 1.2 g/L for YA1669 compared to 0.43 g/L for YA2087. The reduced fusel alcohols also allowed YA2087 to grow to OD₆₀₀ 206 compared to YA1669 which grew to OD₆₀₀ 170.

### Example 20: Platform microbial strains engineered to product thebaine using the arogenate pathway to make tyrosine

Yeast strains that produce thebaine can be further engineered using a heterologous arogenate pathway to make tyrosine. This pathway does not have phenylpyruvate as an intermediate and can further reduce phenylethanol production. The platform yeast producing thebaine from glucose and using 4HPAAS from Example 19 was further engineered to use the arogenate pathway for the production of phenyalanine and tyrosine. An expression cassette (*P_{TEF1}-AtPAT*) was integrated at the neutral locus SWH1 to make arogenate. A second expression cassette was assembled (*P_{GAL10}*-*AtPAT*, *P_{GALI}-AtAAT*) to add another copy of the PAT gene and integrate the AAT gene at the CIN5 locus. A third expression cassette was assembled (*P_{TDH1}*-*PsADT*) and integrated at the PHA2 locus to disrupt the gene. The native aromatic amino acid biosynthesis pathway was removed by additional deletions of ARO8, ARO9, and TYR1. This resulted in the strain YA2696.

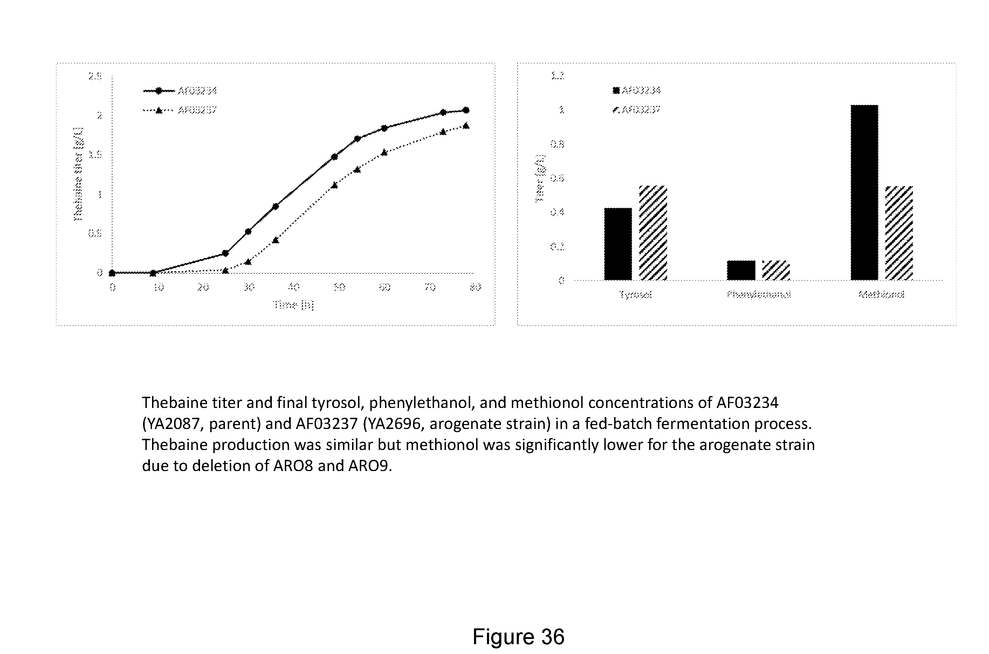

The yeast strain YA2696 containing the arogenate pathway was run in a 1 L fermentor with fed-batch glucose (fermentation AF(3237). The medium was further supplemented with amino acids, vitamins, salts, and trace elements. After 78 h, the thebaine titer was 1.9 g/L with an OD₆₀₀ 205 (**FIG. 36**). This is comparable to the parent strain YA2087. Phenylethanol and tyrosol were also comparable to parent at 0.12 g/L and 0.56 g/L, respectively. Methionol was decreased from 1.0 g/L to 0.55 g/L.

### Example 21: Platform microbial strains engineered to product thebaine with reduced ethanol and fusel alcohol production

Yeast strains that produce thebaine and use 4HPAAS can be further engineered to reduce ethanol and fusel alcohol accumulation. The platform yeast producing thebaine from glucose and using 4HPAAS was further engineered to reduce production of acetaldehydes by disruption of pyruvate decarboxylase genes PDC1, PDC5. and PDC6. Without pyruvate decarboxylase activity, an alternative route to acetyl-CoA is required for growth on glucose. An expression cassette was constructed (*P_{TDH3}*-*CaPK*) to introduce a phosphoketolase enzyme that converts fructose-6-phosphate to acetyl-P and erythrose-4-phosphate. Acetyl-P is spontaneously converted to acetate which can then go to acetyl-CoA using the native acetyl-CoA synthetases (ACS1 or ACS2). Alternatively, a phosphotransacetylase enzyme (PTA) can be used to convert acetyl-P directly to acetyl-CoA which uses less ATP. With an alternative route to acetyl-CoA in place, the pyruvate decarboxylase genes PDC1, PDC5, and PDC6 were disrupted.

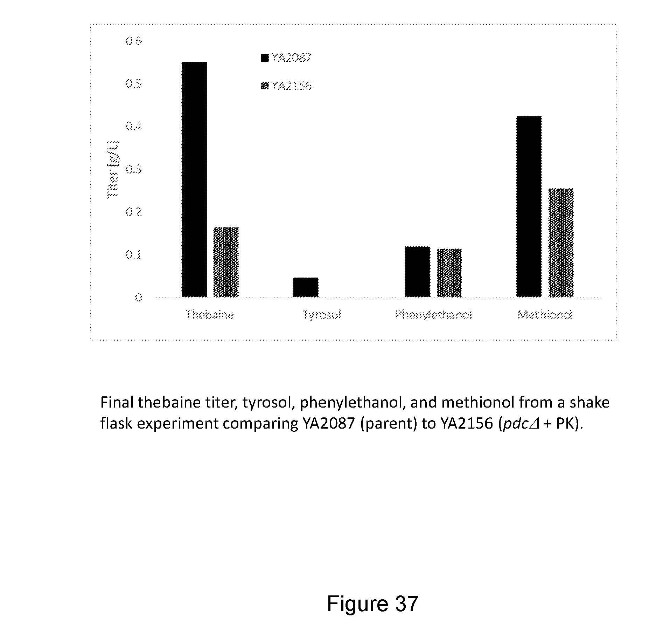

The resulting strain YA2156 with no PDC activity and PK integrated was compared to parent YA2087 in a shake flask experiment. After overnight growth in minimal media, strains were diluted 1 to 100 in fresh media containing 8% maltodextrin plus amylase to provide a slow release of glucose along with additional vitamins and amino acids. Shake flask cultures were incubated with shaking at 30°C for 72 hours. Thebaine titer for YA2156 was 0.17 g/L and titer for YA2087 was 0.55 g/L (**FIG. 37**). In YA2156, tyrosol was not detected, phenylethanol was 0.11 g/L. and methionol was 0.25 g/L. In YA2087, tyrosol was 0.05 g/L. phenylethanol was 0.12 g/L. and methionol was 0.42 g/L. Neither strain accumulated significant ethanol under this condition.

### Example 22: Platform microbial strains engineered to produce thebaine with increased SAM and improved methyltransferase activity

Yeast strains that produce thebaine can be further engineered to increase the co-factor S-adenosyl-L-metruonine (SAM) and improve the flux through several methyltransferase enzymes in the pathway. In a platform yeast strain producing thebaine, additional constructs were integrated to add two additional copies of SAH1 and one copy of SAM2 from *S. cerevisiae* (*P_{TDH3}*-*SAH1* at the PAU24 locus and *S4M2-P_{GALI,10}-SAH1* at the BAS1 locus).

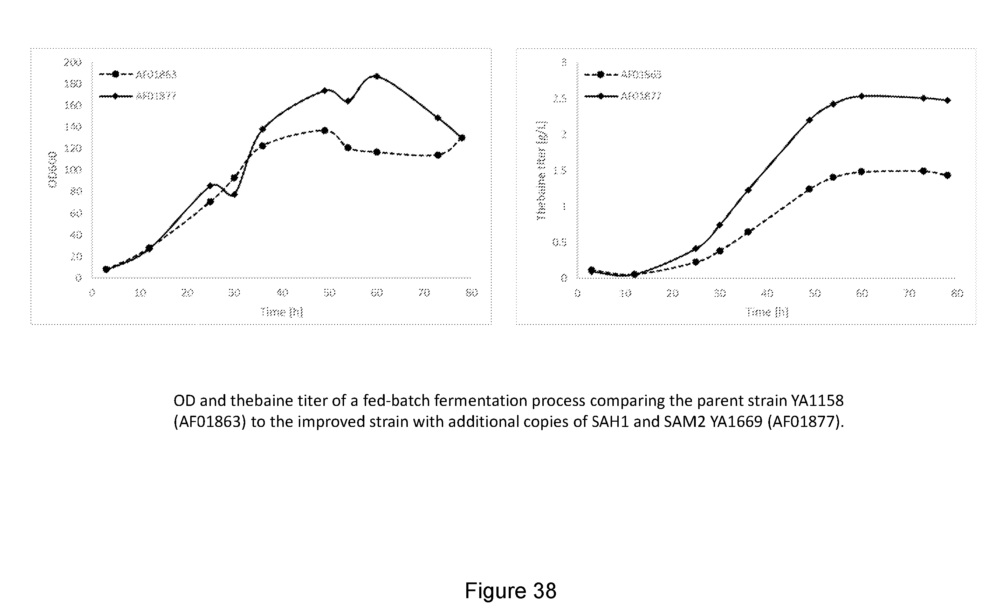

The resulting strain YA1669 with additional copies of SAM2 and SAH1 was run in a 1 L fermentor in a glucose fed-batch process (fermentation AF01877) The medium was supplemented with vitamins. trace elements, and amino acids. After 78 h, the thebaine titer of YA1669 was 2.4 g/L compared to only 1.3 g/L for the parent strain (YA1158, AF01863) (**FIG. 38**). The methyltransferase substrates norcoclaurine, coclaurin, and 3'OH-N-metllylcoclaurine were significantly reduced.

### Example 23: Platform microbial strains engineered to produce thebaine that converts aromatic fusel alcohols to less toxic by-products

Yeast strains that produce thebaine can be further engineered to convert excess fusel alcohols tyrosol and/or phenylethanol to salidroside and phenylethyl beta-D-glucoside. In a platform yeast strain producing thebaine, an additional constructs were integrated to express the UGT33 enzyme from *Rhodiola rosea* (*P_{TDH3}*-*UGT33* at the HXT5 locus and *P_{GAL7}-UGT33* deleting the EGH1 locus). A third copy of UGT from *Oryza sativa* was integrated (*P_{TDH3}*-UGT45) at the HST2 locus and BAT2 was also deleted in this background.

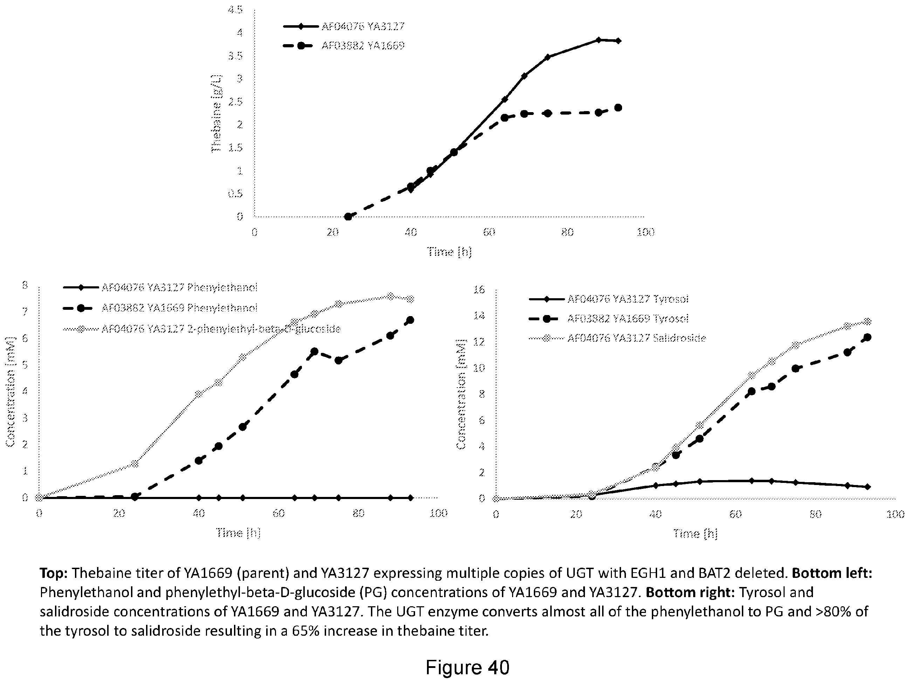

The resulting strain YA3127 producing thebaine and also expressing UGT33 was nm in a 1 L fermentor in a glucose fed-batch process (fermentation AF04076). YA3127 produced 3.8 g/L thebaine compared to the parent strain YA1669 which made 2.4 g/L thebaine (**FIG. 40**). The UGT preferentially used phen) lethanol as a substrate and converted nearly all of the phenylethanol to the glucoside (PG). Over 80% of the tyrosol was also converted to salidroside, resulting in a 65% increase in thebaine titer.

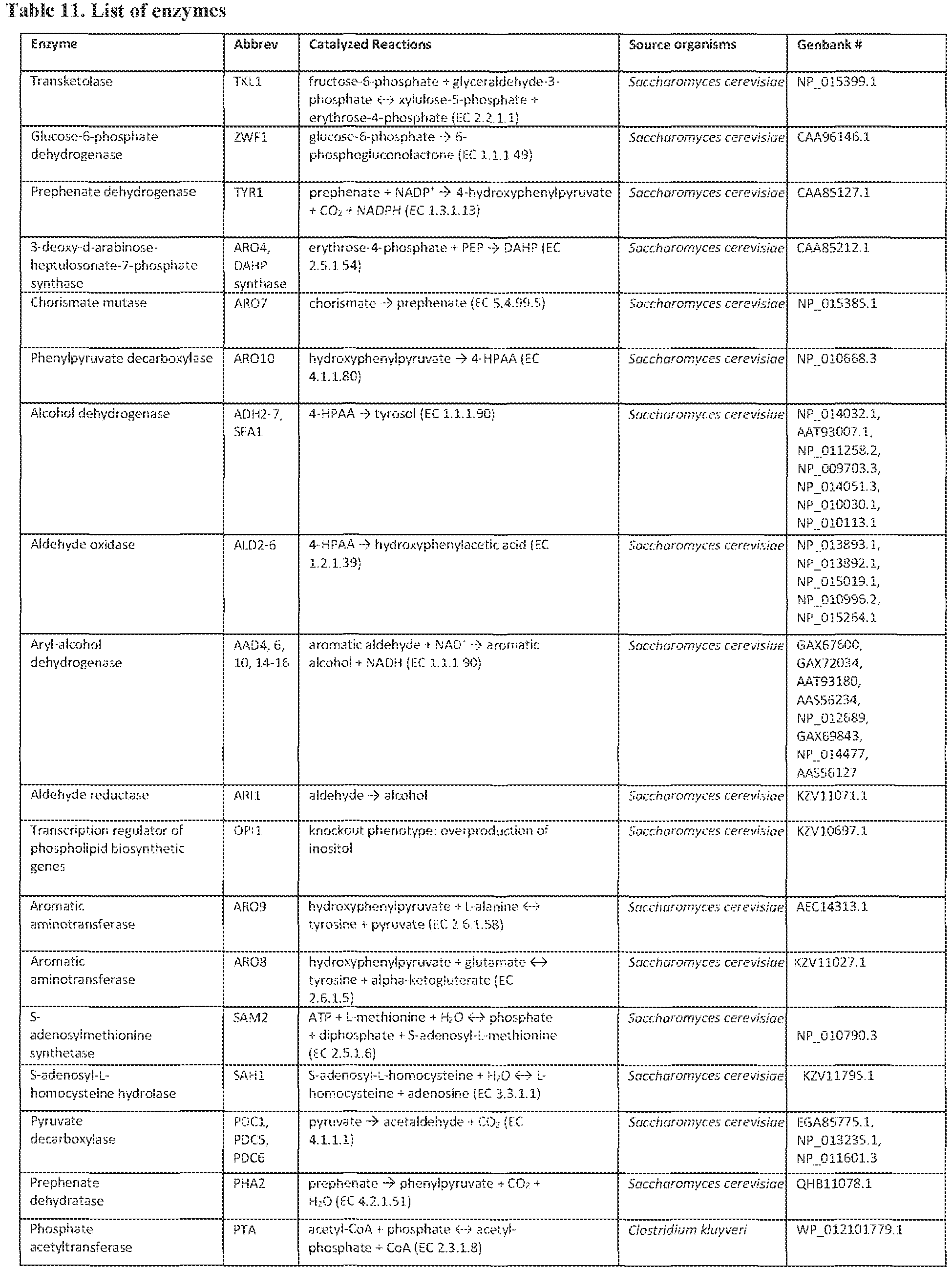

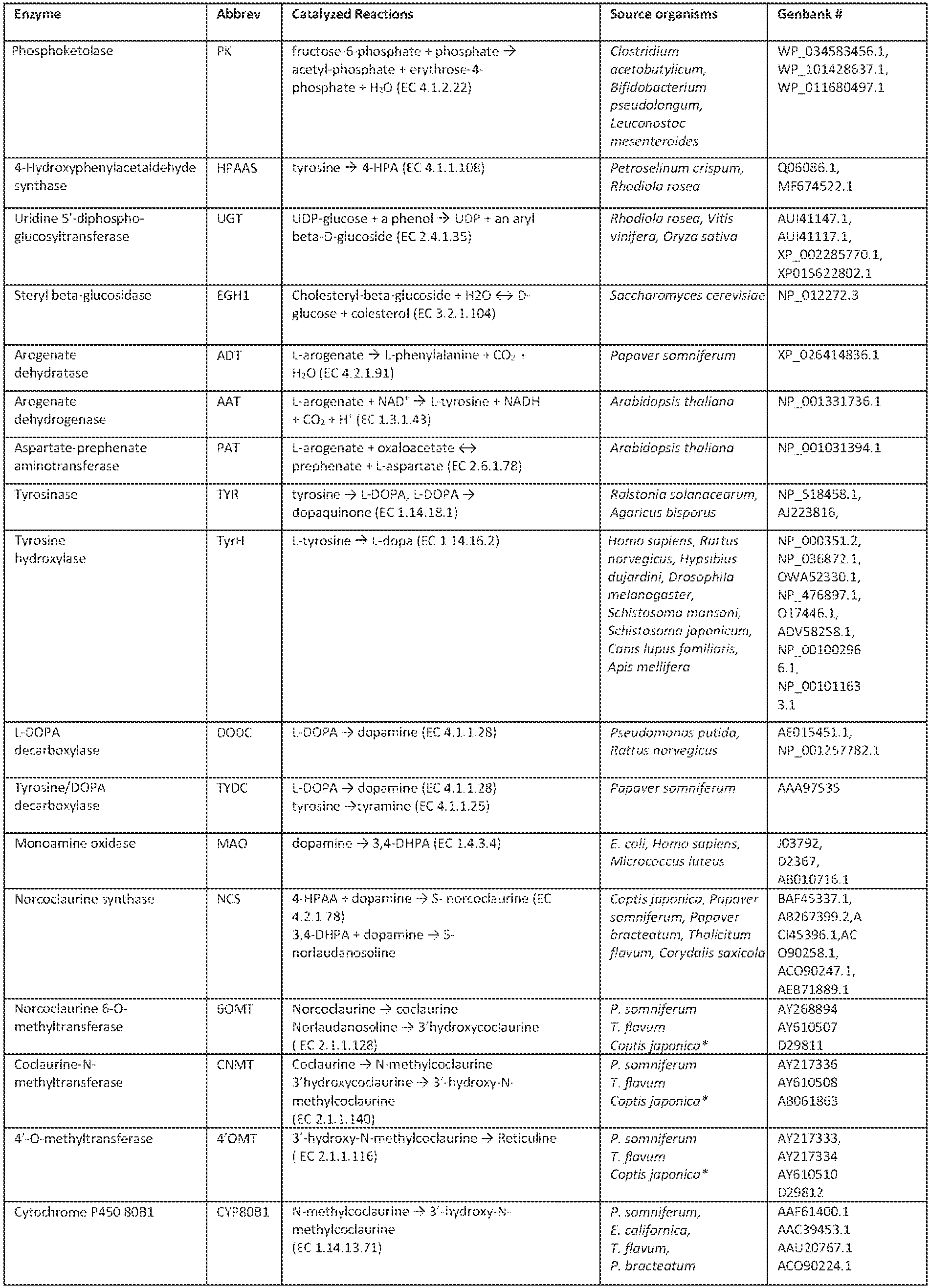

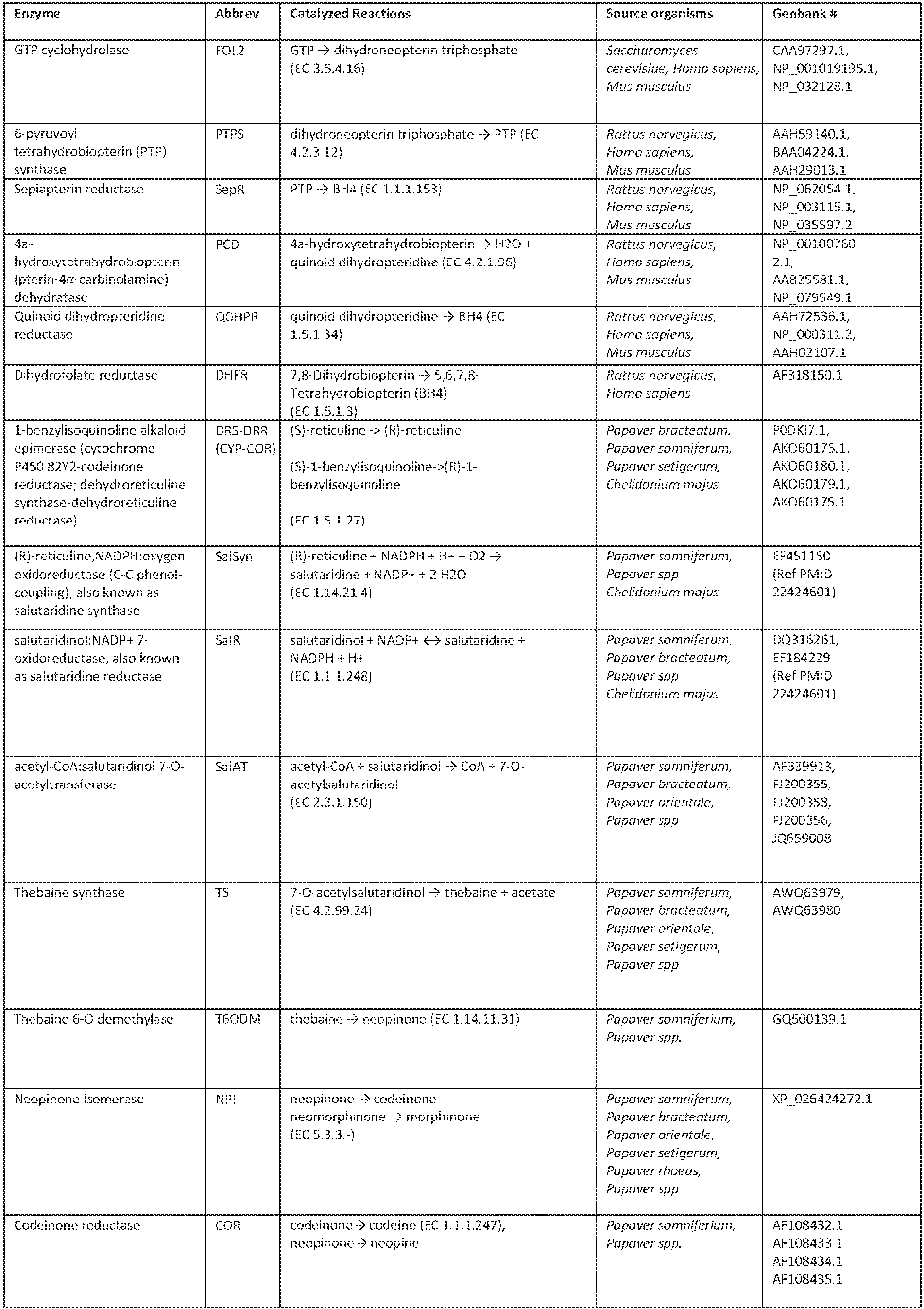

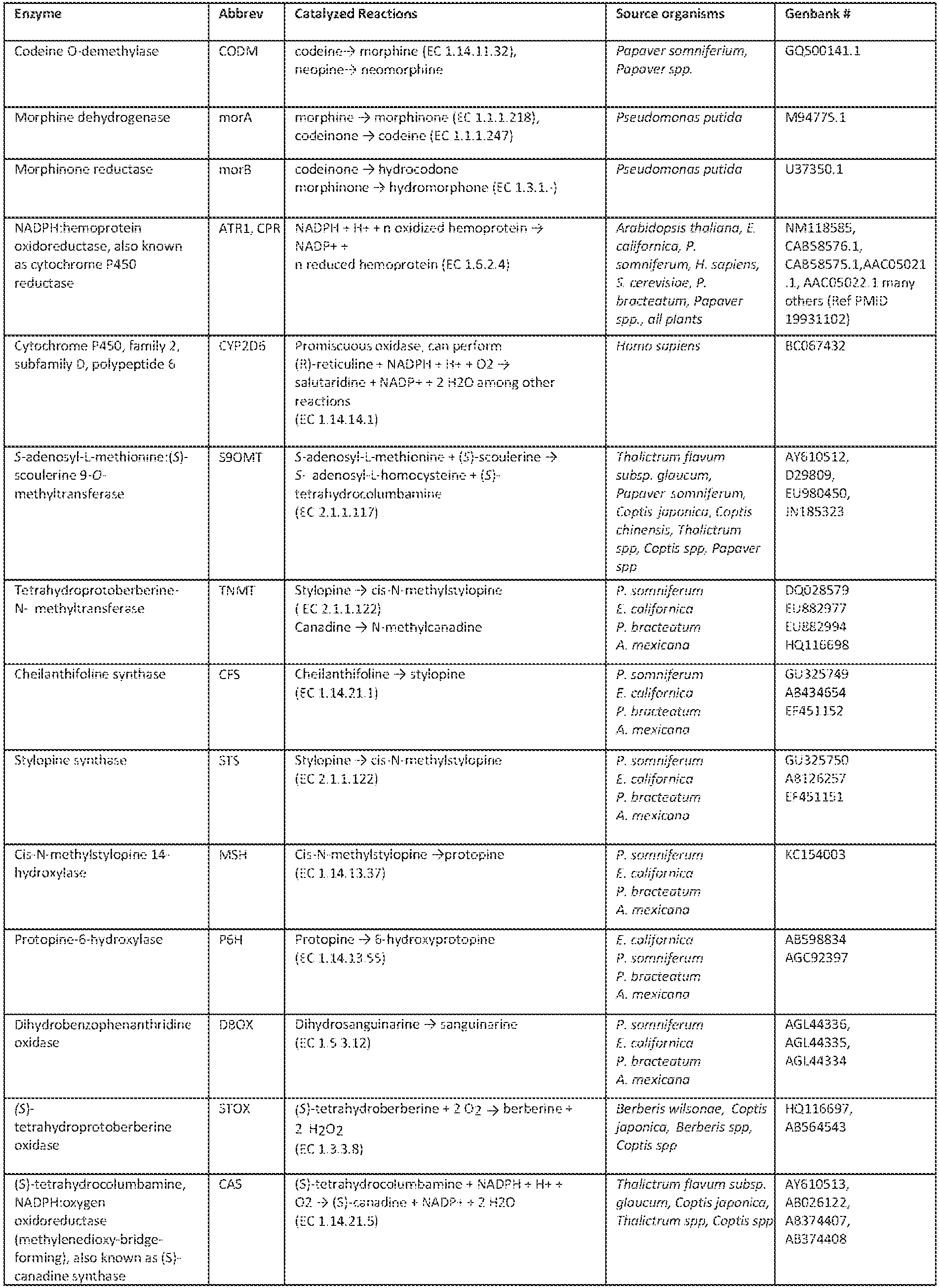

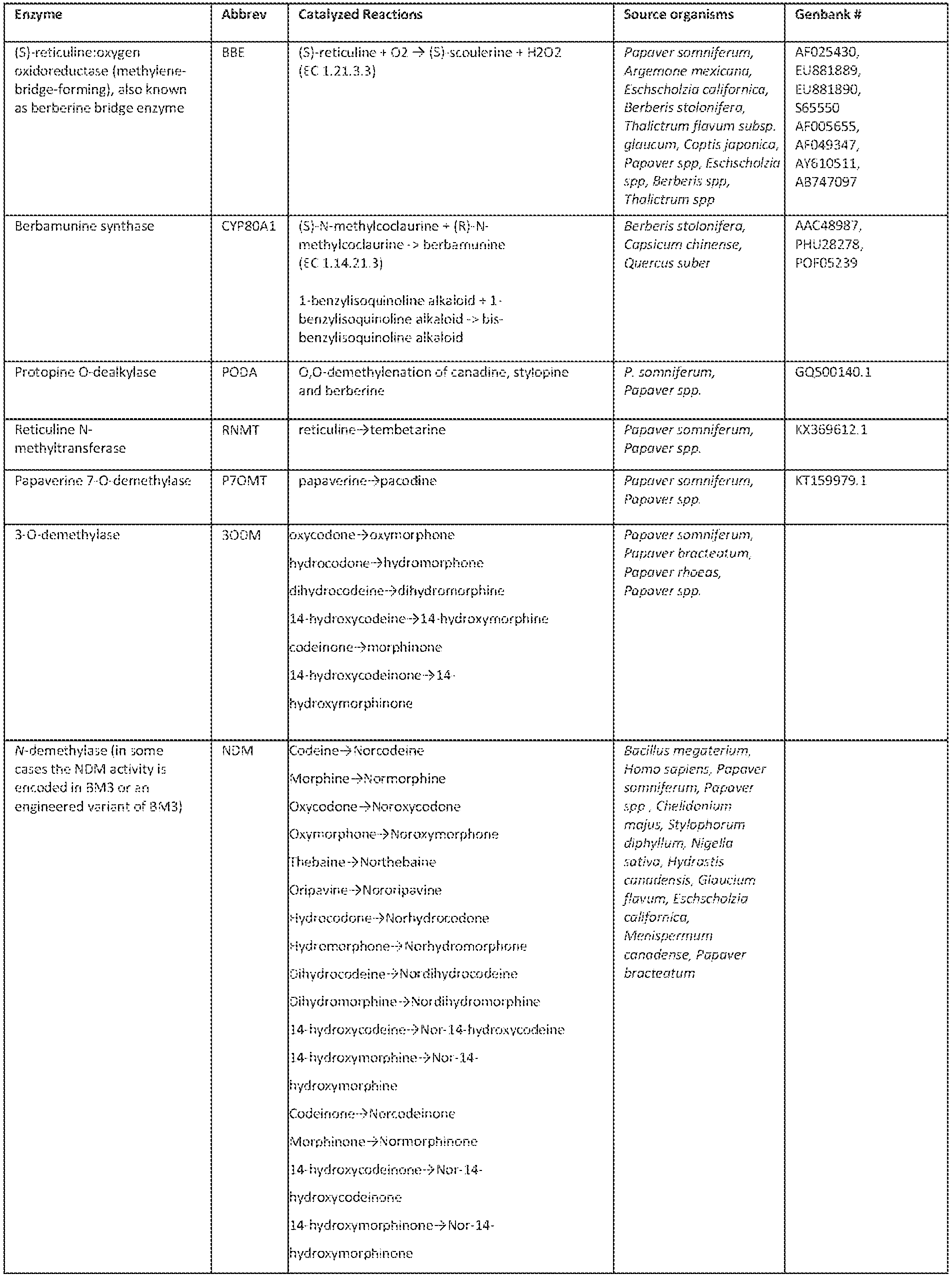

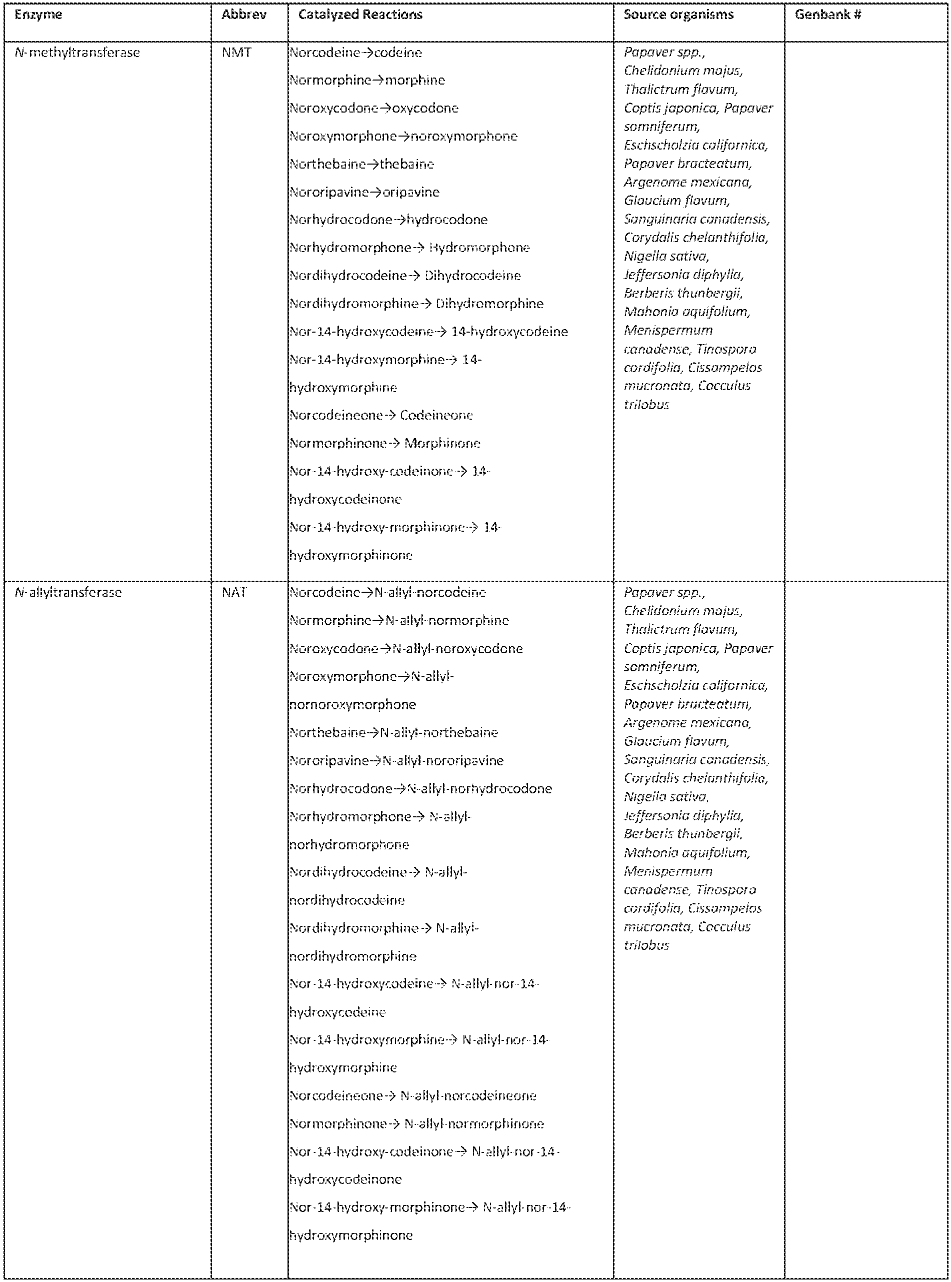

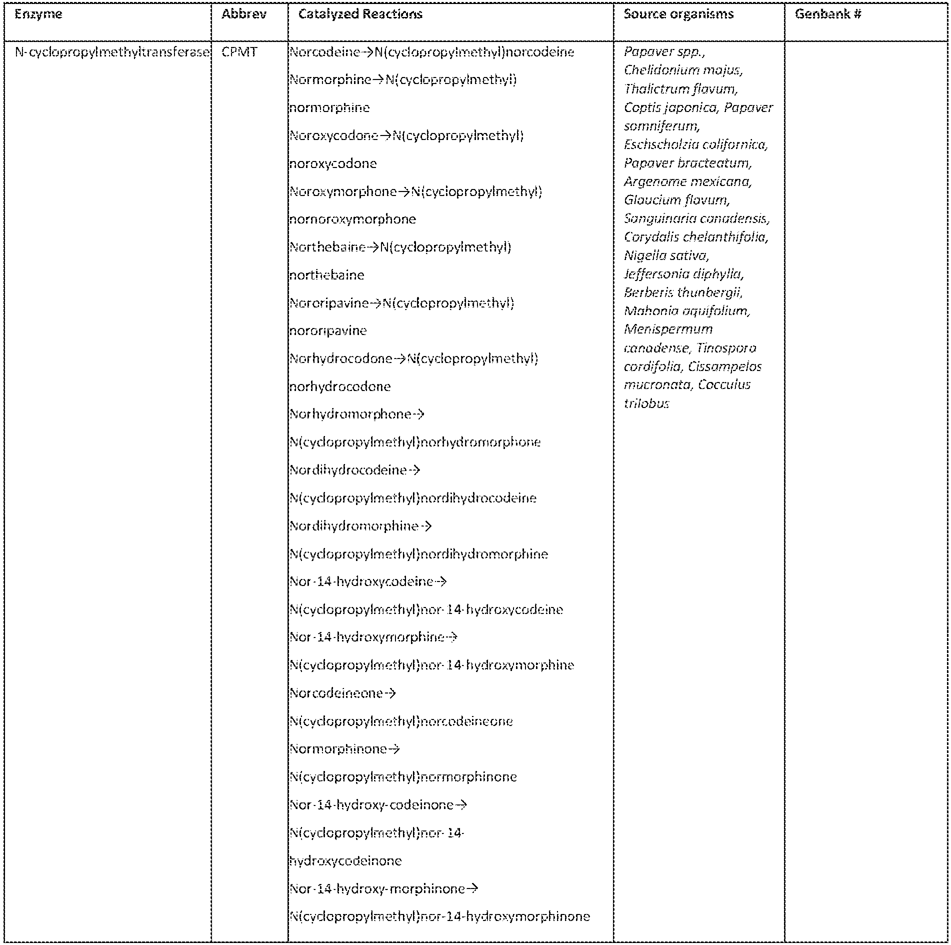

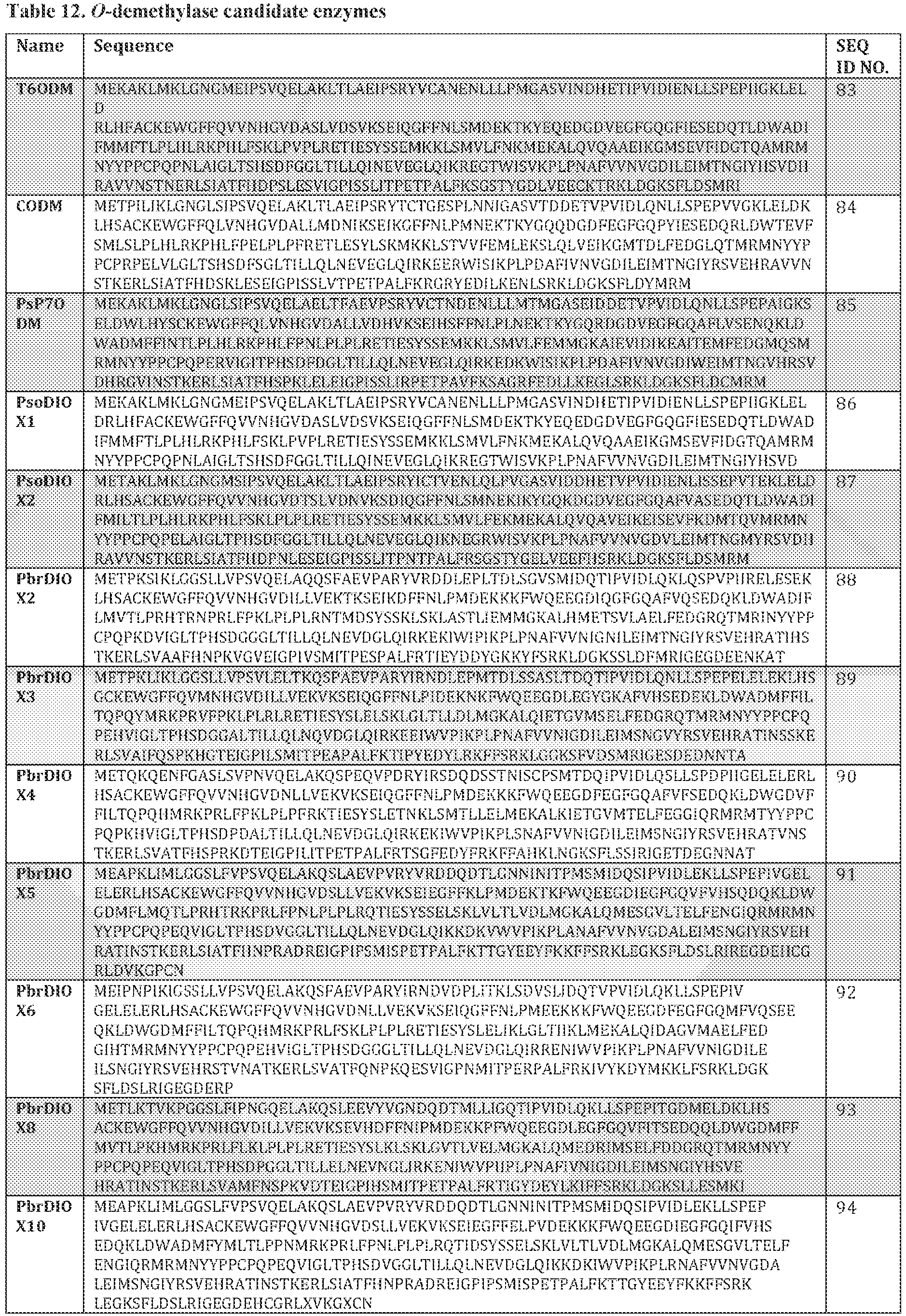

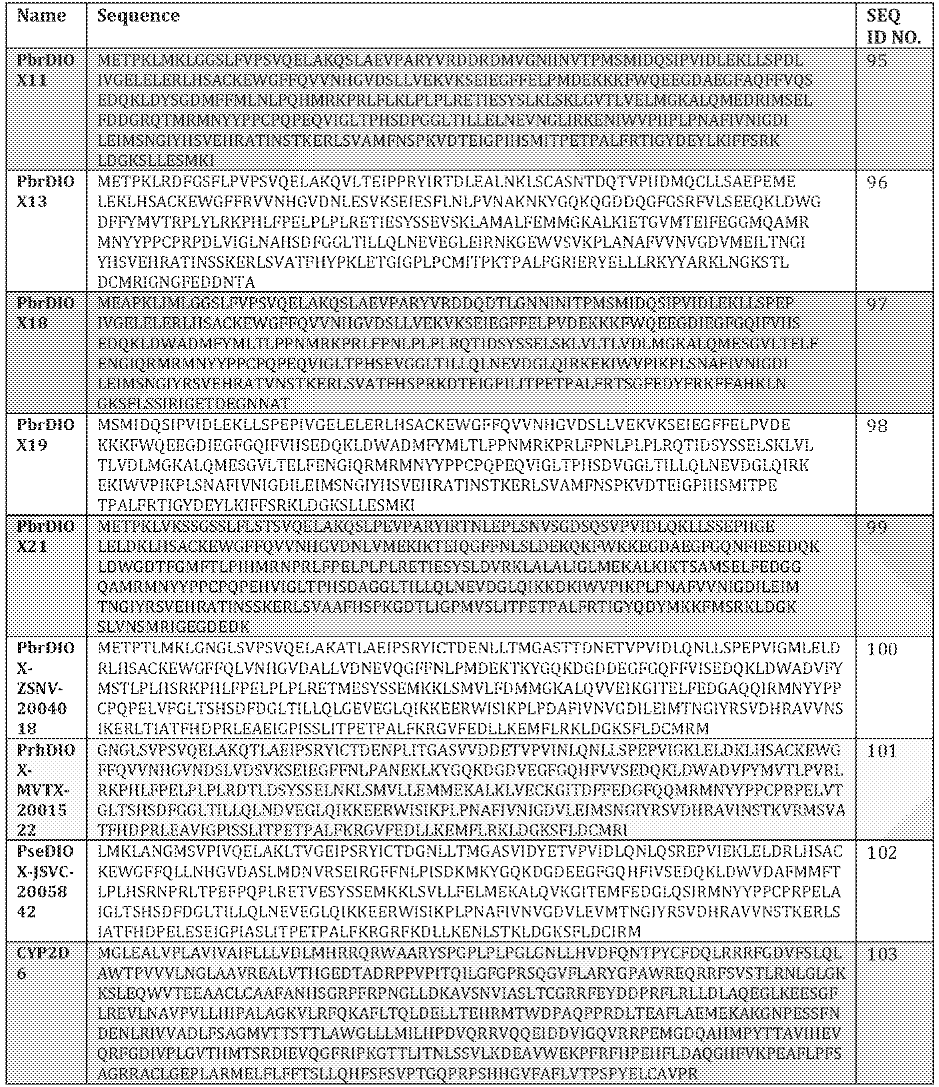

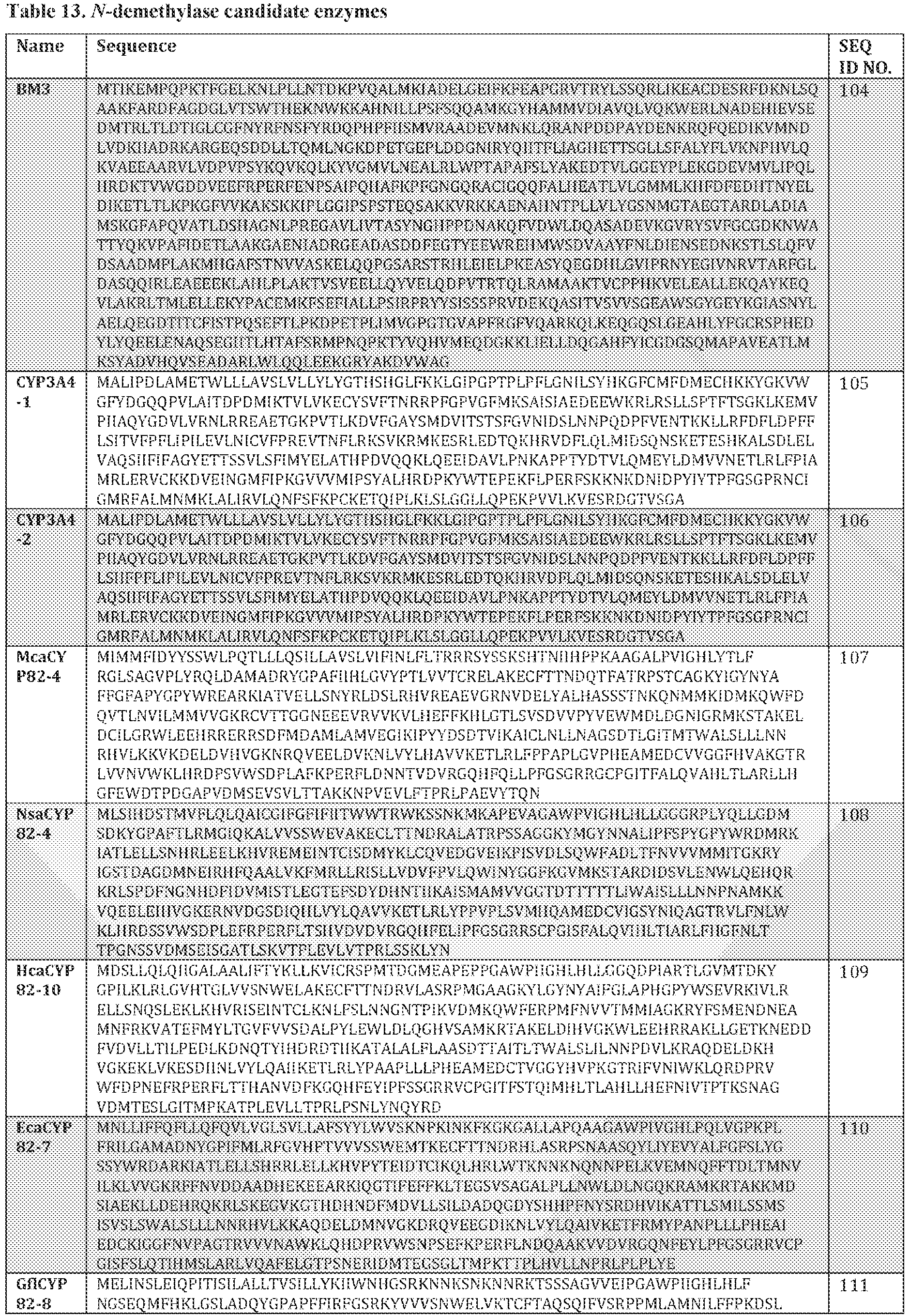

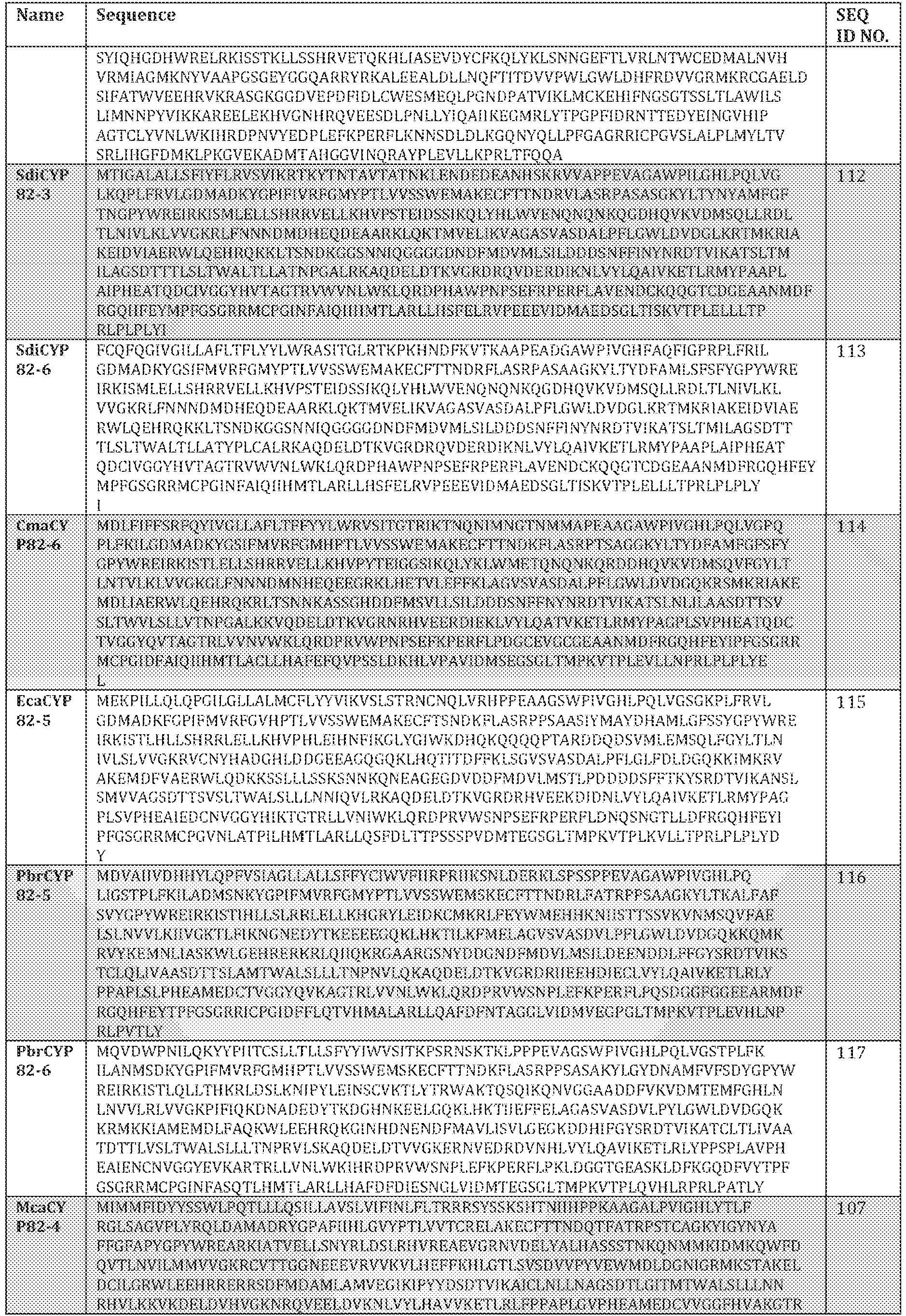

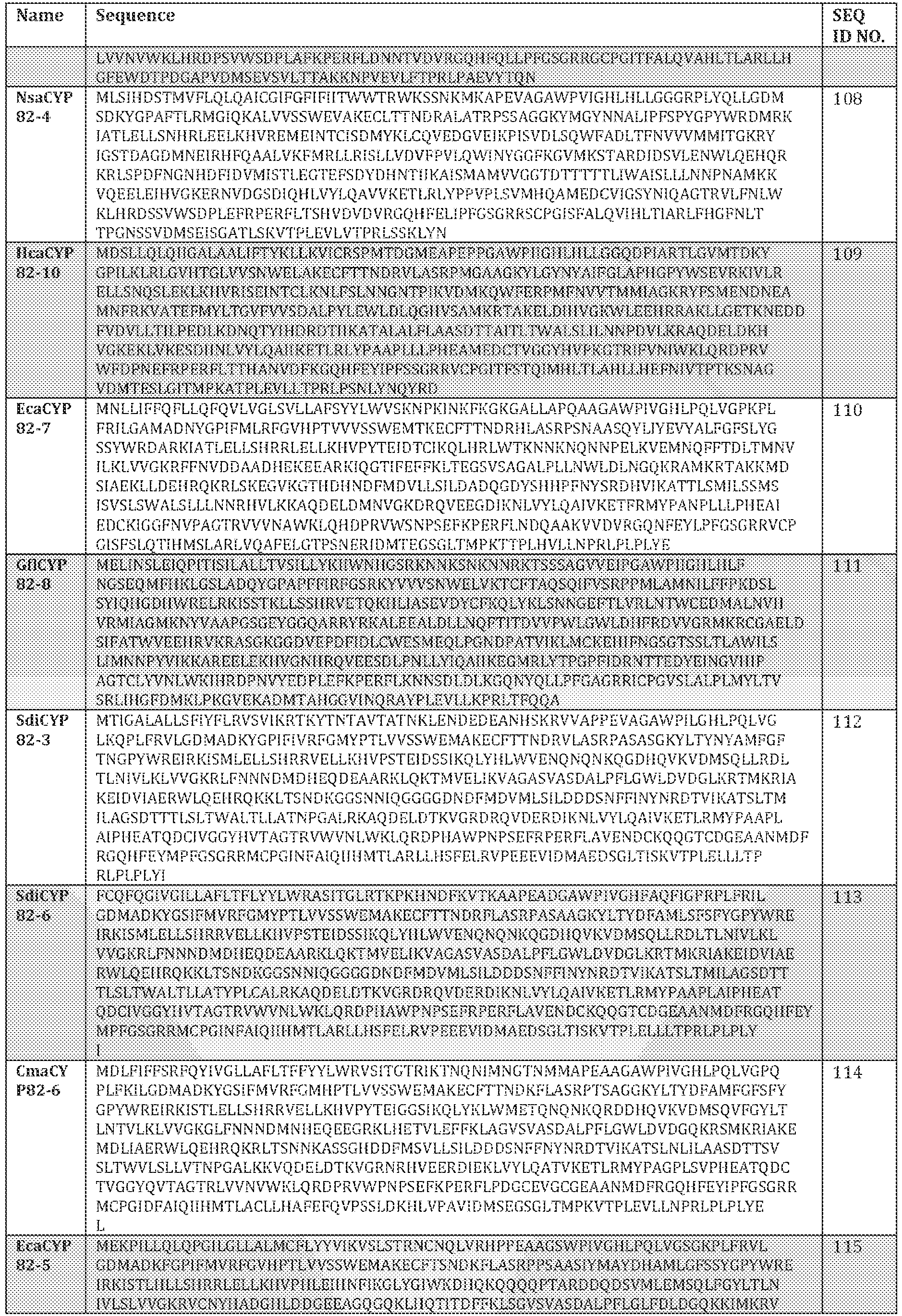

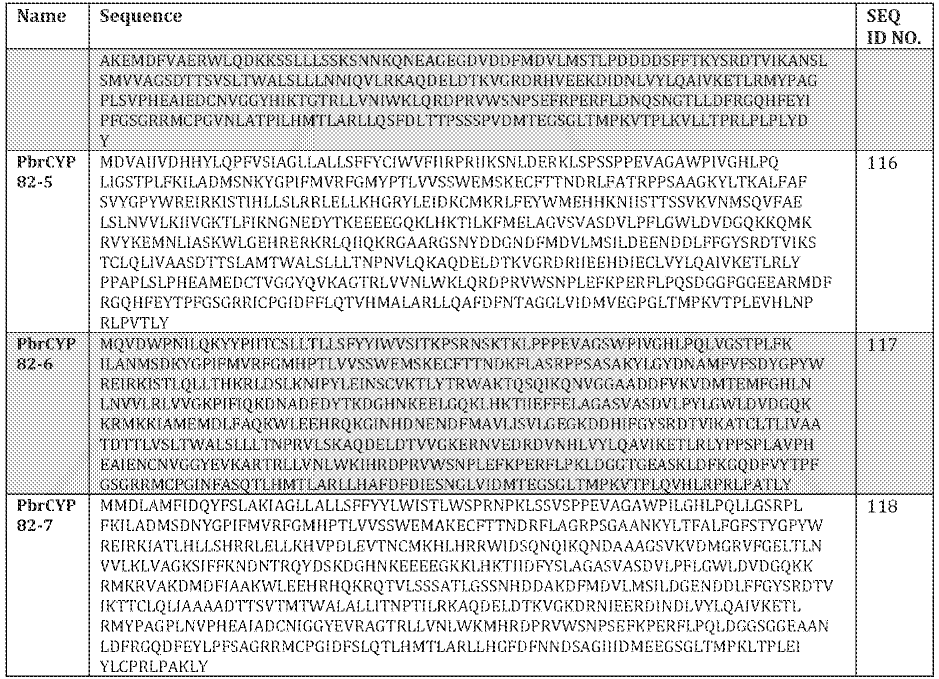

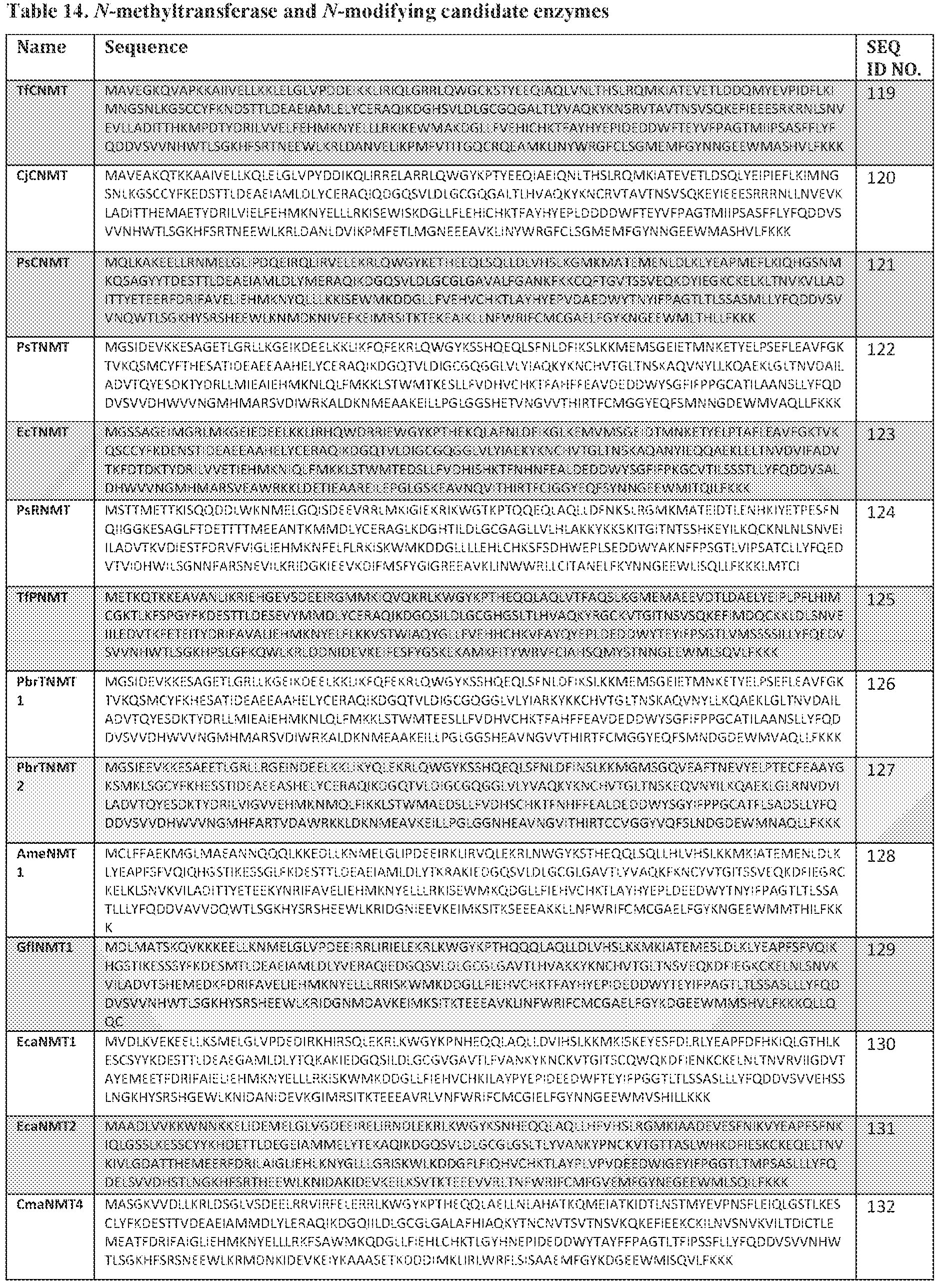

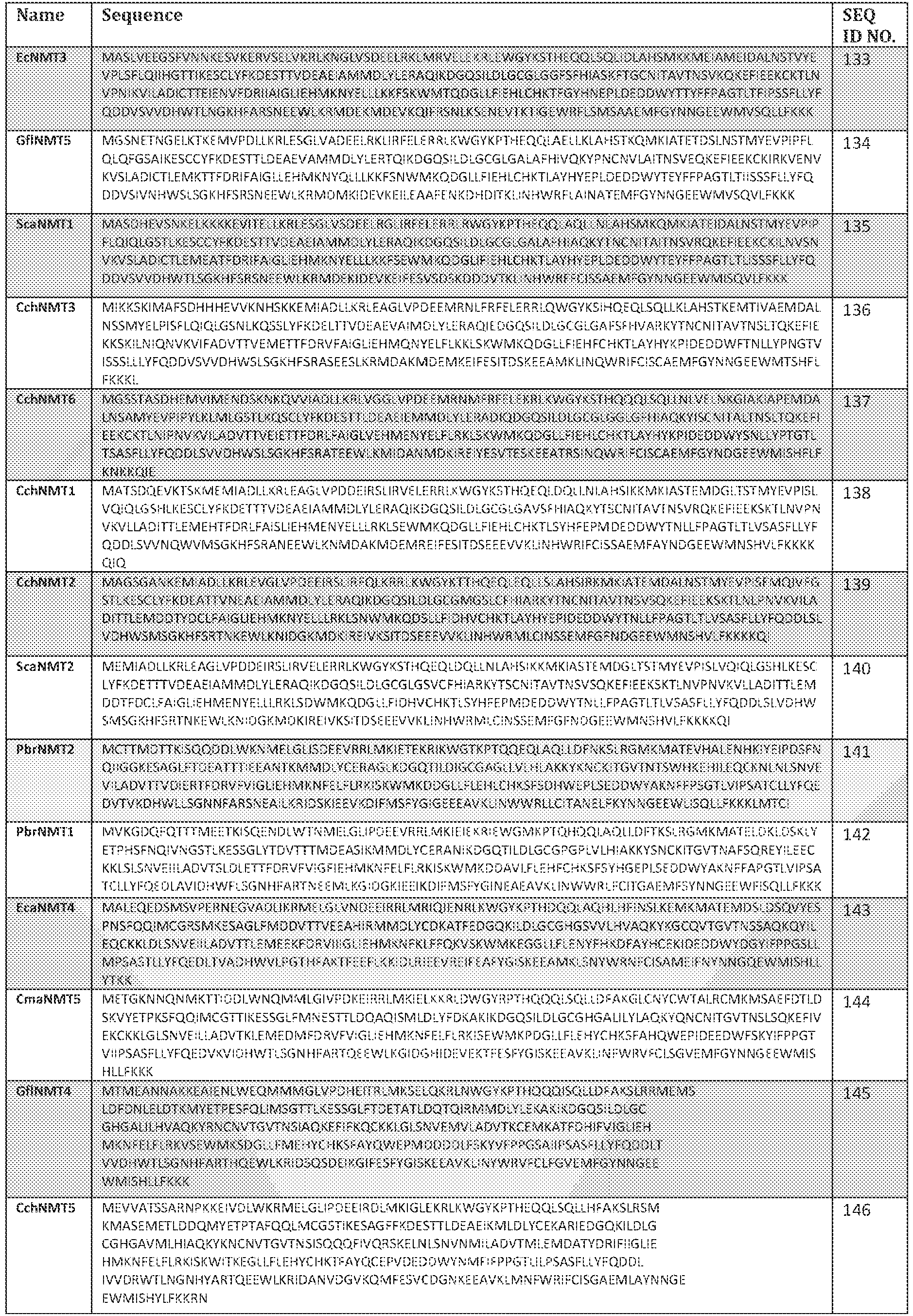

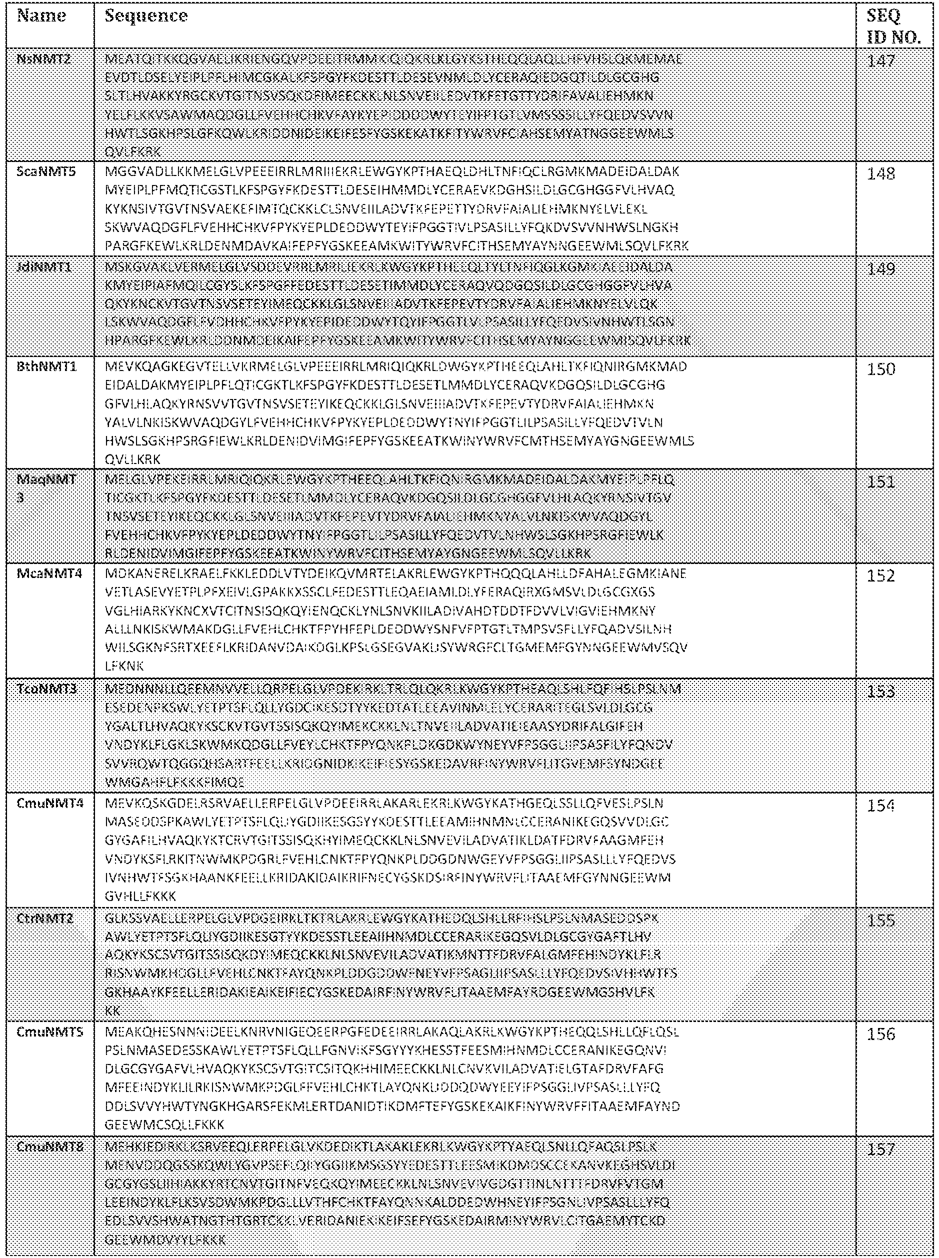

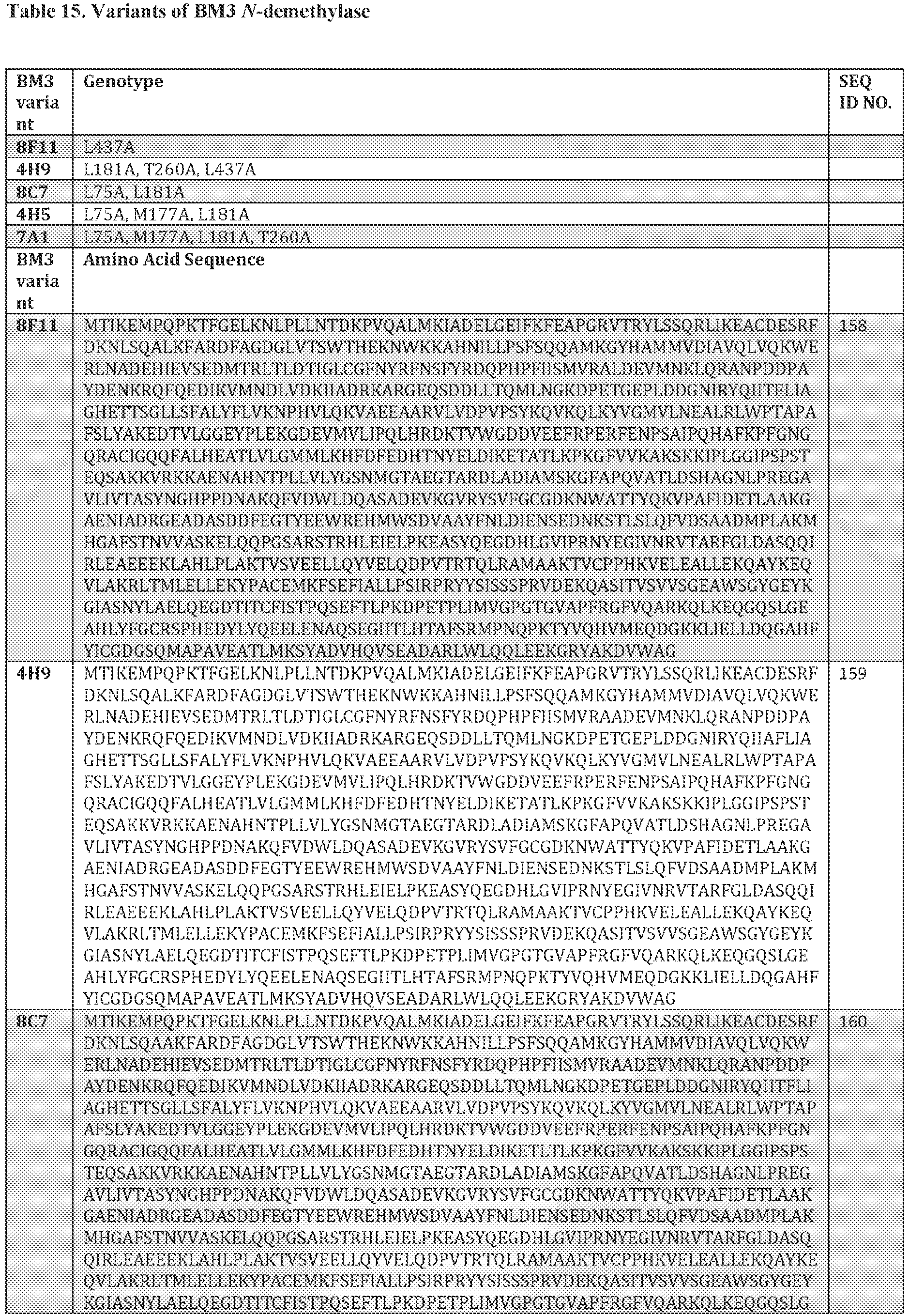

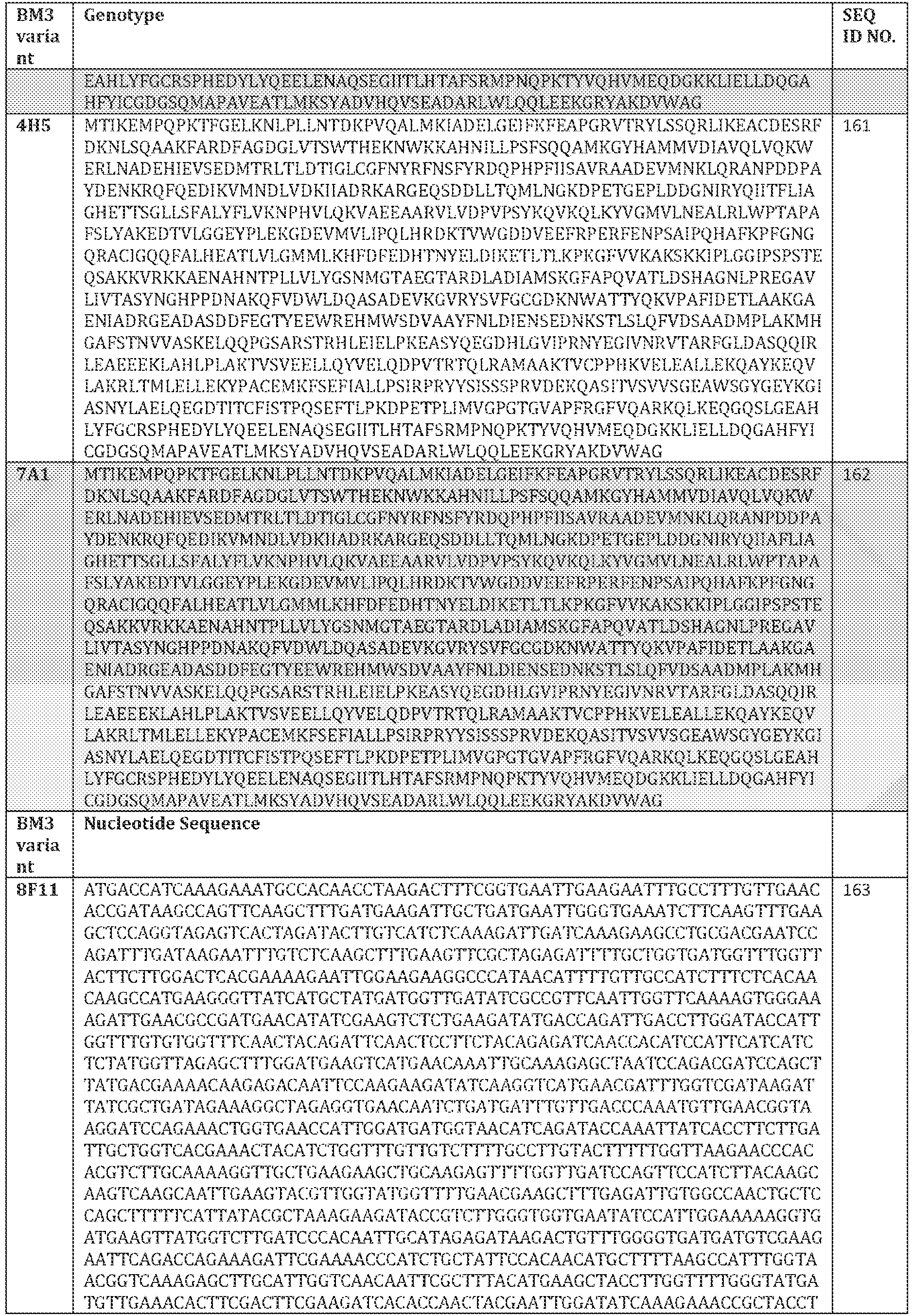

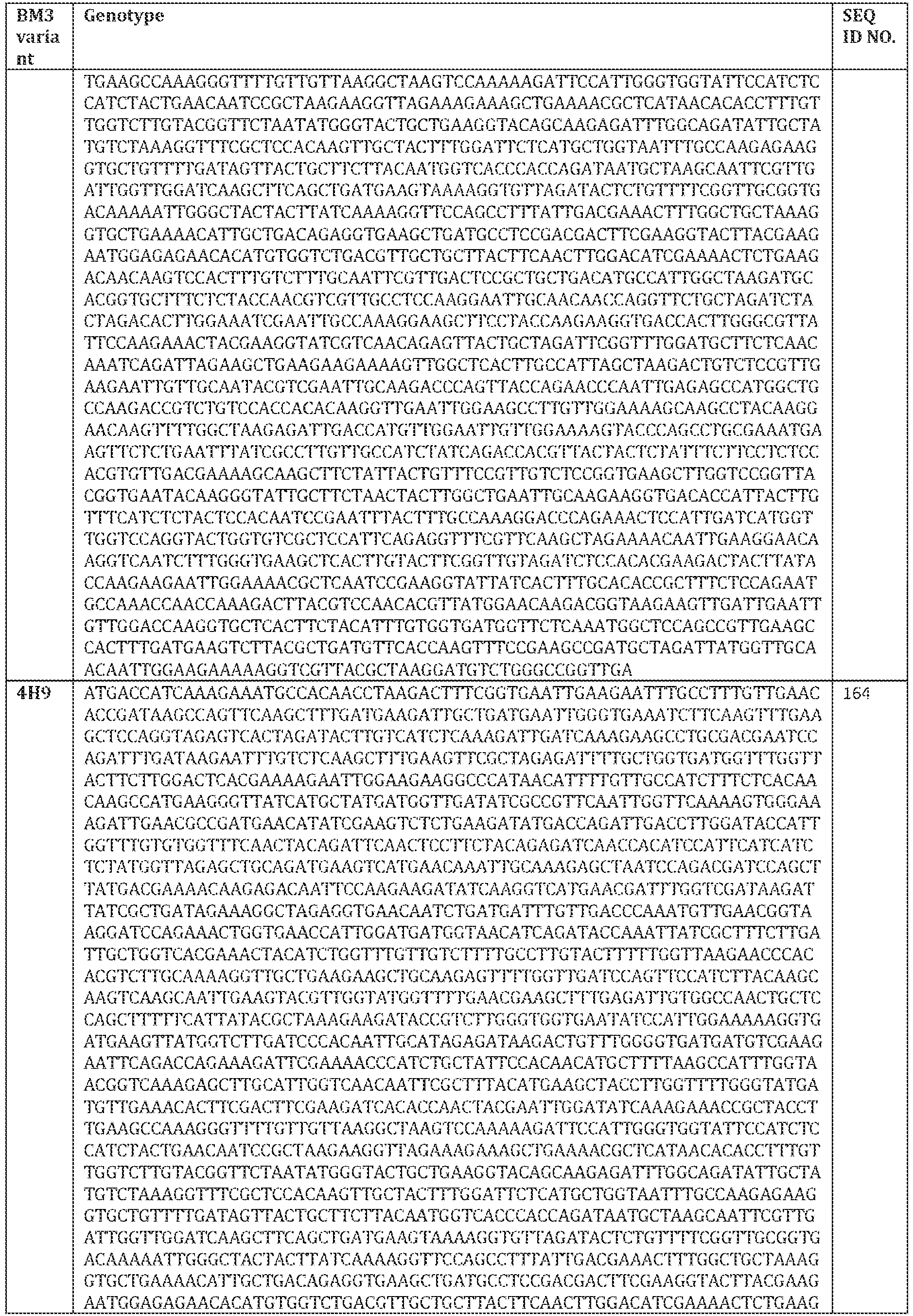

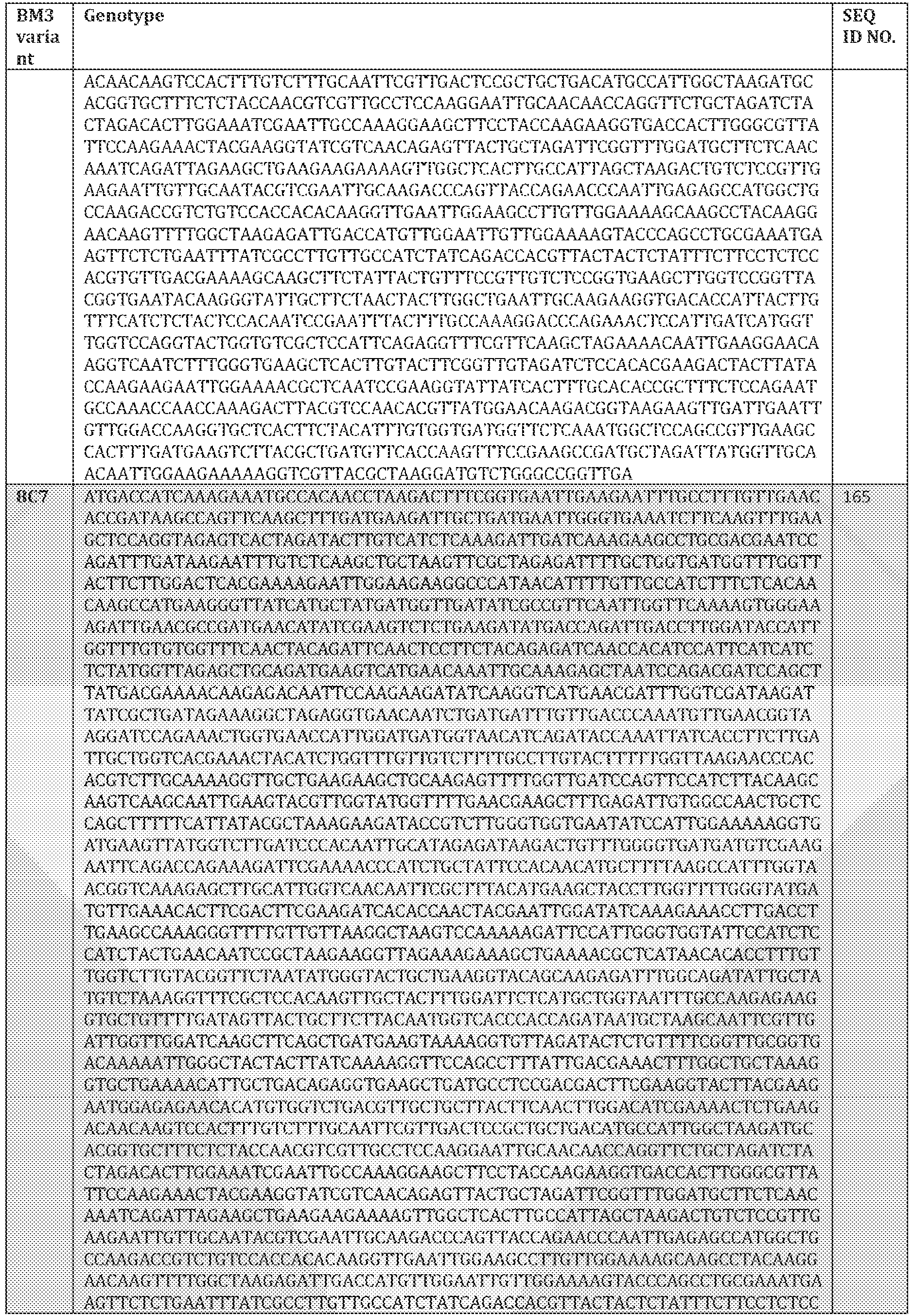

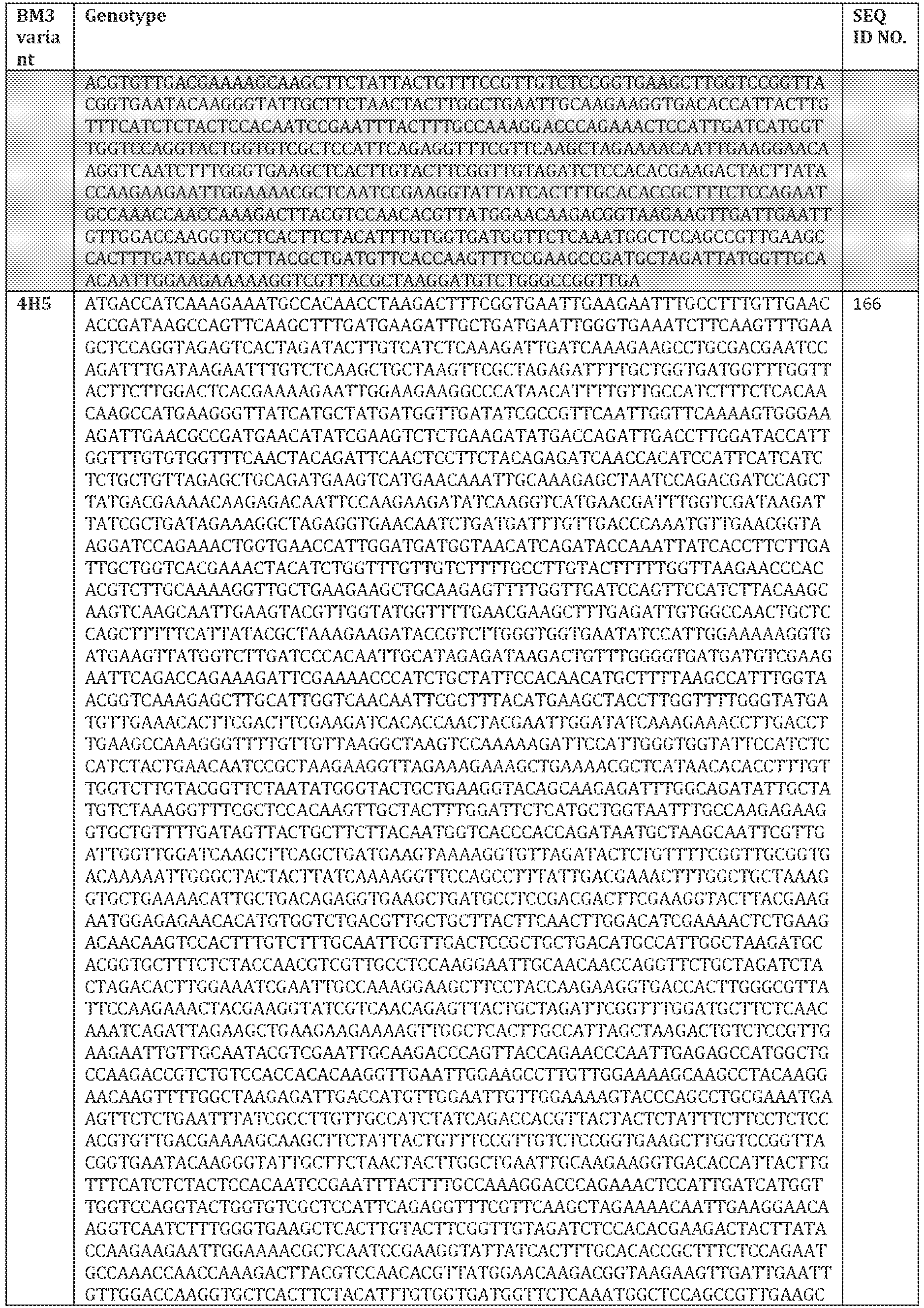

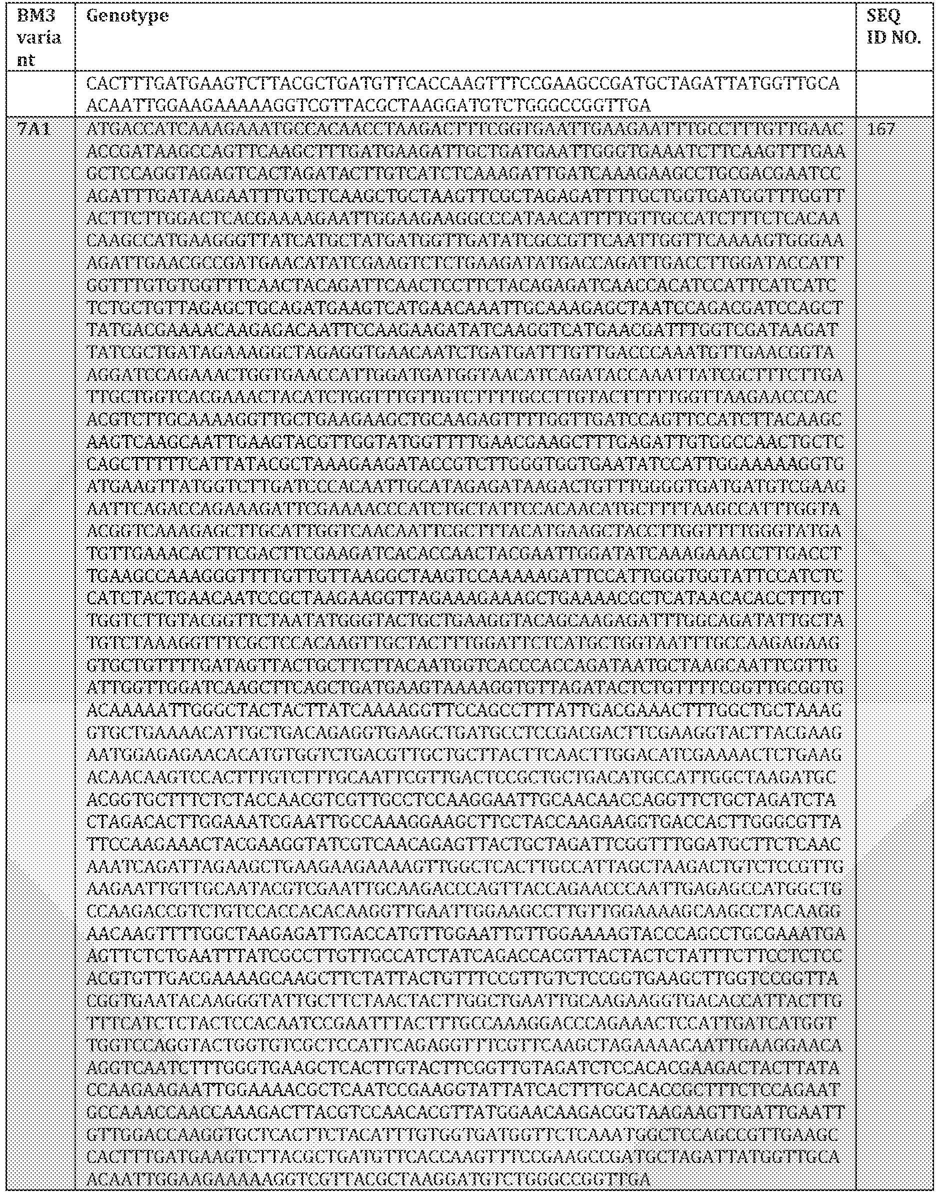

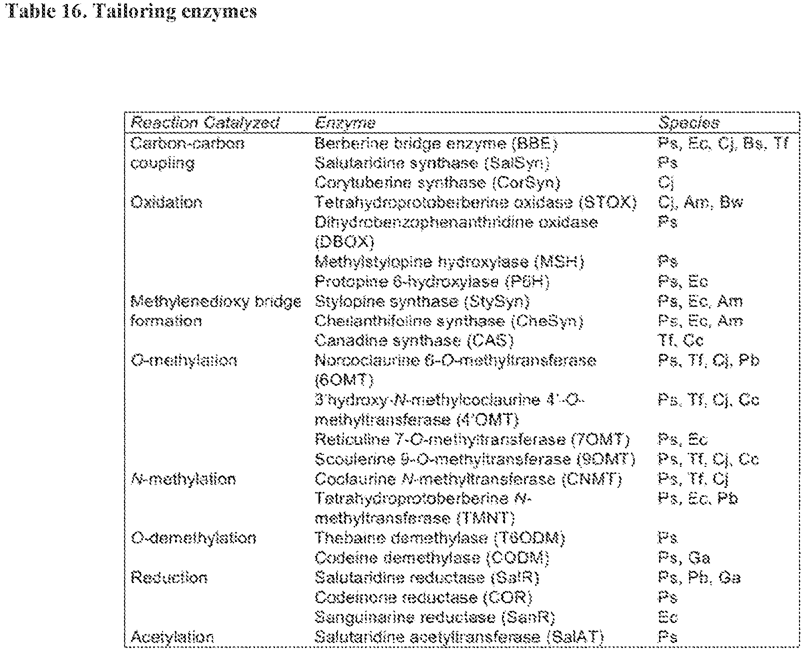

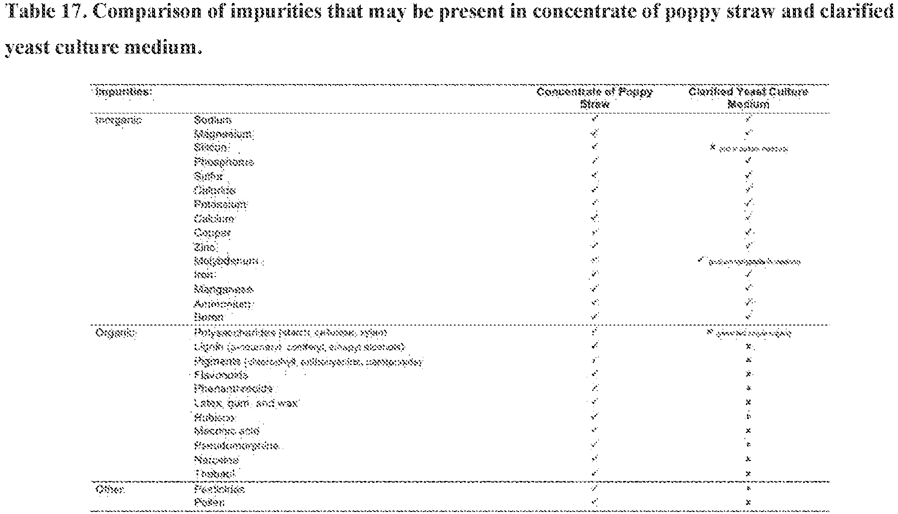

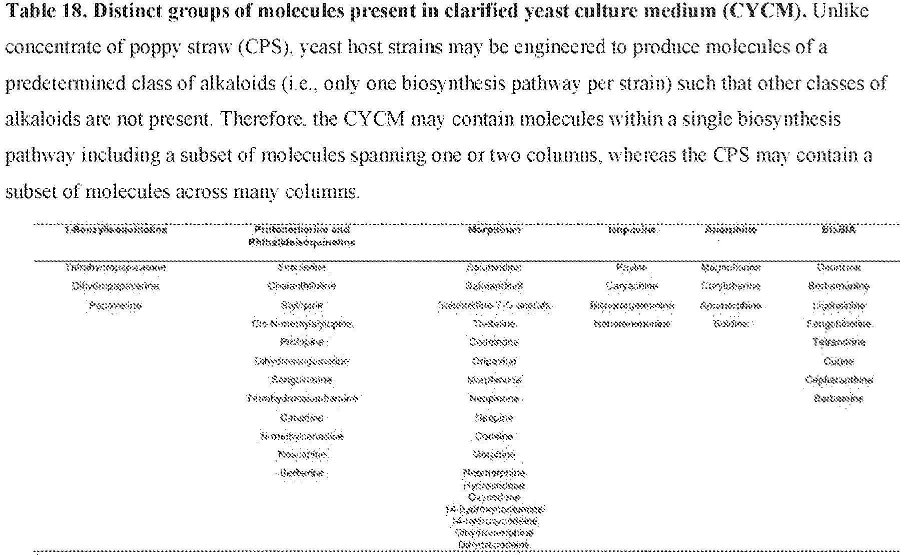

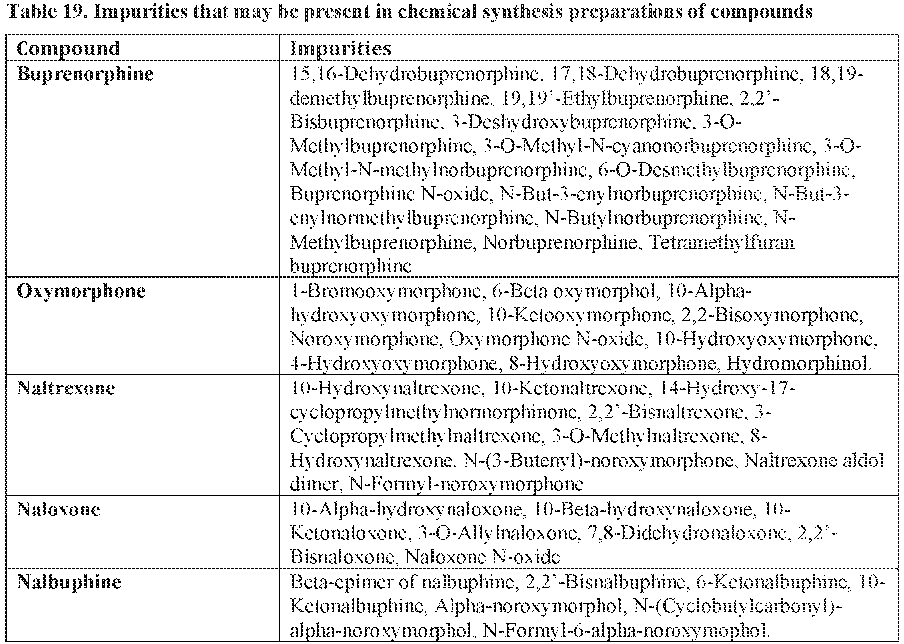

While preferred embodiments of the invention have been shown and described herein. it will be obvious to those skilled in the art that such embodiments are provided by way of example only. Numerous variations. changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.
The invention further provides the following numbered embodiments:
1. A method of producing a benzylisoquinoline alkaloid (BIA) product in an engineered host cell, the method comprising:
   (a) expressing an engineered norcoclaurine synthase in the engineered host cell;
   (b) contacting the engineered norcoclaurine synthase with a BIA-precursor substrate; and
   (c) producing the BIA product within the host cell;

   wherein the engineered norcoclaurine synthase comprises an N-terminal truncation and/or one or more amino acid mutations compared to a non-engineered wild-type norcoclaurine synthase, and has increased condensation activity compared to the non-engineered wild-type norcoclaurine synthase; and
   wherein the engineered host cell produces more BIA product than a non-engineered host cell.
2. The method of embodiment 1, wherein the engineered norcoclaurine synthase comprises an N-terminal truncation compared to a non-engineered wild-type norcoclaurine synthase.
3. The method of embodiment 1 or 2, wherein the engineered norcoclaurine synthase comprises one or more amino acid mutations compared to a non-engineered wild-type norcoclaurine synthase.
4. The method of any one embodiments 1-3, wherein the engineered norcoclaurine synthase comprises at least 90% sequence identity to the amino acid sequence of SEQ ID NO: 69, 70, 71, 72, 73, 74, 75, 76, 77, 78, 79, 80, 81, or 82.
5. The method of any one of embodiments 1-4, wherein the engineered norcoclaurine synthase comprises at least one amino acid mutation at one or more residue positions selected from the group consisting of amino acid residue 70, 81, 91, 101, 104, 147, 149, 151, and 155 with reference to the amino acid sequence of SEQ. ID. NO: 70.
6. The method of any one of embodiments 1-5, wherein the engineered host cell produces a BIA selected from the group consisting of benzylisoquinolines, promorphinans, morphinans, protoberberines, protopines, benzophenanthridines, secoberberines, phthalideisoquinolines, aporphines, bisbenzylisoquinolines, nal-opioids, and nor-opioids.
7. An engineered host cell that produces a benzylisoquinoline alkaloid (BIA) product, the engineered host cell comprising:
   (i) one or more engineered biosynthetic enzymes that reduces accumulation of one or more byproducts that inhibits the production of the BIA product; and/or
   (ii) an inactivation of one or more genes that results in the reduction of accumulation of one or more byproduct that inhibits the production of the BIA product.
8. The engineered host cell of embodiment 7, wherein the one or more engineered biosynthetic enzymes comprises at least one amino acid modification compared to a non-engineered wild-type biosynthetic enzyme.
9. The engineered host cell of embodiment 7 or 8, wherein the inactivation of one or more genes occurs in one or more genes that encodes an enzyme selected from the group consisting of aromatic aminotransferase (ARO8), aromatic aminotransferase (ARO9), phenylpyruvate decarboxylase (ARO10), pyruvate decarboxylase (PDC1), pyruvate decarboxylase (PDC5), pyruvate decarboxylase (PDC6), aldehyde reductase (ARI1), alcohol acetyltransferase 1 (ATF1), alcohol acetyltransferase 2 (ATF2), octanoyl-coenzyme A:ethanol acyltransferase (EHT1), acyl-coenzyme A:ethanol O-acyltransferase (EEB1), (putative) aryl-alcohol dehydrogenase (AAD3), NADPH-dependent aldo-keto reductase (YPR1), 3-methylbutanal reductase and NADPH-dependent methylglyoxal reductase (GRE2), alcohol dehydrogenase 1 (ADH1), alcohol dehydrogenase 2 (ADH2), alcohol dehydrogenase 3 (ADH3), alcohol dehydrogenase 4 (ADH4), alcohol dehydrogenase 5 (ADH5), alcohol dehydrogenase 6 (ADH6), alcohol dehydrogenase 7 (ADH7), aldehyde reductase (YDR541c), branched-chain amino-acid aminotransferase (BAT2), hexadecenal dehydrogenase (HFD1), prephenate dehydrogenase (TYR1), and prephenate dehydratase (PHA2).
10. The engineered host cell of any one of embodiments 7-9, wherein the one or more engineered biosynthetic enzymes is selected from the group consisting of (4-Hydroxyphenylacetaldehyde synthase (HPAAS), aspartate-prephenate aminotransferase (PAT), arogenate dehydratase (ADT), arogenate dehydrogenase (AAT), phosphoketolase (PK), and Uridine 5'-diphosphoglucosyltransferase (UGT).

## Claims

1. An engineered host cell that produces a benzylisoquinoline alkaloid (BIA) product, the engineered host cell comprising:
(i) one or more engineered biosynthetic enzymes that reduces accumulation of one or more byproducts that inhibits the production of the BIA product, wherein the one or more engineered biosynthetic enzymes comprises uridine 5'-diphosphoglucosyltransferase (UGT); and/or
(ii) an inactivation of one or more genes that results in the reduction of accumulation of one or more byproduct that inhibits the production of the BIA product.

2. The engineered host cell of claim 1, wherein the one or more engineered biosynthetic enzymes and/or the inactivation of one or more genes reduces the accumulation of a byproduct selected from the group consisting of tyrosol, phenylethanol, and methionol.

3. The engineered host cell of claim 1 or claim 2, wherein the engineered host cell produces a BIA product selected from the group consisting of benzylisoquinolines, promorphinans, morphinans, protoberberines, protopines, benzophenanthridines, secoberberines, phthalideisoquinolines, aporphines, bisbenzylisoquinolines, nal-opioids, and nor-opioids.

4. The engineered host cell of any one of claims 1-3, wherein UGT is expressed in combination with an inactivating mutation in EGH1 to increase the availability of the substrate UDP-glucose.

5. The engineered host cell of any one of claims 1-4, wherein:
(a) the engineered host cell is modified to include constitutive expression of the UGT gene in the engineered host cell;
(b) the engineered host cell is modified to synthetically regulate the expression of the UGT gene in the engineered host cell;
(c) the engineered host cell is modified to incorporate a copy, copies, or additional copies, of the UGT gene;
(d) the engineered host cell is modified to incorporate the introduction of a strong promoter element for the overexpression of the UGT gene within the engineered host cell;
(e) the UGT gene is codon optimized for expression in *Saccharomyces cerevisiae;* and/or
(f) the UGT gene is derived from *Rhodiola rosea.*

6. The engineered host cell of any one of claims 1-5, wherein the host cell is:
(a) a fungal cell;
(b) a yeast cell; or
(c) a *Saccharomyces cerevisiae* cell.

7. The engineered host cell of any one of claims 1-6, wherein the one or more engineered biosynthetic enzymes comprises at least one amino acid modification compared to a non-engineered wild-type biosynthetic enzyme.

8. The engineered host cell of any one of claims 1-7, further comprising one or more engineered biosynthetic enzymes selected from the group consisting of 4-Hydroxyphenylacetaldehyde synthase (HPAAS), aspartate-prephenate aminotransferase (PAT), arogenate dehydratase (ADT), arogenate dehydrogenase (AAT), and phosphoketolase (PK).

9. The engineered host cell of any one of claims 1-8, wherein the inactivation of one or more genes occurs in one or more genes that encodes an enzyme selected from the group consisting of aromatic aminotransferase (ARO8), aromatic aminotransferase (ARO9), phenylpyruvate decarboxylase (ARO10), pyruvate decarboxylase (PDC1), pyruvate decarboxylase (PDC5), pyruvate decarboxylase (PDC6), aldehyde reductase (ARI1), alcohol acetyltransferase 1 (ATF1), alcohol acetyltransferase 2 (ATF2), octanoyl-coenzyme A:ethanol acyltransferase (EHT1), acyl-coenzyme A:ethanol O-acyltransferase (EEB1), (putative) aryl-alcohol dehydrogenase (AAD3), NADPH-dependent aldo-keto reductase (YPR1), 3-methylbutanal reductase and NADPH-dependent methylglyoxal reductase (GRE2), alcohol dehydrogenase 1 (ADH1), alcohol dehydrogenase 2 (ADH2), alcohol dehydrogenase 3 (ADH3), alcohol dehydrogenase 4 (ADH4), alcohol dehydrogenase 5 (ADH5), alcohol dehydrogenase 6 (ADH6), alcohol dehydrogenase 7 (ADH7), aldehyde reductase (YDR541c), branched-chain amino-acid aminotransferase (BAT2), hexadecenal dehydrogenase (HFD1), prephenate dehydrogenase (TYR1), and prephenate dehydratase (PHA2).

10. A method of increasing production of thebaine in an engineered microbial cell, the method comprising: (a) introducing into the engineered microbial cell a heterologous polynucleotide sequence that encodes a uridine 5'-diphospho-glucosyltransferase; (b) expressing the uridine 5'-diphospho-glucosyltransferase within the engineered microbial cell; and (c) producing increased levels of thebaine within the engineered microbial cell compared to a non-engineered microbial cell, wherein the uridine 5'diphospho-glucosyltransferase converts a phenol to an aryl beta-D-glucoside within the engineered microbial cell.

11. The method of claim 10, wherein:
(a) the heterologous polynucleotide sequence that encodes the uridine 5'-diphospho-glucosyltransferase is positioned at a HXT5 locus in the engineered microbial cell;
(b) the heterologous polynucleotide sequence that encodes the uridine 5'-diphospho-glucosyltransferase construct is positioned at an EGH1 locus in the engineered microbial cell; and/or
(c) the heterologous polynucleotide sequence that encodes the uridine 5'-diphospho-glucosyltransferase construct is positioned at a HST2 locus in the engineered microbial cell.

12. The method of claim 11(c), wherein BAT2 is deleted from the background of the microbial cell.

13. The method of any of claims 10-12, wherein the engineered microbial cell further comprises an inactivation mutation comprising a deletion of EGH1.

14. The method of any of claims 10-13, wherein the expression of the uridine 5'-diphospho-glucosyltransferase within the engineered microbial cell reduces the accumulation of fusel alcohols.

15. The method of claim 14, wherein:
(a) the expression of the uridine 5'-diphospho-glucosyltransferase reduces the accumulation of tyrosol within the engineered microbial cell;
(b) the expression of the uridine 5'-diphospho-glucosyltransferase reduces the accumulation of phenylethanol within the engineered microbial cell; or
(c) the expression of the uridine 5'-diphospho-glucosyltransferase reduces the accumulation of methionol within the engineered microbial cell.
